# EUROPEAN PATENT APPLICATION

(11) **EP 4 650 365 A2**
(43) Date of publication of application: **19.11.2025**
(21) Application number: 25193554.0
(22) Date of filing: 19.12.2019
(51) Int. Cl.: C07K 14/705

(54) **MULTIMERIC T-CELL MODULATORY POLYPEPTIDES AND METHODS OF USE THEREOF**

(30) Priority: 19.12.2018 US 201862782167 P; 06.03.2019 US 201962814684 P
(62) Divisional of application: 19900784.0
(71) Applicant: Cue Biopharma, Inc., Boston, MA 02135 (US)
(72) Inventor: SEIDEL, Ronald D. III, Cambridge, 02139 (US); CHAPARRO, Rodolfo J., Cambridge, 02139 (US)
(74) Representative: Mewburn Ellis LLP

(57) **Abstract**

The present disclosure provides T-cell modulatory multimeric polypeptides that comprise an immunomodulatory polypeptide and that comprise an epitope-presenting Wilms tumor peptide. A T-cell modulatory multimeric polypeptide is useful for modulating the activity of a T cell, and for modulating an immune response in an individual.

## Description

### CROSS-REFERENCE

This application claims the benefit of U.S. Provisional Patent Application No. 62/782,167, filed December 19, 2018, and U.S. Provisional Patent Application No. 62/814,684, filed March 6, 2019, which applications are incorporated herein by reference in their entirety.

### INTRODUCTION

An adaptive immune response involves the engagement of the T cell receptor (TCR), present on the surface of a T cell, with a small peptide antigen non-covalently presented on the surface of an antigen presenting cell (APC) by a major histocompatibility complex (MHC; also referred to in humans as a human leukocyte antigen (HLA) complex). This engagement represents the immune system's targeting mechanism and is a requisite molecular interaction for T cell modulation (activation or inhibition) and effector function. Following epitope-specific cell targeting, the targeted T cells are activated through engagement of costimulatory proteins found on the APC with counterpart costimulatory proteins the T cells. Both signals - epitope/TCR binding and engagement of APC costimulatory proteins with T cell costimulatory proteins - are required to drive T cell specificity and activation or inhibition. The TCR is specific for a given epitope; however, the costimulatory protein not epitope specific and instead is generally expressed on all T cells or on large T cell subsets.

### SUMMARY

The present disclosure provides T-cell modulatory multimeric polypeptides (TMMPs) that comprise an immunomodulatory polypeptide and that comprise an epitope-presenting Wilms tumor peptide. A T-cell modulatory multimeric polypeptide is useful for modulating the activity of a T cell, and for modulating an immune response in an individual.

### BRIEF DESCRIPTION OF THE DRAWINGS

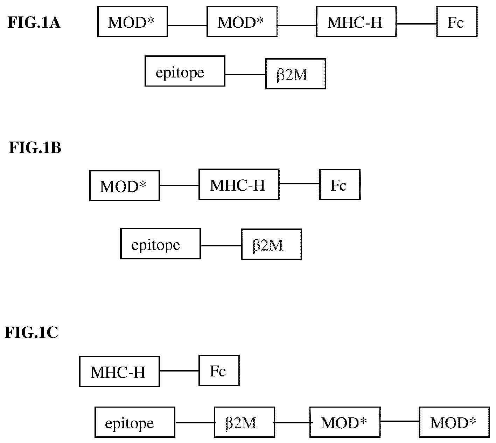

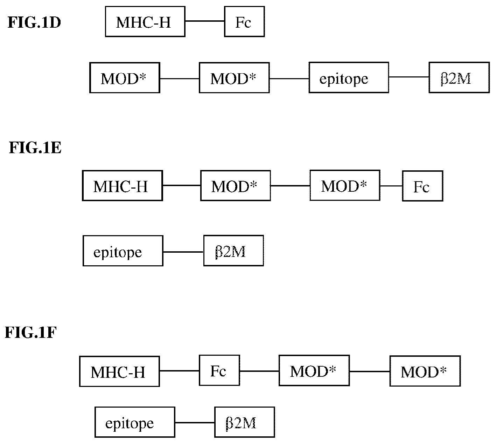

**FIG. 1A-1F** are schematic depictions of various TMMPs of the present disclosure.
**FIG. 2A-2F** are schematic depictions of various disulfide-linked TMMPs of the present disclosure.
**FIG. 3A-3E** provides an amino acid sequence of WT-1 polypeptides. The sequences of FIGs. 3A-3E are set forth in SEQ ID NOs: 399-403, respectively.
**FIG. 4A-4E** provide amino acid sequences of exemplary polypeptide chains of TMMPs of the present disclosure. The sequences of the exemplary polypeptide chains of FIGs. 4A-4E are set forth in SEQ ID NOs: 405-409, respectively. The epitope sequences are set forth as follows: FIG. 4D: CMTWNQMNL (SEQ ID NO:266); FIG. 4E: CYTWNQMNL (SEQ ID NO:267).
**FIG. 5A-5G** provide amino acid sequences of immunoglobulin Fc polypeptides. The sequences of FIGs. 5A-5G are set forth in SEQ ID NOs: 410-421, respectively.
**FIG. 6** provides a multiple amino acid sequence alignment of beta-2 microglobulin (β2M) precursors (i.e., including the leader sequence) from *Homo sapiens* (NP_004039.1; SEQ ID NO: *19), Pan troglodytes* (NP_001009066.1; SEQ ID NO: 19), *Macaca mulatta* (NP_001040602.1; SEQ ID NO: 20), *Bos taurus* (NP_776318.1; SEQ ID NO: 21) and *Mus musculus* (NP_033865.2; SEQ ID NO: 22). Amino acids 1-20 are a signal peptide.
**FIG. 7A-7C** provide amino acid sequences of full-length human HLA heavy chains of alleles A*0101 (SEQ ID NO: 23), A*1101 (SEQ ID NO: 24), A*2402 (SEQ ID NO: 25), and A*3303 (SEQ ID NO: 26) (FIG. 7A); full-length human HLA heavy chain of allele B*0702 (SEQ ID NO: 27) (FIG. 7B); and a full-length human HLA-C heavy chain (SEQ ID NO: 28) (FIG. 7C).
**FIG. 8** provides an alignment of eleven mature MHC class I heavy chain amino acid sequences without their leader sequences, transmembrane domains, and intracellular domains. Top to bottom: SEQ ID NOs: 41-51.
**FIGs. 9A-9B** provide an alignment of HLA-A heavy chain amino acid sequences (FIG. 9A; SEQ ID NOs: 198-206, respectively) and a consensus sequence (FIG. 9B; SEQ ID NO: 29).
**FIGs. 10A-10B** provide an alignment of HLA-B heavy chain amino acid sequences (FIG. 10A; SEQ ID NOs: 207-213, respectively) and a consensus sequence (FIG. 10B; SEQ ID NO: 30).
**FIGs. 11A-11B** provide an alignment of HLA-C heavy chain amino acid sequences (FIG. 11A; SEQ ID NOs: 214-222, respectively) and a consensus sequence (FIG. 11B; SEQ ID NO: 31).
**FIG. 12** provides a consensus amino acid sequence for each of HLA-E, -F, and -G heavy chains (SEQ ID NOs: 32-34, respectively). Variable amino acid (aa) positions are indicated as "X" residues sequentially numbered; the locations of amino acids 84, 139, and 236 are double underlined.
**FIG. 13** provides an alignment of consensus amino acid sequences for HLA-A (SEQ ID NO: 35), -B (SEQ ID NO: 36), -C (SEQ ID NO: 37), -E (SEQ ID NO: 38), -F (SEQ ID NO: 39), and -G (SEQ ID NO: 40).
**FIG. 14A-14I** provide amino acid sequences of polypeptide chains of double disulfide-linked TMMP of the present disclosure. The sequences of the polypeptide chains of FIGs. 14A-14I are set forth in SEQ ID NOs: 422-430, respectively. The epitope sequences are set forth as follows: FIG. 14B: VLDFAPPGA (SEQ ID NO: 259); FIG. 14C: RMFPNAPYL (SEQ ID NO: 260); FIG. 14F: VLDFAPPGA (SEQ ID NO: 259); FIG. 14G: RMFPNAPYL (SEQ ID NO: 260); FIG. 14H: YMFPNAPYL (SEQ ID NO: 264); FIG. 14I: YMFPNAPYL (SEQ ID NO: 264).
**FIG. 15** depicts expression and stability data for a WT1(37-45) epitope-containing TMMP of the present disclosure.
**FIG. 16** depicts expression and stability data for a WT1(126-134) epitope-containing TMMP of the present disclosure.
**FIG. 17A-17D** provide schematic depictions of double disulfide-linked TMMP of the present disclosure.
**FIG. 18A-18C** provide schematic depictions of examples of configurations of disulfide-linked TMMPs of the present disclosure.
**FIG. 19** provide schematic depictions of examples of positions of immunomodulatory polypeptides in TMMPs of the present disclosure.

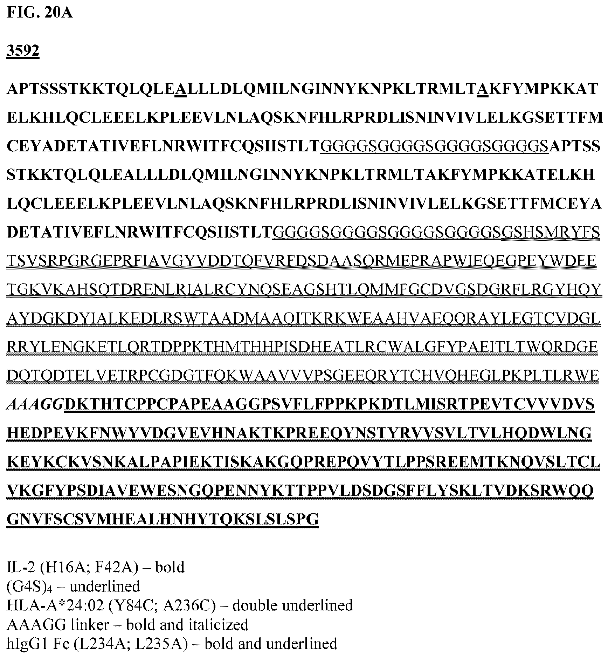

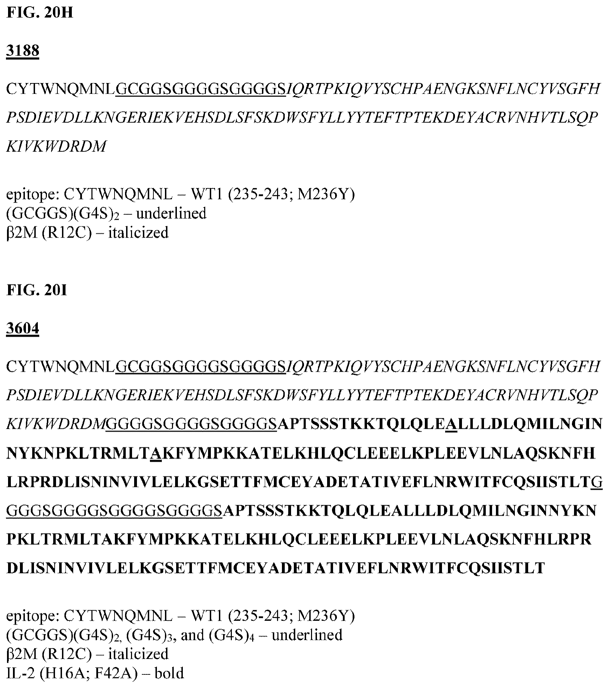

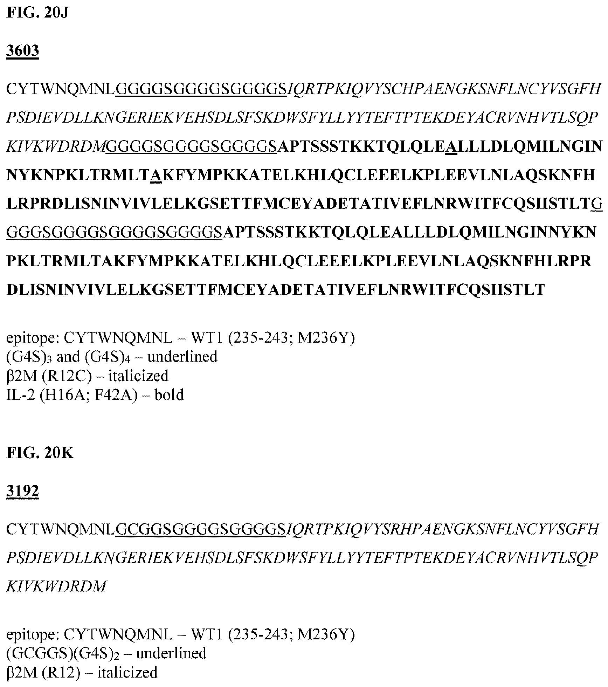

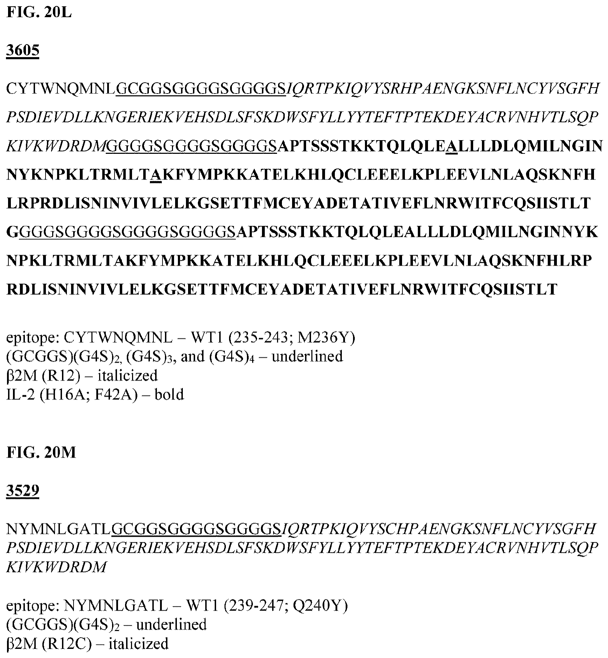

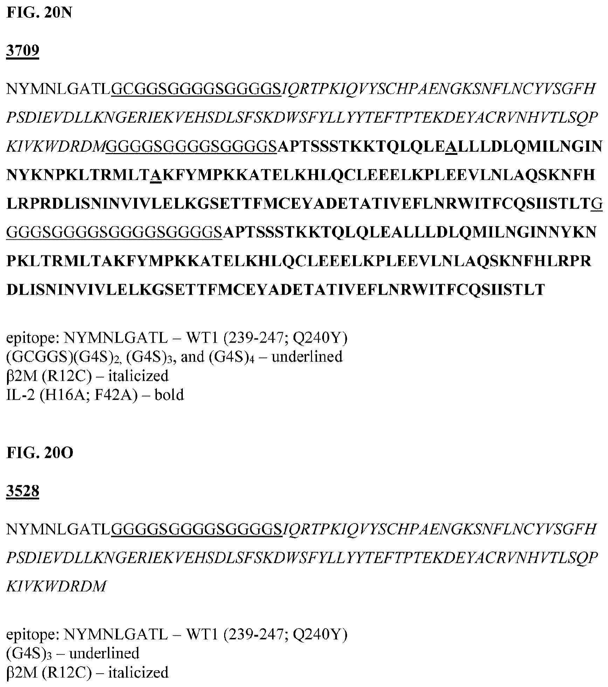

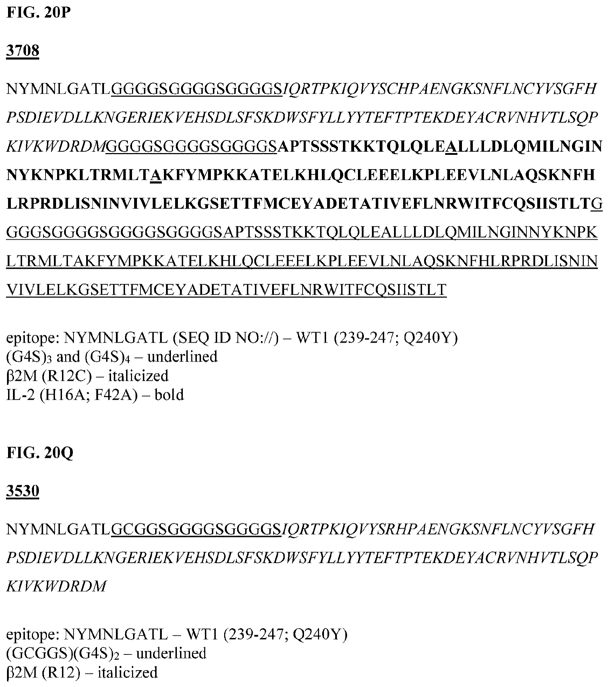

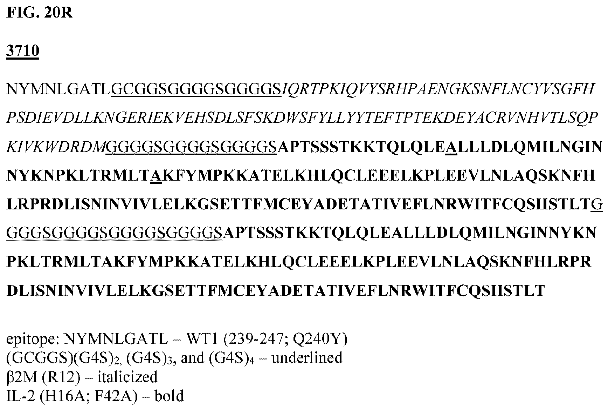

**FIG. 20A-20R** provide amino acid sequences of exemplary polypeptide chains of TMMPs of the present disclosure. The sequences of the exemplary polypeptide chains of FIGs. 20A-20R are set forth in SEQ ID NOs: 431-448, respectively. The epitope sequences are set forth as follows: FIG. 20H: CYTWNQMNL (SEQ ID NO: 262); FIG. 20I: CYTWNQMNL (SEQ ID NO: 262); FIG. 20J: CYTWNQMNL (SEQ ID NO: 262); FIG. 20K: CYTWNQMNL (SEQ ID NO: 262); FIG. 20L: CYTWNQMNL (SEQ ID NO: 262); FIG. 20M: NYMNLGATL (SEQ ID NO: 263); FIG. 20N: NYMNLGATL (SEQ ID NO: 263); FIG. 20O: NYMNLGATL (SEQ ID NO: 263); FIG. 20P: NYMNLGATL (SEQ ID NO: 263); FIG. 20Q: NYMNLGATL (SEQ ID NO: 263); FIG. 20R: NYMNLGATL (SEQ ID NO: 263).
**FIG. 21** depicts the effect of TMMPs, containing WT1 peptide epitopes and HLA-A*02 heavy chains, on antigen-specific CD8+ T cell expansion.
**FIG. 22** depicts the effect of TMMPs containing WT1 peptide epitopes on expansion of WT1-specific CD8+ T cells from total PBMCs over a course of an 8-day re-stimulation culture following a 10-day priming culture.
**FIG. 23** depicts production of TNF-§ and IFN-γ by WT1-specific CD8+ T cells expanded with WT1 37-45 containing TMMPs having either the G2C or R12C/G2C framework.
**FIG. 24** depicts production of TNF-§ and IFN-γ by WT1-specific CD8+ T cells expanded with WT1 126-134 containing TMMPs having the R12C/G2C framework.
**FIG. 25** depicts the effect of disulfide bonds on IL-2-driven immune cell activation.
**FIG. 26** depicts the effect of TMMP containing variant IL-2 as the immunomodulatory polypeptide on CGLL-2 proliferation, compared to proleukine.
**FIG. 27** depicts binding of the "1715 + 2380" TMPP to various Fc receptors.

### DEFINITIONS

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases.

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

A polynucleotide or polypeptide has a certain percent "sequence identity" to another polynucleotide or polypeptide, meaning that, when aligned, that percentage of bases or amino acids are the same, and in the same relative position, when comparing the two sequences. Sequence identity can be determined in a number of different ways. To determine sequence identity, sequences can be aligned using various convenient methods and computer programs (e.g., BLAST, T-COFFEE, MUSCLE, MAFFT, etc.), available over the world wide web at sites including ncbi.nlm.nili.gov/BLAST, ebi.ac.uk/Tools/msa/tcoffee/, ebi.ac.uk/Tools/msa/muscle/, mafft.cbrc.jp/alignment/software/. See, e.g., Altschul et al. (1990), J. Mol. Bioi. 215:403-10.

The term "conservative amino acid substitution" refers to the interchangeability in proteins of amino acid residues having similar side chains. For example, a group of amino acids having aliphatic side chains consists of glycine, alanine, valine, leucine, and isoleucine; a group of amino acids having aliphatic-hydroxyl side chains consists of serine and threonine; a group of amino acids having amide containing side chains consisting of asparagine and glutamine; a group of amino acids having aromatic side chains consists of phenylalanine, tyrosine, and tryptophan; a group of amino acids having basic side chains consists of lysine, arginine, and histidine; a group of amino acids having acidic side chains consists of glutamate and aspartate; and a group of amino acids having sulfur containing side chains consists of cysteine and methionine. Exemplary conservative amino acid substitution groups are: valine-leucine-isoleucine, phenylalanine-tyrosine, lysine-arginine, alanine-valine-glycine, and asparagine-glutamine.

The term "immunological synapse" or "immune synapse" as used herein generally refers to the natural interface between two interacting immune cells of an adaptive immune response including, e.g., the interface between an antigen-presenting cell (APC) or target cell and an effector cell, e.g., a lymphocyte, an effector T cell, a natural killer cell, and the like. An immunological synapse between an APC and a T cell is generally initiated by the interaction of a T cell antigen receptor and major histocompatibility complex molecules, e.g., as described in Bromley et al., Annu Rev Immunol. 2001; 19:375-96; the disclosure of which is incorporated herein by reference in its entirety.

"T cell" includes all types of immune cells expressing CD3, including T-helper cells (CD4⁺ cells), cytotoxic T-cells (CD8⁺ cells), T-regulatory cells (Treg), and NK-T cells.

The term "immunomodulatory polypeptide" (also referred to as a "co-stimulatory polypeptide"), as used herein, includes a polypeptide on an antigen presenting cell (APC) (e.g., a dendritic cell, a B cell, and the like) that specifically binds a cognate co-immunomodulatory polypeptide on a T cell, thereby providing a signal which, in addition to the primary signal provided by, for instance, binding of a TCR/CD3 complex with a major histocompatibility complex (MHC) polypeptide loaded with peptide, mediates a T cell response, including, but not limited to, proliferation, activation, differentiation, and the like. An immunomodulatory polypeptide can include, but is not limited to, CD7, B7-1 (CD80), B7-2 (CD86), PD-L1, PD-L2, 4-1BBL, OX40L, Fas ligand (FasL), inducible costimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3.

As noted above, an "immunomodulatory polypeptide" (also referred to herein as a "MOD") specifically binds a cognate co-immunomodulatory polypeptide on a T cell.

An "immunomodulatory domain" ("MOD") of a TMMP of the present disclosure binds a cognate co-immunomodulatory polypeptide, which may be present on a target T cell.

"Heterologous," as used herein, means a nucleotide or polypeptide that is not found in the native nucleic acid or protein, respectively.

"Recombinant," as used herein, means that a particular nucleic acid (DNA or RNA) is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides, to provide a synthetic nucleic acid which is capable of being expressed from a recombinant transcriptional unit contained in a cell or in a cell-free transcription and translation system.

The terms "recombinant expression vector," or "DNA construct" are used interchangeably herein to refer to a DNA molecule comprising a vector and at least one insert. Recombinant expression vectors are usually generated for the purpose of expressing and/or propagating the insert(s), or for the construction of other recombinant nucleotide sequences. The insert(s) may or may not be operably linked to a promoter sequence and may or may not be operably linked to DNA regulatory sequences.

As used herein, the term "affinity" refers to the equilibrium constant for the reversible binding of two agents (e.g., an antibody and an antigen) and is expressed as a dissociation constant (K_{D}). Affinity can be at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, at least 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1,000-fold greater, or more, than the affinity of an antibody for unrelated amino acid sequences. Affinity of an antibody to a target protein can be, for example, from about 100 nanomolar (nM) to about 0.1 nM, from about 100 nM to about 1 picomolar (pM), or from about 100 nM to about 1 femtomolar (fM) or more. As used herein, the term "avidity" refers to the resistance of a complex of two or more agents to dissociation after dilution. The terms "immunoreactive" and "preferentially binds" are used interchangeably herein with respect to antibodies and/or antigen-binding fragments.

The term "binding," as used herein (e.g. with reference to binding of a TMMP to a polypeptide (e.g., a T-cell receptor) on a T cell), refers to a non-covalent interaction between two molecules. Non-covalent binding refers to a direct association between two molecules, due to, for example, electrostatic, hydrophobic, ionic, and/or hydrogen-bond interactions, including interactions such as salt bridges and water bridges. Non-covalent binding interactions are generally characterized by a dissociation constant (K_{D}) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. "Affinity" refers to the strength of non-covalent binding, increased binding affinity being correlated with a lower K_{D}. "Specific binding" generally refers to binding with an affinity of at least about 10⁻⁷ M or greater, *e.g.,* 5x 10⁻⁷ M, 10⁻⁸ M, 5 x 10⁻⁸ M, 10⁻⁹ M, and greater. "Non-specific binding" generally refers to binding (e.g., the binding of a ligand to a moiety other than its designated binding site or receptor) with an affinity of less than about 10⁻⁷ M (*e.g.,* binding with an affinity of 10⁻⁶ M, 10⁻⁵ M, 10⁻⁴ M). However, in some contexts, e.g., binding between a TCR and a peptide/MHC complex, "specific binding" can be in the range of from 1 µM to 100 µM, or from 100 µM to 1 mM. "Covalent binding" or "covalent bond," as used herein, refers to the formation of one or more covalent chemical binds between two different molecules.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease or symptom in a mammal, and includes: (a) preventing the disease or symptom from occurring in a subject which may be predisposed to acquiring the disease or symptom but has not yet been diagnosed as having it; (b) inhibiting the disease or symptom, i.e., arresting its development; and/or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease.

The terms "individual," "subject," "host," and "patient," are used interchangeably herein and refer to any mammalian subject for whom diagnosis, treatment, or therapy is desired. Mammals include, e.g., humans, non-human primates, rodents (e.g., rats; mice), lagomorphs (e.g., rabbits), ungulates (e.g., cows, sheep, pigs, horses, goats, and the like), etc.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a," "an," and "the" include plural referents unless the context clearly dictates otherwise. Thus, for example, reference to a "T-cell modulatory multimeric polypeptide" includes a plurality of such polypeptides and reference to "the immunomodulatory polypeptide" includes reference to one or more immunomodulatory polypeptides and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present disclosure provides T-cell modulatory multimeric polypeptides that comprise an immunomodulatory polypeptide and that comprise an epitope-presenting Wilms tumor-1 (WT-1) peptide. A TMMP is useful for modulating the activity of a T cell, and for modulating an immune response in an individual.

### T-CELL MODULATORY MULTIMERIC POLYPEPTIDES

The present disclosure provides a T-cell modulatory multimeric polypeptide (TMMP) comprising: a) a first polypeptide; and b) a second polypeptide, wherein the TMMP comprises an epitope; a first major histocompatibility complex (MHC) polypeptide; a second MHC polypeptide; one or more immunomodulatory polypeptides; and optionally an immunoglobulin (Ig) Fc polypeptide or a non-Ig scaffold. The present disclosure provides a TMMP, wherein the TMMP is a heterodimer comprising: a) a first polypeptide comprising a first MHC polypeptide; and b) a second polypeptide comprising a second MHC polypeptide, wherein the first polypeptide or the second polypeptide comprises an epitope (e.g., a peptide that presents an epitope); wherein the first polypeptide and/or the second polypeptide comprises one or more immunomodulatory polypeptides that can be the same or different; and optionally an Ig F c polypeptide or a non-Ig scaffold. A TMMP of the present disclosure is also referred to herein as a "multimeric polypeptide of the present disclosure" or a "synTac." In some cases, the peptide epitope present in a TMMP of the present disclosure is a WT-1 peptide.

The present disclosure provides a TMMP comprising a heterodimeric polypeptide comprising: a) a first polypeptide comprising: i) a peptide epitope; and ii) a first MHC polypeptide; b) a second polypeptide comprising a second MHC polypeptide; and c) at least one immunomodulatory polypeptide, where the first and/or the second polypeptide comprises the at least one (i.e., one or more) immunomodulatory polypeptide. Optionally, the first or the second polypeptide comprises an Ig Fc polypeptide or a non-Ig scaffold. At least one of the one or more immunomodulatory polypeptides is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide. The epitope present in a TMMP of the present disclosure binds to a T-cell receptor (TCR) on a T cell with an affinity of at least 100 µM (e.g., at least 10 µM, at least 1 µM, at least 100 nM, at least 10 nM, or at least 1 nM). A TMMP of the present disclosure binds to a first T cell with an affinity that is at least 25% higher than the affinity with which the TMMP binds a second T cell, where the first T cell expresses on its surface the cognate co-immunomodulatory polypeptide and a TCR that binds the epitope with an affinity of at least 100 µM, and where the second T cell expresses on its surface the cognate co-immunomodulatory polypeptide but does not express on its surface a TCR that binds the epitope with an affinity of at least 100 µM (e.g., at least 10 µM, at least 1 µM, at least 100 nM, at least 10 nM, or at least 1 nM). In some cases, the peptide epitope present in a TMMP of the present disclosure is a WT-1 peptide.

The present disclosure provides a TMMP, wherein the TMMP is:
A) a heterodimer comprising: a) a first polypeptide comprising a first MHC polypeptide; and b) a second polypeptide comprising a second MHC polypeptide, wherein the first polypeptide or the second polypeptide comprises an epitope (e.g., a peptide that presents an epitope to a T cell); wherein the first polypeptide and/or the second polypeptide comprises one or more immunomodulatory polypeptides that can be the same or different, and wherein at least one of the one or more immunomodulatory polypeptides may be a wild-type immunomodulatory polypeptide or a variant of a wild-type immunomodulatory polypeptide, wherein the variant immunomodulatory polypeptide comprises 1, 2, 3, 4, 5, 6, 7, 8, 9,10, 11, 12, 13, 14, 15, 16, 17, 18, 19, or 20 amino acid substitutions compared to the amino acid sequence of the corresponding wild-type immunomodulatory polypeptide; and wherein the first polypeptide or the second polypeptide optionally comprises an Ig Fc polypeptide or a non-Ig scaffold; or
B) a heterodimer comprising: a) a first polypeptide comprising a first MHC polypeptide; and b) a second polypeptide comprising a second MHC polypeptide, wherein the first polypeptide or the second polypeptide comprises an epitope; wherein the first polypeptide and/or the second polypeptide comprises one or more immunomodulatory polypeptides that can be the same or different,
   wherein at least one of the one or more immunomodulatory polypeptides is a variant of a wild-type immunomodulatory polypeptide, wherein the variant immunomodulatory polypeptide comprises 1, 2, 3, 4, 5, 6, 7, 8, 9,10, 11, 12, 13, 14, 15, 16, 17, 18, 19, or 20 amino acid substitutions compared to the amino acid sequence of the corresponding wild-type immunomodulatory polypeptide,
   wherein at least one of the one or more immunomodulatory domains is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide, and wherein the epitope binds to a TCR on a T cell with an affinity of at least 10⁻⁷ M, such that: i) the TMMP polypeptide binds to a first T cell with an affinity that is at least 25% higher than the affinity with which the TMMP binds a second T cell, wherein the first T cell expresses on its surface the cognate co-immunomodulatory polypeptide and a TCR that binds the epitope with an affinity of at least 10⁻⁷ M, and wherein the second T cell expresses on its surface the cognate co-immunomodulatory polypeptide but does not express on its surface a TCR that binds the epitope with an affinity of at least 10⁻⁷ M; and/or ii) the ratio of the binding affinity of a control TMMP, wherein the control comprises a wild-type immunomodulatory polypeptide, to a cognate co-immunomodulatory polypeptide to the binding affinity of the TMMP comprising a variant of the wild-type immunomodulatory polypeptide to the cognate co-immunomodulatory polypeptide, when measured by bio-layer interferometry, is in a range of from 1.5:1 to 10⁶:1; and wherein the variant immunomodulatory polypeptide comprises 1, 2, 3, 4, 5, 6, 7, 8, 9,10, 11, 12, 13, 14, 15, 16, 17, 18, 19, or 20 amino acid substitutions compared to the amino acid sequence of the corresponding wild-type immunomodulatory polypeptide; and
   wherein the first polypeptide or the second polypeptide optionally comprises an Ig Fc polypeptide or a non-Ig scaffold; or
C) a heterodimer comprising: a) a first polypeptide comprising, in order from N-terminus to C-terminus: i) an epitope; ii) a first MHC polypeptide; and b) a second polypeptide comprising, in order from N-terminus to C-terminus: i) a second MHC polypeptide; and ii) optionally an immunoglobulin (Ig) Fc polypeptide or a non-Ig scaffold, wherein the TMMP comprises one or more immunomodulatory domains that can be the same or different, wherein at least one of the one or more immunomodulatory domain is: A) at the C-terminus of the first polypeptide; B) at the N-terminus of the second polypeptide; C) at the C-terminus of the second polypeptide; or D) at the C-terminus of the first polypeptide and at the N-terminus of the second polypeptide, and wherein at least one of the one or more immunomodulatory domains may be a wild-type immunomodulatory polypeptide or a variant of a wild-type immunomodulatory polypeptide, wherein the variant immunomodulatory polypeptide comprises 1, 2, 3, 4, 5, 6, 7, 8, 9,10, 11, 12, 13, 14, 15, 16, 17, 18, 19, or 20 amino acid substitutions compared to the amino acid sequence of the corresponding wild-type immunomodulatory polypeptide; and
optionally wherein at least one of the one or more immunomodulatory domains is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide, and wherein the epitope binds to a TCR on a T cell with an affinity of at least 10⁻⁷ M, such that: i) the TMMP binds to a first T cell with an affinity that is at least 25% higher than the affinity with which the TMMP binds a second T cell, wherein the first T cell expresses on its surface the cognate co-immunomodulatory polypeptide and a TCR that binds the epitope with an affinity of at least 10⁻⁷ M, and wherein the second T cell expresses on its surface the cognate co-immunomodulatory polypeptide but does not express on its surface a TCR that binds the epitope with an affinity of at least 10⁻⁷ M; and/or ii) the ratio of the binding affinity of a control TMMP, wherein the control comprises a wild-type immunomodulatory polypeptide, to a cognate co-immunomodulatory polypeptide to the binding affinity of the TMMP comprising a variant of the wild-type immunomodulatory polypeptide to the cognate co-immunomodulatory polypeptide, when measured by bio-layer interferometry, is in a range of from 1.5:1 to 10⁶:1; and wherein the variant immunomodulatory polypeptide comprises 1, 2, 3, 4, 5, 6, 7, 8, 9,10, 11, 12, 13, 14, 15, 16, 17, 18, 19, or 20 amino acid substitutions compared to the amino acid sequence of the corresponding wild-type immunomodulatory polypeptide. In some cases, the peptide epitope present in a TMMP of the present disclosure is a WT-1 peptide.

The present disclosure provides a TMMP comprising: a) a first polypeptide comprising, in order from N-terminus to C-terminus: i) an epitope; ii) a first MHC polypeptide; and b) a second polypeptide comprising, in order from N-terminus to C-terminus: i) a second MHC polypeptide; and ii) optionally an Ig Fc polypeptide or a non-Ig scaffold. A TMMP of the present disclosure comprises one or more immunomodulatory polypeptides, wherein at least one of the one or more immunomodulatory polypeptides is: A) at the C-terminus of the first polypeptide; B) at the N-terminus of the second polypeptide; C) at the C-terminus of the second polypeptide; or D) at the C-terminus of the first polypeptide and at the N-terminus of the second polypeptide. At least one of the one or more immunomodulatory polypeptides is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide. The epitope present in a TMMP of the present disclosure binds to a T-cell receptor (TCR) on a T cell with an affinity of at least 100 µM (e.g., at least 10 µM, at least 1 µM, at least 100 nM, at least 10 nM, or at least 1 nM). A TMMP of the present disclosure binds to a first T cell with an affinity that is at least 25% higher than the affinity with which the TMMP binds a second T cell, where the first T cell expresses on its surface the cognate co-immunomodulatory polypeptide and a TCR that binds the epitope with an affinity of at least 100 µM, and where the second T cell expresses on its surface the cognate co-immunomodulatory polypeptide but does not express on its surface a TCR that binds the epitope with an affinity of at least 100 µM (e.g., at least 10 µM, at least 1 µM, at least 100 nM, at least 10 nM, or at least 1 nM).

In some cases, the epitope present in a TMMP of the present disclosure binds to a TCR on a T cell with an affinity of from about 10⁻⁴ M to about 5 x 10⁻⁴ M, from about 5 x 10⁻⁴ M to about 10⁻⁵ M, from about 10⁻⁵ M to 5 x 10⁻⁵ M, from about 5 x 10⁻⁵ M to 10⁻⁶ M, from about 10⁻⁶ M to about 5 x 10⁻⁶ M, from about 5 x 10⁻⁶ M to about 10⁻⁷ M, from about 10⁻⁷ M to about 5 x 10⁻⁷ M, from about 5 x 10⁻⁷ M to about 10⁻⁴ M, or from about 10⁻⁸ M to about 10⁻⁹ M. Expressed another way, in some cases, the epitope present in a TMMP of the present disclosure binds to a TCR on a T cell with an affinity of from about 1 nM to about 5 nM, from about 5 nM to about 10 nM, from about 10 nM to about 50 nM, from about 50 nM to about 100 nM, from about 0.1 µM to about 0.5 µM, from about 0.5 µM to about 1 µM, from about 1 µM to about 5 µM, from about 5 µM to about 10 µM, from about 10 µM to about 25 µM, from about 25 µM to about 50 µM, from about 50 µM to about 75 µM, from about 75 µM to about 100 µM.

An immunomodulatory polypeptide present in a TMMP of the present disclosure binds to its cognate co-immunomodulatory polypeptide with an affinity that it at least 10% less, at least 15% less, at least 20% less, at least 25% less, at least 30% less, at least 35% less, at least 40% less, at least 45% less, at least 50% less, at least 55% less, at least 60% less, at least 65% less, at least 70% less, at least 75% less, at least 80% less, at least 85% less, at least 90% less, at least 95% less, or more than 95% less, than the affinity of a corresponding wild-type immunomodulatory polypeptide for the cognate co-immunomodulatory polypeptide.

In some cases, a variant immunomodulatory polypeptide present in a TMMP of the present disclosure has a binding affinity for a cognate co-immunomodulatory polypeptide that is from 1 nM to 100 nM, or from 100 nM to 100 µM. For example, in some cases, a variant immunomodulatory polypeptide present in a TMMP of the present disclosure has a binding affinity for a cognate co-immunomodulatory polypeptide that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM. In some cases, a variant immunomodulatory polypeptide present in a TMMP of the present disclosure has a binding affinity for a cognate co-immunomodulatory polypeptide that is from about 1 nM to about 5 nM, from about 5 nM to about 10 nM, from about 10 nM to about 50 nM, from about 50 nM to about 100 nM.

The combination of the reduced affinity of the immunomodulatory polypeptide for its cognate co-immunomodulatory polypeptide, and the affinity of the epitope for a TCR, provides for enhanced selectivity of a TMMP of the present disclosure. For example, a TMMP of the present disclosure binds selectively to a first T cell that displays both: i) a TCR specific for the epitope present in the TMMP; and ii) a co-immunomodulatory polypeptide that binds to the immunomodulatory polypeptide present in the TMMP, compared to binding to a second T cell that displays: i) a TCR specific for an epitope other than the epitope present in the TMMP; and ii) a co-immunomodulatory polypeptide that binds to the immunomodulatory polypeptide present in the TMMP. For example, a TMMP of the present disclosure binds to the first T cell with an affinity that is at least 10%, at least 15%, at least 20%, at least 25%, at least 30%, at least 40%, at least 50%, at least 60%, at least 70%, at least 80%, at least 90%, at least 2-fold, at least 2.5-fold, at least 5-fold, at least 10-fold, at least 15-fold, at least 20-fold, at least 25-fold, at least 50-fold, at least 100-fold, or more than 100-fold, higher than the affinity to which it binds the second T cell.

In some cases, a TMMP of the present disclosure, when administered to an individual in need thereof, induces both an epitope-specific T cell response and an epitope non-specific T cell response. In other words, in some cases, a TMMP of the present disclosure, when administered to an individual in need thereof, induces an epitope-specific T cell response by modulating the activity of a first T cell that displays both: i) a TCR specific for the epitope present in the TMMP; ii) a co-immunomodulatory polypeptide that binds to the immunomodulatory polypeptide present in the TMMP; and induces an epitope non-specific T cell response by modulating the activity of a second T cell that displays: i) a TCR specific for an epitope other than the epitope present in the TMMP; and ii) a co-immunomodulatory polypeptide that binds to the immunomodulatory polypeptide present in the TMMP. The ratio of the epitope-specific T cell response to the epitope-non-specific T cell response is at least 2:1, at least 5:1, at least 10:1, at least 15:1, at least 20:1, at least 25:1, at least 50:1, or at least 100:1. The ratio of the epitope-specific T cell response to the epitope-non-specific T cell response is from about 2:1 to about 5:1, from about 5:1 to about 10:1, from about 10:1 to about 15:1, from about 15:1 to about 20:1, from about 20:1 to about 25:1, from about 25:1 to about 50:1, or from about 50:1 to about 100:1, or more than 100:1. "Modulating the activity" of a T cell can include one or more of: i) activating a cytotoxic (e.g., CD8⁺) T cell; ii) inducing cytotoxic activity of a cytotoxic (e.g., CD8⁺) T cell; iii) inducing production and release of a cytotoxin (e.g., a perforin; a granzyme; a granulysin) by a cytotoxic (e.g., CD8⁺) T cell; iv) inhibiting activity of an autoreactive T cell; and the like.

The combination of the reduced affinity of the immunomodulatory polypeptide for its cognate co-immunomodulatory polypeptide, and the affinity of the epitope for a TCR, provides for enhanced selectivity of a TMMP of the present disclosure. Thus, for example, a TMMP of the present disclosure binds with higher avidity to a first T cell that displays both: i) a TCR specific for the epitope present in the TMMP; and ii) a co-immunomodulatory polypeptide that binds to the immunomodulatory polypeptide present in the TMMP, compared to the avidity to which it binds to a second T cell that displays: i) a TCR specific for an epitope other than the epitope present in the TMMP; and ii) a co-immunomodulatory polypeptide that binds to the immunomodulatory polypeptide present in the TMMP.

Binding affinity between an immunomodulatory polypeptide and its cognate co-immunomodulatory polypeptide can be determined by bio-layer interferometry (BLI) using purified immunomodulatory polypeptide and purified cognate co-immunomodulatory polypeptide. Binding affinity between a TMMP and its cognate co-immunomodulatory polypeptide can be determined by BLI using purified TMMP and the cognate co-immunomodulatory polypeptide. BLI methods are well known to those skilled in the art. See, e.g., Lad et al. (2015) J. Biomol. Screen. 20(4):498-507; and Shah and Duncan (2014) J. Vis. Exp. 18:e51383.

A BLI assay can be carried out using an Octet RED 96 (Pal FortéBio) instrument, or a similar instrument, as follows. A TMMP (e.g., a TMMP of the present disclosure; a control TMMP (where a control TMMP comprises a wild-type immunomodulatory polypeptide)) is immobilized onto an insoluble support (a "biosensor"). The immobilized TMMP is the "target." Immobilization can be effected by immobilizing a capture antibody onto the insoluble support, where the capture antibody immobilizes the TMMP. For example, immobilization can be effected by immobilizing anti-Fe (e.g., anti-human IgG Fc) antibodies onto the insoluble support, where the immobilized anti-Fc antibodies bind to and immobilize the TMMP (where the TMMP comprises an IgFc polypeptide). A co-immunomodulatory polypeptide is applied, at several different concentrations, to the immobilized TMMP, and the instrument's response recorded. Assays are conducted in a liquid medium comprising 25mM HEPES pH 6.8, 5% poly(ethylene glycol) 6000, 50 mM KCl, 0.1% bovine serum albumin, and 0.02% Tween 20 nonionic detergent. Binding of the co-immunomodulatory polypeptide to the immobilized TMMP is conducted at 30°C. As a positive control for binding affinity, an anti-MHC Class I monoclonal antibody can be used. For example, anti-HLA Class I monoclonal antibody W6/32 (American Type Culture Collection No. HB-95; Parham et al. (1979) J. Immunol. 123:342), which has a K_{D} of 7 nM, can be used. A standard curve can be generated using serial dilutions of the anti-MHC Class I monoclonal antibody. The co-immunomodulatory polypeptide, or the anti-MHC Class I mAb, is the "analyte." BLI analyzes the interference pattern of white light reflected from two surfaces: i) from the immobilized polypeptide ("target"); and ii) an internal reference layer. A change in the number of molecules ("analyte"; e.g., co-immunomodulatory polypeptide; anti-HLA antibody) bound to the biosensor tip causes a shift in the interference pattern; this shift in interference pattern can be measured in real time. The two kinetic terms that describe the affinity of the target/analyte interaction are the association constant (*k*ₐ) and dissociation constant (*k*_{d}). The ratio of these two terms (*k*_{d}/ₐ) gives rise to the affinity constant K_{D}.

The BLI assay is carried out in a multi-well plate. To run the assay, the plate layout is defined, the assay steps are defined, and biosensors are assigned in Octet Data Acquisition software. The biosensor assembly is hydrated. The hydrated biosensor assembly and the assay plate are equilibrated for 10 minutes on the Octet instrument. Once the data are acquired, the acquired data are loaded into the Octet Data Analysis software. The data are processed in the Processing window by specifying method for reference subtraction, y-axis alignment, inter-step correction, and Savitzky-Golay filtering. Data are analyzed in the Analysis window by specifying steps to analyze (Association and Dissociation), selecting curve fit model (1:1), fitting method (global), and window of interest (in seconds). The quality of fit is evaluated. K_{D} values for each data trace (analyte concentration) can be averaged if within a 3-fold range. K_{D} error values should be within one order of magnitude of the affinity constant values; R² values should be above 0.95. See, e.g., Abdiche et al. (2008) J. Anal. Biochem. 377:209.

Unless otherwise stated herein, the affinity of a TMMP of the present disclosure for a cognate co-immunomodulatory polypeptide, or the affinity of a control TMMP (where a control TMMP comprises a wild-type immunomodulatory polypeptide) for a cognate co-immunomodulatory polypeptide, is determined using BLI, as described above.

In some cases, the ratio of: i) the binding affinity of a control TMMP (where the control comprises a wild-type immunomodulatory polypeptide) to a cognate co-immunomodulatory polypeptide to ii) the binding affinity of a TMMP of the present disclosure comprising a variant of the wild-type immunomodulatory polypeptide to the cognate co-immunomodulatory polypeptide, when measured by BLI (as described above), is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1. In some cases, the ratio of: i) the binding affinity of a control TMMP (where the control comprises a wild-type immunomodulatory polypeptide) to a cognate co-immunomodulatory polypeptide to ii) the binding affinity of a TMMP of the present disclosure comprising a variant of the wild-type immunomodulatory polypeptide to the cognate co-immunomodulatory polypeptide, when measured by BLI, is in a range of from 1.5:1 to 10⁶:1, e.g., from 1.5:1 to 10:1, from 10:1 to 50:1, from 50:1 to 10²:1, from 10²:1 to 10³:1, from 10³:1 to 10⁴:1, from 10⁴:1 to 10⁵:1, or from 10⁵:1 to 10⁶:1.

As an example, where a control TMMP comprises a wild-type IL-2 polypeptide, and where a TMMP of the present disclosure comprises a variant IL-2 polypeptide (comprising from 1 to 10 amino acid substitutions relative to the amino acid sequence of the wild-type IL-2 polypeptide) as the immunomodulatory polypeptide, the ratio of: i) the binding affinity of the control TMMP to an IL-2 receptor (i.e., the cognate co-immunomodulatory polypeptide) to ii) the binding affinity of the TMMP of the present disclosure to the IL-2 receptor, when measured by BLI, is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1. In some cases, where a control TMMP comprises a wild-type IL-2 polypeptide, and where a TMMP of the present disclosure comprises a variant IL-2 polypeptide (comprising from 1 to 10 amino acid substitutions relative to the amino acid sequence of the wild-type IL-2 polypeptide) as the immunomodulatory polypeptide, the ratio of: i) the binding affinity of the control TMMP to an IL-2 receptor (i.e., the cognate co-immunomodulatory polypeptide) to ii) the binding affinity of the TMMP of the present disclosure to the IL-2 receptor, when measured by BLI, is in a range of from 1.5:1 to 10⁶:1, e.g., from 1.5:1 to 10:1, from 10:1 to 50:1, from 50:1 to 10²:1, from 10²:1 to 10³:1, from 10³:1 to 10⁴:1, from 10⁴:1 to 10⁵:1, or from 10⁵:1 to 10⁶:1.

As another example, where a control TMMP comprises a wild-type PD-L1 polypeptide, and where a TMMP of the present disclosure comprises a variant PD-L1 polypeptide (comprising from 1 to 10 amino acid substitutions relative to the amino acid sequence of the wild-type PD-L1 polypeptide) as the immunomodulatory polypeptide, the ratio of: i) the binding affinity of the control TMMP to a PD-1 polypeptide (i.e., the cognate co-immunomodulatory polypeptide) to ii) the binding affinity of the TMMP of the present disclosure to the PD-1 polypeptide, when measured by BLI, is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1.

As another example, where a control TMMP comprises a wild-type CD80 polypeptide, and where a TMMP of the present disclosure comprises a variant CD80 polypeptide (comprising from 1 to 10 amino acid substitutions relative to the amino acid sequence of the wild-type CD80 polypeptide) as the immunomodulatory polypeptide, the ratio of: i) the binding affinity of the control TMMP to a CTLA4 polypeptide (i.e., the cognate co-immunomodulatory polypeptide) to ii) the binding affinity of the TMMP of the present disclosure to the CTLA4 polypeptide, when measured by BLI, is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1.

As another example, where a control TMMP comprises a wild-type CD80 polypeptide, and where a TMMP of the present disclosure comprises a variant CD80 polypeptide (comprising from 1 to 10 amino acid substitutions relative to the amino acid sequence of the wild-type CD80 polypeptide) as the immunomodulatory polypeptide, the ratio of: i) the binding affinity of the control TMMP to a CD28 polypeptide (i.e., the cognate co-immunomodulatory polypeptide) to ii) the binding affinity of the TMMP of the present disclosure to the CD28 polypeptide, when measured by BLI, is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1.

As another example, where a control TMMP comprises a wild-type 4-1BBL polypeptide, and where a TMMP of the present disclosure comprises a variant 4-1BBL polypeptide (comprising from 1 to 10 amino acid substitutions relative to the amino acid sequence of the wild-type 4-1BBL polypeptide) as the immunomodulatory polypeptide, the ratio of: i) the binding affinity of the control TMMP to a 4-1BB polypeptide (i.e., the cognate co-immunomodulatory polypeptide) to ii) the binding affinity of the TMMP of the present disclosure to the 4-1BB polypeptide, when measured by BLI, is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1.

As another example, where a control TMMP comprises a wild-type CD86 polypeptide, and where a TMMP of the present disclosure comprises a variant CD86 polypeptide (comprising from 1 to 10 amino acid substitutions relative to the amino acid sequence of the wild-type CD86 polypeptide) as the immunomodulatory polypeptide, the ratio of: i) the binding affinity of the control TMMP to a CD28 polypeptide (i.e., the cognate co-immunomodulatory polypeptide) to ii) the binding affinity of the TMMP of the present disclosure to the CD28 polypeptide, when measured by BLI, is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at least 10⁵:1, or at least 10⁶:1.

Binding affinity of a TMMP of the present disclosure to a target T cell can be measured in the following manner: A) contacting a TMMP of the present disclosure with a target T-cell expressing on its surface: i) a cognate co-immunomodulatory polypeptide that binds the parental wild-type immunomodulatory polypeptide; and ii) a T-cell receptor that binds to the epitope, where the TMMP comprises an epitope tag, such that the TMMP binds to the target T-cell; B) contacting the target T-cell-bound TMMP with a fluorescently labeled binding agent (e.g., a fluorescently labeled antibody) that binds to the epitope tag, generating a TMMP/target T-cell/binding agent complex; C) measuring the mean fluorescence intensity (MFI) of the TMMP/target T-cell/binding agent complex using flow cytometry. The epitope tag can be, e.g., a FLAG tag, a hemagglutinin tag, a c-myc tag, a poly(histidine) tag, etc. The MFI measured over a range of concentrations of the TMMP library member provides a measure of the affinity. The MFI measured over a range of concentrations of the TMMP library member provides a half maximal effective concentration (EC₅₀) of the TMMP. In some cases, the EC₅₀ of a TMMP of the present disclosure for a target T cell is in the nM range; and the EC₅₀ of the TMMP for a control T cell (where a control T cell expresses on its surface: i) a cognate co-immunomodulatory polypeptide that binds the parental wild-type immunomodulatory polypeptide; and ii) a T-cell receptor that does not bind to the epitope present in the TMMP) is in the µM range. In some cases, the ratio of the EC₅₀ of a TMMP of the present disclosure for a control T cell to the EC₅₀ of the TMMP for a target T cell is at least 1.5:1, at least 2:1, at least 5:1, at least 10:1, at least 15:1, at least 20:1, at least 25:1, at least 50:1, at least 100:1, at least 500:1, at least 10²:1, at least 5 x 10²:1, at least 10³:1, at least 5 x 10³:1, at least 10⁴:1, at lease 10⁵:1, or at least 10⁶:1. The ratio of the EC₅₀ of a TMMP of the present disclosure for a control T cell to the EC₅₀ of the TMMP for a target T cell is an expression of the selectivity of the TMMP.

In some cases, when measured as described in the preceding paragraph, a TMMP of the present disclosure exhibits selective binding to target T-cell, compared to binding of the TMMP library member to a control T cell that comprises: i) the cognate co-immunomodulatory polypeptide that binds the parental wild-type immunomodulatory polypeptide; and ii) a T-cell receptor that binds to an epitope other than the epitope present in the TMMP library member.

### Dimerized TMMPs

A TMMP of the present disclosure can be dimerized; i.e., the present disclosure provides a multimeric polypeptide comprising a dimer of a TMMP of the present disclosure. Thus, the present disclosure provides a TMMP comprising: A) a first heterodimer comprising: a) a first polypeptide comprising: i) a peptide epitope; and ii) a first major histocompatibility complex (MHC) polypeptide; and b) a second polypeptide comprising: i) a second MHC polypeptide, wherein the first heterodimer comprises one or more immunomodulatory polypeptides; and B) a second heterodimer comprising: a) a first polypeptide comprising: i) a peptide epitope; and ii) a first MHC polypeptide; and b) a second polypeptide comprising: i) a second MHC polypeptide, wherein the second heterodimer comprises one or more immunomodulatory polypeptides, and wherein the first heterodimer and the second heterodimer are covalently linked to one another. In some cases, the two TMMPs are identical to one another in amino acid sequence. In some cases, the first heterodimer and the second heterodimer are covalently linked to one another via a C-terminal region of the second polypeptide of the first heterodimer and a C-terminal region of the second polypeptide of the second heterodimer. In some cases, first heterodimer and the second heterodimer are covalently linked to one another via the C-terminal amino acid of the second polypeptide of the first heterodimer and the C-terminal region of the second polypeptide of the second heterodimer; for example, in some cases, the C-terminal amino acid of the second polypeptide of the first heterodimer and the C-terminal region of the second polypeptide of the second heterodimer are linked to one another, either directly or via a linker. The linker can be a peptide linker. The peptide linker can have a length of from 1 amino acid to 200 amino acids (e.g., from 1 amino acid (aa) to 5 aa, from 5 aa to 10 aa, from 10 aa to 25 aa, from 25 aa to 50 aa, from 50 aa to 100 aa, from 100 aa to 150 aa, or from 150 aa to 200 aa). In some cases, the peptide epitope of the first heterodimer and the peptide epitope of the second heterodimer comprise the same amino acid sequence. In some cases, the first MHC polypeptide of the first and the second heterodimer is an MHC Class I β2-microglobulin, and wherein the second MHC polypeptide of the first and the second heterodimer is an MHC Class I heavy chain. In some cases, the immunomodulatory polypeptide of the first heterodimer and the immunomodulatory polypeptide of the second heterodimer comprise the same amino acid sequence. In some cases, the immunomodulatory polypeptide of the first heterodimer and the immunomodulatory polypeptide of the second heterodimer are variant immunomodulatory polypeptides that comprise from 1 to 10 amino acid substitutions compared to a corresponding parental wild-type immunomodulatory polypeptide, and wherein the from 1 to 10 amino acid substitutions result in reduced affinity binding of the variant immunomodulatory polypeptide to a cognate co-immunomodulatory polypeptide. In some cases, the immunomodulatory polypeptide of the first heterodimer and the immunomodulatory polypeptide of the second heterodimer are each independently selected from the group consisting of IL-2, 4-1BBL, PD-L1, CD80, CD86, ICOS-L, OX-40L, FasL, JAG1 (CD339), TGFβ, CD70, and ICAM. Examples, of suitable MHC polypeptides, immunomodulatory polypeptides, and peptide epitopes are described below.

### MHC polypeptides

As noted above, a TMMP of the present disclosure includes MHC polypeptides. For the purposes of the instant disclosure, the term "major histocompatibility complex (MHC) polypeptides" is meant to include MHC polypeptides of various species, including human MHC (also referred to as human leukocyte antigen (HLA)) polypeptides, rodent (e.g., mouse, rat, etc.) MHC polypeptides, and MHC polypeptides of other mammalian species (e.g., lagomorphs, non-human primates, canines, felines, ungulates (e.g., equines, bovines, ovines, caprines, etc.), and the like. The term "MHC polypeptide" is meant to include Class I MHC polypeptides (e.g., β-2 microglobulin and MHC class I heavy chain).

In some cases, the first MHC polypeptide is an MHC Class I β2M (β2M) polypeptide, and the second MHC polypeptide is an MHC Class I heavy chain (H chain) ("MHC-H")). In other instances, the first MHC polypeptide is an MHC Class I heavy chain polypeptide; and the second MHC polypeptide is a β2M polypeptide. In some cases, both the β2M and MHC-H chain are of human origin; i.e., the MHC-H chain is an HLA heavy chain, or a variant thereof. Unless expressly stated otherwise, a TMMP of the present disclosure does not include membrane anchoring domains (transmembrane regions) of an MHC Class I heavy chain, or a part of MHC Class I heavy chain sufficient to anchor the resulting TMMP to a cell *(e.g.,* eukaryotic cell such as a mammalian cell) in which it is expressed. In some cases, the MHC Class I heavy chain present in a TMMP of the present disclosure does not include a signal peptide, a transmembrane domain, or an intracellular domain (cytoplasmic tail) associated with a native MHC Class I heavy chain. Thus, e.g., in some cases, the MHC Class I heavy chain present in a TMMP of the present disclosure includes only the α1, α2, and α3 domains of an MHC Class I heavy chain. In some cases, the MHC Class I heavy chain present in a TMMP of the present disclosure has a length of from about 270 amino acids (aa) to about 290 aa. In some cases, the MHC Class I heavy chain present in a TMMP of the present disclosure has a length of 270 aa, 271 aa, 272 aa, 273 aa, 274 aa, 275 aa, 276 aa, 277 aa, 278 aa, 279 aa, 280 aa, 281 aa, 282 aa, 283 aa, 284 aa, 285 aa, 286 aa, 287 aa, 288 aa, 289 aa, or 290 aa.

In some cases, an MHC polypeptide of a TMMP is a human MHC polypeptide, where human MHC polypeptides are also referred to as "human leukocyte antigen" ("HLA") polypeptides. In some cases, an MHC polypeptide of a TMMP is a Class I HLA polypeptide, e.g., a β2-microglobulin polypeptide, or a Class I HLA heavy chain polypeptide. Class I HLA heavy chain polypeptides include HLA-A heavy chain polypeptides, HLA-B heavy chain polypeptides, HLA-C heavy chain polypeptides, HLA-E heavy chain polypeptides, HLA-F heavy chain polypeptides, and HLA-G heavy chain polypeptides.

### MHC Class I heavy chains

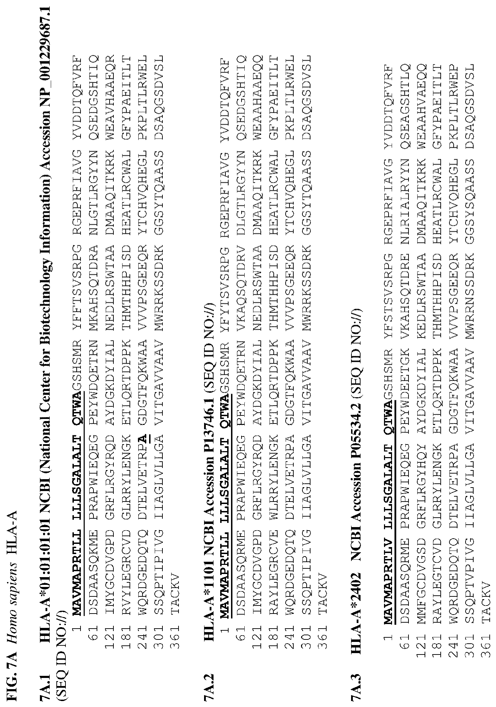

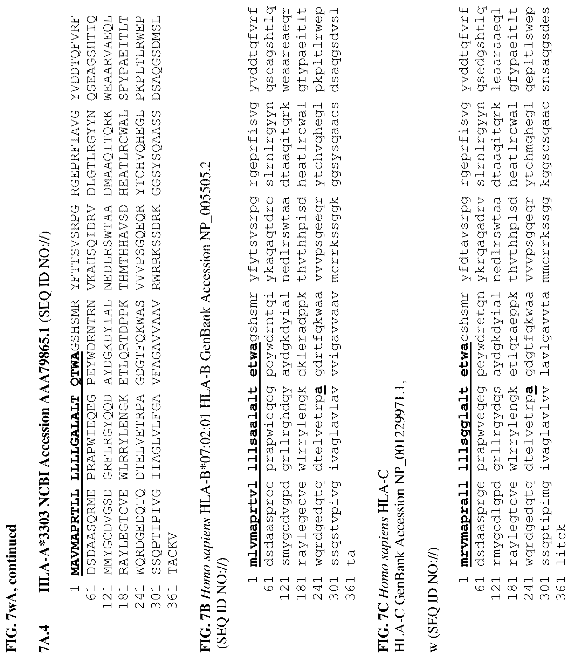

In some cases, an MHC Class I heavy chain polypeptide present in a TMMP of the present disclosure comprises an amino acid sequence having at least 75%, at least 80%, at least 85%, at least 90%, at least 95%, at least 98%, at least 99%, or 100%, amino acid sequence identity to all or part (e.g., 50, 75, 100, 150, 200, or 250 contiguous amino acids) of the amino acid sequence of any of the human HLA heavy chain polypeptides depicted in **FIGs. 7-13****.** In some cases, the MHC Class I heavy chain has a length of 270 aa, 271 aa, 272 aa, 273 aa, 274 aa, 275 aa, 276 aa, 277 aa, 278 aa, 279 aa, 280 aa, 281 aa, 282 aa, 283 aa, 284 aa, 285 aa, 286 aa, 287 aa, 288 aa, 289 aa, or 290 aa. In some cases, an MHC Class I heavy chain polypeptide present in a TMMP of the present disclosure comprises 1-30, 1-5, 5-10, 10-15, 15-20, 20-25 or 25-30 amino acid insertions, deletions, and/or substitutions (in addition to those locations indicated as being variable in the heavy chain consensus sequences) of any one of the amino acid sequences depicted in **FIGs 7-13****.** In some cases, the MHC Class I heavy chain does not include transmembrane or cytoplasmic domains. As an example, a MHC Class I heavy chain polypeptide of a TMMP of the present disclosure can comprise an amino acid sequence having at least 75%, at least 80%, at least 85%, at least 90%, at least 95%, at least 98%, at least 99%, or 100%, amino acid sequence identity to amino acids 25-300 (lacking all, or substantially all, of the leader, transmembrane and cytoplasmic sequence) or amino acids 25-365 (lacking the leader) of a human HLA-A heavy chain polypeptides depicted in any one of **FIG. 7A****,** **7B, and 7C****.**

FIGs. 7A, 7B and 7C provide amino acid sequences of human leukocyte antigen (HLA) Class I heavy chain polypeptides. Signal sequences, amino acids 1-24, are bolded and underlined. FIG. 7A entry: 3A.1 is the HLA-A heavy chain (HLA-A*01:01:01:01 or A*0101) (NCBI accession NP_001229687.1), SEQ ID NO:23; entry 3A.2 is from HLA-A*1101 SEQ ID NO:24; entry 3A.3 is from HLA-A*2402 SEQ ID NO:25 and entry 3A.4 is from HLA-A*3303 SEQ ID NO:26. FIG. 7B provides the sequence HLA-B*07:02:01 (HLA-B*0702) NCBI GenBank Accession NP_005505.2 (see also GenBank Accession AUV50118.1.). FIG. 7C provides the sequence HLA- C*0701 (GenBank Accession NP_001229971.1) (HLA-C*07:01:01:01 or HLA-Cw*070101, HLA-Cw*07 see GenBank Accession CAO78194.1).

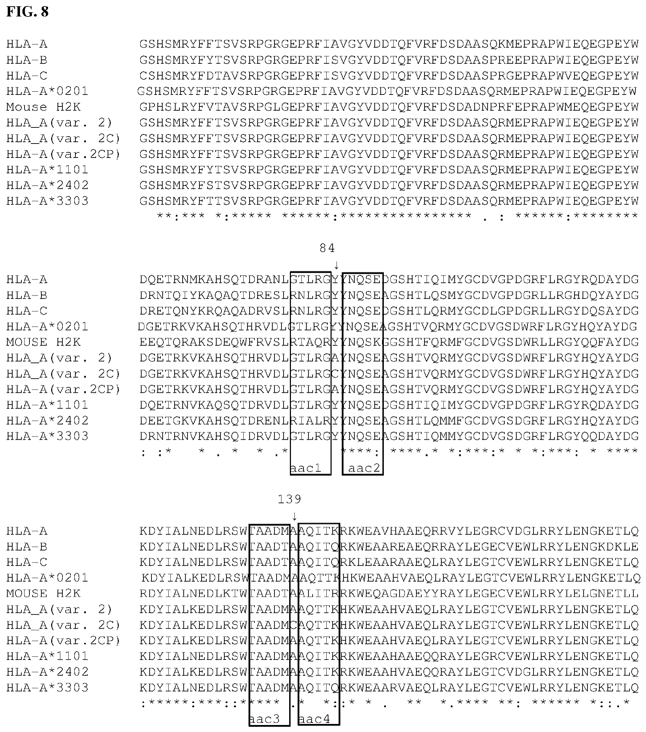

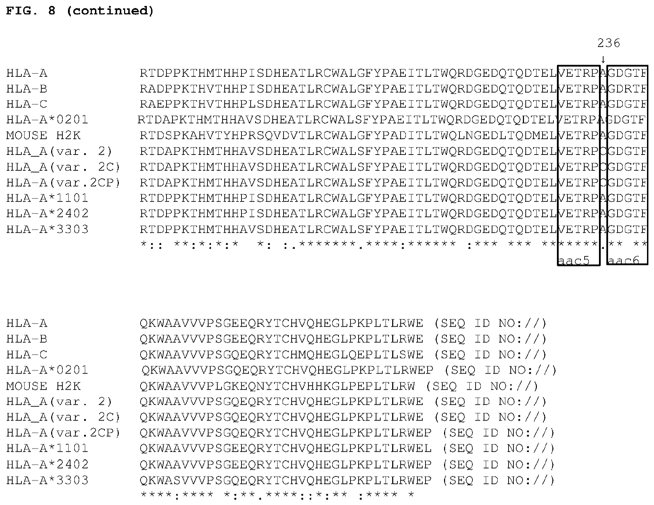

**FIG. 8** provides an alignment of eleven mature MHC class I heavy chain amino acid sequences without their leader sequences or transmembrane domains or intracellular domains. The aligned sequences are human HLA-A, HLA-B, and HLA-C, a mouse H2K protein sequence, three variants of HLA-A (var.1, var. 2C, and var.2CP), and 3 human HLA-A variants (HLA-A*1101; HLA-A*2402; and HLA-A*3303). Indicated in the alignment are the locations (84 and 139 of the mature proteins) where cysteine residues may be introduced (e.g., by substitution) for the formation of a disulfide bond to stabilize the MHC H chain - β2M complex. Also shown in the alignment is position 236 (of the mature polypeptide), which may be substituted by a cysteine residue that can form an inter-chain disulfide bond with β2M (*e.g.,* at aa 12). An arrow appears above each of those locations and the residues are bolded. The seventh HLA-A sequence shown in the alignment (var. 2c), shows the sequence of variant 2 substituted with C residues at positions 84, 139 and 236. The boxes flanking residues 84, 139 and 236 show the groups of five amino acids on either sides of those six sets of five residues, denoted aac1 (for "amino acid cluster 1"), aac2 (for "amino acid cluster 2"), aac3 (for "amino acid cluster 3"), aac4 (for "amino acid cluster 4"), aac5 (for "amino acid cluster 5"), and aac6 (for "amino acid cluster 6"), that may be replaced by 1 to 5 amino acids selected independently from (i) any naturally occurring amino acid or (ii) any naturally occurring amino acid except proline or glycine.

With regard to **FIG. 8****,** in some cases: i) aac1 (amino acid cluster 1) may be the amino acid sequence GTLRG (SEQ ID NO:287) or that sequence with one or two amino acids deleted or substituted with other naturally occurring amino acids (*e.g.,* L replaced by I, V, A or F); ii) aac2 (amino acid cluster 2) may be the amino acid sequence YNQSE (SEQ ID NO:288) or that sequence with one or two amino acids deleted or substituted with other naturally occurring amino acids (*e.g.,* N replaced by Q, Q replaced by N, and/or E replaced by D); iii) aac3 (amino acid cluster 3) may be the amino acid sequence TAADM (SEQ ID NO:289) or that sequence with one or two amino acids deleted or substituted with other naturally occurring amino acids (*e.g.,* T replaced by S, A replaced by G, D replaced by E, and/or M replaced by L, V, or I); iv) aac4 (amino acid cluster 4) may be the amino acid sequence AQTTK (SEQ ID NO:290) or that sequence with one or two amino acids deleted or substituted with other naturally occurring amino acids (*e.g.,* A replaced by G, Q replaced by N, or T replaced by S, and/or K replaced by R or Q); v) aac5 (amino acid cluster 5) may be the amino acid sequence VETRP (SEQ ID NO:291) or that sequence with one or two amino acids deleted or substituted with other naturally occurring amino acids (*e.g.,* V replaced by I or L, E replaced by D, T replaced by S, and/or R replaced by K); and/or vi) aac6 (amino acid cluster 6) may be the amino acid sequence GDGTF (SEQ ID NO:292) or that sequence with one or two amino acids deleted or substituted with other naturally occurring amino acids (*e.g.,* D replaced by E, T replaced by S, or F replaced by L, W, or Y).

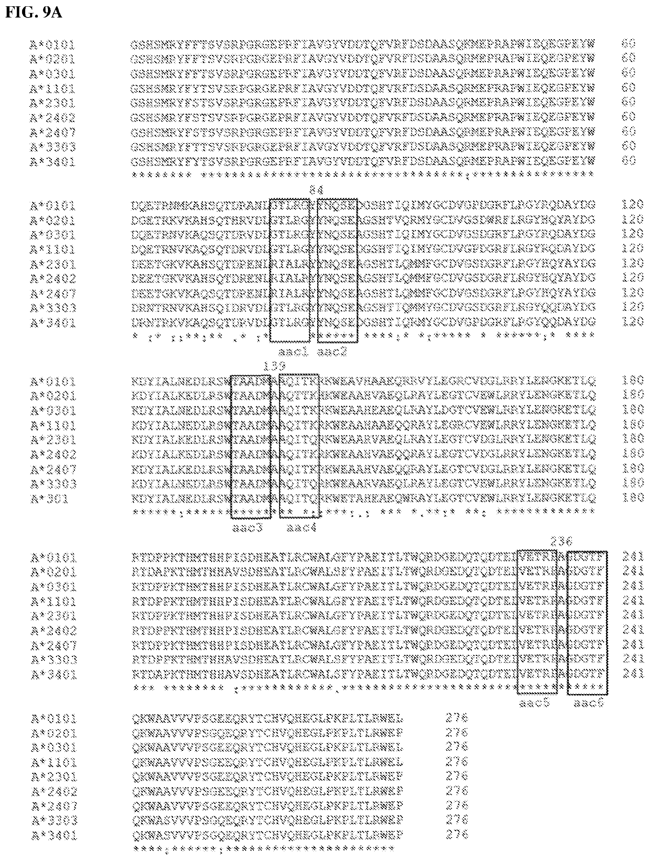

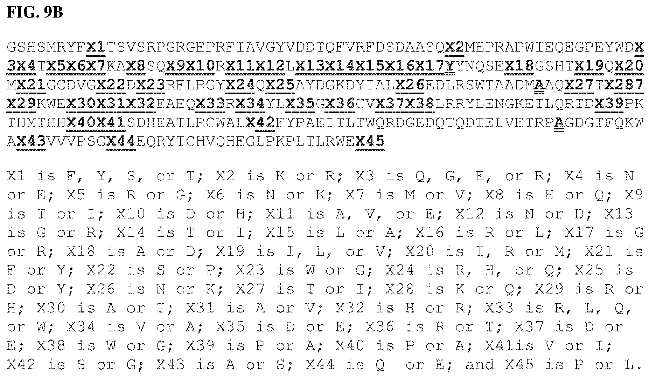

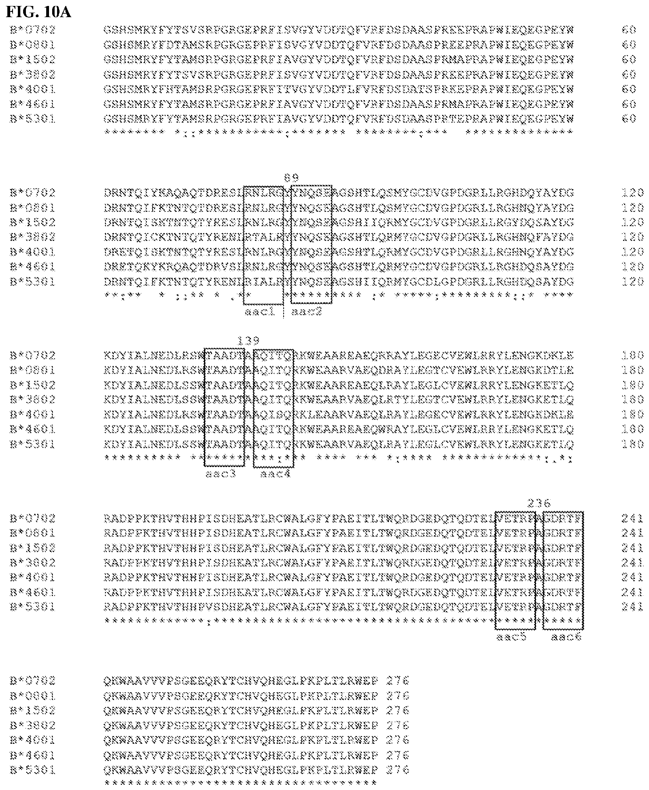

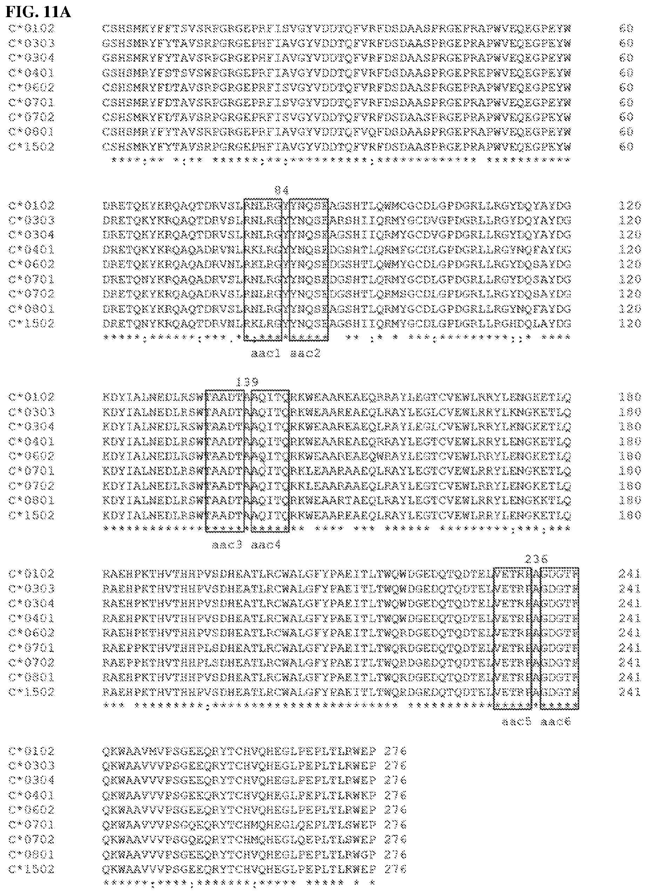

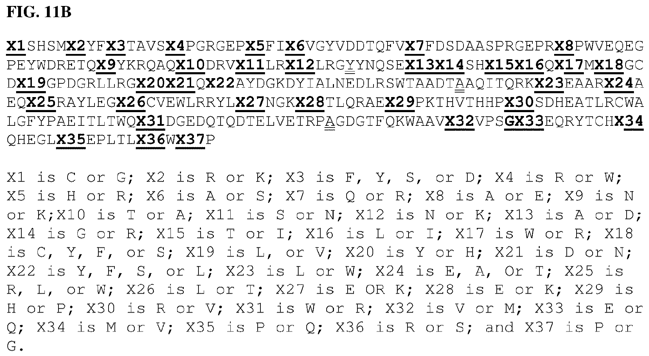

**FIGs. 9-11** provide alignments of mature HLA class I heavy chain amino acid sequences (without leader sequences or transmembrane domains or intracellular domains). The aligned amino acid sequences in **FIG. 9A** are HLA-A class I heavy chains of the following alleles: A*0101, A*0201, A*0301, A*1101, A*2301, A*2402, A*2407, A*3303, and A*3401. The aligned amino acid sequences in **FIG. 10A** are HLA-B class I heavy chains of the following alleles: B*0702, B*0801, B*1502, B*3802, B*4001, B*4601, and B*5301. The aligned amino acid sequences in **FIG. 11A** are HLA-C class I heavy chains of the following alleles: C*0102, C*0303, C*0304, C*0401, C*0602, C*0701, C*0801, and C*1502. Indicated in the alignments are the locations (84 and 139 of the mature proteins) where cysteine residues may be introduced (e.g., by substitution) for the formation of a disulfide bond to stabilize the HLA H chain - β2M complex. Also shown in the alignment is position 236 (of the mature polypeptide), which may be substituted by a cysteine residue that can form an inter-chain disulfide bond with β2M (*e.g.,* at aa 12). The boxes flanking residues 84, 139 and 236 show the groups of five amino acids on either sides of those six sets of five residues, denoted aac1 (for "amino acid cluster 1"), aac2 (for "amino acid cluster 2"), aac3 (for "amino acid cluster 3"), aac4 (for "amino acid cluster 4"), aac5 (for "amino acid cluster 5"), and aac6 (for "amino acid cluster 6"), that may be replaced by 1 to 5 amino acids selected independently from (i) any naturally occurring amino acid or (ii) any naturally occurring amino acid except proline or glycine.

**FIGs. 9A****,** **10A****, and** **11A** provide alignments of the amino acid sequences of mature HLA-A, -B, and -C class I heavy chains, respectively. The sequences are provided for the extracellular portion of the mature protein (without leader sequences or transmembrane domains or intracellular domains). As described in **FIG. 8****,** the positions of aa residues 84, 139, and 236 and their flanking residues (aac1 to aac6) that may be replaced by 1 to 5 amino acids selected independently from (i) any naturally occurring amino acid or (ii) any naturally occurring amino acid except proline or glycine ae also shown. **FIG. 9B****,** **10B****, and** **11B** provide consensus amino acid sequences for the HLA-A, -B, and -C sequences, respectively, provide in FIG. 9A, 10A, and 11A. The consensus sequences show the variable amino acid positions as "X" residues sequentially numbered and the locations of amino acids 84, 139 and 236 double underlined.

With regard to **FIG. 9A****,** in some cases: i) aac1 (amino acid cluster 1) may be the amino acid sequence GTLRG (SEQ ID NO:287) or that sequence with one or two amino acids deleted or substituted with other naturally occurring amino acids *(e.g.,* L replaced by I, V, A or F); ii) aac2 (amino acid cluster 2) may be the amino acid sequence YNQSE (SEQ ID NO:288) or that sequence with one or two amino acids deleted or substituted with other naturally occurring amino acids (*e.g.,* N replaced by Q, Q replaced by N, and/or E replaced by D); iii) aac3 (amino acid cluster 3) may be the amino acid sequence TAADM (SEQ ID NO:289) or that sequence with one or two amino acids deleted or substituted with other naturally occurring amino acids (*e.g.,* T replaced by S, A replaced by G, D replaced by E, and/or M replaced by L, V, or I); iv) aac4 (amino acid cluster 4) may be the amino acid sequence AQTTK (SEQ ID NO:290) or that sequence with one or two amino acids deleted or substituted with other naturally occurring amino acids (*e.g.,* A replaced by G, Q replaced by N, or T replaced by S, and or K replaced by R or Q); v) aac5 (amino acid cluster 5) may be the amino acid sequence VETRP (SEQ ID NO:291) or that sequence with one or two amino acids deleted or substituted with other naturally occurring amino acids (*e.g.,* V replaced by I or L, E replaced by D, T replaced by S, and/or R replaced by K); and/or vi) aac6 (amino acid cluster 6) may be the amino acid sequence GDGTF (SEQ ID NO:292) or that sequence with one or two amino acids deleted or substituted with other naturally occurring amino acids (*e.g.,* D replaced by E, T replaced by S, or F replaced by L, W, or Y).

With regard to **FIG. 10A****,** in some cases: i) aac1 (amino acid cluster 1) may be the amino acid sequence RNLRG (SEQ ID NO:293) or that sequence with one or two amino acids deleted or substituted with other naturally occurring amino acids (*e.g.,* N replaced by T or I; and/or L replaced by A; and/or the second R replaced by L; and/or the G replaced by R); ii) aac2 (amino acid cluster 2) may be the amino acid sequence YNQSE (SEQ ID NO:288) or that sequence with one or two amino acids deleted or substituted with other naturally occurring amino acids (*e.g.,* N replaced by Q, Q replaced by N, and/or E replaced by D); iii) aac3 (amino acid cluster 3) may be the amino acid sequence TAADT (SEQ ID NO:294) or that sequence with one or two amino acids deleted or substituted with other naturally occurring amino acids (*e.g.,* the first T replaced by S; and/or A replaced by G; and/or D replaced by E; and/or the second T replaced by S); iv) aac4 (amino acid cluster 4) may be the amino acid sequence AQITQ (SEQ ID NO:295) or that sequence with one or two amino acids deleted or substituted with other naturally occurring amino acids (*e.g.,* A replaced by G; and/or the first Q replaced by N; and/or I replaced by L or V; and/or the T replaced by S; and/or the second Q replaced by N); v) aac5 (amino acid cluster 5) may be the amino acid sequence VETRP (SEQ ID NO:291) or that sequence with one or two amino acids deleted or substituted with other naturally occurring amino acids (*e.g.,* V replaced by I or L, E replaced by D, T replaced by S, and/or R replaced by K); and/or vi) aac6 (amino acid cluster 6) may be the amino acid sequence GDRTF (SEQ ID NO:296) or that sequence with one or two amino acids deleted or substituted with other naturally occurring amino acids (*e.g.,* D replaced by E; and/or T replaced by S; and/or R replaced by K or H; and/or F replaced by L, W, or Y).

With regard to **FIG. 11A****,** in some cases: i) aac1 (amino acid cluster 1) may be the amino acid sequence RNLRG (SEQ ID NO:293) or that sequence with one or two amino acids deleted or substituted with other naturally occurring amino acids (*e.g.,* N replaced by K; and/or L replaced by A or I; and/or the second R replaced by H; and/or the G replaced by T or S); ii) aac2 (amino acid cluster 2) may be the amino acid sequence YNQSE (SEQ ID NO:288) or that sequence with one or two amino acids deleted or substituted with other naturally occurring amino acids (*e.g.,* N replaced by Q, Q replaced by N, and/or E replaced by D); iii) aac3 (amino acid cluster 3) may be the amino acid sequence TAADT (SEQ ID NO:294) or that sequence with one or two amino acids deleted or substituted with other naturally occurring amino acids (*e.g.,* the first T replaced by S; and/or A replaced by G; and/or D replaced by E; and/or the second T replaced by S); iv) aac4 (amino acid cluster 4) may be the amino acid sequence AQITQ (SEQ ID NO:295) or that sequence with one or two amino acids deleted or substituted with other naturally occurring amino acids (*e.g.,* A replaced by G; and/or the first Q replaced by N; and/or I replaced by L; and/or the second Q replaced by N or K); v) aac5 (amino acid cluster 5) may be the amino acid sequence VETRP (SEQ ID NO:291) or that sequence with one or two amino acids deleted or substituted with other naturally occurring amino acids (*e.g.,* V replaced by I or L, E replaced by D, T replaced by S, and/or R replaced by K or H); and/or vi) aac6 (amino acid cluster 6) may be the amino acid sequence GDGTF (SEQ ID NO:292) or that sequence with one or two amino acids deleted or substituted with other naturally occurring amino acids (*e.g.,* D replaced by E; and/or T replaced by S; and/or F replaced by L, W, or Y).

### HLA-A

In some cases, a TMMP of the present disclosure comprises an HLA-A heavy chain polypeptide. The HLA-A heavy chain peptide sequences, or portions thereof, that may be that may be incorporated into a TMMP of the present disclosure include, but are not limited to, the alleles: A*0101, A*0201, A*0301, A*1101, A*2301, A*2402, A*2407, A*3303, and A*3401, which are aligned without all, or substantially all, of the leader, transmembrane and cytoplasmic sequences in **FIG. 9A****.** Any of those alleles may comprise a mutation at one or more of positions 84, 139 and/or 236 (as shown in **FIG. 9A****)** selected from: a tyrosine to alanine at position 84 (Y84A); a tyrosine to cysteine at position 84 (Y84C); an alanine to cysteine at position 139 (A139C); and an alanine to cysteine substitution at position 236 (A236C). In addition, HLA-A sequence having at least 75% (e.g., at least 80%, at least 85%, at least 90%, at least 95%, at least 98%, at least 99%) or 100% amino acid sequence identity to all or part (e.g., 50, 75, 100, 150, 200, or 250 contiguous amino acids) of the sequence of those HLA-A alleles may also be employed (*e.g.,* it may comprise 1-25, 1-5, 5-10, 10-15, 15-20, 20-25, or 25-30 amino acid insertions, deletions, and/or substitutions).

In some cases, a TMMP of the present disclosure comprises an HLA-A heavy chain polypeptide comprising the following HLA-A consensus amino acid sequence:
GSHSMRYF**X1**TSVSRPGRGEPRFIA VGYVDDTQFVRFDSDAASQ**X2**MEPRAPWIE QEGPEYWD**X3X4**T**X5X6X7**KA**X8**SQ**X9X10**R**X11X12**L**X13X14X15X16X17Y**YNQSE**X18**GSHT**X1 9**Q**X20**M**X21**GCDVG**X22**D**X23**RFLRGY**X24**Q**X25**AYDGKDYIAL**X26**EDLRSWTAADM**A**AQ**X27**T **X287X29**KWE**X30X31X32**EAEQ**X33**R**X34**YL**X35**G**X36**CV**X37X38**LRRYLENGKETLQRTD**X39**PK THMTHH**X40X41**SDHEATLRCWAL**X42**FYPAEITLTWQRDGEDQTQDTELVETRP**A**GDGTFQK WA**X43**VVVPSG**X44**EQRYTCHVQHEGLPKPLTLRWE**X45** (SEQ ID NO:29), wherein X1 is F. Y, S, or T; X2 is K or R; X3 is Q. G, E, or R; X4 is N or E; X5 is R or G; X6 is N or K; X7 is M or V; X8 is H or Q; X9 is T or I: X10 is D or H; X11 is A, V, or E; X12 is N or D; X13 is G or R; X14 is T or I; X15 is L or A; X16 is R or L; X17 is G or R; X18 is A or D; X19 is I, L, or V; X20 is I, R or M; X21 is F or Y; X22 is S or P; X23 is W or G; X24 is R, H, or Q; X25 is D or Y; X26 is N or K; X27 is T or 1; X28 is K or Q; X29 is R or H; X30 is A or T; X31 is A or V; X32 is H or R; X33 is R, L, Q, or W; X34 is V or A; X35 is D or E; X36 is R or T; X37 is D or E; X38 is W or G; X39 is P or A; X40 is P or A; X41is V or I; X42 is S or G; X43 is A or S; X44 is Q or E; and X45 is P or L.

As one example, an MHC Class I heavy chain polypeptide of a TMMP can comprise an amino acid sequence having at least 75%, at least 80%, at least 85%, at least 90%, at least 95%, at least 98%, at least 99%, or 100%, amino acid sequence identity to the following human HLA-A heavy chain amino acid sequence:





 (SEQ ID NO:297).

In some cases, an HLA-A heavy chain polypeptide suitable for inclusion in a TMMP of the present disclosure comprises the following amino acid sequence:
GSHSMRYFFTSVSRPGRGEPRFIAVGYVDDTQFVRFDSDAASQRMEPRAPWIEQEGPEYWDGET RKVKAHSQTHRVDLGTLRGYYNQSEAGSHTVQRMYGCDVGSDWRFLRGYHQYAYDGKDYIA LKEDLRSWTAADMAAQTTKHKWEAAHVAEQLRAYLEGTCVEWLRRYLENGKETLQRTDAPK THMTHHAVSDHEATLRCWALSFYPAEITLTWQRDGEDQTQDTELVETRPAGDGTFQKWAAVV VPSGQEQRYTCHVQHEGLPKPLTLRWEP (SEQ ID NO:297). This HLA-A heavy chain polypeptide is also referred to as "HLA-A*0201" or simply "HLA-A02." In some cases, the C-terminal Pro is not included in a TMMP of the present disclosure. For example, in some cases, an HLA-A02 polypeptide suitable for inclusion in a TMMP of the present disclosure comprises the following amino acid sequence:






 (SEQ ID NO:449).

### HLA-A (Y84A; A236C)

In some cases, the MHC Class I heavy chain polypeptide comprises Y84A and A236C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-A heavy chain (Y84A; A236C) amino acid sequence:
GSHSMRYFFTSVSRPGRGEPRFIAVGYVDDTQFVRFDSDAASQRMEPRAPWIEQEGPEYWDGET RKVKAHSQTHRVDLGTLRG**A**YNQSEAGSHTVQRMYGCDVGSDWRFLRGYHQYAYDGKDYIA LKEDLRSWTAADMAAQTTKHKWEAAHVAEQLRAYLEGTCVEWLRRYLENGKETLQRTDAPK THMTHHAVSDHEATLRCWALSFYPAEITLTWQRDGEDQTQDTELVETRP**C**GDGTFQKWAAVV VPSGQEQRYTCHVQHEGLPKPLTLRWEP (SEQ ID NO:298), where amino acid 84 is Ala and amino acid 236 is Cys. In some cases, the Cys-236 forms an interchain disulfide bond with Cys-12 of a variant β2M polypeptide that comprises an R12C substitution.

In some cases, an HLA-A heavy chain polypeptide suitable for inclusion in a TMMP of the present disclosure is an HLA-A02 (Y84A; A236C) polypeptide comprising the following amino acid sequence:

GSHSMRYFFTSVSRPGRGEPRFIAVGYVDDTQFVRFDSDAASQRMEPRAPWIEQEGPEYWDGET
RKVKAHSQTHRVDLGTLRGAYNQSEAGSHTVQRMYGCDVGSDWRFLRGYHQYAYDGKDYIA
LKEDLRSWTAADMAAQTTKHKWEAAHVAEQLRAYLEGTCVEWLRRYLENGKETLQRTDAPK
THMTHHAVSDHEATLRCWALSFYPAEITLTWQRDGEDQTQDTELVETRPCGDGTFQKWAAVV
VPSGQEQRYTCHVQHEGLPKPLTLRWEP (SEQ ID NO:298).

In some cases, an HLA-A heavy chain polypeptide suitable for inclusion in a TMMP of the present disclosure is an HLA-A02 (Y84A; A236C) polypeptide comprising the following amino acid sequence:

GSHSMRYFFTSVSRPGRGEPRFIAVGYVDDTQFVRFDSDAASQRMEPRAPWIEQEGPEYWDGET
RKVKAHSQTHRVDLGTLRGAYNQSEAGSHTVQRMYGCDVGSDWRFLRGYHQYAYDGKDYIA
LKEDLRSWTAADMAAQTTKHKWEAAHVAEQLRAYLEGTCVEWLRRYLENGKETLQRTDAPK
THMTHHAVSDHEATLRCWALSFYPAEITLTWQRDGEDQTQDTELVETRPCGDGTFQKWAAVV
VPSGQEQRYTCHVQHEGLPKPLTLRWE (SEQ ID NO:450).

### HLA-A (Y84C; A139C)

In some cases, the MHC Class I heavy chain polypeptide comprises Y84C and A139C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-A heavy chain (Y84C; A139C) amino acid sequence:
GSHSMRYFFTSVSRPGRGEPRFIAVGYVDDTQFVRFDSDAASQRMEPRAPWIEQEGPEYWDGET RKVKAHSQTHRVDLGTLRG**C**YNQSEAGSHTVQRMYGCDVGSDWRFLRGYHQYAYDGKDYIA LKEDLRSWTAADM**C**AQTTKHKWEAAHVAEQLRAYLEGTCVEWLRRYLENGKETLQRTDAPK THMTHHAVSDHEATLRCWALSFYPAEITLTWQRDGEDQTQDTELVETRPAGDGTFQKWAAVV VPSGQEQRYTCHVQHEGLPKPLTLRWEP (SEQ ID NO:299), where amino acid 84 is Cys and amino acid 139 is Cys. In some cases, Cys-84 forms an intrachain disulfide bond with Cys-139.

### HLA-A11 (HLA-A*1101)

As one non-limiting example, an MHC Class I heavy chain polypeptide of a TMMP can comprise an amino acid sequence having at least 75%, at least 80%, at least 85%, at least 90%, at least 95%, at least 98%, at least 99%, or 100%, amino acid sequence identity to the following human HLA-A11 heavy chain amino acid sequence:
GSHSMRYFYTSVSRPGRGEPRFIAVGYVDDTQFVRFDSDAASQRMEPRAPWIEQEGPEYWDQE TRNVKAQSQTDRVDLGTLRGYYNQSEDGSHTIQIMYGCDVGPDGRFLRGYRQDAYDGKDYIA LNEDLRSWTAADMAAQITKRKWEAAHAAEQQRAYLEGTCVEWLRRYLENGKETLQRTDPPKT HMTHHPISDHEATLRCWALGFYPAEITLTWQRDGEDQTQDTELVETRPAGDGTFQKWAAVVV PSGEEQRYTCHVQHEGLPKPLTLRWE (SEQ ID NO:300). Such an MHC Class I heavy chain may be prominent in Asian populations, including populations of individuals of Asian descent.

### HLA-A11 (Y84A; A236C)

As one non-limiting example, in some cases, the MHC Class I heavy chain polypeptide is an HLA-A11 allele that comprises Y84A and A236C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-A A11 heavy chain (Y84A; A236C) amino acid sequence:
GSHSMRYFYTSVSRPGRGEPRFIAVGYVDDTQFVRFDSDAASQRMEPRAPWIEQEGPEYWDQE TRNVKAQSQTDRVDLGTLRG**A**YNQSEDGSHTIQIMYGCDVGPDGRFLRGYRQDAYDGKDYIA LNEDLRSWTAADMAAQITKRKWEAAHAAEQQRAYLEGTCVEWLRRYLENGKETLQRTDPPKT HMTHHPISDHEATLRCWALGFYPAEITLTWQRDGEDQTQDTELVETRP**C**GDGTFQKWAAVVV PSGEEQRYTCHVQHEGLPKPLTLRWE (SEQ ID NO:301), where amino acid 84 is Ala and amino acid 236 is Cys. In some cases, the Cys-236 forms an interchain disulfide bond with Cys-12 of a variant β2M polypeptide that comprises an R12C substitution.

### HLA-A24 (HLA-A *2402)

As one non-limiting example, an MHC Class I heavy chain polypeptide of a TMMP of the present disclosure can comprise an amino acid sequence having at least 75%, at least 80%, at least 85%, at least 90%, at least 95%, at least 98%, at least 99%, or 100%, amino acid sequence identity to the following human HLA-A24 heavy chain amino acid sequence:
GSHSMRYFSTSVSRPGRGEPRFIAVGYVDDTQFVRFDSDAASQRMEPRAPWIEQEGPEYWDEET GKVKAHSQTDRENLRIALRYYNQSEAGSHTLQMMFGCDVGSDGRFLRGYHQYAYDGKDYIAL KEDLRSWTAADMAAQITKRKWEAAHVAEQQRAYLEGTCVDGLRRYLENGKETLQRTDPPKT HMTHHPISDHEATLRCWALGFYPAEITLTWQRDGEDQTQDTELVETRPAGDGTFQKWAAVVV PSGEEQRYTCHVQHEGLPKPLTLRWEPSSQPTVPIVGIIAGLVLLGAVITGAVVAAVMWRRNSS DRKGGSYSQAASSDSAQGSDVSLTACKV (SEQ ID NO:302). Such an MHC Class I heavy chain may be prominent in Asian populations, including populations of individuals of Asian descent. In some cases, amino acid 84 is an Ala. In some cases, amino acid 84 is a Cys. In some cases, amino acid 236 is a Cys. In some cases, amino acid 84 is an Ala and amino acid 236 is a Cys. In some cases, amino acid 84 is an Cys and amino acid 236 is a Cys.

### HLA-A33 (HLA-A*3303)

As one non-limiting example, an MHC Class I heavy chain polypeptide of a TMMP of the present disclosure can comprise an amino acid sequence having at least 75%, at least 80%, at least 85%, at least 90%, at least 95%, at least 98%, at least 99%, or 100%, amino acid sequence identity to the following human HLA-A33 heavy chain amino acid sequence:
GSHSMRYFTTSVSRPGRGEPRFIAVGYVDDTQFVRFDSDAASQRMEPRAPWIEQEGPEYWDRN TRNVKAHSQIDRVDLGTLRGYYNQSEAGSHTIQMMYGCDVGSDGRFLRGYQQDAYDGKDYIA LNEDLRSWTAADMAAQITQRKWEAARVAEQLRAYLEGTCVEWLRRYLENGKETLQRTDPPKT HMTHHAVSDHEATLRCWALSFYPAEITLTWQRDGEDQTQDTELVETRPAGDGTFQKWASVVV PSGQEQRYTCHVQHEGLPKPLTLRWEPSSQPTIPIVGIIAGLVLFGAVFAGAVVAAVRWRRKSSD RKGGSYSQAASSDSAQGSDMSLTACKV (SEQ ID NO:303). Such an MHC Class I heavy chain may be prominent in Asian populations, including populations of individuals of Asian descent. In some cases, amino acid 84 is an Ala. In some cases, amino acid 84 is a Cys. In some cases, amino acid 236 is a Cys. In some cases, amino acid 84 is an Ala and amino acid 236 is a Cys. In some cases, amino acid 84 is an Cys and amino acid 236 is a Cys.

### HLA-B

In some cases, a TMMP of the present disclosure comprises an HLA-B heavy chain polypeptide. The HLA-B heavy chain peptide sequences, or portions thereof, that may be that may be incorporated into a TMMP of the present disclosure include, but are not limited to, the alleles: B*0702, B*0801, B*1502, B*3802, B*4001, B*4601, and B*5301, which are aligned without all, or substantially all, of the leader, transmembrane and cytoplasmic sequences in **FIG. 10A****.** Any of those alleles may comprise a mutation at one or more of positions 84, 139 and/or 236 (as shown in **FIG. 10A****)** selected from: a tyrosine to alanine at position 84 (Y84A); a tyrosine to cysteine at position 84 (Y84C); an alanine to cysteine at position 139 (A139C); and an alanine to cysteine substitution at position 236 (A236C). In addition, a HLA-B polypeptide comprising an amino acid sequence having at least 75% (e.g., at least 80%, at least 85%, at least 90%, at least 95%, at least 98%, at least 99%) or 100% amino acid sequence identity to all or part (e.g., 50, 75, 100, 150, 200, or 250 contiguous amino acids) of the sequence of those HLA-B alleles may also be employed (*e.g.,* it may comprise 1-25, 1-5, 5-10, 10-15, 15-20, 20-25, or 25-30 amino acid insertions, deletions, and/or substitutions).

In some cases, a TMMP of the present disclosure comprises an HLA-B heavy chain polypeptide comprising the following HLA-B consensus amino acid sequence:
GSHSMRYF**X1**T**X2X3**SRPGRGEPRFI**X4**VGYVDDT**X5**FVRFDSDA**X6**SPR**X7X8**PR APWIEQEGPEYWDR**X9**TQ**X10X11**KT**X12X13**TQ**X14**Y**X15X16**NL**X17X18X19X20**YYNQSEAGS H**X21X22QX23**MYGCDLGPDGRLLRGHDQSAYDGKDYIALNEDL**X24**SWTAADTAAQI**X25**QRK **X26**EAAR**X27**AEQ**X28**R**X29**YLEG**X30**CVEWLRRYLENGK**X31X32**L**X33**RADPPKTHVTHHP**X34** SDHEATLRCWALGFYPAEITLTWQRDGEDQTQDTELVETRPAGDRTFQKWAAVVVPSGEEQR YTCHVQHEGLPKPLTLRWEP (SEQ ID NO:30), wherein X1 is H, Y, or D; X2 is A or S; X3 is M or V; X4 is A, S, or T; X5 is Q or L; X6 is A or T; X7 is E, M K, or T; X8 is A or T; X9 is E or N; X10 is I or K; X11 is Y, F, S, or C; X12 is N or Q; X13 is A or T; X14 is D or Y; X15 is E or V; X16 is S or N; X17 is T, N, or I; X18 is A or L; X19 is L, or R; X20 is R or G; X21 is T or I; X22 is L or I; X23 is R or S; X24 is R or S; X25 is S or T; X26 is L or W; X27 is E OR V; X28 is R, D, L or W; X29 is A or T; X30 is L, E or T; X31 is E or D; X32 is K or T; X33 is E or Q; and X34 is I or V.

As an example, an MHC Class I heavy chain polypeptide of a TMMP of the present disclosure can comprise an amino acid sequence having at least 75%, at least 80%, at least 85%, at least 90%, at least 95%, at least 98%, at least 99%, or 100%, amino acid sequence identity to the following human HLA-B heavy chain amino acid sequence:





 (SEQ ID NO:304).

### HLA-B (Y84A; A236C)

As one non-limiting example, in some cases, the MHC Class I heavy chain polypeptide is an HLA-B polypeptide that comprises Y84A and A236C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-B heavy chain (Y84A; A236C) amino acid sequence:
GSHSMRYFYTSVSRPGRGEPRFISVGYVDDTQFVRFDSDAASPREEPRAPWIEQEGPEYWDRNT QIYKAQAQTDRESLRNLRG**A**YNQSEAGSHTLQSMYGCDVGPDGRLLRGHDQYAYDGKDYIAL NEDLRSWTAADTAAQITQRKWEAAREAEQRRAYLEGECVEWLRRYLENGKDKLERADPPKTH VTHHPISDHEATLRCWALGFYPAEITLTWQRDGEDQTQDTELVETRP**C**GDRTFQKWAAVVVPS GEEQRYTCHVQHEGLPKPLTLRWEP (SEQ ID NO:305), where amino acid 84 is Ala and amino acid 236 is Cys. In some cases, the Cys-236 forms an interchain disulfide bond with Cys-12 of a variant β2M polypeptide that comprises an R12C substitution.

### HLA-B (Y84C; A139C)

In some cases, the MHC Class I heavy chain polypeptide comprises Y84C and A139C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-B heavy chain (Y84C; A139C) amino acid sequence:
GSHSMRYFYTSVSRPGRGEPRFISVGYVDDTQFVRFDSDAASPREEPRAPWIEQEGPEYWDRNT QIYKAQAQTDRESLRNLRG**C**YNQSEAGSHTLQSMYGCDVGPDGRLLRGHDQYAYDGKDYIAL NEDLRSWTAADT**C**AQITQRKWEAAREAEQRRAYLEGECVEWLRRYLENGKDKLERADPPKTH VTHHPISDHEATLRCWALGFYPAEITLTWQRDGEDQTQDTELVETRPAGDRTFQKWAAVVVPS GEEQRYTCHVQHEGLPKPLTLRWEP (SEQ ID NO:306), where amino acid 84 is Cys and amino acid 139 is Cys. In some cases, Cys-84 forms an intrachain disulfide bond with Cys-139.

### HLA-B*0702

As an example, in some cases, a MHC Class I heavy chain polypeptide present in a TMMP of the present disclosure comprises an amino acid sequence of HLA-B*0702 (SEQ ID NO:207) in **FIG. 10A****,** or a sequence having at least 75% (e.g., at least 80%, at least 85%, at least 90%, at least 95%, at least 98%, at least 99%) or 100%, amino acid sequence identity to all or part (e.g., 50, 75, 100, 150, 200, or 250 contiguous amino acids) of that sequence (*e.g.,* it may comprise 1-25, 1-5, 5-10, 10-15, 15-20, 20-25, or 25-30 amino acid insertions, deletions, and/or substitutions). In some cases, where the HLA-B heavy chain polypeptide of TMMP of the present disclosure has less than 100% identity to the sequence labeled HLA-B in **FIG. 8****,** or labeled "B*0702 in **FIG. 10A****,** it may comprise a mutation at one or more of positions 84, 139 and/or 236 selected from: a tyrosine to alanine substitution at position 84 (Y84A); a tyrosine to cysteine substitution at position 84 (Y84C); an alanine to cysteine at position 139 (A139C); and an alanine to cysteine substitution at position 236 (A236C). In some cases, the HLA-B heavy chain polypeptide of TMMP of the present disclosure comprises Y84A and A236C substitutions. In some cases, the HLA-B*0702 heavy chain polypeptide of TMMP of the present disclosure comprises Y84C and A139C substitutions. In some cases, the HLA-B heavy chain polypeptide of TMMP of the present disclosure comprises Y84C, A139C, and A236C substitutions.

### HLA-C

In some cases, a TMMP of the present disclosure comprises an HLA-C heavy chain polypeptide. The HLA-C heavy chain polypeptide, or portions thereof, that may be that may be incorporated into a TMMP of the present disclosure include, but are not limited to, the alleles: C*0102, C*0303, C*0304, C*0401, C*0602, C*0701, C*0801, and C*1502, which are aligned without all, or substantially all, of the leader, transmembrane and cytoplasmic sequences in **FIG. 11A****.** Any of those alleles may comprise a mutation at one or more of positions 84, 139 and/or 236 (as shown in **FIG. 11A****)** selected from: a tyrosine to alanine substitution at position 84 (Y84A); a tyrosine to cysteine substitution at position 84 (Y84C); an alanine to cysteine substitution at position 139 (A139C); and an alanine to cysteine substitution at position 236 (A236C). In addition, an HLA-C polypeptide comprising an amino acid sequence having at least 75% (e.g., at least 80%, at least 85%, at least 90%, at least 95%, at least 98%, at least 99%) or 100% amino acid sequence identity to all or part (e.g., 50, 75, 100, 150, 200, or 250 contiguous amino acids) of the sequence of those HLA-C alleles may also be employed *(e.g.,* it may comprise 1-25, 1-5, 5-10, 10-15, 15-20, 20-25, or 25-30 amino acid insertions, deletions, and/or substitutions).

In some cases, a TMMP of the present disclosure comprises an HLA-C heavy chain polypeptide comprising the following HLA-C consensus amino acid sequence:
**X1**SHSM**X2**YF**X3**TAVS**X4**PGRGEP**X5**FI**X6**VGYVDDTQFV**X7**FDSDAASPRGEPR**X 8**PWVEQEGPEYWDRETQ**X9**YKRQAQ**X10**DRV**X11**LR**X12**LRGYYNQSE**X13X14**SH**X15X16**QX**1 7**M**X18**GCD**X19**GPDGRLLRG**X20X21**Q**X22**AYDGKDYIALNEDLRSWTAADTAAQITQRK**X23**EA AR**X24**AEQ**X25**RAYLEG**X26**CVEWLRRYL**X27**NGK**X28**TLQRAE**X29**PKTHVTHHP**X30**SDHEAT LRCWALGFYPAEITLTWQ**X31**DGEDQTQDTELVETRPAGDGTFQKWAAV**X32**VPS**GX33**EQRYT CH**X34**QHEGL**X35**EPLTL**X36**W**X37**P (SEQ ID NO:31), wherein X1 is C or G; X2 is R or K; X3 is F, Y, S, or D; X4 is R or W; X5 is H or R; X6 is A or S; X7 is Q or R; X8 is A or E; X9 is N or K;X10 is T or A; X11 is S or N; X12 is N or K; X13 is A or D; X14 is G or R; X15 is T or I; X16 is L or I; X17 is W or R; X18 is C, Y, F, or S; X19 is L, or V; X20 is Y or H; X21 is D or N; X22 is Y, F, S, or L; X23 is L or W; X24 is E, A, Or T; X25 is R, L, or W; X26 is L or T; X27 is E OR K; X28 is E or K; X29 is H or P; X30 is R or V; X31 is W or R; X32 is V or M; X33 is E or Q; X34 is M or V; X35 is P or Q; X36 is R or S; and X37 is P or G.

As an example, an MHC Class I heavy chain polypeptide of a TMMP of the present disclosure can comprise an amino acid sequence having at least 75%, at least 80%, at least 85%, at least 90%, at least 95%, at least 98%, at least 99%, or 100%, amino acid sequence identity to the following human HLA-C heavy chain amino acid sequence:

CSHSMRYFDTAVSRPGRGEPRFISVGYVDDTQFVRFDSDAASPRGEPRAPWVEQEGPEYWDRE
TQNYKRQAQADRVSLRNLRGYYNQSEDGSHTLQRMYGCDLGPDGRLLRGYDQSAYDGKDYI
ALNEDLRSWTAADTAAQITQRKLEAARAAEQLRAYLEGTCVEWLRRYLENGKETLQRAEPPKT
HVTHHPLSDHEATLRCWALGFYPAEITLTWQRDGEDQTQDTELVETRPAGDGTFQKWAAVVV
PSGQEQRYTCHMQHEGLQEPLTLSWEP (SEQ ID NO:307).

### HLA-C (Y84A; A236C)

As one non-limiting example, in some cases, the MHC Class I heavy chain polypeptide is an HLA-C polypeptide that comprises Y84A and A236C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-C heavy chain (Y84A; A236C) amino acid sequence:
CSHSMRYFDTAVSRPGRGEPRFISVGYVDDTQFVRFDSDAASPRGEPRAPWVEQEGPEYWDRE TQNYKRQAQADRVSLRNLRG**A**YNQSEDGSHTLQRMYGCDLGPDGRLLRGYDQSAYDGKDYI ALNEDLRSWTAADTAAQITQRKLEAARAAEQLRAYLEGTCVEWLRRYLENGKETLQRAEPPKT HVTHHPLSDHEATLRCWALGFYPAEITLTWQRDGEDQTQDTELVETRP**C**GDGTFQKWAAVVV PSGQEQRYTCHMQHEGLQEPLTLSWEP (SEQ ID NO:308), where amino acid 84 is Ala and amino acid 236 is Cys. In some cases, the Cys-236 forms an interchain disulfide bond with Cys-12 of a variant β2M polypeptide that comprises an R12C substitution.

### HLA-C (Y84C; A139C)

In some cases, the MHC Class I heavy chain polypeptide comprises Y84C and A139C substitutions. For example, in some cases, the MHC Class I heavy chain polypeptide comprises an amino acid sequence having at least 75%, at least 80%, at least 85%, at least 90%, at least 95%, at least 98%, at least 99%, or 100%, amino acid sequence identity to the following human HLA-C heavy chain (Y84C; A139C) amino acid sequence:
CSHSMRYFDTAVSRPGRGEPRFISVGYVDDTQFVRFDSDAASPRGEPRAPWVEQEGPEYWDRE TQNYKRQAQADRVSLRNLRG**C**YNQSEDGSHTLQRMYGCDLGPDGRLLRGYDQSAYDGKDYI ALNEDLRSWTAADT**C**AQITQRKLEAARAAEQLRAYLEGTCVEWLRRYLENGKETLQRAEPPKT HVTHHPLSDHEATLRCWALGFYPAEITLTWQRDGEDQTQDTELVETRPAGDGTFQKWAAVVV PSGQEQRYTCHMQHEGLQEPLTLSWEP (SEQ ID NO:397), where amino acid 84 is Cys and amino acid 139 is Cys. In some cases, Cys-84 forms an intrachain disulfide bond with Cys-139.

### HLA-C*0701

In some cases, a MHC Class I heavy chain polypeptide of a TMMP of the present disclosure comprises an amino acid sequence of HLA-C*0701 of **FIG. 11A** (labeled HLA-C in **FIG. 8****),** or an amino acid sequence having at least 75% (e.g., at least 80%, at least 85%, at least 90%, at least 95%, at least 98%, at least 99%) or 100% amino acid sequence identity to all or part (e.g., 50, 75, 100, 150, 200, or 250 contiguous amino acids) of that sequence (*e.g.,* it may comprise 1-25, 1-5, 5-10, 10-15, 15-20, 20-25, or 25-30 amino acid insertions, deletions, and/or substitutions). In some cases, where the HLA-C heavy chain polypeptide of a TMMP of the present disclosure has less than 100% identity to the sequence labeled HLA-C*0701 in **FIG. 11A****,** it may comprise a mutation at one or more of positions 84, 139 and/or 236 selected from: a tyrosine to alanine substitution at position 84 (Y84A); a tyrosine to cysteine substitution at position 84 (Y84C); an alanine to cysteine at position 139 (A139C); and an alanine to cysteine substitution at position 236 (A236C). In some cases, the HLA-C heavy chain polypeptide of a TMMP of the present disclosure comprises Y84A and A236C substitutions. In some cases, the HLA-C*0701 heavy chain polypeptide of a T-Cell-MMP or its epitope conjugate comprises Y84C and A139C substitutions. In some cases, the HLA-C heavy chain polypeptide of a TMMP of the present disclosure comprises Y84C, A139C, and A236C substitutions.

### Non-classical HLA-E, -F, and -G MHC Class I heavy chains

In some cases, a TMMP of the present disclosure comprises a non-classical MHC Class I heavy chain polypeptide. Among the non-classical HLA heavy chain polypeptides, or portions thereof, that may be that may be incorporated into a TMMP of the present disclosure include, but are not limited to, those of HLA-E, -F, and -G alleles. Amino acid sequences for HLA-E, -F, and -G heavy chain polypeptides, (and the HLA-A, B and C alleles) may be found on the world wide web hla.alleles.org/ nomenclature/index.html, the European Bioinformatics Institute (www(dot)ebi(dot)ac(dot)uk), which is part of the European Molecular Biology Laboratory(EMBL), and at the National Center for Biotechnology Information (www(dot)ncbi(dot)nlm(dot)nih(dot)gov).

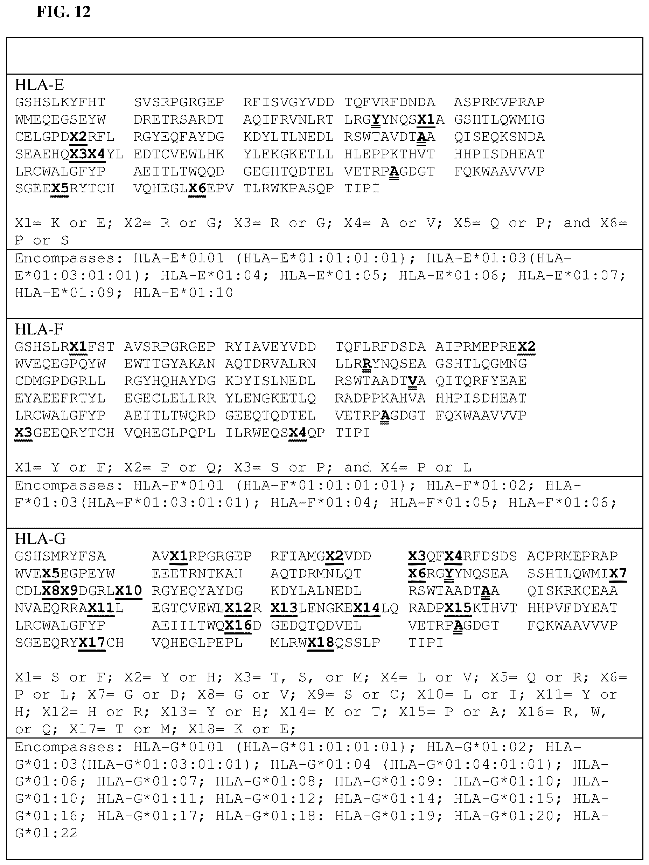

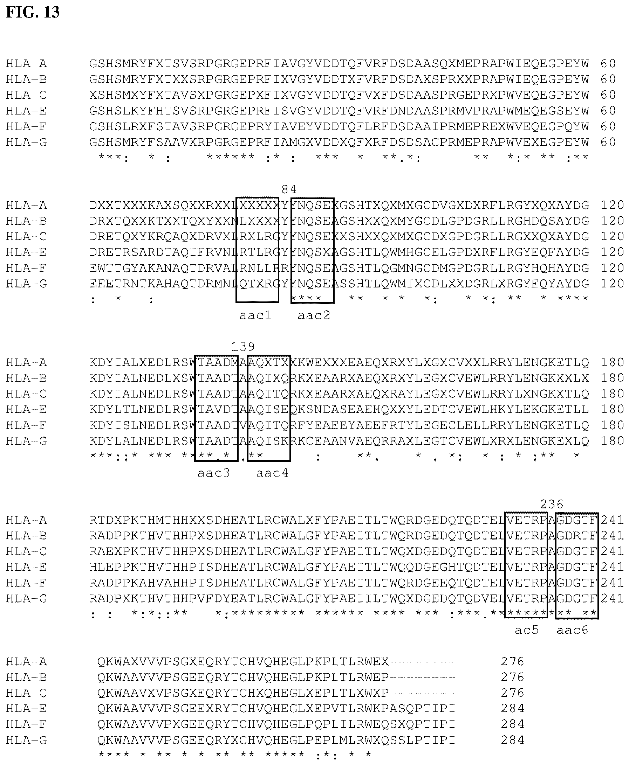

Non-limiting examples of suitable HLA-E alleles include, but are not limited to, HLA-E*0101 (HLA-E*01:01:01:01), HLA-E*01:03(HLA-E*01:03:01:01), HLA-E*01:04, HLA-E*01:05, HLA-E*01:06, HLA-E*01:07, HLA-E*01:09, and HLA-E*01:10. Non-limiting examples of suitable HLA-F alleles include, but are not limited to, HLA-F*0101 (HLA-F*01:01:01:01), HLA-F*01:02, HLA-F*01:03(HLA-F*01:03:01:01), HLA-F*01:04, HLA-F*01:05, and HLA-F*01:06. Non-limiting examples of suitable HLA-G alleles include, but are not limited to, HLA-G*0101 (HLA-G*01:01:01:01), HLA-G*01:02, HLA-G*01:03(HLA-G*01:03:01:01), HLA-G*01:04 (HLA-G*01:04:01:01), HLA-G*01:06, HLA-G*01:07, HLA-G*01:08, HLA-G*01:09: HLA-G*01:10, HLA-G*01:10, HLA-G*01:11, HLA-G*01:12, HLA-G*01:14, HLA-G*01:15, HLA-G*01:16, HLA-G*01:17, HLA-G*01:18: HLA-G*01:19, HLA-G*01:20, and HLA-G*01:22. Consensus sequences for those HLA E, -F and -G alleles without all, or substantially all, of the leader, transmembrane and cytoplasmic sequences are provided in **FIG. 12****,** and aligned with consensus sequences of the above-mentioned HLA-A, -B and -C alleles in **FIG. 13****.**

**FIG. 12** provides a consensus sequence for each of HLA-E, -F, and -G with the variable aa positions indicated as "X" residues sequentially numbered and the locations of aas 84, 139 and 236 double underlined.

**FIG. 13** provides an alignment of the consensus amino acid sequences for HLA-A, -B, - C, -E, -F, and -G, which are given in FIGs. 9-13. Variable residues in each sequence are listed as "X" with the sequential numbering removed. As indicated in FIG. 8, the locations of aas 84, 139 and 236 are indicated with their flanking five-amino acid clusters that may be replaced by 1 to 5 amino acids selected independently from (i) any naturally occurring amino acid or (ii) any naturally occurring amino acid except proline or glycine are also shown.

Any of the above-mentioned HLA-E, -F, and/or -G alleles may comprise a substitution at one or more of positions 84, 139 and/or 236 as shown in **FIG. 13** for the consensus sequences. In some cases, the substitutions may be selected from a: position 84 tyrosine to alanine (Y84A) or cysteine (Y84C), or, in the case of HLA-F, an R84A or R84C substitution; a position 139 alanine to cysteine (A139C), or, in the case of HLA-F, a V139C; and an alanine to cysteine substitution at position 236 (A236C). In addition, an HLA-E, -F and /or -G sequence having at least 75% (e.g., at least 80%, at least 85%, at least 90%, at least 95%, at least 98%, at least 99%) or 100% amino acid sequence identity to all or part (e.g., 50, 75, 100, 150, 200, or 250 contiguous amino acids) of any of the consensus sequences of set forth in **FIG. 13** may also be employed (*e.g.,* the sequences may comprise 1-25, 1-5, 5-10, 10-15, 15-20, 20-25, or 25-30 amino acid insertions, deletions, and/or substitutions in addition to changes at variable residues listed therein). Mouse H2K

In some cases, a MHC Class I heavy chain polypeptide present in a TMMP of the present disclosure comprises an amino acid sequence of MOUSE H2K (SEQ ID NO:45) (MOUSE H2K in **FIG.** 8), or a sequence having at least 75%, at least 80%, at least 85%, at least 90%, at least 95%, at least 98%, at least 99%, or 100% amino acid sequence identity to all or part (e.g., 50, 75, 100, 150, 200, or 250 contiguous amino acids) of that sequence (*e.g.,* it may comprise 1-25, 1-5, 5-10, 10-15, 15-20, 20-25, or 25-30 amino acid insertions, deletions, and/or substitutions). In some cases, where the MOUSE H2K heavy chain polypeptide of a TMMP of the present disclosure has less than 100% identity to the sequence labeled MOUSE H2K in **FIG. 8****,** it may comprise a mutation at one or more of positions 84, 139 and/or 236 selected from: a tyrosine to alanine at position 84 (Y84A); a tyrosine to cysteine at position 84 (Y84C); an alanine to cysteine at position 139 (A139C); and an alanine to cysteine substitution at position 236 (A236C). In some cases, the MOUSE H2K heavy chain polypeptide of a TMMP of the present disclosure comprises Y84A and A236C substitutions. In some cases, the MOUSE H2K heavy chain polypeptide of a TMMP of the present disclosure comprises Y84C and A139C substitutions. In some cases, the MOUSE H2K heavy chain polypeptide of a TMMP of the present disclosure comprises Y84C, A139C and A236C substitutions.

### Exemplary combinations

Table 1, below, presents various combinations of MHC Class I heavy chain sequence modifications that can be incorporated in a TMMP of the present disclosure.

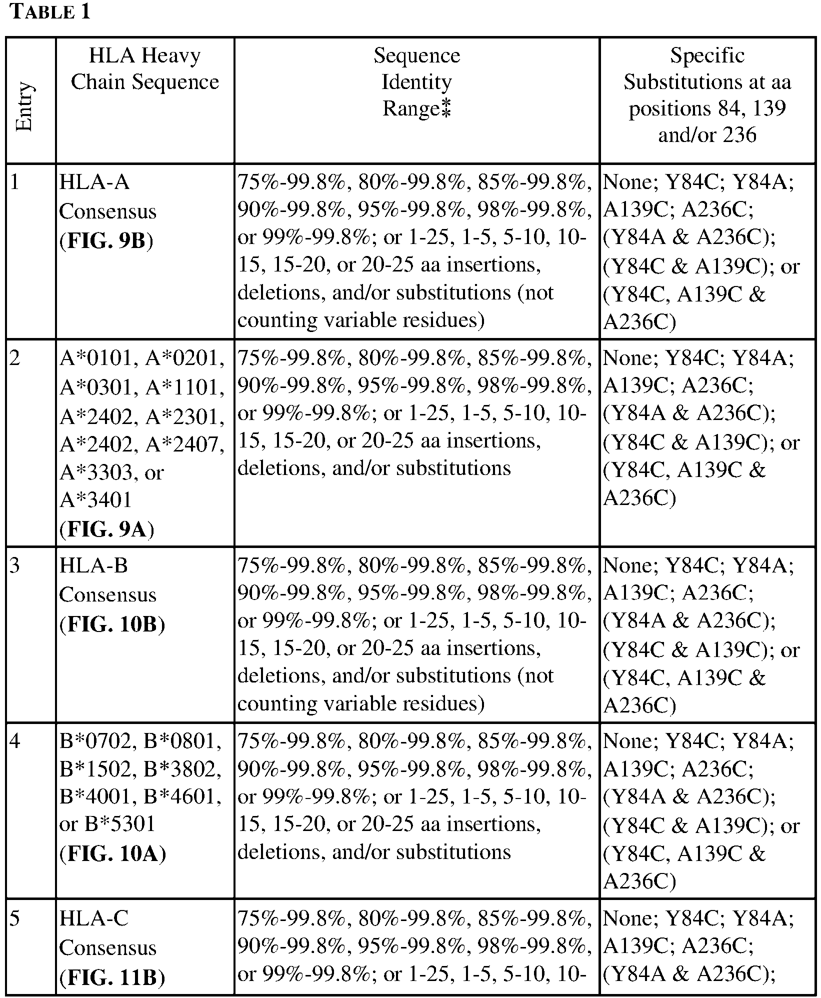

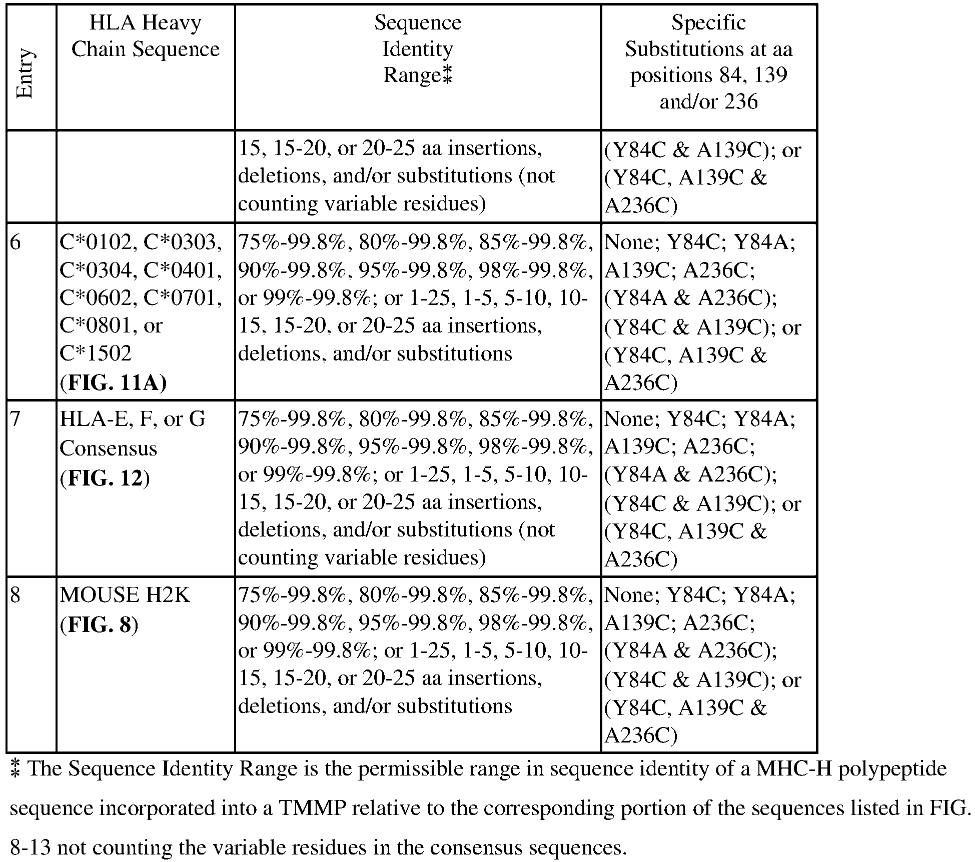

### Beta-2 micro globulin

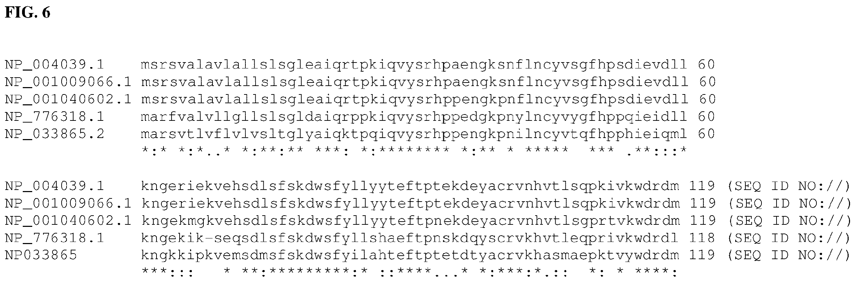

A β2-microglobulin (β2M) polypeptide of a TMMP of the present disclosure can be a human β2M polypeptide, a non-human primate β2M polypeptide, a murine β2M polypeptide, and the like. In some instances, a β2M polypeptide comprises an amino acid sequence having at least 75%, at least 80%, at least 85%, at least 90%, at least 95%, at least 98%, at least 99%, or 100%, amino acid sequence identity to a β2M amino acid sequence depicted in FIG. 6. In some instances, a β2M polypeptide comprises an amino acid sequence having at least 75%, at least 80%, at least 85%, at least 90%, at least 95%, at least 98%, at least 99%, or 100%, amino acid sequence identity to amino acids 21 to 119 of a β2M amino acid sequence depicted in FIG. 6.

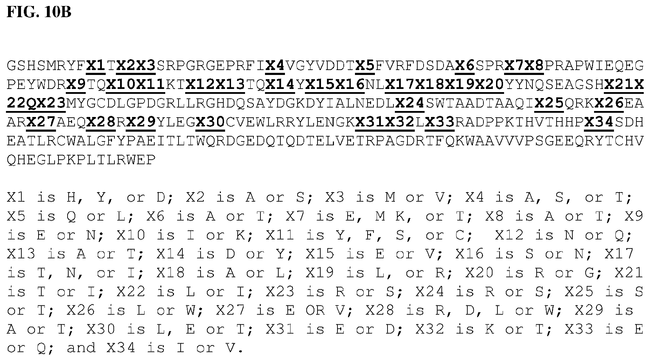

In some cases, a suitable β2M polypeptide comprises the following amino acid sequence: 
IQRTPKIQVY SCHPAENGKS NFLNCYVSGF HPSDIEVDLLKNGERIEKVE
HSDLSFSKDW SFYLLYYTEF TPTEKDEYAC RVNHVTLSQP KIVKWDRDM (SEQ ID NO:311);
 and the HLA Class I heavy chain polypeptide comprises the following amino acid sequence:
GSHSMRYFFTSVSRPGRGEPRFIAVGYVDDTQFVRFDSDAASQRMEPRAPWIEQE GPEYWDGETRKVKAHSQTHRVDL(aa1){C}(aa2)AGSHTVQRMYGCDVGSDWRFLRGYHQYAY DGKDYIALKEDLRSW(aa3){C}(aa4))HKWEAAHVAEQLRAYLEGTCVEWLRRYLENGKETLQR TDAPKTHMTHHAVSDHEATLRCWALSFYPAEITLTWQRDGEDQTQDTEL(aa5)(C)(aa6)QKWAA VVVPSGQEQRYTCHVQHEGLPKPLTLRWEP (SEQ ID NO:309), where the cysteine residues indicated as {C} form an disulfide bond between the α1 and α2-1 helices and the (C) residue forms a disulfide bond with the β2M polypeptide cysteine at position 12. In the sequence above, "aa1" is "amino acid cluster 1" ; "aa2" is "amino acid cluster 2"; "aa3" is "amino acid cluster 3" ; "aa4" is "amino acid cluster 4" ; "aa5" is "amino acid cluster 5"; and "aa6" is "amino acid cluster 6"; see, e.g., FIG. 10. Each occurrence of aa1, aa2, aa3, aa4, aa5, and aa6 is and independently selected to be 1-5 amino acid residues, wherein the amino acid residues are i) selected independently from any naturally occurring (e.g., encoded) amino acid or ii) any naturally occurring amino acid except proline or glycine.

In some cases, an MHC polypeptide comprises a single amino acid substitution relative to a reference MHC polypeptide (where a reference MHC polypeptide can be a wild-type MHC polypeptide), where the single amino acid substitution substitutes an amino acid with a cysteine (Cys) residue. Such cysteine residues, when present in an MHC polypeptide of a first polypeptide of a TMMP of the present disclosure, can form a disulfide bond with a cysteine residue present in a second polypeptide chain of a TMMP of the present disclosure.

In some cases, a first MHC polypeptide in a first polypeptide of a TMMP of the present disclosure, and/or the second MHC polypeptide in the second polypeptide of a TMMP of the present disclosure, includes an amino acid substitution to substitute an amino acid with a cysteine, where the substituted cysteine in the first MHC polypeptide forms a disulfide bond with a cysteine in the second MHC polypeptide, where a cysteine in the first MHC polypeptide forms a disulfide bond with the substituted cysteine in the second MHC polypeptide, or where the substituted cysteine in the first MHC polypeptide forms a disulfide bond with the substituted cysteine in the second MHC polypeptide.

For example, in some cases, one of following pairs of residues in an HLA β2-microglobulin and an HLA Class I heavy chain is substituted with cysteines (where residue numbers are those of the mature polypeptide): 1) β2M residue 12, HLA Class I heavy chain residue 236; 2) β2M residue 12, HLA Class I heavy chain residue 237; 3) β2M residue 8, HLA Class I heavy chain residue 234; 4) β2M residue 10, HLA Class I heavy chain residue 235; 5) β2M residue 24, HLA Class I heavy chain residue 236; 6) β2M residue 28, HLA Class I heavy chain residue 232; 7) β2M residue 98, HLA Class I heavy chain residue 192; 8) β2M residue 99, HLA Class I heavy chain residue 234; 9) β2M residue 3, HLA Class I heavy chain residue 120; 10) β2M residue 31, HLA Class I heavy chain residue 96; 11) β2M residue 53, HLA Class I heavy chain residue 35; 12) β2M residue 60, HLA Class I heavy chain residue 96; 13) β2M residue 60, HLA Class I heavy chain residue 122; 14) β2M residue 63, HLA Class I heavy chain residue 27; 15) β2M residue Arg3, HLA Class I heavy chain residue Gly120; 16) β2M residue His31, HLA Class I heavy chain residue Gln96; 17) β2M residue Asp53, HLA Class I heavy chain residue Arg35; 18) β2M residue Trp60, HLA Class I heavy chain residue Gln96; 19) β2M residue Trp60, HLA Class I heavy chain residue Asp122; 20) β2M residue Tyr63, HLA Class I heavy chain residue Tyr27; 21) β2M residue Lys6, HLA Class I heavy chain residue Glu232; 22) β2M residue Gln8, HLA Class I heavy chain residue Arg234; 23) β2M residue Tyr10, HLA Class I heavy chain residue Pro235; 24) β2M residue Ser11, HLA Class I heavy chain residue Gln242; 25) β2M residue Asn24, HLA Class I heavy chain residue Ala236; 26) β2M residue Ser28, HLA Class I heavy chain residue Glu232; 27) β2M residue Asp98, HLA Class I heavy chain residue His192; and 28) β2M residue Met99, HLA Class I heavy chain residue Arg234. The amino acid numbering of the MHC/HLA Class I heavy chain is in reference to the mature MHC/HLA Class I heavy chain, without a signal peptide. For example, in some cases, residue 236 of the mature HLA-A amino acid sequence is substituted with a Cys. In some cases, residue 236 of the mature HLA-B amino acid sequence is substituted with a Cys. In some cases, residue 236 of the mature HLA-C amino acid sequence is substituted with a Cys. In some cases, residue 32 (corresponding to Arg-12 of mature β2M) of an amino acid sequence depicted in FIG. 6 is substituted with a Cys.

In some cases, a β2M polypeptide comprises the amino acid sequence: IQRTPKIQVY SRHPAENGKS NFLNCYVSGF HPSDIEVDLLKNGERIEKVE HSDLSFSKDW SFYLLYYTEF TPTEKDEYAC RVNHVTLSQP KIVKWDRDM (SEQ ID NO:310). In some cases, a β2M polypeptide comprises the amino acid sequence: IQRTPKIQVY SCHPAENGKS NFLNCYVSGF HPSDIEVDLLKNGERIEKVE HSDLSFSKDW SFYLLYYTEF TPTEKDEYAC RVNHVTLSQP KIVKWDRDM (SEQ ID NO:311).

In some cases, an HLA Class I heavy chain polypeptide comprises the amino acid sequence:





 (SEQ ID NO:297).

In some cases, an HLA Class I heavy chain polypeptide comprises the amino acid sequence:

GSHSMRYFFTSVSRPGRGEPRFIAVGYVDDTQFVRFDSDAASQRMEPRAPWIEQEGPEYWDGET
RKVKAHSQTHRVDLGTLRGYYNQSEAGSHTVQRMYGCDVGSDWRFLRGYHQYAYDGKDYIA
LKEDLRSWTAADMAAQTTKHKWEAAHVAEQLRAYLEGTCVEWLRRYLENGKETLQRTDAPK

THHMTHHAVSDHEATLRCWALSFYPAEITLTWQRDGEDQTQDTELVETRPCGDGTFQKWAAVV
VPSGQEQRYTCHVQHEGLPKPLTLRWEP (SEQ ID NO:312).

In some cases, an HLA Class I heavy chain polypeptide comprises the amino acid sequence:

GSHSMRYFFTSVSRPGRGEPRFIAVGYVDDTQFVRFDSDAASQRMEPRAPWIEQEGPEYWDGET
RKVKAHSQTHRVDLGTLRGAYNQSEAGSHTVQRMYGCDVGSDWRFLRGYHQYAYDGKDYIA
LKEDLRSWTAADMAAQTTKHKWEAAHVAEQLRAYLEGTCVEWLRRYLENGKETLQRTDAPK
THMTHHAVSDHEATLRCWALSFYPAEITLTWQRDGEDQTQDTELVETRPCGDGTFQKWAAVV
VPSGQEQRYTCHVQHEGLPKPLTLRWE (SEQ ID NO:313).

In some cases, the β2M polypeptide comprises the following amino acid sequence: 
IQRTPKIQVY SCHPAENGKS NFLNCYVSGF HPSDIEVDLLKNGERIEKVE
HSDLSFSKDW SFYLLYYTEF TPTEKDEYAC RVNHVTLSQP KIVKWDRDM (SEQ ID NO:311);
 and the HLA Class I heavy chain polypeptide of a TMMP of the present disclosure comprises the following amino acid sequence:
GSHSMRYFFTSVSRPGRGEPRFIAVGYVDDTQFVRFDSDAASQRMEPRAPWIEQE GPEYWDGETRKVKAHSQTHRVDLGTLRGYYNQSEAGSHTVQRMYGCDVGSDWRFLRGYHQY AYDGKDYIALKEDLRSWTAADMAAQTTKHKWEAAHVAEQLRAYLEGTCVEWLRRYLENGKE TLQRTDAPKTHMTHHAVSDHEATLRCWALSFYPAEITLTWQRDGEDQTQDTELVETRP**C**GDGT FQKWAAVVVPSGQEQRYTCHVQHEGLPKPLTLRWEP (SEQ ID NO:312), where the Cys residues that are underlined and in bold form a disulfide bond with one another in the TMMP.

In some cases, the β2M polypeptide comprises the amino acid sequence:

IQRTPKIQVYSCHPAENGKSNFLNCYVSGFHPSDIEVDLLKNGERIEKVEHSDLSFSKDWSFYLL
YYTEFTPTEKDEYACRVNHVTLSQPKIVKWDRDM (SEQ ID NO:314).

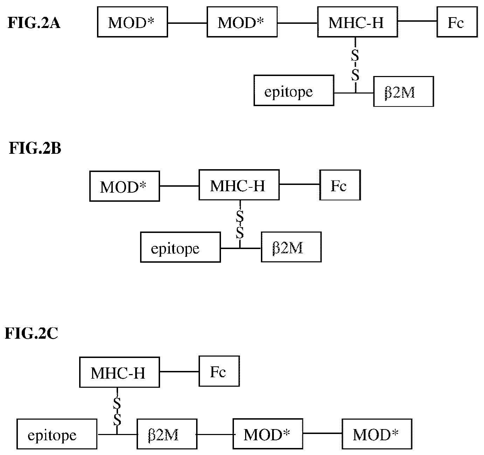

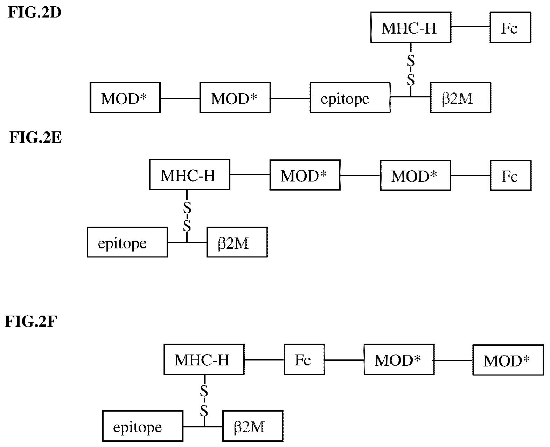

In some cases, the first polypeptide and the second polypeptide of a TMMP of the present disclosure are disulfide linked to one another through: i) a Cys residue present in a linker connecting the peptide epitope and a β2M polypeptide in the first polypeptide chain; and ii) a Cys residue present in an MHC Class I heavy chain in the second polypeptide chain. In some cases, the Cys residue present in the MHC Class I heavy chain is a Cys introduce as a Y84C substitution. In some cases, the linker connecting the peptide epitope and the β2M polypeptide in the first polypeptide chain is GCGGS(G4S)n (SEQ ID NO:315), where n is 1, 2, 3, 4, 5, 6, 7, 8, or 9. For example, in some cases, the linker comprises the amino acid sequence GCGGSGGGGSGGGGSGGGGS (SEQ ID NO:316). As another example, the linker comprises the amino acid sequence GCGGSGGGGSGGGGS (SEQ ID NO:317). Examples of disulfide-linked first and second polypeptides of a TMMP of the present disclosure are depicted schematically in FIG. 2A-2F.

### Multiple disulfide bonded TMMPs

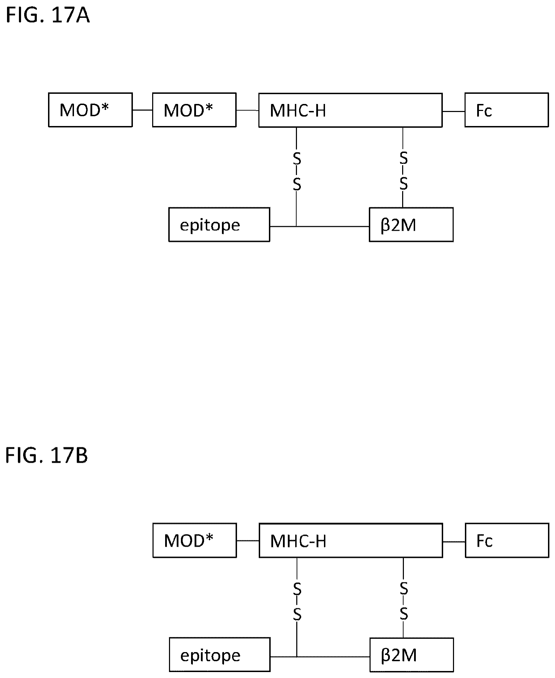

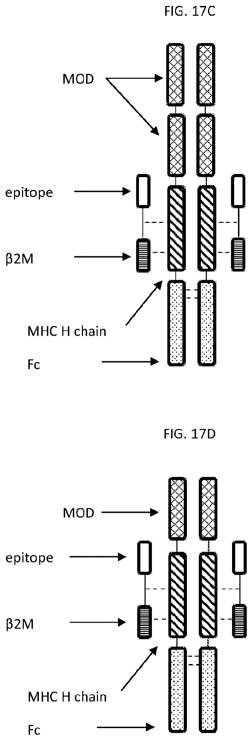

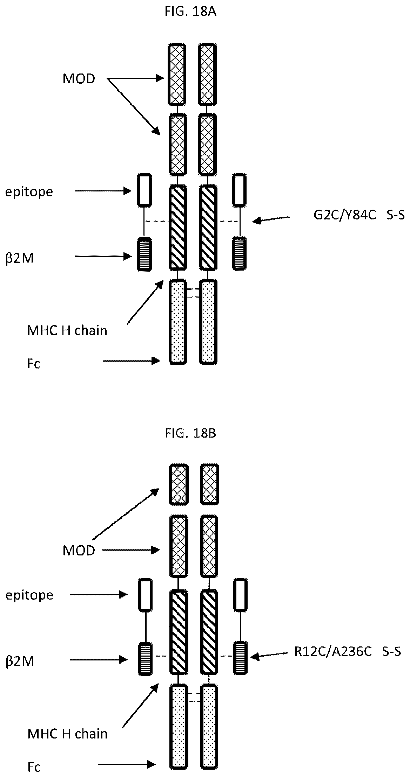

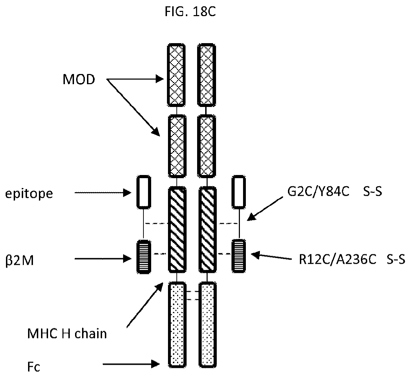

In some cases, the first polypeptide and the second polypeptide of a TMMP of the present disclosure are linked to one another by at least two disulfide bonds (i.e., two interchain disulfide bonds). Examples of such multiple disulfide-linked TMMP are depicted schematically in FIG. 17A and 17B and FIG. 18A-18C. In addition, where a TMMP of the present disclosure comprises an IgFc polypeptide, a heterodimeric TMMP can be dimerized, such that disulfide bonds link the IgFc polypeptides in the two heterodimeric TMMPs. Such an arrangement is depicted schematically in FIG. 17C and 17D, where disulfide bonds are represented by dashed lines. Unless otherwise stated, the at least two disulfide bonds described in the multiple disulfide-linked TMMPPs in this section are not referring to disulfide bonds linking IgFc polypeptides in dimerized TMMPs.

As noted above, in some cases, the first polypeptide and the second polypeptide of a TMMP of the present disclosure are linked to one another by at least two disulfide bonds (i.e., two interchain disulfide bonds). For example, in some instances, the first polypeptide and the second polypeptide of a TMMP of the present disclosure are linked to one another by 2 interchain disulfide bonds. As another example, in some instances, the first polypeptide and the second polypeptide of a TMMP of the present disclosure are linked to one another by 3 interchain disulfide bonds. As another example, in some instances, the first polypeptide and the second polypeptide of a TMMP of the present disclosure are linked to one another by 4 interchain disulfide bonds.

In some cases where a peptide epitope in a first polypeptide of a TMMP of the present disclosure is linked to a β2M polypeptide by a linker comprising a Cys, at least one of the at least two disulfide bonds links a Cys in the linker to a Cys in an MHC Class I heavy chain in the second polypeptide. In some cases, where a peptide epitope in a first polypeptide of a TMMP of the present disclosure is linked to an MHC Class I heavy chain polypeptide by a linker, at least one of the at least two disulfide bonds links a Cys in the linker to a Cys in a β2M polypeptide present in the second polypeptide.

In some cases, a multiple disulfide-linked TMMP of the present disclosure (e.g., a double disulfide-linked TMMP) exhibits increased stability, compared to a control TMMP that includes only one of the at least two disulfide bonds. In some cases, a multiple disulfide-linked TMMP (e.g., a double disulfide-linked TMMP) of the present disclosure exhibits increased *in vitro* stability, compared to a control TMMP that includes only one of the at least two disulfide bonds. For example, in some cases, a multiple disulfide-linked TMMP of the present disclosure (e.g., a double disulfide-linked TMMP) exhibits at least 5%, at least 10%, at least 15%, at least 20%, at least 25%, at least 50%, at least 2-fold, at least 5-fold, or at least 10-fold, greater *in vitro* stability, compared to a control TMMP that includes only one of the at least two disulfide bonds.

Whether a multiple disulfide-linked TMMP of the present disclosure (e.g., a double disulfide-linked TMMP) exhibits increased *in vitro* stability, compared to a control TMMP that includes only one of the at least two disulfide bonds, can be determined by measuring the amount disulfide-linked heterodimeric TMMP present in a sample over time and/or under a specified condition and/or during purification of the TMMP.

For example, in some cases, a multiple disulfide-linked TMMP (e.g., a double disulfide-linked TMMP) of the present disclosure exhibits at least 5%, at least 10%, at least 15%, at least 20%, at least 25%, at least 50%, at least 2-fold, at least 5-fold, or at least 10-fold, greater *in vitro* stability, compared to a control TMMP that includes only one of the at least two disulfide bonds, when the TMMP is stored at 37°C for a period of time (e.g., for a period of time of from about 1 week to about 2 weeks, from about 2 weeks to about 4 weeks, or from about 4 weeks to about 2 months). For example, in some cases, the amount of disulfide-linked heterodimeric TMMP remaining after storing a multiple disulfide-linked TMMP (e.g., a double disulfide-linked TMMP) of the present disclosure *in vitro* at 37°C for 28 days is at least at least 5%, at least 10%, at least 15%, at least 20%, at least 25%, at least 50%, at least 2-fold, at least 5-fold, or at least 10-fold, greater than the amount of disulfide-linked heterodimeric TMMP remaining after storing a control TMMP (a TMMP that includes only one of the at least two disulfide bonds present in the multiple disulfide-linked TMMP) *in vitro* at 37°C for 28 days.

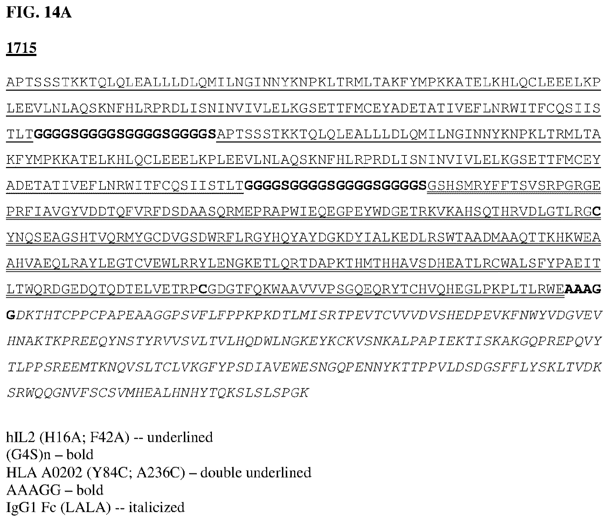

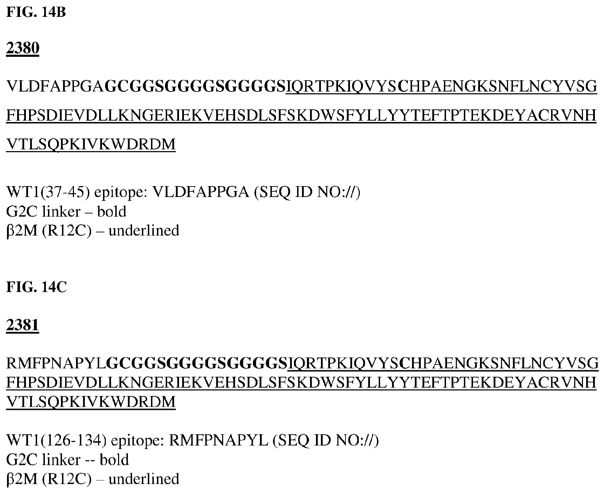

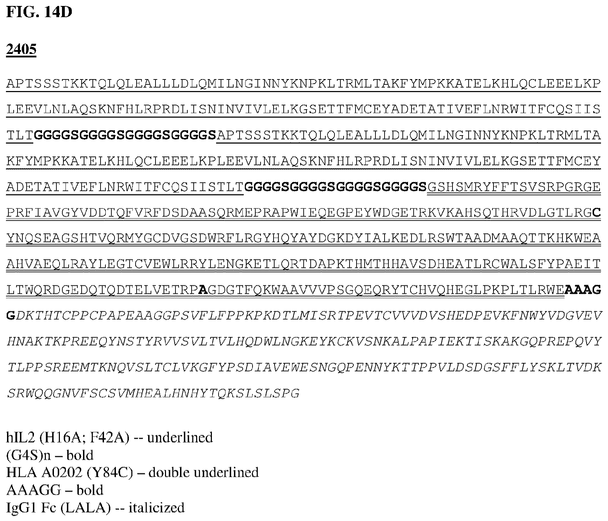

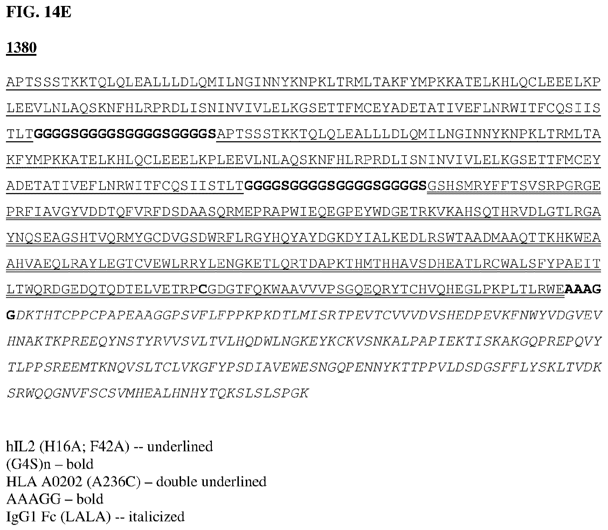

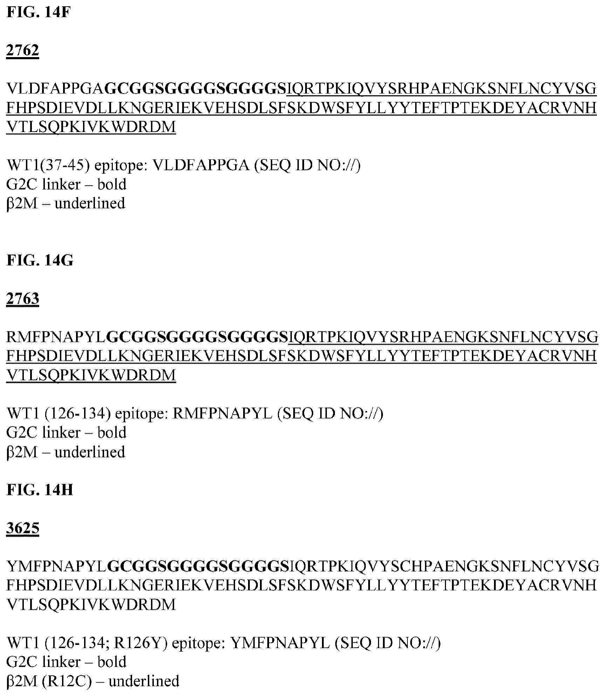

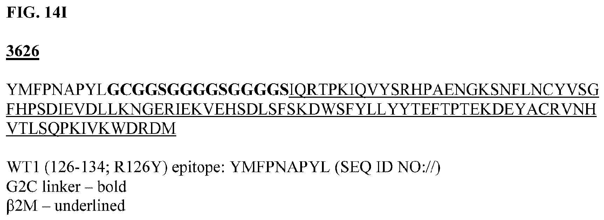

As an example, a double disulfide-linked TMMP comprising polypeptides 1715 and 2380, as depicted in FIG. 14A and 14B, exhibits greater *in vitro* stability, compared to a TMMP comprising polypeptides 2405 and 2380, where polypeptide 2405 is depicted in FIG. 14D, where such TMMP comprises only one disulfide linkage, where the single disulfide linkage is formed between: i) the Cys of the G2C linker between the epitope and the β2M; and ii) the Cys provided by a Y84C substitution in the MHC Class 1 heavy chain. As another example, a double disulfide-linked TMMP comprising polypeptides 1715 and 2380, as depicted in FIG. 14A and 14B, exhibits greater in *vitro* stability, compared to a TMMP comprising polypeptides 1380 and 2380, where polypeptide 1380 is depicted in FIG. 14E, where such TMMP comprises only one disulfide linkage, where the single disulfide linkage is formed between: i) the Cys provided by an R12C substitution in the β2M polypeptide; and ii) the Cys provided by the A236C substitution in the MHC Class I heavy chain.

In some cases, a multiple disulfide-linked TMMP of the present disclosure exhibits increased *in* vivo stability, compared to a control TMMP that includes only one of the at least two disulfide bonds. For example, in some cases, a multiple disulfide-linked TMMP of the present disclosure exhibits at least 5%, at least 10%, at least 15%, at least 20%, at least 25%, at least 50%, at least 2-fold, at least 5-fold, or at least 10-fold, greater *in vivo* stability, compared to a control TMMP that includes only one of the at least two disulfide bonds.

In some cases, the presence of two disulfide bonds in a multiple disulfide-linked TMMP of the present disclosure (e.g., a double disulfide-linked TMMP) provides for increased production of disulfide-linked heterodimeric TMMP, compared to the amount of disulfide-linked heterodimeric TMMP produced when the TMMP is a control TMMP that includes only one of the at least two disulfide bonds. For example, a multiple disulfide-linked TMMP of the present disclosure (e.g., a double disulfide-linked TMMP) can be produced in a mammalian cell in *in vitro* cell culture, where the mammalian cell is cultured in a liquid cell culture medium. The TMMP can be secreted into the cell culture medium. The cells can be lysed, generating a cell lysate, and the TMMP can be present in the cell lysate. The TMMP can be purified from the cell culture medium and/or the cell lysate. For example, where the TMMP comprises an IgG1 Fc polypeptide, the cell culture medium and/or the cell lysate can be contacted with immobilized protein A (e.g., the cell culture medium and/or the cell lysate can be applied to a protein A column, where protein A is immobilized onto beads). TMMP present in the cell culture medium and/or the cell lysate becomes bound to the immobilized protein A. After washing the column to remove unbound materials, the bound TMMP is eluted, generating a protein A eluate. The amount of disulfide-linked heterodimeric TMMP present in the protein A eluate is a least 0.5%, at least 1%, at least 2%, at least 3%, at least 4%, at least 5%, at least 6%, at least 7%, at least 8%, at least 9%, or at least 10%, higher than the amount of disulfide-linked heterodimeric TMMP present in the protein A eluate when the TMMP is a control TMMP that includes only one of the at least two disulfide bonds present in the multiple disulfide-linked TMMP (e.g., a double disulfide-linked TMMP). In some cases, the percent of the total TMMP protein in the eluate that is non-aggregated disulfide-linked heterodimeric TMMP is at least 70%, at least 75%, at least 80%, at least 85%, at least 90%, at least 95%, or at least 99%. The protein A eluate can be subjected to size exclusion chromatography (SEC) and/or one or more other additional purification steps.

In some cases, a T-cell modulatory multimeric polypeptide of the present disclosure comprises at least one heterodimer comprising: a) a first polypeptide comprising: i) a WT1 peptide epitope, where the WT1 peptide has a length of at least 4 amino acids (e.g., from 4 amino acids to 25 amino acids; e.g., the WT1 peptide has a length of 4, 5, 6, 7, 8, 9, 10-15, 15-20, or 20-25 amino acids); and ii) first MHC polypeptide; b) a second polypeptide comprising a second MHC polypeptide, and c) at least one immunomodulatory polypeptide, where the first and/or the second polypeptide comprises the immunomodulatory polypeptide, and where the heterodimer comprises 2 disulfide bonds between the first polypeptide and the second polypeptide (i.e., the heterodimer comprises: i) a first disulfide bond linking the first polypeptide and the second polypeptide; and ii) a second disulfide bond linking the first polypeptide and the second polypeptide). Expressed another way, the first polypeptide comprises a first Cys residue that forms a disulfide bond (a first disulfide bond) with a first Cys residue in the second polypeptide; and the first polypeptide comprises a second Cys residue that forms a disulfide bond (a second disulfide bond) with a second Cys residue in the second polypeptide.

In some cases, a TMMP of the present disclosure comprises: a) a first polypeptide comprising, in order from N-terminus to C-terminus: i) a peptide epitope; ii) a peptide linker; and iii) a β2M polypeptide; and b) a second polypeptide comprising an MHC Class I heavy chain polypeptide, where one or both of the first and the second polypeptides comprises at least one immunomodulatory polypeptide, where the TMMP comprises: a) a first disulfide linkage between: i) a Cys present in the linker between the peptide epitope and the β2M polypeptide; and ii) a first Cys introduced into the MHC Class I heavy chain polypeptide; and b) at least a second disulfide linkage between the first polypeptide and the second polypeptide, where the at least a second disulfide linkage is between: i) a Cys in the first polypeptide that is C-terminal to the Cys present in the linker; and ii) a Cys in the second polypeptide that is C-terminal to the first Cys introduced into the MHC Class I heavy chain polypeptide.

In some cases, a first and a second disulfide bond-forming Cys residues in a first or a second polypeptide of a TMMP of the present disclosure are from about 10 amino acids to about 200 amino acids apart from one another. For example, in some cases, a first and a second disulfide bond-forming Cys residues in a first or a second polypeptide of a TMMP are from about 10 amino acids (aa) to about 15 aa, from about 15 aa to about 20 aa, from about 20 aa to about 25 aa, from about 25 aa to about 30 aa, from about 30 aa to about 40 aa, from about 40 aa to about 50 aa, from about 50 aa to about 60 aa, from about 60 aa to about 70 aa, from about 70 aa to about 80 aa, from about 80 aa to about 90 aa, from about 90 aa to about 100 aa, from about 100 aa to about 110 aa, from about 110 aa to about 120 aa, from about 120 aa to about 130 aa, from about 130 aa to about 140 aa, from about 140 aa to about 150 aa, from about 150 aa to about 160 aa, from about 160 aa to about 170 aa, from about 170 aa to about 180 aa, from about 180 aa to about 190 aa, or from about 190 aa to about 200 aa.

As an example, in some cases, the first and second disulfide bond-forming Cys residues in the first polypeptide of a TMMP of the present disclosure are from about 10 amino acids to about 80 amino acid residues apart from one another. For example, in some cases, the second disulfide bond-forming Cys residue in the first polypeptide is from about 10 amino acids to about 80 amino acids (e.g., from about 10 amino acids (aa) to about 15 aa, from about 15 aa to about 20 aa, from about 20 aa to about 25 aa, from about 25 aa to about 30 aa, from about 30 aa to about 40 aa, from about 40 aa to about 50 aa, from about 50 aa to about 60 aa, from about 60 aa to about 70 aa, or from about 70 aa to about 80 aa) C-terminal to the first disulfide bond-forming Cys residue in the first polypeptide. In some cases, the second disulfide bond-forming Cys residue in the first polypeptide is 10 aa, 11 aa, 12 aa, 13 aa, 14 aa, 15 aa, 16 aa, 17 aa, 18 aa, 19 aa, 20 aa, 21 aa, 22 aa, 23 aa, 24 aa, or 25 aa, C-terminal to the first disulfide bond-forming Cys residue in the first polypeptide. In some cases, the second disulfide bond-forming Cys residue in the first polypeptide is 15 aa C-terminal to the first disulfide bond-forming Cys residue in the first polypeptide. In some cases, the second disulfide bond-forming Cys residue in the first polypeptide is 20 aa C-terminal to the first disulfide bond-forming Cys residue in the first polypeptide. In some cases, the second disulfide bond-forming Cys residue in the first polypeptide is 25 aa C-terminal to the first disulfide bond-forming Cys residue in the first polypeptide.

In some cases, the first and second disulfide bond-forming Cys residues in the second polypeptide of a TMMP of the present disclosure are from about 140 amino acids to about 160 amino acids apart from one another. For example, in some cases, the second disulfide bond-forming Cys residue in the second polypeptide is from about 140 amino acids to about 160 amino acids C-terminal to the first disulfide bond-forming Cys residue in the second polypeptide. In some cases, the second disulfide bond-forming Cys residue in the second polypeptide is 140 amino acids (aa), 141 aa, 142 aa, 143 aa, 144 aa, 145 aa, 146 aa, 147 aa, 148 aa, 149 aa, 150 aa, 151 aa, 152 aa, 153 aa, 154 aa, 155 aa, 156 aa, 157 aa, 158 aa, 159 aa, or 160 aa, C-terminal to the first disulfide bond-forming Cys residue in the second polypeptide.

A multiple disulfide-linked TMMP of the present disclosure (e.g., a double disulfide-linked TMMP) can comprise: a) a first polypeptide comprising: i) a WT1 peptide (e.g., a WT1 peptide of from 4 amino acids to about 25 amino acids); and ii) a first MHC polypeptide, where the first polypeptide comprises a peptide linker between the WT1 peptide and the first MHC polypeptide, where the peptide linker comprises a Cys residue, and where the first MHC polypeptide is a β2M polypeptide that comprises an amino acid substitution that introduces a Cys residue; b) and a second polypeptide comprising a second MHC polypeptide, where the second MHC polypeptide is a Class I heavy chain comprising a Y84C substitution and an A236C substitution, based on the amino acid numbering of HLA-A*0201 (depicted in FIG. 9A), or at corresponding positions in another Class I heavy chain allele, where the TMMP comprises a disulfide bond between the Cys residue in the peptide linker and the Cys residue at amino acid position 84 of the Class I heavy chain or corresponding position of another Class I heavy chain allele, and where the TMMP comprises a disulfide bond between the introduced Cys residue in the β2M polypeptide and the Cys at amino acid position 236 of the Class I heavy chain or corresponding position of another Class I heavy chain allele; and c) at least one immunomodulatory polypeptide, where the first and/or the second polypeptide comprises the at least one immunomodulatory polypeptide. Examples are depicted schematically in FIG. 17A and FIG. 17B.

In some cases, the peptide linker comprises the amino acid sequence GCGGS (SEQ ID NO:318). In some cases, the peptide linker comprises the amino acid sequence GCGGS(GGGGS)n (SEQ ID NO:319), where n is an integer from 1 to 10. In some cases, the peptide linker comprises the amino acid sequence GCGGS(GGGGS)n (SEQ ID NO:398), where n is 1. In some cases, the peptide linker comprises the amino acid sequence GCGGS(GGGGS)n (SEQ ID NO:320), where n is 2. In some cases, the peptide linker comprises the amino acid sequence GCGGS(GGGGS)n (SEQ ID NO:321), where n is 3. In some cases, the peptide linker comprises the amino acid sequence GCGGS(GGGGS)n (SEQ ID NO:322), where n is 4. In some cases, the peptide linker comprises the amino acid sequence GCGGS(GGGGS)n (SEQ ID NO:323), where n is 5. In some cases, the peptide linker comprises the amino acid sequence GCGGS(GGGGS)n (SEQ IDNO:324), where n is 6. In some cases, the peptide linker comprises the amino acid sequence GCGGS(GGGGS)n (SEQ ID NO:325), where n is 7. In some cases, the peptide linker comprises the amino acid sequence GCGGS(GGGGS)n (SEQ ID NO:326), where n is 8. In some cases, the peptide linker comprises the amino acid sequence GCGGS(GGGGS)n (SEQ ID NO: 327), where n is 9. In some cases, the peptide linker comprises the amino acid sequence GCGGS(GGGGS)n (SEQ ID NO:328), where n is 10.

In some cases, the peptide linker comprises the amino acid sequence CGGGS (SEQ ID NO:329). In some cases, the peptide linker comprises the amino acid sequence CGGGS(GGGGS)n (SEQ ID NO: 330), where n is an integer from 1 to 10. In some cases, the peptide linker comprises the amino acid sequence CGGGS(GGGGS)n (SEQ ID NO:331), where n is 1. In some cases, the peptide linker comprises the amino acid sequence CGGGS(GGGGS)n (SEQ ID NO:332), where n is 2. In some cases, the peptide linker comprises the amino acid sequence CGGGS(GGGGS)n (SEQ ID NO:333), where n is 3. In some cases, the peptide linker comprises the amino acid sequence CGGGS(GGGGS)n (SEQ ID NO:334), where n is 4. In some cases, the peptide linker comprises the amino acid sequence CGGGS(GGGGS)n (SEQ ID NO:335), where n is 5. In some cases, the peptide linker comprises the amino acid sequence CGGGS(GGGGS)n (SEQ ID NO:336), where n is 6. In some cases, the peptide linker comprises the amino acid sequence CGGGS(GGGGS)n (SEQ ID NO:337), where n is 7. In some cases, the peptide linker comprises the amino acid sequence CGGGS(GGGGS)n (SEQ ID NO:338), where n is 8. In some cases, the peptide linker comprises the amino acid sequence CGGGS(GGGGS)n (SEQ ID NO:339), where n is 9. In some cases, the peptide linker comprises the amino acid sequence CGGGS(GGGGS)n (SEQ ID NO:340), where n is 10.

The following are non-limiting examples of MHC Class I heavy chain comprising a Y84C substitution and an A236C substitution, based on the amino acid numbering of HLA-A*0201 (depicted in FIG. 9A), or at corresponding positions in another Class I heavy chain allele.

### HLA-A

In some cases, a multiple disulfide-linked TMMP of the present disclosure (e.g., a double disulfide-linked TMMP) comprises: a) a first polypeptide comprising: i) a WT1 peptide (e.g., a WT1 peptide of from 4 amino acids to about 25 amino acids); and ii) a first MHC polypeptide, where the first polypeptide comprises a peptide linker between the WT1 peptide and the first MHC polypeptide, where the peptide linker comprises a Cys residue, and where the first MHC polypeptide is a β2M polypeptide that comprises an amino acid substitution that introduces a Cys residue; and b) a second polypeptide comprising an HLA-A MHC Class I heavy chain comprising an amino acid sequence having at least 60%, at least 70%, at least 80%, at least 90%, at least 95%, at least 98%, at least 99%, or 100%, amino acid sequence identity to the following amino acid sequence:
GSHSMRYFFTSVSRPGRGEPRFIAVGYVDDTQFVRFDSDAASQRMEPRAPWIEQEGPEYWDGET RKVKAHSQTHRVDLGTLRGCYNQSEAGSHTVQRMYGCDVGSDWRFLRGYHQYAYDGKDYIA LKEDLRSWTAADMAAQTTKHKWEAAHVAEQLRAYLEGTCVEWLRRYLENGKETLQRTDAPK THMTHHAVSDHEATLRCWALSFYPAEITLTWQRDGEDQTQDTELVETRPCGDGTFQKWAAVV VPSGQEQRYTCHVQHEGLPKPLTLRWEP (SEQ ID NO:341), where amino acid 84 is a Cys and amino acid 236 is a Cys; and c) at least one immunomodulatory polypeptide, where the first and/or the second polypeptide comprises the at least one immunomodulatory polypeptide. In some cases, the peptide linker comprises the amino acid sequence GCGGS (SEQ ID NO:318). In some cases, the peptide linker comprises the amino acid sequence GCGGS(GGGGS)n (SEQ ID NO:319), where n is an integer from 1 to 10. In some cases, the β2M polypeptide comprises an R12C substitution. For example, the β2M polypeptide can comprises an amino acid sequence having at least 90%, at least 95%, at least 98%, at least 99%, or 100%, amino acid sequence identity to the following amino acid sequence: IQRTPKIQVYSCHPAENGKSNFLNCYVSGFHPSDIEVDLLKNGERIEKVEHSDLSFSKDWSFYLL YYTEFTPTEKDEYACRVNHVTLSQPKIVKWDRDM (SEQ ID NO:314), where amino acid 12 is a Cys. The at least one immunomodulatory polypeptide can be a polypeptide that exerts an activating/stimulating effect on the target T cell or a suppressing/inhibitory effect on the target T cell. For example, the at least one immunomodulatory polypeptide can be a cytokine (e.g., an IL2 polypeptide, an IL7 polypeptide, an IL12 polypeptide, an IL15 polypeptide, an IL17 polypeptide, an IL21 polypeptide, an IL27 polypeptide, an IL-23 polypeptide, a TGFβ polypeptide, and the like; and including all family members, e.g., IL17A, IL-17B, IL-17C, IL-17D, IL-17E, IL-17F, IL-17E), a 4-1BBL polypeptide, an ICOS-L polypeptide, an OX-40L polypeptide, a CD80 polypeptide, a CD86 polypeptide, (CD80 and CD86 are also known as B7-1 and B7-2, respectively), a CD40 polypeptide, a CD70 polypeptide, a JAG1 (CD339) polypeptide, an ICAM (CD540 polypeptide, a PD-L1 polypeptide, a FasL polypeptide, a PD-L2 polypeptide, a PD-1H (VISTA) polypeptide, an ICOS-L (CD275) polypeptide, a GITRL polypeptide, an HVEM polypeptide, a CXCL10 polypeptide, a CXCL9 polypeptide, a CXCL11 polypeptide, a CXCL13 polypeptide, and a CX3CL1 polypeptide, a Galectin-9 polypeptide, a CD83 polypeptide, a CD30L polypeptide, a HLA-G polypeptide, a MICA polypeptide, a MICB polypeptide, a HVEM (CD270) polypeptide, a lymphotoxin beta receptor polypeptide, a 3/TR6 polypeptide, an ILT3 polypeptide, an ILT4 polypeptide, a CXCL10 polypeptide, a CXCL9 polypeptide, a CXCL11 polypeptide, a CXCL13 polypeptide, or a CX3CL1 polypeptide. These immunomodulatory polypeptides may be the wild type polypeptide or a variant of a wild type polypeptide. In some cases, the immunomodulatory polypeptide is an activating ("stimulatory") immunomodulatory polypeptide; e.g., the immunomodulatory polypeptide may produce an activating/stimulating effect on a T cell. Examples of activating immunomodulatory polypeptides include, e.g., CD80, CD86, 4-1BBL, OX40L, CD70, ICOS-L, CD40, ICAM (CD54), IL2, IL7, IL12, IL15, IL17, IL21, IL27, IL23, GITRL, TGFβ, and lymphotoxin beta receptor. In some cases, the immunomodulatory polypeptide is an inhibitory ("suppressing") immunomodulatory polypeptide; e.g., the immunomodulatory polypeptide s may produce a suppressing/inhibitory effect on a T cell. Examples of inhibitory immunomodulatory polypeptides include, e.g., PD-1H, PD-L1, PD-L2, TGFβ, FasL, HVEM, Galectin-9, ILT3, and ILT4. TGFβ polypeptides may produce either an activating/stimulating effect or a suppressing/inhibitory effect, depending on the context.

In some cases, the at least one immunomodulatory polypeptide is a reduced affinity variant, as described elsewhere herein. In some cases, the first or the second polypeptide comprises an Ig Fc polypeptide.

In some cases, a multiple disulfide-linked TMMP of the present disclosure (e.g., a double disulfide-linked TMMP) comprises an HLA-A Class I heavy chain polypeptide. In some cases, the HLA-A heavy chain polypeptide present in a multiple disulfide-linked TMMP of the present disclosure (e.g., a double disulfide-linked TMMP) comprises an amino acid sequence having at least 95%, at least 98%, or at least 99%, amino acid sequence identity to the HLA-A*0101, HLA-A*0201, HLA-A*0202, HLA-A*1101, HLA-A*2301, HLA-A*2402, HLA-A*2407, HLA-A*3303, or HLA-A*3401 amino acid sequence depicted in FIG. 9A, where the HLA-A heavy chain polypeptide comprises Y84C and A236C substitutions.

### HLA-A*0101 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*0101 (Y84C; A236C) amino acid sequence:
GSHSMRYFFTSVSRPGRGEPRFIAVGYVDDTQFVRFDSDAASQKMEPRAPWIEQE GPEYWDQETRNMKAHSQTDRANLGTLRGCYNQSEDGSHTIQIMYGCDVGPDGRFLRGYRQDA YDGKDYIALNEDLRSWTAADMAAQITKRKWEAVHAAEQRRVYLEGRCVDGLRRYLENGKET LQRTDPPKTHMTHHPISDHEATLRCWALGFYPAEITLTWQRDGEDQTQDTELVETRPCGDGTF QKWAAVVVPSGEEQRYTCHVQHEGLPKPLTLRWE (SEQ ID NO:343), where amino acid 84 is a Cys and amino acid 236 is a Cys.

### HLA-A*0201 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*0201 (Y84C; A236C) amino acid sequence:
GSHSMRYFFTSVSRPGRGEPRFIAVGYVDDTQFVRFDSDAASQRMEPRAPWIEQEGPEYWDGET RKVKAHSQTHRVDLGTLRGCYNQSEAGSHTVQRMYGCDVGSDWRFLRGYHQYAYDGKDYIA LKEDLRSWTAADMAAQTTKHKWEAAHVAEQLRAYLEGTCVEWLRRYLENGKETLQRTDAPK THMTHHAVSDHEATLRCWALSFYPAEITLTWQRDGEDQTQDTELVETRPCGDGTFQKWAAVV VPSGQEQRYTCHVQHEGLPKPLTLRWEP (SEQ ID NO:341), where amino acid 84 is a Cys and amino acid 236 is a Cys.

### HLA-A*0202 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*0202 (Y84C; A236C) amino acid sequence:
GSHSMRYFFTSVSRPGRGEPRFIAVGYVDDTQFVRFDSDAASQRMEPRAPWIEQEGPEYWDGET RKVKAHSQTHRVDLGTLRGCYNQSEAGSHTVQRMYGCDVGSDWRFLRGYHQYAYDGKDYIA LKEDLRSWTAADMAAQTTKHKWEAAHVAEQLRAYLEGTCVEWLRRYLENGKETLQRTDAPK THMTHHAVSDHEATLRCWALSFYPAEITLTWQRDGEDQTQDTELVETRPCGDGTFQKWAAVV VPSGQEQRYTCHVQHEGLPKPLTLRWE (SEQ ID NO:341), where amino acid 84 is a Cys and amino acid 236 is a Cys.

### HLA-A*1101 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*1101 (Y84C; A236C) amino acid sequence:
GSHSMRYFYTSVSRPGRGEPRFIAVGYVDDTQFVRFDSDAASQRMEPRAPWIEQEGPEYWDQE TRNVKAQSQTDRVDLGTLRGCYNQSEDGSHTIQIMYGCDVGPDGRFLRGYRQDAYDGKDYIA LNEDLRSWTAADMAAQITKRKWEAAHAAEQQRAYLEGRCVEWLRRYLENGKETLQRTDPPK THMTHHPISDHEATLRCWALGFYPAEITLTWQRDGEDQTQDTELVETRPCGDGTFQKWAAVV VPSGEEQRYTCHVQHEGLPKPLTLRWE (SEQ ID NO:344), where amino acid 84 is a Cys and amino acid 236 is a Cys.

### HLA-A*2301 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*2301 (Y84C; A236C) amino acid sequence:
GSHSMRYFSTSVSRPGRGEPRFIAVGYVDDTQFVRFDSDAASQRMEPRAPWIEQEGPEYWDEET GKVKAHSQTDRENLRIALRCYNQSEAGSHTLQMMFGCDVGSDGRFLRGYHQYAYDGKDYIAL KEDLRSWTAADMAAQITQRKWEAARVAEQLRAYLEGTCVDGLRRYLENGKETLQRTDPPKTH MTHHPISDHEATLRCWALGFYPAEITLTWQRDGEDQTQDTELVETRPCGDGTFQKWAAVVVPS GEEQRYTCHVQHEGLPKPLTLRWE (SEQ ID NO:345), where amino acid 84 is a Cys and amino acid 236 is a Cys.

### HLA-A*2402 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*2402 (Y84C; A236C) amino acid sequence:
GSHSMRYFSTSVSRPGRGEPRFIAVGYVDDTQFVRFDSDAASQRMEPRAPWIEQEGPEYWDEET GKVKAHSQTDRENLRIALRCYNQSEAGSHTLQMMFGCDVGSDGRFLRGYHQYAYDGKDYIAL KEDLRSWTAADMAAQITKRKWEAAHVAEQQRAYLEGTCVDGLRRYLENGKETLQRTDPPKT HMTHHPISDHEATLRCWALGFYPAEITLTWQRDGEDQTQDTELVETRPCGDGTFQKWAAVVV PSGEEQRYTCHVQHEGLPKPLTLRWE (SEQ ID NO:346), where amino acid 84 is a Cys and amino acid 236 is a Cys.

### HLA-A*2407 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*2407 (Y84C; A236C) amino acid sequence:
GSHSMRYFSTSVSRPGRGEPRFIAVGYVDDTQFVRFDSDAASQRMEPRAPWIEQEGPEYWDEET GKVKAQSQTDRENLRIALRCYNQSEAGSHTLQMMFGCDVGSDGRFLRGYHQYAYDGKDYIAL KEDLRSWTAADMAAQITKRKWEAAHVAEQQRAYLEGTCVDGLRRYLENGKETLQRTDPPKT HMTHHPISDHEATLRCWALGFYPAEITLTWQRDGEDQTQDTELVETRPCGDGTFQKWAAVVV PSGEEQRYTCHVQHEGLPKPLTLRWE (SEQ ID NO:347), where amino acid 84 is a Cys and amino acid 236 is a Cys.

### HLA-A*3303 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*3303 (Y84C; A236C) amino acid sequence:
GSHSMRYFTTSVSRPGRGEPRFIAVGYVDDTQFVRFDSDAASQRMEPRAPWIEQE GPEYWDRNTRNVKAHSQIDRVDLGTLRGCYNQSEAGSHTIQMMYGCDVGSDGRFLRGYQQD AYDGKDYIALNEDLRSWTAADMAAQITQRKWEAARVAEQLRAYLEGTCVEWLRRYLENGKE TLQRTDPPKTHMTHHAVSDHEATLRCWALSFYPAEITLTWQRDGEDQTQDTELVETRPCGDGT FQKWASVVVPSGQEQRYTCHVQHEGLPKPLTLRWE (SEQ ID NO:348), where amino acid 84 is a Cys and amino acid 236 is a Cys.

### HLA-A*3401 (Y84C; A236C)

In some cases, the HLA-A heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-A*3401 (Y84C; A236C) amino acid sequence:
GSHSMRYFYTSVSRPGRGEPRFIAVGYVDDTQFVRFDSDAASQRMEPRAPWIEQEGPEYWDRN TRKVKAQSQTDRVDLGTLRGCYNQSEDGSHTIQRMYGCDVGPDGRFLRGYQQDAYDGKDYIA LNEDLRSWTAADMAAQITQRKWETAHEAEQWRAYLEGTCVEWLRRYLENGKETLQRTDAPK THMTHHAVSDHEATLRCWALSFYPAEITLTWQRDGEDQTQDTELVETRPCGDGTFQKWASVV VPSGQEQRYTCHVQHEGLPKPLTLRWE (SEQ ID NO:349), where amino acid 84 is a Cys and amino acid 236 is a Cys.

### HLA-B

In some cases, a multiple disulfide-linked TMMP of the present disclosure (e.g., a double disulfide-linked TMMP) comprises: a) a first polypeptide comprising: i) a WT1 peptide (e.g., a WT1 peptide of from 4 amino acids to about 25 amino acids); and ii) a first MHC polypeptide, where the first polypeptide comprises a peptide linker between the WT1 peptide and the first MHC polypeptide, where the peptide linker comprises a Cys residue, and where the first MHC polypeptide is a β2M polypeptide that comprises an amino acid substitution that introduces a Cys residue; and b) a second polypeptide comprising an HLA-B MHC Class I heavy chain comprising an amino acid sequence having at least 60%, at least 70%, at least 80%, at least 90%, at least 95%, at least 98%, at least 99%, or 100%, amino acid sequence identity to the following amino acid sequence:
GSHSMRYFYTSVSRPGRGEPRFISVGYVDDTQFVRFDSDAASPREEPRAPWIEQEGPEYWDRNT QIYKAQAQTDRESLRNLRGCYNQSEAGSHTLQSMYGCDVGPDGRLLRGHDQYAYDGKDYIAL NEDLRSWTAADTAAQITQRKWEAAREAEQRRAYLEGECVEWLRRYLENGKDKLERADPPKTH VTHHPISDHEATLRCWALGFYPAEITLTWQRDGEDQTQDTELVETRPCGDRTFQKWAAVVVPS GEEQRYTCHVQHEGLPKPLTLRWEP (SEQ ID NO:350), where amino acid 84 is a Cys and amino acid 236 is a Cys; and c) at least one immunomodulatory polypeptide, where the first and/or the second polypeptide comprises the at least one immunomodulatory polypeptide. In some cases, the peptide linker comprises the amino acid sequence GCGGS (SEQ ID NO:318). In some cases, the peptide linker comprises the amino acid sequence GCGGS(GGGGS)n (SEQ ID NO:342), where n is an integer from 1 to 10. In some cases, the β2M polypeptide comprises an R12C substitution. For example, the β2M polypeptide can comprises an amino acid sequence having at least 90%, at least 95%, at least 98%, at least 99%, or 100%, amino acid sequence identity to the following amino acid sequence: IQRTPKIQVYSCHPAENGKSNFLNCYVSGFHPSDIEVDLLKNGERIEKVEHSDLSFSKDWSFYLL YYTEFTPTEKDEYACRVNHVTLSQPKIVKWDRDM (SEQ ID NO:314), where amino acid 12 is a Cys. The at least one immunomodulatory polypeptide can be a polypeptide that exerts an activating/stimulating effect on the target T cell or a suppressing/inhibitory effect on the target T cell. For example, the at least one immunomodulatory polypeptide can be a cytokine (e.g., an IL2 polypeptide, an IL7 polypeptide, an IL12 polypeptide, an IL15 polypeptide, an IL17 polypeptide, an IL21 polypeptide, an IL27 polypeptide, an IL-23 polypeptide, a TGFβ polypeptide, and the like; and including all family members, e.g., IL17A, IL-17B, IL-17C, IL-17D, IL-17E, IL-17F, IL-17E), a 4-1BBL polypeptide, an ICOS-L polypeptide, an OX-40L polypeptide, a CD80 polypeptide, a CD86 polypeptide, (CD80 and CD86 are also known as B7-1 and B7-2, respectively), a CD40 polypeptide, a CD70 polypeptide, a JAG1 (CD339) polypeptide, an ICAM (CD540 polypeptide, a PD-L1 polypeptide, a FasL polypeptide, a PD-L2 polypeptide, a PD-1H (VISTA) polypeptide, an ICOS-L (CD275) polypeptide, a GITRL polypeptide, an HVEM polypeptide, a CXCL10 polypeptide, a CXCL9 polypeptide, a CXCL11 polypeptide, a CXCL13 polypeptide, and a CX3CL1 polypeptide, a Galectin-9 polypeptide, a CD83 polypeptide, a CD30L polypeptide, a HLA-G polypeptide, a MICA polypeptide, a MICB polypeptide, a HVEM (CD270) polypeptide, a lymphotoxin beta receptor polypeptide, a 3/TR6 polypeptide, an ILT3 polypeptide, an ILT4 polypeptide, a CXCL10 polypeptide, a CXCL9 polypeptide, a CXCL11 polypeptide, a CXCL13 polypeptide, or a CX3CL1 polypeptide. These immunomodulatory polypeptides may be the wild type polypeptide or a variant of a wild type polypeptide. In some cases, the immunomodulatory polypeptide is an activating ("stimulatory") immunomodulatory polypeptide; e.g., the immunomodulatory polypeptide may produce an activating/stimulating effect on a T cell. Examples of activating immunomodulatory polypeptides include, e.g., CD80, CD86, 4-1BBL, OX40L, CD70, ICOS-L, CD40, ICAM (CD54), IL2, IL7, IL12, IL15, IL17, IL21, IL27, IL23, GITRL, TGFβ, and lymphotoxin beta receptor. In some cases, the immunomodulatory polypeptide is an inhibitory ("suppressing") immunomodulatory polypeptide; e.g., the immunomodulatory polypeptide s may produce a suppressing/inhibitory effect on a T cell. Examples of inhibitory immunomodulatory polypeptides include, e.g., PD-1H, PD-L1, PD-L2, TGFB, FasL, HVEM, Galectin-9, ILT3, and ILT4. TGFβ polypeptides may produce either an activating/stimulating effect or a suppressing/inhibitory effect, depending on the context.

. In some cases, the at least one immunomodulatory polypeptide is a reduced affinity variant, as described elsewhere herein. In some cases, the first or the second polypeptide comprises an Ig Fc polypeptide.

In some cases, a multiple disulfide-linked TMMP of the present disclosure (e.g., a double disulfide-linked TMMP) comprises an HLA-B Class I heavy chain polypeptide. In some cases, the HLA-B heavy chain polypeptide present in a multiple disulfide-linked TMMP of the present disclosure (e.g., a double disulfide-linked TMMP) comprises an amino acid sequence having at least 95%, at least 98%, or at least 99%, amino acid sequence identity to the HLA-B*0702, HLA-B*0801, HLA-B*1502, HLA-B*3802, HLA-B*4001, HLA-B*4601, or HLA-B*5301 amino acid sequence depicted in FIG. 10A, where the HLA-B heavy chain polypeptide comprises Y84C and A236C substitutions.

### HLA-B*0702 (Y84C; A236C)

In some cases, the HLA-B heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-B*0702 (Y84C; A236C) amino acid sequence:
GSHSMRYFYTSVSRPGRGEPRFISVGYVDDTQFVRFDSDAASPREEPRAPWIEQE GPEYWDRNTQIYKAQAQTDRESLRNLRGCYNQSEAGSHTLQSMYGCDVGPDGRLLRGHDQYA YDGKDYIALNEDLRSWTAADTAAQITQRKWEAAREAEQRRAYLEGECVEWLRRYLENGKDKL ERADPPKTHVTHHPISDHEATLRCWALGFYPAEITLTWQRDGEDQTQDTELVETRPCGDRTFQK WAAVVVPSGEEQRYTCHVQHEGLPKPLTLRWE (SEQ ID NO:350), where amino acid 84 is a Cys and amino acid 236 is a Cys.

### HLA-B*0801 (Y84C; A236C)

In some cases, the HLA-B heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-B*0801 (Y84C; A236C) amino acid sequence:
GSHSMRYFDTAMSRPGRGEPRFISVGYVDDTQFVRFDSDAASPREEPRAPWIEQE GPEYWDRNTQIFKTNTQTDRESLRNLRGCYNQSEAGSHTLQSMYGCDVGPDGRLLRGHNQYA YDGKDYIALNEDLRSWTAADTAAQITQRKWEAARVAEQDRAYLEGTCVEWLRRYLENGKDTL ERADPPKTHVTHHPISDHEATLRCWALGFYPAEITLTWQRDGEDQTQDTELVETRPCGDRTFQK WAAVVVPSGEEQRYTCHVQHEGLPKPLTLRWE (SEQ ID NO:351), where amino acid 84 is a Cys and amino acid 236 is a Cys.

### HLA-B*1502 (Y84C; A236C)

In some cases, the HLA-B heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-B*1502 (Y84C; A236C) amino acid sequence:
GSHSMRYFYTAMSRPGRGEPRFIAVGYVDDTQFVRFDSDAASPRMAPRAPWIEQ EGPEYWDRNTQISKTNTQTYRESLRNLRGCYNQSEAGSHIIQRMYGCDVGPDGRLLRGYDQSA YDGKDYIALNEDLSSWTAADTAAQITQRKWEAAREAEQLRAYLEGLCVEWLRRYLENGKETL QRADPPKTHVTHHPISDHEATLRCWALGFYPAEITLTWQRDGEDQTQDTELVETRPCGDRTFQ KWAAVVVPSGEEQRYTCHVQHEGLPKPLTLRWE (SEQ ID NO:352), where amino acid 84 is a Cys and amino acid 236 is a Cys.

### HLA-B*3802 (Y84C; A236C)

In some cases, the HLA-B heavy chain polypeptide present in a multiple disulfide-linked TMMP of the present disclosure (*e.g*., a double disulfide-linked TMMP) comprises an amino acid sequence having at least 95%, at least 98%, at least 99%, or 100%, amino acid sequence identity to the following HLA-B*3802 (Y84C; A236C) amino acid sequence:
GSHSMRYFYTSVSRPGRGEPRFISVGYVDDTQFVRFDSDAASPREEPRAPWIEQE GPEYWDRNTQICKTNTQTYRENLRTALRCYNQSEAGSHTLQRMYGCDVGPDGRLLRGHNQFA YDGKDYIALNEDLSSWTAADTAAQITQRKWEAARVAEQLRTYLEGTCVEWLRRYLENGKETL QRADPPKTHVTHHPISDHEATLRCWALGFYPAEITLTWQRDGEDQTQDTELVETRPCGDRTFQ KWAAVVVPSGEEQRYTCHVQHEGLPKPLTLRWE (SEQ ID NO:353), where amino acid 84 is a Cys and amino acid 236 is a Cys.

### HLA-B*4001 (Y84C; A2346C)

In some cases, the HLA-B heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-B*4001 (Y84C; A236C) amino acid sequence:
GSHSMRYFHTAMSRPGRGEPRFITVGYVDDTLFVRFDSDATSPRKEPRAPWIEQE GPEYWDRETQISKTNTQTYRESLRNLRGCYNQSEAGSHTLQRMYGCDVGPDGRLLRGHNQYA YDGKDYIALNEDLRSWTAADTAAQISQRKLEAARVAEQLRAYLEGECVEWLRRYLENGKDKL ERADPPKTHVTHHPISDHEATLRCWALGFYPAEITLTWQRDGEDQTQDTELVETRPCGDRTFQK WAAVVVPSGEEQRYTCHVQHEGLPKPLTLRWE (SEQ ID NO:354) where amino acid 84 is a Cys and amino acid 236 is a Cys.

### HLA-B*4601 (Y84C; A236C)

In some cases, the HLA-B heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-B*4601 (Y84C; A236C) amino acid sequence:
GSHSMRYFYTAMSRPGRGEPRFIAVGYVDDTQFVRFDSDAASPRMAPRAPWIEQ EGPEYWDRETQKYKRQAQTDRVSLRNLRGCYNQSEAGSHTLQRMYGCDVGPDGRLLRGHDQ SAYDGKDYIALNEDLSSWTAADTAAQITQRKWEAAREAEQWRAYLEGLCVEWLRRYLENGKE TLQRADPPKTHVTHHPISDHEATLRCWALGFYPAEITLTWQRDGEDQTQDTELVETRPCGDRTF QKWAAVVVPSGEEQRYTCHVQHEGLPKPLTLRWE (SEQ ID NO:355) where amino acid 84 is a Cys and amino acid 236 is a Cys.

### HLA-B*5301 (Y84C; A236C)

In some cases, the HLA-B heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-B*5301 (Y84C; A236C) amino acid sequence:
GSHSMRYFYTAMSRPGRGEPRFIAVGYVDDTQFVRFDSDAASPRTEPRAPWIEQE GPEYWDRNTQIFKTNTQTYRENLRIALRCYNQSEAGSHIIQRMYGCDLGPDGRLLRGHDQSAY DGKDYIALNEDLSSWTAADTAAQITQRKWEAARVAEQLRAYLEGLCVEWLRRYLENGKETLQ RADPPKTHVTHHPVSDHEATLRCWALGFYPAEITLTWQRDGEDQTQDTELVETRPCGDRTFQK WAAVVVPSGEEQRYTCHVQHEGLPKPLTLRWE (SEQ ID NO:356) where amino acid 84 is a Cys and amino acid 236 is a Cys.

### HLA-C

In some cases, a multiple disulfide-linked TMMP of the present disclosure (e.g., a double disulfide-linked TMMP) comprises: a) a first polypeptide comprising: i) a WT-1 peptide (e.g., 1 WT-1 peptide of from 4 amino acids to about 25 amino acids); and ii) a first MHC polypeptide, where the first polypeptide comprises a peptide linker between the WT-1 peptide and the first MHC polypeptide, where the peptide linker comprises a Cys residue, and where the first MHC polypeptide is a β2M polypeptide that comprises an amino acid substitution that introduces a Cys residue; and b) a second polypeptide comprising an HLA-C MHC Class I heavy chain comprising an amino acid sequence having at least 60%, at least 70%, at least 80%, at least 90%, at least 95%, at least 98%, at least 99%, or 100%, amino acid sequence identity to the following amino acid sequence:
CSHSMRYFDTAVSRPGRGEPRFISVGYVDDTQFVRFDSDAASPRGEPRAPWVEQEGPEYWDRE TQNYKRQAQADRVSLRNLRGCYNQSEDGSHTLQRMYGCDLGPDGRLLRGYDQSAYDGKDYI ALNEDLRSWTAADTAAQITQRKLEAARAAEQLRAYLEGTCVEWLRRYLENGKETLQRAEPPKT HVTHHPLSDHEATLRCWALGFYPAEITLTWQRDGEDQTQDTELVETRPCGDGTFQKWAAVVV PSGQEQRYTCHMQHEGLQEPLTLSWEP (SEQ ID NO:357), where amino acid 84 is a Cys and amino acid 236 is a Cys; and c) at least one immunomodulatory polypeptide, where the first and/or the second polypeptide comprises the at least one immunomodulatory polypeptide. In some cases, the peptide linker comprises the amino acid sequence GCGGS (SEQ ID NO:318). In some cases, the peptide linker comprises the amino acid sequence GCGGS(GGGGS)n (SEQ ID NO:342), where n is an integer from 1 to 10. In some cases, the β2M polypeptide comprises an R12C substitution. For example, the β2M polypeptide can comprises an amino acid sequence having at least 90%, at least 95%, at least 98%, at least 99%, or 100%, amino acid sequence identity to the following amino acid sequence: IQRTPKIQVYSCHPAENGKSNFLNCYVSGFHPSDIEVDLLKNGERIEKVEHSDLSFSKDWSFYLL YYTEFTPTEKDEYACRVNHVTLSQPKIVKWDRDM (SEQ ID NO:314), where amino acid 12 is a Cys. The at least one immunomodulatory polypeptide can be a polypeptide that exerts an activating/stimulating effect on the target T cell or a suppressing/inhibitory effect on the target T cell. For example, the at least one immunomodulatory polypeptide can be a cytokine (e.g., an IL2 polypeptide, an IL7 polypeptide, an IL12 polypeptide, an IL15 polypeptide, an IL17 polypeptide, an IL21 polypeptide, an IL27 polypeptide, an IL-23 polypeptide, a TGFβ polypeptide, and the like; and including all family members, e.g., IL17A, IL-17B, IL-17C, IL-17D, IL-17E, IL-17F, IL-17E), a 4-1BBL polypeptide, an ICOS-L polypeptide, an OX-40L polypeptide, a CD80 polypeptide, a CD86 polypeptide, (CD80 and CD86 are also known as B7-1 and B7-2, respectively), a CD40 polypeptide, a CD70 polypeptide, a JAG1 (CD339) polypeptide, an ICAM (CD540 polypeptide, a PD-L1 polypeptide, a FasL polypeptide, a PD-L2 polypeptide, a PD-1H (VISTA) polypeptide, an ICOS-L (CD275) polypeptide, a GITRL polypeptide, an HVEM polypeptide, a CXCL10 polypeptide, a CXCL9 polypeptide, a CXCL11 polypeptide, a CXCL13 polypeptide, and a CX3CL1 polypeptide, a Galectin-9 polypeptide, a CD83 polypeptide, a CD30L polypeptide, a HLA-G polypeptide, a MICA polypeptide, a MICB polypeptide, a HVEM (CD270) polypeptide, a lymphotoxin beta receptor polypeptide, a 3/TR6 polypeptide, an ILT3 polypeptide, an ILT4 polypeptide, a CXCL10 polypeptide, a CXCL9 polypeptide, a CXCL11 polypeptide, a CXCL13 polypeptide, or a CX3CL1 polypeptide. These immunomodulatory polypeptides may be the wild type polypeptide or a variant of a wild type polypeptide. In some cases, the immunomodulatory polypeptide is an activating ("stimulatory") immunomodulatory polypeptide; e.g., the immunomodulatory polypeptide may produce an activating/stimulating effect on a T cell. Examples of activating immunomodulatory polypeptides include, e.g., CD80, CD86, 4-1BBL, OX40L, CD70, ICOS-L, CD40, ICAM (CD54), IL2, IL7, IL12, IL15, IL17, IL21, IL27, IL23, GITRL, TGFβ, and lymphotoxin beta receptor. In some cases, the immunomodulatory polypeptide is an inhibitory ("suppressing") immunomodulatory polypeptide; e.g., the immunomodulatory polypeptide s may produce a suppressing/inhibitory effect on a T cell. Examples of inhibitory immunomodulatory polypeptides include, e.g., PD-1H, PD-L1, PD-L2, TGFB, FasL, HVEM, Galectin-9, ILT3, and ILT4. TGFβ polypeptides may produce either an activating/stimulating effect or a suppressing/inhibitory effect, depending on the context.

. In some cases, the at least one immunomodulatory polypeptide is a reduced affinity variant, as described elsewhere herein. In some cases, the first or the second polypeptide comprises an Ig Fc polypeptide.

In some cases, a multiple disulfide-linked TMMP of the present disclosure (e.g., a double disulfide-linked TMMP) comprises an HLA-C Class I heavy chain polypeptide. In some cases, the HLA-C heavy chain polypeptide present in a multiple disulfide-linked TMMP of the present disclosure (e.g., a double disulfide-linked TMMP) comprises an amino acid sequence having at least 95%, at least 98%, or at least 99%, amino acid sequence identity to the HLA-C*0102, HLA-C*0303, HLA-C*0304, HLA-C*0401, HLA-C*0602, HLA-C*0701, HLA-C*0702, HLA-C*0801, or HLA-C*1502 amino acid sequence depicted in FIG. 11A, where the HLA-C heavy chain polypeptide comprises Y84C and A236C substitutions.

### HLA-C*01:02 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*01:02 (Y84C; A236C) amino acid sequence:
CSHSMKYFFTSVSRPGRGEPRFISVGYVDDTQFVRFDSDAASPRGEPRAPWVEQE GPEYWDRETQKYKRQAQTDRVSLRNLRGCYNQSEAGSHTLQWMCGCDLGPDGRLLRGYDQY AYDGKDYIALNEDLRSWTAADTAAQITQRKWEAAREAEQRRAYLEGTCVEWLRRYLENGKET LQRAEHPKTHVTHHPVSDHEATLRCWALGFYPAEITLTWQWDGEDQTQDTELVETRPCGDGTF QKWAAVMVPSGEEQRYTCHVQHEGLPEPLTLRWEP (SEQ ID NO:358), where amino acid 84 is a Cys and amino acid 236 is a Cys.

### HLA-C*0303 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*03:03 (Y84C; A236C) amino acid sequence:
GSHSMRYFYTAVSRPGRGEPHFIAVGYVDDTQFVRFDSDAASPRGEPRAPWVEQ EGPEYWDRETQKYKRQAQTDRVSLRNLRGCYNQSEARSHIIQRMYGCDVGPDGRLLRGYDQY AYDGKDYIALNEDLRSWTAADTAAQITQRKWEAAREAEQLRAYLEGLCVEWLRRYLKNGKET LQRAEHPKTHVTHHPVSDHEATLRCWALGFYPAEITLTWQWDGEDQTQDTELVETRPCGDGTF QKWAAVVVPSGEEQRYTCHVQHEGLPEPLTLRWEP (SEQ ID NO:359), where amino acid 84 is a Cys and amino acid 236 is a Cys.

### HLA-C*0304 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*03:04 (Y84C; A236C) amino acid sequence:
GSHSMRYFYTAVSRPGRGEPHFIAVGYVDDTQFVRFDSDAASPRGEPRAPWVEQ EGPEYWDRETQKYKRQAQTDRVSLRNLRGCYNQSEAGSHIIQRMYGCDVGPDGRLLRGYDQY AYDGKDYIALNEDLRSWTAADTAAQITQRKWEAAREAEQLRAYLEGLCVEWLRRYLKNGKET LQRAEHPKTHVTHHPVSDHEATLRCWALGFYPAEITLTWQWDGEDQTQDTELVETRPCGDGTF QKWAAVVVPSGEEQRYTCHVQHEGLPEPLTLRWEP (SEQ ID NO:360), where amino acid 84 is a Cys and amino acid 236 is a Cys.

### HLA-C*0401 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*04:01 (Y84C; A236C) amino acid sequence:
GSHSMRYFSTSVSWPGRGEPRFIAVGYVDDTQFVRFDSDAASPRGEPREPWVEQ EGPEYWDRETQKYKRQAQADRVNLRKLRGCYNQSEDGSHTLQRMFGCDLGPDGRLLRGYNQ FAYDGKDYIALNEDLRSWTAADTAAQITQRKWEAAREAEQRRAYLEGTCVEWLRRYLENGKE TLQRAEHPKTHVTHHPVSDHEATLRCWALGFYPAEITLTWQWDGEDQTQDTELVETRPCGDGT FQKWAAVVVPSGEEQRYTCHVQHEGLPEPLTLRWKP (SEQ ID NO:361), where amino acid 84 is a Cys and amino acid 236 is a Cys.

### HLA-C*0602 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*06:02 (Y84C; A236C) amino acid sequence:
CSHSMRYFDTAVSRPGRGEPRFISVGYVDDTQFVRFDSDAASPRGEPRAPWVEQ EGPEYWDRETQKYKRQAQADRVNLRKLRGCYNQSEDGSHTLQWMYGCDLGPDGRLLRGYD QSAYDGKDYIALNEDLRSWTAADTAAQITQRKWEAAREAEQWRAYLEGTCVEWLRRYLENG KETLQRAEHPKTHVTHHPVSDHEATLRCWALGFYPAEITLTWQRDGEDQTQDTELVETRPCGD GTFQKWAAVVVPSGEEQRYTCHVQHEGLPEPLTLRWEP (SEQ ID NO:362), where amino acid 84 is a Cys and amino acid 236 is a Cys.

### HLA-C*0701 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*07:01 (Y84C; A236C) amino acid sequence:
CSHSMRYFDTAVSRPGRGEPRFISVGYVDDTQFVRFDSDAASPRGEPRAPWVEQ EGPEYWDRETQNYKRQAQADRVSLRNLRGCYNQSEDGSHTLQRMYGCDLGPDGRLLRGYDQ SAYDGKDYIALNEDLRSWTAADTAAQITQRKLEAARAAEQLRAYLEGTCVEWLRRYLENGKE TLQRAEPPKTHVTHHPLSDHEATLRCWALGFYPAEITLTWQRDGEDQTQDTELVETRPCGDGT FQKWAAVVVPSGQEQRYTCHMQHEGLQEPLTLSWEP (SEQ ID NO:357), where amino acid 84 is a Cys and amino acid 236 is a Cys.

### HLA-C*0702 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*07:02 (Y84C; A236C) amino acid sequence:
CSHSMRYFDTAVSRPGRGEPRFISVGYVDDTQFVRFDSDAASPRGEPRAPWVEQ EGPEYWDRETQKYKRQAQADRVSLRNLRGCYNQSEDGSHTLQRMSGCDLGPDGRLLRGYDQS AYDGKDYIALNEDLRSWTAADTAAQITQRKLEAARAAEQLRAYLEGTCVEWLRRYLENGKET LQRAEPPKTHVTHHPLSDHEATLRCWALGFYPAEITLTWQRDGEDQTQDTELVETRPCGDGTF QKWAAVVVPSGQEQRYTCHMQHEGLQEPLTLSWEP (SEQ ID NO:404), where amino acid 84 is a Cys and amino acid 236 is a Cys.

### HLA-C*0801 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*08:01 (Y84C; A236C) amino acid sequence:
CSHSMRYFYTAVSRPGRGEPRFIAVGYVDDTQFVQFDSDAASPRGEPRAPWVEQ EGPEYWDRETQKYKRQAQTDRVSLRNLRGCYNQSEAGSHTLQRMYGCDLGPDGRLLRGYNQ FAYDGKDYIALNEDLRSWTAADTAAQITQRKWEAARTAEQLRAYLEGTCVEWLRRYLENGKK TLQRAEHPKTHVTHHPVSDHEATLRCWALGFYPAEITLTWQRDGEDQTQDTELVETRPCGDGT FQKWAAVVVPSGEEQRYTCHVQHEGLPEPLTLRWGP (SEQ ID NO:363), where amino acid 84 is a Cys and amino acid 236 is a Cys.

### HLA-C*1502 (Y84C; A236C)

In some cases, the HLA-C heavy chain polypeptide present in a multiple disulfide-linked TMMP of the present disclosure (e.g., a double disulfide-linked TMMP) comprises an amino acid sequence having at least 95%, at least 98%, at least 99%, or 100%, amino acid sequence identity to the following HLA-C*15:02 (Y84C; A236C) amino acid sequence:
CSHSMRYFYTAVSRPGRGEPHFIAVGYVDDTQFVRFDSDAASPRGEPRAPWVEQ EGPEYWDRETQNYKRQAQTDRVNLRKLRGCYNQSEAGSHIIQRMYGCDLGPDGRLLRGHDQL AYDGKDYIALNEDLRSWTAADTAAQITQRKWEAAREAEQLRAYLEGTCVEWLRRYLENGKET LQRAEHPKTHVTHHPVSDHEATLRCWALGFYPAEITLTWQRDGEDQTQDTELVETRPCGDGTF QKWAAVVVPSGEEQRYTCHVQHEGLPEPLTLRWEP (SEQ ID NO:364), where amino acid 84 is a Cys and amino acid 236 is a Cys.

### Scaffold polypeptides

A TMMP can comprise an Fc polypeptide, or can comprise another suitable scaffold polypeptide.

Suitable scaffold polypeptides include antibody-based scaffold polypeptides and non-antibody-based scaffolds. Non-antibody-based scaffolds include, e.g., albumin, an XTEN (extended recombinant) polypeptide, transferrin, an Fc receptor polypeptide, an elastin-like polypeptide (see, e.g., Hassouneh et al. (2012) Methods Enzymol. 502:215; e.g., a polypeptide comprising a pentapeptide repeat unit of (Val-Pro-Gly-X-Gly; SEQ ID NO:59), where X is any amino acid other than proline), an albumin-binding polypeptide, a silk-like polypeptide (see, e.g., Valluzzi et al. (2002) Philos Trans R Soc Lond B Biol Sci. 357:165), a silk-elastin-like polypeptide (SELP; see, e.g., Megeed et al. (2002) Adv Drug Deliv Rev. 54:1075), and the like. Suitable XTEN polypeptides include, e.g., those disclosed in WO 2009/023270, WO 2010/091122, WO 2007/103515, US 2010/0189682, and US 2009/0092582; see also Schellenberger et al. (2009) Nat Biotechnol. 27:1186). Suitable albumin polypeptides include, e.g., human serum albumin.

Suitable scaffold polypeptides will in some cases be a half-life extending polypeptides. Thus, in some cases, a suitable scaffold polypeptide increases the *in vivo* half-life (e.g., the serum half-life) of the TMMP, compared to a control TMMP lacking the scaffold polypeptide. For example, in some cases, a scaffold polypeptide increases the *in vivo* half-life (e.g., the serum half-life) of the TMMP, compared to a control TMMP lacking the scaffold polypeptide, by at least about 10%, at least about 15%, at least about 20%, at least about 25%, at least about 50%, at least about 2-fold, at least about 2.5-fold, at least about 5-fold, at least about 10-fold, at least about 25-fold, at least about 50-fold, at least about 100-fold, or more than 100-fold. As an example, in some cases, an Fc polypeptide increases the *in vivo* half-life (e.g., the serum half-life) of the TMMP, compared to a control TMMP lacking the Fc polypeptide, by at least about 10%, at least about 15%, at least about 20%, at least about 25%, at least about 50%, at least about 2-fold, at least about 2.5-fold, at least about 5-fold, at least about 10-fold, at least about 25-fold, at least about 50-fold, at least about 100-fold, or more than 100-fold.

### Fc polypeptides

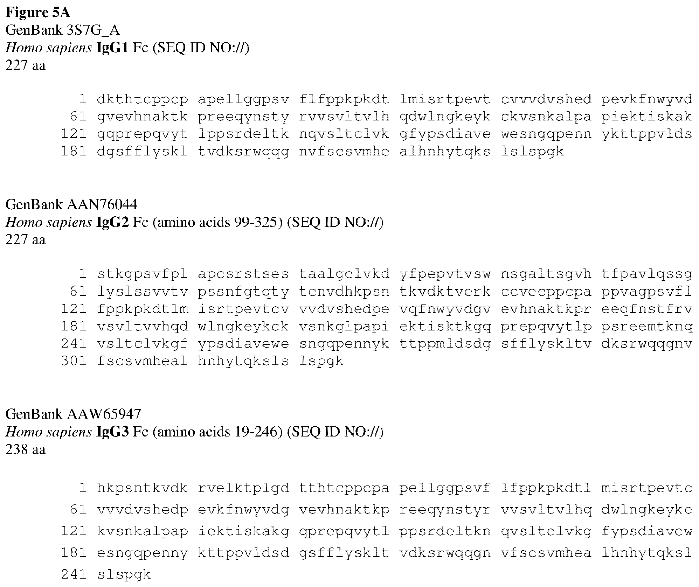

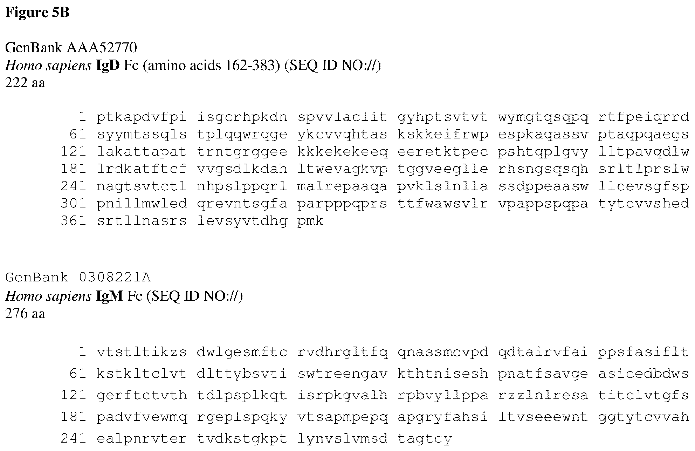

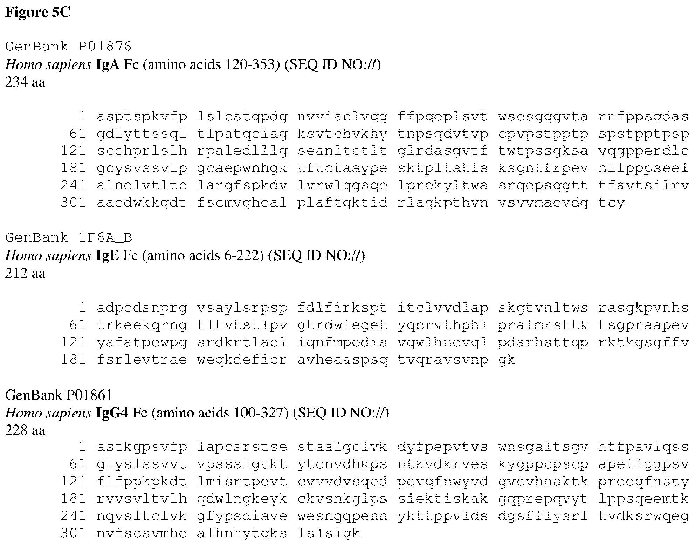

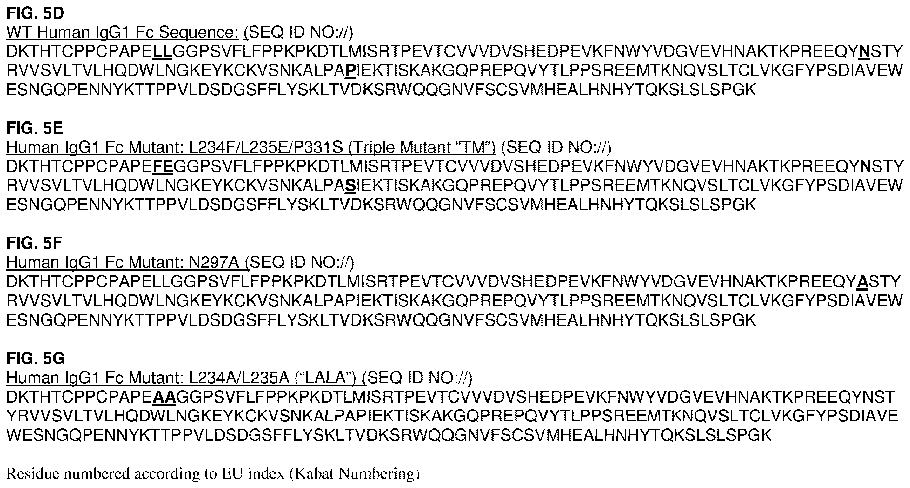

In some cases, the first and/or the second polypeptide chain of a TMMP of the present disclosure comprises an Fc polypeptide. The Fc polypeptide of a TMMP of the present disclosure can be a human IgG1 Fc, a human IgG2 Fc, a human IgG3 Fc, a human IgG4 Fc, etc. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to an amino acid sequence of an Fc region depicted in FIG. 5A-5G. In some cases, the Fc region comprises an amino acid sequence having at least about 70%, at least about 75%, at least about 80%, at least about 85%, at least about 90%, at least about 95%, at least about 98%, at least about 99%, or 100%, amino acid sequence identity to the human IgG1 Fc polypeptide depicted in FIG. 5A. In some cases, the Fc region comprises an amino acid sequence having at least about 70%, at least about 75%, at least about 80%, at least about 85%, at least about 90%, at least about 95%, at least about 98%, at least about 99%, or 100%, amino acid sequence identity to the human IgG1 Fc polypeptide depicted in FIG. 5A; and comprises a substitution of N77; e.g., the Fc polypeptide comprises an N77A substitution. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgG2 Fc polypeptide depicted in FIG. 5A; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 99-325 of the human IgG2 Fc polypeptide depicted in FIG. 5A. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgG3 Fc polypeptide depicted in FIG. 5A; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 19-246 of the human IgG3 Fc polypeptide depicted in FIG. 5A. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgM Fc polypeptide depicted in FIG. 5B; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 1-276 to the human IgM Fc polypeptide depicted in FIG. 5B. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgA Fc polypeptide depicted in FIG. 5C; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 1-234 to the human IgA Fc polypeptide depicted in FIG. 5C.

In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgG4 Fc polypeptide depicted in FIG. 5C. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 100 to 327 of the human IgG4 Fc polypeptide depicted in FIG. 5C.

In some cases, the IgG4 Fc polypeptide comprises the following amino acid sequence:

PPCPSCPAPEFLGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRLTVDKS
RWQEGNVFSCSVMHEALHNHYTQKSLSLSPG (SEQ ID NO:365).

In some cases, the Fc polypeptide present in a TMMP comprises the amino acid sequence depicted in FIG. 5A (human IgG1 Fc). In some cases, the Fc polypeptide present in a TMMP comprises the amino acid sequence depicted in FIG. 5A (human IgG1 Fc), except for a substitution of N297 (N77 of the amino acid sequence depicted in FIG. 5A) with an amino acid other than asparagine. In some cases, the Fc polypeptide present in a TMMP comprises the amino acid sequence depicted in FIG. 5C (human IgG1 Fc comprising an N297A substitution, which is N77 of the amino acid sequence depicted in FIG. 5A). In some cases, the Fc polypeptide present in a TMMP comprises the amino acid sequence depicted in FIG. 5A (human IgG1 Fc), except for a substitution of L234 (L14 of the amino acid sequence depicted in FIG. 5A) with an amino acid other than leucine. In some cases, the Fc polypeptide present in a TMMP comprises the amino acid sequence depicted in FIG. 5A (human IgG1 Fc), except for a substitution of L235 (L15 of the amino acid sequence depicted in FIG. 5A) with an amino acid other than leucine.

In some cases, the Fc polypeptide present in a TMMP comprises the amino acid sequence depicted in FIG. 5E. In some cases, the Fc polypeptide present in a TMMP comprises the amino acid sequence depicted in FIG. 5F. In some cases, the Fc polypeptide present in a TMMP comprises the amino acid sequence depicted in FIG. 5G (human IgG1 Fc comprising an L234A substitution and an L235A substitution, corresponding to positions 14 and 15 of the amino acid sequence depicted in FIG. 5G). In some cases, the Fc polypeptide present in a TMMP comprises the amino acid sequence depicted in FIG. 5A (human IgG1 Fc), except for a substitution of P331 (P111 of the amino acid sequence depicted in FIG. 5A) with an amino acid other than proline; in some cases, the substitution is a P331S substitution. In some cases, the Fc polypeptide present in a TMMP comprises the amino acid sequence depicted in FIG. 5A (human IgG1 Fc), except for substitutions at L234 and L235 (L14 and L15 of the amino acid sequence depicted in FIG. 5A) with amino acids other than leucine. In some cases, the Fc polypeptide present in a TMMP comprises the amino acid sequence depicted in FIG. 5A (human IgG1 Fc), except for substitutions at L234 and L235 (L14 and L15 of the amino acid sequence depicted in FIG. 5A) with amino acids other than leucine, and a substitution of P331 (P111 of the amino acid sequence depicted in FIG. 5A) with an amino acid other than proline. In some cases, the Fc polypeptide present in a TMMP comprises the amino acid sequence depicted in FIG. 5E (human IgG1 Fc comprising L234F, L235E, and P331S substitutions (corresponding to amino acid positions 14, 15, and 111 of the amino acid sequence depicted in FIG. 5E). In some cases, the Fc polypeptide present in a TMMP is an IgG1 Fc polypeptide that comprises L234A and L235A substitutions (substitutions of L14 and L15 of the amino acid sequence depicted in FIG. 5A with Ala), as depicted in FIG. 5G.

### Linkers

A TMMP of the present disclosure can include one or more linkers, where the one or more linkers are between one or more of: i) an MHC Class I polypeptide and an Ig Fc polypeptide, where such a linker is referred to herein as "L1"; ii) an immunomodulatory polypeptide and an MHC Class I polypeptide, where such a linker is referred to herein as "L2"; iii) a first immunomodulatory polypeptide and a second immunomodulatory polypeptide, where such a linker is referred to herein as "L3"; iv) a peptide antigen ("epitope") and an MHC Class I polypeptide; v) an MHC Class I polypeptide and a dimerization polypeptide (e.g., a first or a second member of a dimerizing pair); and vi) a dimerization polypeptide (e.g., a first or a second member of a dimerizing pair) and an IgFc polypeptide.

Suitable linkers (also referred to as "spacers") can be readily selected and can be of any of a number of suitable lengths, such as from 1 amino acid to 25 amino acids, from 3 amino acids to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids. A suitable linker can be 1, 2, 3, 4, 5, 6, 7, 8, 9, 10, 11, 12, 13, 14, 15, 16, 17, 18, 19, 20, 21, 22, 23, 24, or 25 amino acids in length. In some cases, a linker has a length of from 25 amino acids to 50 amino acids, e.g., from 25 to 30, from 30 to 35, from 35 to 40, from 40 to 45, or from 45 to 50 amino acids in length.

Exemplary linkers include glycine polymers (G)ₙ, glycine-serine polymers (including, for example, (GS)ₙ, (GSGGS)ₙ (SEQ ID NO:366) and (GGGS)ₙ (SEQ ID NO:367), where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains (see Scheraga, Rev. Computational Chem. 11173-142 (1992)). Exemplary linkers can comprise amino acid sequences including, but not limited to, GGSG (SEQ ID NO:368), GGSGG (SEQ ID NO:369), GSGSG (SEQ ID NO:370), GSGGG (SEQ ID NO:371), GGGSG (SEQ ID NO:372), GSSSG (SEQ ID NO:373), and the like. Exemplary linkers can include, e.g., Gly(Ser₄)n (SEQ ID NO:374), where n is 1, 2, 3, 4, 5, 6, 7, 8, 9, or 10. In some cases, a linker comprises the amino acid sequence (GSSSS)n (SEQ ID NO:375), where n is 4. In some cases, a linker comprises the amino acid sequence (GSSSS)n (SEQ ID NO:376), where n is 5. In some cases, a linker comprises the amino acid sequence (GGGGS)n (SEQ ID NO:377), where n is 1. In some cases, a linker comprises the amino acid sequence (GGGGS)n (SEQ ID NO:378), where n is 2. In some cases, a linker comprises the amino acid sequence (GGGGS)n (SEQ ID NO:379), where n is 3. In some cases, a linker comprises the amino acid sequence (GGGGS)n (SEQ ID NO:380), where n is 4. In some cases, a linker comprises the amino acid sequence (GGGGS)n (SEQ ID NO:381), where n is 5. In some cases, a linker comprises the amino acid sequence (GGGGS)n (SEQ ID NO:382), where n is 6. In some cases, a linker comprises the amino acid sequence (GGGGS)n (SEQ ID NO:383), where n is 7, In some cases, a linker comprises the amino acid sequence (GGGGS)n (SEQ ID NO:384), where n is 8, In some cases, a linker comprises the amino acid sequence (GGGGS)n (SEQ ID NO:385), where n is 9, In some cases, a linker comprises the amino acid sequence (GGGGS)n (SEQ ID NO:386), where n is 10. In some cases, a linker comprises the amino acid sequence AAAGG (SEQ ID NO:387).

In some cases, a linker polypeptide, present in a first polypeptide of a TMMP of the present disclosure, includes a cysteine residue that can form a disulfide bond with a cysteine residue present in a second polypeptide of a TMMP of the present disclosure. In some cases, for example, a suitable linker comprises the amino acid sequence G**C**GGSGGGGSGGGGS (SEQ ID NO:317). As another example, a suitable linker can comprise the amino acid sequence GCGGS(G4S)n (SEQ ID NO:315), where n is 1, 2, 3, 4, 5, 6, 7, 8, or 9. For example, in some cases, the linker comprises the amino acid sequence GCGGSGGGGSGGGGSGGGGS (SEQ ID NO:316). As another example, the linker comprises the amino acid sequence GCGGSGGGGSGGGGS (SEQ ID NO:317).

### Epitopes

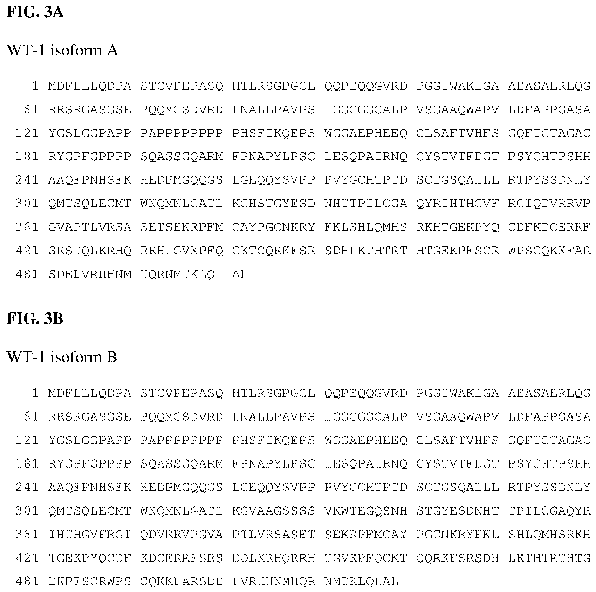

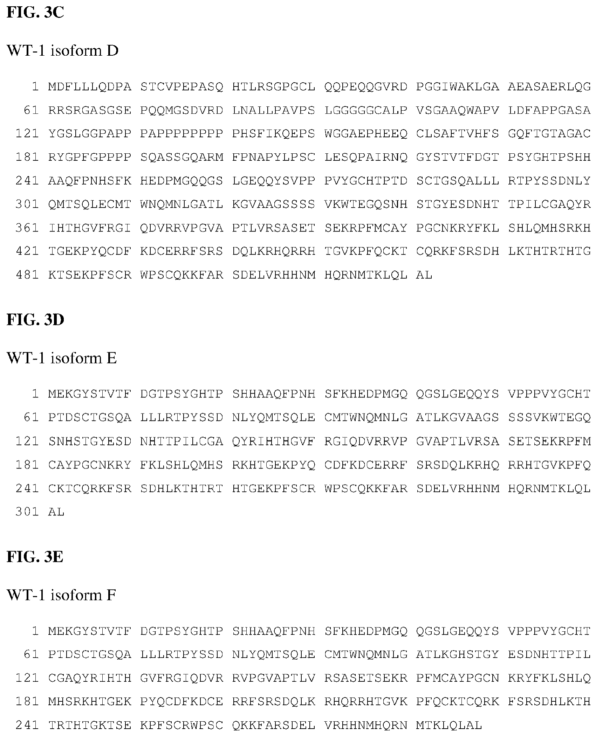

In some cases, an epitope (a peptide presenting one or more epitopes) present in a TMMP of the present disclosure is a WT-1 peptide, e.g., a WT-1 peptide that, together with MHC, presents an epitope to a TCR. Amino acid sequences of WT-1 isoforms are presented in FIG. 3A-3E. A WT-1 peptide that presents one or more epitopes is referred to herein as a "WT-1 peptide" or a "WT-1 epitope." In some cases, a WT-1 epitope present in a TMMP of the present disclosure can be a peptide of from 4 to 25 contiguous amino acids (e.g., 4 amino acids (aa), 5 aa, 6 aa, 7 aa, 8 aa, 9 aa, 10-15 aa, 15-20 aa, or 20-25 aa) of an amino acid sequence having at least 90%, at least 95%, at least 98%, at least 99%, or 100%, amino acid sequence identity to the WT-1 amino acid sequence depicted in any one of FIG. 3A-3E. In some cases, a WT-1 epitope present in a TMMP of the present disclosure can be a peptide of from 4 to 25 contiguous amino acids (e.g., 4 amino acids (aa), 5 aa, 6 aa, 7 aa, 8 aa, 9 aa, 10-15 aa, 15-20 aa, or 20-25 aa) of an amino acid sequence having at least 90%, at least 95%, at least 98%, at least 99%, or 100%, amino acid sequence identity to the WT-1 amino acid sequence depicted in FIG. 3A. In some cases, a WT-1 epitope present in a TMMP of the present disclosure can be a peptide of from 4 to 25 contiguous amino acids (e.g., 4 amino acids (aa), 5 aa, 6 aa, 7 aa, 8 aa, 9 aa, 10-15 aa, 15-20 aa, or 20-25 aa) of an amino acid sequence having at least 90%, at least 95%, at least 98%, at least 99%, or 100%, amino acid sequence identity to the WT-1 amino acid sequence depicted in FIG. 3B. In some cases, a WT-1 epitope present in a TMMP of the present disclosure can be a peptide of from 4 to 25 contiguous amino acids (e.g., 4 amino acids (aa), 5 aa, 6 aa, 7 aa, 8 aa, 9 aa, 10-15 aa, 15-20 aa, or 20-25 aa) of an amino acid sequence having at least 90%, at least 95%, at least 98%, at least 99%, or 100%, amino acid sequence identity to the WT-1 amino acid sequence depicted in FIG. 3C. In some cases, a WT-1 epitope present in a TMMP of the present disclosure can be a peptide of from 4 to 25 contiguous amino acids (e.g., 4 amino acids (aa), 5 aa, 6 aa, 7 aa, 8 aa, 9 aa, 10-15 aa, 15-20 aa, or 20-25 aa) of an amino acid sequence having at least 90%, at least 95%, at least 98%, at least 99%, or 100%, amino acid sequence identity to the WT-1 amino acid sequence depicted in FIG. 3D. In some cases, a WT-1 epitope present in a TMMP of the present disclosure can be a peptide of from 4 to 25 contiguous amino acids (e.g., 4 amino acids (aa), 5 aa, 6 aa, 7 aa, 8 aa, 9 aa, 10-15 aa, 15-20 aa, or 20-25 aa) of an amino acid sequence having at least 90%, at least 95%, at least 98%, at least 99%, or 100%, amino acid sequence identity to the WT-1 amino acid sequence depicted in FIG. 3E. In some cases, a WT-1 epitope present in a TMMP of the present disclosure is 6 amino acids in length. In some cases, a WT-1 epitope present in a TMMP of the present disclosure is 7 amino acids in length. In some cases, a WT-1 epitope present in a TMMP of the present disclosure is 8 amino acids in length. In some cases, a WT-1 epitope present in a TMMP of the present disclosure is 9 amino acids in length. In some cases, a WT-1 epitope present in a TMMP of the present disclosure is 10 amino acids in length. In some cases, a WT-1 epitope present in a TMMP of the present disclosure is 11 amino acids in length. In some cases, a WT-1 epitope present in a TMMP of the present disclosure is from 6 amino acids to 25 amino acids in length. In some cases, a WT-1 epitope present in a TMMP of the present disclosure is from 6 amino acids to 20 amino acids in length. In some cases, a WT-1 epitope present in a TMMP of the present disclosure is from 7 amino acids to 25 amino acids in length. In some cases, a WT-1 epitope present in a TMMP of the present disclosure is from 7 amino acids to 20 amino acids in length. In some cases, a WT-1 epitope present in a TMMP of the present disclosure is at least 4 amino acids in length, at least 6 amino acids in length, or at least 7 amino acids in length.

An epitope present in a TMMP of the present disclosure can have a length of from about 4 amino acids to about 25 amino acids, e.g., the epitope can have a length of from 4 amino acids (aa) to 10 aa, from 10 aa to 15 aa, from 15 aa to 20 aa, or from 20 aa to 25 aa. For example, an epitope present in a TMMP of the present disclosure can have a length of 4 amino acids (aa), 5 aa, 6 aa, 7, aa, 8 aa, 9 aa, 10 aa, 11 aa, 12 aa, 13 aa, 14 aa, 15 aa, 16 aa, 17 aa, 18 aa, 19 aa, 20 aa, 21 aa, 22 aa, 23 aa, 24 aa, or 25 aa. In some cases, an epitope present in a TMMP has a length of from 5 amino acids to 10 amino acids, e.g., 5 aa, 6 aa, 7 aa, 8 aa, 9 aa, or 10 aa.

A WT-1 epitope present in a TMMP of the present disclosure is a peptide specifically bound by a T-cell, i.e., the epitope is specifically bound by a WT-1 epitope-specific T cell. An epitope-specific T cell binds an epitope having a reference amino acid sequence, but does not substantially bind an epitope that differs from the reference amino acid sequence. For example, an epitope-specific T cell binds an epitope having a reference amino acid sequence, and binds an epitope that differs from the reference amino acid sequence, if at all, with an affinity that is less than 10⁻⁶ M, less than 10⁻⁵ M, or less than 10⁻⁴ M. An epitope-specific T cell can bind an epitope for which it is specific with an affinity of at least 10⁻⁷ M, at least 10⁻⁸ M, at least 10⁻⁹ M, or at least 10⁻¹⁰ M.

Examples of WT-1 peptides suitable for inclusion in a TMMP of the present disclosure include, but are not limited to, CMTWNQMNLGATLKG (SEQ ID NO:223), WNQMNLGATLKGVAA (SEQ ID NO:224), CMTWNYMNLGATLKG (SEQ ID NO:225), WNYMNLGATLKGVAA (SEQ ID NO:226), MTWNQMNLGATLKGV (SEQ ID NO:227), TWNQMNLGATLKGVA (SEQ ID NO:228), CMTWNLMNLGATLKG (SEQ ID NO:229), MTWNLMNLGATLKGV (SEQ ID NO:230), TWNLMNLGATLKGVA (SEQ ID NO:231), WNLMNLGATLKGVAA (SEQ ID NO:232), MNLGATLK (SEQ ID NO:233), MTWNYMNLGATLKGV (SEQ ID NO:234), TWNYMNLGATLKGVA (SEQ ID NO:235), CMTWNQMNLGATLKGVA (SEQ ID NO:236), CMTWNLMNLGATLKGVA (SEQ ID NO:237), CMTWNYMNLGATLKGVA (SEQ ID NO:238), GYLRNPTAC (SEQ ID NO:239), GALRNPTAL (SEQ ID NO:240), YALRNPTAC (SEQ ID NO:241), GLLRNPTAC (SEQ ID NO:242), RYRPHPGAL (SEQ ID NO:243), YQRPHPGAL (SEQ ID NO:244), RLRPHPGAL (SEQ ID NO:245), RIRPHPGAL (SEQ ID NO:246), QFPNHSFKHEDPMGQ (SEQ ID NO:247), HSFKHEDPY (SEQ ID NO:248), QFPNHSFKHEDPM (SEQ ID NO:249), QFPNHSFKHEDPY (SEQ ID NO:250), KRPFMCAYPGCNK (SEQ ID NO:251), KRPFMCAYPGCYK (SEQ ID NO:252), FMCAYPGCY (SEQ ID NO:253), FMCAYPGCK (SEQ ID NO:254), KRPFMCAYPGCNKRY (SEQ ID NO:255), SEKRPFMCAYPGCNK (SEQ ID NO:256), KRPFMCAYPGCYKRY (SEQ ID NO:257), NLMNLGATL (SEQ ID NO:258), VLDFAPPGA (SEQ ID NO:259); RMFPNAPYL (SEQ ID NO:260); CMTWNQMN (SEQ ID NO:261); CYTWNQMNL (SEQ ID NO:269); NYMNLGATL (SEQ ID NO:263); YMFPNAPYL (SEQ ID NO:264); SLGEQQYSV (SEQ ID NO:265); CMTWNQMNL (SEQ ID NO:266); and NQMNLGATL (SEQ ID NO:267). In some cases, the WT-1 peptide present in a TMMP of the present disclosure is CMTWNQMN (SEQ ID NO:261). In some cases, the WT-1 peptide present in a TMMP of the present disclosure is CYTWNQMNL (SEQ ID NO:269).

In some cases, the WT-1 peptide present in a TMMP of the present disclosure presents an HLA-A*2402-restricted epitope. WT-1 peptides that present an HLA-A*2402-restricted epitope include, e.g., CMTWNQMN (SEQ ID NO:261); NYMNLGATL (SEQ ID NO:263) (WT-1 239-247; Q240Y); CYTWNQMNL (SEQ ID NO:269) (WT-1 235-243); CMTWNQMNL (SEQ ID NO:266) (WT-1 235-243); NQMNLGATL (SEQ ID NO:267) (WT-1 239-247); and NLMNLGATL (SEQ ID NO:258) (WT-1 239-247; Q240L).

In some cases, the WT-1 peptide present in a TMMP of the present disclosure presents an HLA-A*0201-restricted epitope. WT-1 peptides that present an HLA-A*0201-restricted epitope include, e.g., VLDFAPPGA (SEQ ID NO:259) (WT-1 37-45); RMFPNAPYL (SEQ ID NO:260) (WT-1 126-134); YMFPNAPYL (SEQ ID NO:264) (WT-1 126-134; R126Y); SLGEQQYSV (SEQ ID NO:265) (WT-1 187-195); and NLMNLGATL (SEQ ID NO:258) (WT-1 239-247; Q240L).

### HLA/peptide binding assays

Whether a given peptide (e.g., WT-1 peptide) binds a class I HLA (comprising an HLA heavy chain and a β2M polypeptide), and, when bound to the HLA complex, can effectively present an epitope to a TCR, can be determined using any of a number of well-known methods. Assays include binding assays and T-cell activation assays.

### Cell-based binding assay

As one example, a cell-based peptide-induced stabilization assay can be used to determine peptide-HLA class I binding. In this assay, a peptide of interest is allowed to bind to a TAP-deficient cell, i.e., a cell that has defective transporter associated with antigen processing (TAP) machinery, and consequently, few surface class I molecules. Such cells include, e.g., the human T2 cell line (T2 (174 x CEM.T2; American Type Culture Collection (ATCC) No. CRL-1992). Henderson et al. (1992) Science 255:1264. Without efficient TAP-mediated transport of cytosolic peptides into the endoplasmic reticulum, assembled class I complexes are structurally unstable, and retained only transiently at the cell surface. However, when T2 cells are incubated with an exogenous peptide capable of binding class I, surface peptide-HLA class I complexes are stabilized and can be detected by flow cytometry with, e.g., a pan anti-class I monoclonal antibody. The stabilization and resultant increased life-span of peptide-HLA complexes on the cell surface by the addition of a peptide validates their identity. Analysis can be carried out using flow cytometry, e.g., where the pan-HLA class I antibody comprises a fluorescent label. Binding of the peptide to various allelic forms of HLA H chains can be tested by genetically modifying the T2 cells to express an allelic HLA H chain of interest.

The following is a non-limiting example of use of a T2 assay to assess peptide binding to HLA A*0201. T2 cells are washed in cell culture medium, and concentrated to 10⁶ cells/ml. Peptides of interest are prepared in cell culture medium and serially diluted providing concentrations of 200 µM, 100 µM, 20 µM and 2 µM. The cells are mixed 1:1 with each peptide dilution to give a final volume of 200 µL and final peptide concentrations of 100 µM, 50 µM, 10 µM and 1 µM. A HLA A*0201 binding peptide, GILGFVFTL (SEQ ID NO: 395), and a non-HLA A*0201-restricted peptide, HPVGEADYF (SEQ ID NO: 396) (HLA-B*3501), are included as positive and negative controls, respectively. The cell/peptide mixtures are kept at 37°C 5% CO₂ for ten minutes; then incubated at room temperature overnight. Cells are then incubated for 2 hours at 37°C and stained with a fluorescently-labeled antihuman HLA antibody. The cells are washed twice with phosphate-buffered saline and analyzed using flow cytometry. The average mean fluorescence intensity (MFI) of the anti-HLA antibody staining is used to measure the strength of binding.

### Biochemical binding assay

HLA polypeptides (HLA heavy chain polypeptide complexed with β2M polypeptide) can be tested for binding to a peptide of interest in a cell-free *in vitro* assay system. For example, a labeled reference peptide (e.g., fluorescently labeled) is allowed to bind to HLA polypeptides (HLA heavy chain polypeptide complexed with β2M polypeptide), to form an HLA-reference peptide complex. The ability of a test peptide of interest to displace the labeled reference peptide from the HLA-reference peptide complex is tested. The relative binding affinity is calculated as the amount of test peptide needed to displace the bound reference peptide. See, e.g., van der Burg et al. (1995) Human Immunol. 44:189.

As another example, a peptide of interest can be incubated with an HLA molecule (HLA heavy chain complexed with a β2M polypeptide), and the stabilization of the HLA/peptide complex can be measured in an immunoassay format. The ability of a peptide of interest to stabilize an HLA molecule is compared to that of a control peptide presenting a known T-cell epitope. Detection of stabilization is based on the presence or absence of the native conformation of the HLA/peptide complex, detected using an anti-HLA antibody. See, e.g., Westrop et al. (2009) J. Immunol. Methods 341:76; Steinitz et al. (2012) Blood 119:4073; and U.S. Patent No. 9,205,144.

### T-cell activation assays

Whether a given peptide binds a class I HLA (comprising an HLA heavy chain and a β2M polypeptide), and, when bound to the HLA complex, can effectively present an epitope to a TCR, can be determined by assessing T-cell response to the peptide-HLA complex. T-cell responses that can be measured include, e.g., interferon-gamma (IFNγ) production, cytotoxic activity, and the like.

### ELISPOT assay

Suitable assays include, e.g., an enzyme linked immunospot (ELISPOT) assay. In this assay, production of IFNγ by CD8⁺ T cells is measured following with an antigen-presenting cell (APC) that presents a peptide of interest complexed with HLA class I. Antibody to IFNγ is immobilized on wells of a multi-well plate. APCs are added to the wells, and incubated for a period of time with a peptide of interest, such that the peptide binds HLA class I on the surface of the APCs. CD8⁺ T cells specific for the peptide are added to the wells, and the plate is incubated for about 24 hours. The wells are then washed, and any IFNγ bound to the immobilized anti-IFNγ antibody is detected using a detectably labeled anti-IFNγ antibody. A colorimetric assay can be used. For example, the detectably labeled anti-IFNγ antibody can be a biotin-labeled anti-IFNγ antibody, which can be detected using, e.g., streptavidin conjugated to alkaline phosphatase. A BCIP/NBT (5-bromo-4-chloro-3-indolyl phosphate/nitro blue tetrazolium) solution is added, to develop the assay. The presence of IFNγ-secreting T cells is identified by colored spots. Negative controls include APCs not contacted with the peptide. APCs expressing various HLA H chain alleles can be used to determine whether a peptide of interest effectively binds to a HLA class I molecule comprising a particular HLA H chain.

### Cytotoxicity assays

Whether a given peptide binds to a particular HLA class I H chain and, when bound to a HLA class I complex comprising the H chain, can effectively present an epitope to a TCR, can also be determined using a cytotoxicity assay. A cytotoxicity assay involves incubation of a target cell with a cytotoxic CD8⁺ T cell. The target cell displays on its surface a peptide/HLA class I complex comprising a peptide of interest and an HLA class I molecule comprising an HLA H chain to be tested. The target cells can be radioactively labeled, e.g., with ⁵¹Cr. Whether the target cell effectively presents an epitope to a TCR on the cytotoxic CD8⁺ T cell, thereby inducing cytotoxic activity by the CD8⁺ T cell toward the target cell, is determined by measuring release of ⁵¹Cr from the lysed target cell. Specific cytotoxicity can be calculated as the amount of cytotoxic activity in the presence of the peptide minus the amount of cytotoxic activity in the absence of the peptide.

### Detection of Antigen-specific T cells with peptide-HLA tetramers

As another example, multimers (e.g., tetramers) of peptide-HLA complexes are generated with fluorescent or heavy metal tags. The multimers can then be used to identify and quantify specific T cells via flow cytometry (FACS) or mass cytometry (CyTOF). Detection of epitope-specific T cells provides direct evidence that the peptide-bound HLA molecule is capable of binding to a specific TCR on a subset of antigen-specific T cells. See, e.g., Klenerman et al. (2002) Nature Reviews Immunol. 2:263.

### Immunomodulatory polypeptides

In some cases, an immunomodulatory polypeptide present in a TMMP of the present disclosure is a wild-type immunomodulatory polypeptide. In other cases, an immunomodulatory polypeptide present in a TMMP of the present disclosure is a variant immunomodulatory polypeptide that has reduced affinity for a co-immunomodulatory polypeptide, compared to the affinity of a corresponding wild-type immunomodulatory polypeptide for the co-immunomodulatory polypeptide. Suitable immunomodulatory domains that exhibit reduced affinity for a co-immunomodulatory domain can have from 1 amino acid (aa) to 20 aa differences from a wild-type immunomodulatory domain. For example, in some cases, a variant immunomodulatory polypeptide present in a TMMP of the present disclosure differs in amino acid sequence by 1 aa, 2 aa, 3 aa, 4 aa, 5 aa, 6 aa, 7 aa, 8 aa, 9 aa, or 10 aa, from a corresponding wild-type immunomodulatory polypeptide. As another example, in some cases, a variant immunomodulatory polypeptide present in a TMMP of the present disclosure differs in amino acid sequence by 11 aa, 12 aa, 13 aa, 14 aa, 15 aa, 16 aa, 17 aa, 18 aa, 19 aa, or 20 aa, from a corresponding wild-type immunomodulatory polypeptide. As an example, in some cases, a variant immunomodulatory polypeptide present in a TMMP of the present disclosure includes 1, 2, 3, 4, 5, 6, 7, 8, 9, or 10 amino acid substitutions, compared to a corresponding reference (e.g., wild-type) immunomodulatory polypeptide. In some cases, variant immunomodulatory polypeptide present in a TMMP of the present disclosure includes a single amino acid substitution compared to a corresponding reference (e.g., wild-type) immunomodulatory polypeptide. In some cases, variant immunomodulatory polypeptide present in a TMMP of the present disclosure includes 2 amino acid substitutions (e.g., no more than 2 amino acid substitutions) compared to a corresponding reference (e.g., wild-type) immunomodulatory polypeptide. In some cases, variant immunomodulatory polypeptide present in a TMMP of the present disclosure includes 3 amino acid substitutions (e.g., no more than 3 amino acid substitutions) compared to a corresponding reference (e.g., wild-type) immunomodulatory polypeptide. In some cases, variant immunomodulatory polypeptide present in a TMMP of the present disclosure includes 4 amino acid substitutions (e.g., no more than 4 amino acid substitutions) compared to a corresponding reference (e.g., wild-type) immunomodulatory polypeptide. In some cases, variant immunomodulatory polypeptide present in a TMMP of the present disclosure includes 5 amino acid substitutions (e.g., no more than 5 amino acid substitutions) compared to a corresponding reference (e.g., wild-type) immunomodulatory polypeptide. In some cases, variant immunomodulatory polypeptide present in a TMMP of the present disclosure includes 6 amino acid substitutions (e.g., no more than 6 amino acid substitutions) compared to a corresponding reference (e.g., wild-type) immunomodulatory polypeptide. In some cases, variant immunomodulatory polypeptide present in a TMMP of the present disclosure includes 7 amino acid substitutions (e.g., no more than 7 amino acid substitutions) compared to a corresponding reference (e.g., wild-type) immunomodulatory polypeptide. In some cases, variant immunomodulatory polypeptide present in a TMMP of the present disclosure includes 8 amino acid substitutions (e.g., no more than 8 amino acid substitutions) compared to a corresponding reference (e.g., wild-type) immunomodulatory polypeptide. In some cases, variant immunomodulatory polypeptide present in a TMMP of the present disclosure includes 9 amino acid substitutions (e.g., no more than 9 amino acid substitutions) compared to a corresponding reference (e.g., wild-type) immunomodulatory polypeptide. In some cases, variant immunomodulatory polypeptide present in a TMMP of the present disclosure includes 10 amino acid substitutions (e.g., no more than 10 amino acid substitutions) compared to a corresponding reference (e.g., wild-type) immunomodulatory polypeptide.

In some cases, variant immunomodulatory polypeptide present in a TMMP of the present disclosure includes 11 amino acid substitutions (e.g., no more than 11 amino acid substitutions) compared to a corresponding reference (e.g., wild-type) immunomodulatory polypeptide.

In some cases, variant immunomodulatory polypeptide present in a TMMP of the present disclosure includes 12 amino acid substitutions (e.g., no more than 12 amino acid substitutions) compared to a corresponding reference (e.g., wild-type) immunomodulatory polypeptide.

In some cases, variant immunomodulatory polypeptide present in a TMMP of the present disclosure includes 13 amino acid substitutions (e.g., no more than 13 amino acid substitutions) compared to a corresponding reference (e.g., wild-type) immunomodulatory polypeptide.

In some cases, variant immunomodulatory polypeptide present in a TMMP of the present disclosure includes 14 amino acid substitutions (e.g., no more than 14 amino acid substitutions) compared to a corresponding reference (e.g., wild-type) immunomodulatory polypeptide.

In some cases, variant immunomodulatory polypeptide present in a TMMP of the present disclosure includes 15 amino acid substitutions (e.g., no more than 15 amino acid substitutions) compared to a corresponding reference (e.g., wild-type) immunomodulatory polypeptide.

In some cases, variant immunomodulatory polypeptide present in a TMMP of the present disclosure includes 16 amino acid substitutions (e.g., no more than 16 amino acid substitutions) compared to a corresponding reference (e.g., wild-type) immunomodulatory polypeptide.

In some cases, variant immunomodulatory polypeptide present in a TMMP of the present disclosure includes 17 amino acid substitutions (e.g., no more than 17 amino acid substitutions) compared to a corresponding reference (e.g., wild-type) immunomodulatory polypeptide.

In some cases, variant immunomodulatory polypeptide present in a TMMP of the present disclosure includes 18 amino acid substitutions (e.g., no more than 18 amino acid substitutions) compared to a corresponding reference (e.g., wild-type) immunomodulatory polypeptide.

In some cases, variant immunomodulatory polypeptide present in a TMMP of the present disclosure includes 19 amino acid substitutions (e.g., no more than 19 amino acid substitutions) compared to a corresponding reference (e.g., wild-type) immunomodulatory polypeptide.

In some cases, variant immunomodulatory polypeptide present in a TMMP of the present disclosure includes 20 amino acid substitutions (e.g., no more than 20 amino acid substitutions) compared to a corresponding reference (e.g., wild-type) immunomodulatory polypeptide.

As discussed above, a variant immunomodulatory polypeptide suitable for inclusion in a TMMP of the present disclosure exhibits reduced affinity for a cognate co-immunomodulatory polypeptide, compared to the affinity of a corresponding wild-type immunomodulatory polypeptide for the cognate co-immunomodulatory polypeptide.

Exemplary pairs of immunomodulatory polypeptide and cognate co-immunomodulatory polypeptide include, but are not limited to:
a) 4-1BBL (immunomodulatory polypeptide) and 4-1BB (cognate co-immunomodulatory polypeptide);
b) PD-L1 (immunomodulatory polypeptide) and PD1 (cognate co-immunomodulatory polypeptide);
c) IL-2 (immunomodulatory polypeptide) and IL-2 receptor (cognate co-immunomodulatory polypeptide);
d) CD80 (immunomodulatory polypeptide) and CD86 (cognate co-immunomodulatory polypeptide);
e) CD86 (immunomodulatory polypeptide) and CD28 (cognate co-immunomodulatory polypeptide);
f) OX40L (CD252) (immunomodulatory polypeptide) and OX40 (CD134) (cognate co-immunomodulatory polypeptide);
g) Fas ligand (immunomodulatory polypeptide) and Fas (cognate co-immunomodulatory polypeptide);
h) ICOS-L (immunomodulatory polypeptide) and ICOS (cognate co-immunomodulatory polypeptide);
i) ICAM (immunomodulatory polypeptide) and LFA-1 (cognate co-immunomodulatory polypeptide);
j) CD30L (immunomodulatory polypeptide) and CD30 (cognate co-immunomodulatory polypeptide);
k) CD40 (immunomodulatory polypeptide) and CD40L (cognate co-immunomodulatory polypeptide);
l) CD83 (immunomodulatory polypeptide) and CD83L (cognate co-immunomodulatory polypeptide);
m) HVEM (CD270) (immunomodulatory polypeptide) and CD160 (cognate co-immunomodulatory polypeptide);
n) JAG1 (CD339) (immunomodulatory polypeptide) and Notch (cognate co-immunomodulatory polypeptide);
o) JAG1 (immunomodulatory polypeptide) and CD46 (cognate co-immunomodulatory polypeptide);
p) CD80 (immunomodulatory polypeptide) and CTLA4 (cognate co-immunomodulatory polypeptide);
q) CD86 (immunomodulatory polypeptide) and CTLA4 (cognate co-immunomodulatory polypeptide); and
r) CD70 (immunomodulatory polypeptide) and CD27 (cognate co-immunomodulatory polypeptide).

In some cases, a variant immunomodulatory polypeptide present in a TMMP of the present disclosure has a binding affinity for a cognate co-immunomodulatory polypeptide that is from 100 nM to 100 µM. For example, in some cases, a variant immunomodulatory polypeptide present in a TMMP of the present disclosure has a binding affinity for a cognate co-immunomodulatory polypeptide that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

A variant immunomodulatory polypeptide present in a TMMP of the present disclosure exhibits reduced affinity for a cognate co-immunomodulatory polypeptide. Similarly, a TMMP of the present disclosure that comprises a variant immunomodulatory polypeptide exhibits reduced affinity for a cognate co-immunomodulatory polypeptide. Thus, for example, a TMMP of the present disclosure that comprises a variant immunomodulatory polypeptide has a binding affinity for a cognate co-immunomodulatory polypeptide that is from 100 nM to 100 µM. For example, in some cases, a TMMP of the present disclosure that comprises a variant immunomodulatory polypeptide has a binding affinity for a cognate co-immunomodulatory polypeptide that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

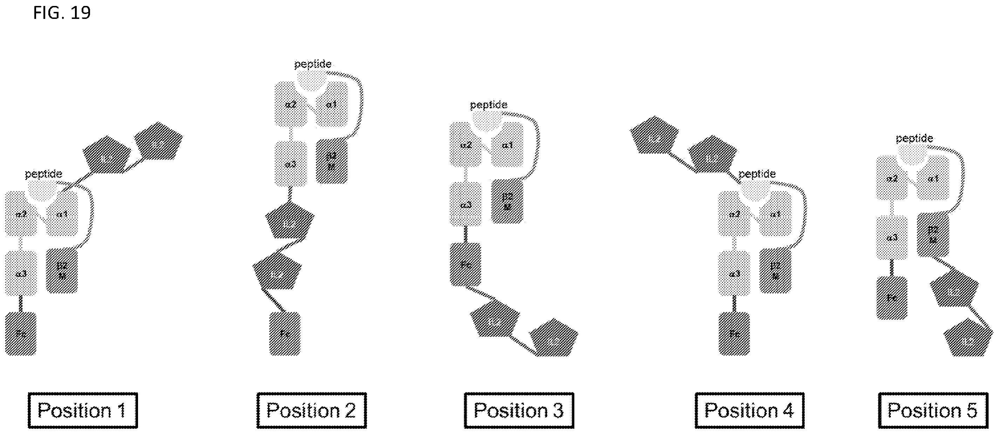

As depicted schematically in FIG. 19, an immunomodulatory polypeptide (i.e., one or more immunomodulatory polypeptides) can be present in a TMMP of the present disclosure at any of a variety of positions. FIG. 19 depicts the position of two copies of a variant IL-2 polypeptide; however, the immunomodulatory polypeptide can be any of a variety of immunomodulatory polypeptide, as described herein. As depicted in FIG. 19, an immunomodulatory polypeptide can be: 1) N-terminal to the MHC class I heavy chain; 2) C-terminal to the MHC class I heavy chain and N-terminal to the Ig Fc polypeptide; in other words, between the MHC class I heavy chain and the Ig Fc polypeptide; 3) C-terminal to the Ig Fc polypeptide; 4) N-terminal to the peptide epitope; or 5) C-terminal to the β2M polypeptide.

### PD-L1 variants

As one non-limiting example, in some cases, a variant immunomodulatory polypeptide present in a TMMP of the present disclosure is a variant PD-L1 polypeptide. Wild-type PD-L1 binds to PD1.
A wild-type human PD-L1 polypeptide can comprise the following amino acid sequence: 
MRIFAVFIFM TYWHLLNAFT VTVPKDLYVV EYGSNMTIEC KFPVEKQLDL AALIVYWEME
DKNIIQFVHG EEDLKVQHSS YRQRARLLKD QLSLGNAALQ ITDVKLQDAG VYRCMISYGG
ADYKRITVKV NAPYNKINQR ILVVDPVTSE HELTCQAEGY PKAEVIWTSS DHQVLSGKTT
TTNSKREEKL FNVTSTLRIN TTTNEIFYCT FRRLDPEENH TAELVIPGNI LNVSIKICLT LSPST
(SEQ ID NO:1).
A wild-type human PD-L1 ectodomain can comprise the following amino acid sequence: 
FT VTVPKDLYVV EYGSNMTIEC KFPVEKQLDL AALIVYWEME DKNIIQFVHG
EEDLKVQHSS YRQRARLLKD QLSLGNAALQ ITDVKLQDAG VYRCMISYGG ADYKRITVKV
NAPYNKINQR ILVVDPVTSE HELTCQAEGY PKAEVIWTSS DHQVLSGKTT TTNSKREEKL
FNVTSTLRIN TTTNEIFYCT FRRLDPEENH TAELVIPGNI LNVSIKI (SEQ ID NO:2).
A wild-type PD-1 polypeptide can comprise the following amino acid sequence:
   PGWFLDSPDR PWNPPTFSPA LLVVTEGDNA TFTCSFSNTS ESFVLNWYRM SPSNQTDKLA AFPEDRSQPG QDCRFRVTQL PNGRDFHMSV VRARRNDSGT YLCGAISLAP KAQIKESLRA ELRVTERRAE VPTAHPSPSP RPAGQFQTLV VGVVGGLLGS LVLLVWVLAV ICSRAARGTI GARRTGQPLK EDPSAVPVFS VDYGELDFQW REKTPEPPVP CVPEQTEYAT IVFPSGMGTS SPARRGSADG PRSAQPLRPE DGHCSWPL (SEQ ID NO:3). In some cases, where a TMMP of the present disclosure comprises a variant PD-L1 polypeptide, a "cognate co-immunomodulatory polypeptide" is a PD-1 polypeptide comprising the amino acid sequence of SEQ ID NO:3.

In some cases, a variant PD-L1 polypeptide exhibits reduced binding affinity to PD-1 (e.g., a PD-1 polypeptide comprising the amino acid sequence set forth in SEQ ID NO:3), compared to the binding affinity of a PD-L1 polypeptide comprising the amino acid sequence set forth in SEQ ID NO:1 or SEQ ID NO:2. For example, in some cases, a variant PD-L1 polypeptide of the present disclosure binds PD-1 (e.g., a PD-1 polypeptide comprising the amino acid sequence set forth in SEQ ID NO:3) with a binding affinity that is at least 10%, at least 15%, at least 20%, at least 25%, at least 30%, at least 35%, at least 40%, at least 45%, at least 50% less, at least 55% less, at least 60% less, at least 65% less, at least 70% less, at least 75% less, at least 80% less, at least 85% less, at least 90% less, at least 95% less, or more than 95% less, than the binding affinity of a PD-L1 polypeptide comprising the amino acid sequence set forth in SEQ ID NO:1 or SEQ ID NO:2.

In some cases, a variant PD-L1 polypeptide has a binding affinity to PD-1that is from 1nM to 1mM. In some cases, a variant PD-L1 polypeptide of the present disclosure has a binding affinity to PD-1 that is from 100 nM to 100 µM. As another example, in some cases, a variant PD-L1 polypeptide has a binding affinity for PD1 (e.g., a PD1 polypeptide comprising the amino acid sequence set forth in SEQ ID NO:3) that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

In some cases, a variant PD-L1 polypeptide has a single amino acid substitution compared to the PD-L1 amino acid sequence set forth in SEQ ID NO:1 or SEQ ID NO:2. In some cases, a variant PD-L1 polypeptide has from 2 to 10 amino acid substitutions compared to the PD-L1 amino acid sequence set forth in SEQ ID NO:1 or SEQ ID NO:2. In some cases, a variant PD-L1 polypeptide has 2 amino acid substitutions compared to the PD-L1 amino acid sequence set forth in SEQ ID NO:1 or SEQ ID NO:2. In some cases, a variant PD-L1 polypeptide has 3 amino acid substitutions compared to the PD-L1 amino acid sequence set forth in SEQ ID NO:1 or SEQ ID NO:2. In some cases, a variant PD-L1 polypeptide has 4 amino acid substitutions compared to the PD-L1 amino acid sequence set forth in SEQ ID NO:1 or SEQ ID NO:2. In some cases, a variant PD-L1 polypeptide has 5 amino acid substitutions compared to the PD-L1 amino acid sequence set forth in SEQ ID NO:1 or SEQ ID NO:2. In some cases, a variant PD-L1 polypeptide has 6 amino acid substitutions compared to the PD-L1 amino acid sequence set forth in SEQ ID NO:1 or SEQ ID NO:2. In some cases, a variant PD-L1 polypeptide has 7 amino acid substitutions compared to the PD-L1 amino acid sequence set forth in SEQ ID NO:1 or SEQ ID NO:2. In some cases, a variant PD-L1 polypeptide has 8 amino acid substitutions compared to the PD-L1 amino acid sequence set forth in SEQ ID NO:1 or SEQ ID NO:2. In some cases, a variant PD-L1 polypeptide has 9 amino acid substitutions compared to the PD-L1 amino acid sequence set forth in SEQ ID NO:1 or SEQ ID NO:2. In some cases, a variant PD-L1 polypeptide has 10 amino acid substitutions compared to the PD-L1 amino acid sequence set forth in SEQ ID NO:1 or SEQ ID NO:2.

A suitable PD-L1 variant includes a polypeptide that comprises an amino acid sequence having at least 90%, at least 95%, at least 98%, at least 99%, or 100%, amino acid sequence identity to the following amino acid sequence:
FT VTVPK**X**LYVV EYGSNMTIEC KFPVEKQLDL AALIVYWEME DKNIIQFVHG EEDLKVQHSS YRQRARLLKD QLSLGNAALQ ITDVKLQDAG VYRCMISYGG ADYKRITVKV NAPYNKINQR ILVVDPVTSE HELTCQAEGY PKAEVIWTSS DHQVLSGKTT TTNSKREEKL FNVTSTLRIN TTTNEIFYCT FRRLDPEENH TAELVIPGNI LNVSIKI (SEQ ID NO: 52), where X is any amino acid other than Asp. In some cases, X is Ala. In some cases, X is Arg.

A suitable PD-L1 variant includes a polypeptide that comprises an amino acid sequence having at least 90%, at least 95%, at least 98%, at least 99%, or 100%, amino acid sequence identity to the following amino acid sequence:
FT VTVPKDLYVV EYGSNMTIEC KFPVEKQLDL AAL**X**VYWEME DKNIIQFVHG EEDLKVQHSS YRQRARLLKD QLSLGNAALQ ITDVKLQDAG VYRCMISYGG ADYKRITVKV NAPYNKINQR ILVVDPVTSE HELTCQAEGY PKAEVIWTSS DHQVLSGKTT TTNSKREEKL FNVTSTLRIN TTTNEIFYCT FRRLDPEENH TAELVIPGNI LNVSIKI (SEQ ID NO: 53), where X is any amino acid other than Ile. In some cases, X is Asp.

A suitable PD-L1 variant includes a polypeptide that comprises an amino acid sequence having at least 90%, at least 95%, at least 98%, at least 99%, or 100%, amino acid sequence identity to the following amino acid sequence:
FT VTVPKDLYVV EYGSNMTIEC KFPVEKQLDL AALIVYWEME DKNIIQFVHG E**X**DLKVQHSS YRQRARLLKD QLSLGNAALQ ITDVKLQDAG VYRCMISYGG ADYKRITVKV NAPYNKINQR ILVVDPVTSE HELTCQAEGY PKAEVIWTSS DHQVLSGKTT TTNSKREEKL FNVTSTLRIN TTTNEIFYCT FRRLDPEENH TAELVIPGNI LNVSIKI (SEQ ID NO:54), where X is any amino acid other than Glu. In some cases, X is Arg.

### CD80 variants

In some cases, a variant immunomodulatory polypeptide present in a TMMP of the present disclosure is a variant CD80 polypeptide. Wild-type CD80 binds to CD28. Wild-type CD80 also binds to CD86.

A wild-type amino acid sequence of the ectodomain of human CD80 can be as follows:




 (SEQ ID NO:4).

A wild-type CD28 amino acid sequence can be as follows: MLRLLLALNL FPSIQVTGNK ILVKQSPMLV AYDNAVNLSC KYSYNLFSRE FRASLHKGLD SAVEVCVVYG NYSQQLQVYS KTGFNCDGKL GNESVTFYLQ NLYVNQTDIY FCKIEVMYPP PYLDNEKSNG TIIHVKGKHL CPSPLFPGPS KPFWVLVVVG GVLACYSLLV TVAFIIFWVR SKRSRLLHSD YMNMTPRRPG PTRKHYQPYA PPRDFAAYRS (SEQ ID NO:5). In some cases, where a TMMP of the present disclosure comprises a variant CD80 polypeptide, a "cognate co-immunomodulatory polypeptide" is a CD28 polypeptide comprising the amino acid sequence of SEQ ID NO:5.

A wild-type CD28 amino acid sequence can be as follows: MLRLLLALNL FPSIQVTGNK ILVKQSPMLV AYDNAVNLSW KHLCPSPLFP GPSKPFWVLV VVGGVLACYS LLVTVAFIIF WVRSKRSRLL HSDYMNMTPR RPGPTRKHYQ PYAPPRDFAA YRS (SEQ ID NO:6)

A wild-type CD28 amino acid sequence can be as follows: MLRLLLALNL FPSIQVTGKH LCPSPLFPGP SKPFWVLVVV GGVLACYSLL VTVAFIIFWV RSKRSRLLHS DYMNMTPRRP GPTRKHYQPY APPRDFAAYR S (SEQ ID NO:7).

In some cases, a variant CD80 polypeptide exhibits reduced binding affinity to CD28, compared to the binding affinity of a CD80 polypeptide comprising the amino acid sequence set forth in SEQ ID NO:4 for CD28. For example, in some cases, a variant CD80 polypeptide binds CD28 with a binding affinity that is at least 10%, at least 15%, at least 20%, at least 25%, at least 30%, at least 35%, at least 40%, at least 45%, at least 50% less, at least 55% less, at least 60% less, at least 65% less, at least 70% less, at least 75% less, at least 80% less, at least 85% less, at least 90% less, at least 95% less, or more than 95% less, than the binding affinity of a CD80 polypeptide comprising the amino acid sequence set forth in SEQ ID NO:4 for CD28 (e.g., a CD28 polypeptide comprising the amino acid sequence set forth in one of SEQ ID NO:5, 6, or 7).

In some cases, a variant CD80 polypeptide has a binding affinity to CD28 that is from 100 nM to 100 µM. As another example, in some cases, a variant CD80 polypeptide of the present disclosure has a binding affinity for CD28 (e.g., a CD28 polypeptide comprising the amino acid sequence set forth in SEQ ID NO:5, SEQ ID NO:6, or SEQ ID NO:7) that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

In some cases, a variant CD80 polypeptide has a single amino acid substitution compared to the CD80 amino acid sequence set forth in SEQ ID NO:4. In some cases, a variant CD80 polypeptide has from 2 to 10 amino acid substitutions compared to the CD80 amino acid sequence set forth in SEQ ID NO:4. In some cases, a variant CD80 polypeptide has 2 amino acid substitutions compared to the CD80 amino acid sequence set forth in SEQ ID NO:4. In some cases, a variant CD80 polypeptide has 3 amino acid substitutions compared to the CD80 amino acid sequence set forth in SEQ ID NO:4. In some cases, a variant CD80 polypeptide has 4 amino acid substitutions compared to the CD80 amino acid sequence set forth in SEQ ID NO:4. In some cases, a variant CD80 polypeptide has 5 amino acid substitutions compared to the CD80 amino acid sequence set forth in SEQ ID NO:4. In some cases, a variant CD80 polypeptide has 6 amino acid substitutions compared to the CD80 amino acid sequence set forth in SEQ ID NO:4. In some cases, a variant CD80 polypeptide has 7 amino acid substitutions compared to the CD80 amino acid sequence set forth in SEQ ID NO:4. In some cases, a variant CD80 polypeptide has 8 amino acid substitutions compared to the CD80 amino acid sequence set forth in SEQ ID NO:4. In some cases, a variant CD80 polypeptide has 9 amino acid substitutions compared to the CD80 amino acid sequence set forth in SEQ ID NO:4. In some cases, a variant CD80 polypeptide has 10 amino acid substitutions compared to the CD80 amino acid sequence set forth in SEQ ID NO:4.

Suitable CD80 variants include a polypeptide that comprises an amino acid sequence having at least 90%, at least 95%, at least 98%, at least 99%, or 100%, amino acid sequence identity to any one of the following amino acid sequences:
VIHVTK EVKEVATLSC GH**X**VSVEELA QTRIYWQKEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO: 55), where X is any amino acid other than Asn. In some cases, X is Ala;
VIHVTK EVKEVATLSC GHNVSVEELA QTRIYWQKEK KMVLTMMSGD MNIWPEYKNR TIFDIT**X**NLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO: 56), where X is any amino acid other than Asn. In some cases, X is Ala;
VIHVTK EVKEVATLSC GHNVSVEELA QTRIYWQKEK KMVLTMMSGD MNIWPEYKNR TIFDITNNLS **X**VILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:57), where X is any amino acid other than Ile. In some cases, X is Ala;
VIHVTK EVKEVATLSC GHNVSVEELA QTRIYWQKEK KMVLTMMSGD MNIWPEYKNR TIFDITNNLS IVILALRPSD EGTYECVVL**X** YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:58), where X is any amino acid other than Lys. In some cases, X is Ala;
VIHVTK EVKEVATLSC GHNVSVEELA QTRIYWQKEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS **X**DPETELYAV SSKLDFNMTT NHSFMCLIKY GHLRVNQTFN WNTTKQEHFP DN (SEQ ID NO:60), where X is any amino acid other than Gln. In some cases, X is Ala;
VIHVTK EVKEVATLSC GHNVSVEELA QTRIYWQKEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS Q**X**PETELYAV SSKLDFNMTT NHSFMCLIKY GHLRVNQTFN WNTTKQEHFP DN (SEQ ID NO:61), where X is any amino acid other than Asp. In some cases, X is Ala;
VIHVTK EVKEVATLSC GHNVSVEE**X**A QTRIYWQKEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:62), where X is any amino acid other than Leu. In some cases, X is Ala;
VIHVTK EVKEVATLSC GHNVSVEELA QTRI**X**WQKEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:63), where X is any amino acid other than Tyr. In some cases, X is Ala;
VIHVTK EVKEVATLSC GHNVSVEELA QTRIYW**X**KEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:64), where X is any amino acid other than Gln. In some cases, X is Ala;
VIHVTK EVKEVATLSC GHNVSVEELA QTRIYWQKEK K**X**VLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:65), where X is any amino acid other than Met. In some cases, X is Ala;
VIHVTK EVKEVATLSC GHNVSVEELA QTRIYWQKEK KM**X**LTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:66), where X is any amino acid other than Val. In some cases, X is Ala;
VIHVTK EVKEVATLSC GHNVSVEELA QTRIYWQKEK KMVLTMMSGD MN**X**WPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:67), where X is any amino acid other than Ile. In some cases, X is Ala;
VIHVTK EVKEVATLSC GHNVSVEELA QTRIYWQKEK KMVLTMMSGD MNIWPE**X**KNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:68), where X is any amino acid other than Tyr. In some cases, X is Ala;
VIHVTK EVKEVATLSC GHNVSVEELA QTRIYWQKEK KMVLTMMSGD MNIWPEYKNR TIF**X**ITNNLS IVILALRPSD EGTYECVVLK YEKDAFKREH LAEVTLSVKA DFPTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:69), where X is any amino acid other than Asp. In some cases, X is Ala;
VIHVTK EVKEVATLSC GHNVSVEELA QTRIYWQKEK KMVLTMMSGD MNIWPEYKNR TIFDITNNLS IVILALRPSD EGTYECVVLK YEKDAFKREH LAEVTLSVKA D**X**PTPSISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:70), where X is any amino acid other than Phe. In some cases, X is Ala;
VIHVTK EVKEVATLSC GHNVSVEELA QTRIYWQKEK KMVLTMMSGD MNIWPEYKNR TIFDITNNLS IVILALRPSD EGTYECVVLK YEKDAFKREH LAEVTLSVKA DFPTPSISDF EIPTSNIRRI ICSTSGGFPE PHLSWLENGE ELNAINTTV**X** QDPETELYAV
SSKLDFNMTT NHSFMCLIKY GHLRVNQTFN WNTTKQEHFP DN (SEQ ID NO:71), where X is any amino acid other than Ser. In some cases, X is Ala; and
VIHVTK EVKEVATLSC GHNVSVEELA QTRIYWQKEK KMVLTMMSGD MNIWPEYKNR TIFDITNNLS IVILALRPSD EGTYECVVLK YEKDAFKREH LAEVTLSVKA DFPT**X**SISDF EIPTSNIRRI ICSTSGGFPE PHLSWLENGE ELNAINTTVS QDPETELYAV SSKLDFNMTT NHSFMCLIKY GHLRVNQTFN WNTTKQEHFP DN (SEQ ID NO:72), where X is any amino acid other than Pro. In some cases, X is Ala.

### CD86 variants

In some cases, a variant immunomodulatory polypeptide present in a TMMP of the present disclosure is a variant CD86 polypeptide. Wild-type CD86 binds to CD28. In some cases, where a TMMP of the present disclosure comprises a variant CD86 polypeptide, a "cognate co-immunomodulatory polypeptide" is a CD28 polypeptide comprising the amino acid sequence of SEQ ID NO:5.

The amino acid sequence of the full ectodomain of a wild-type human CD86 can be as follows:

APLKIQAYFNETADLPCQFANSQNQSLSELVVFWQDQENLVLNEVYLGKEKFDSVHSKYMNRTSFDSDSW
TLRLHNLQIKDKGLYQCIIHHKKPTGMIRIHQMNSELSVLANFSQPEIVPISNITENVYINLTCSSIHGY
PEPKKMSVLLRTKNSTIEYDGIMQKSQDNVTELYDVSISLSVSFPDVTSNMTIFCILETDKTRLLSSPFS
IELEDPQPPPDHIP (SEQ ID NO:8).

The amino acid sequence of the IgV domain of a wild-type human CD86 can be as follows:

APLKIQAYFNETADLPCQFANSQNQSLSELVVFWQDQENLVLNEVYLGKEKFDSVHSKYMNRTSFDSDSW
TLRLHNLQIKDKGLYQCIIHHKKPTGMIRIHQMNSELSVL (SEQ ID NO:9).

In some cases, a variant CD86 polypeptide exhibits reduced binding affinity to CD28, compared to the binding affinity of a CD86 polypeptide comprising the amino acid sequence set forth in SEQ ID NO:8 or SEQ ID NO:9 for CD28. For example, in some cases, a variant CD86 polypeptide binds CD28 with a binding affinity that is at least 10%, at least 15%, at least 20%, at least 25%, at least 30%, at least 35%, at least 40%, at least 45%, at least 50% less, at least 55% less, at least 60% less, at least 65% less, at least 70% less, at least 75% less, at least 80% less, at least 85% less, at least 90% less, at least 95% less, or more than 95% less, than the binding affinity of a CD86 polypeptide comprising the amino acid sequence set forth in SEQ ID NO:8 or SEQ ID NO:9 for CD28 (e.g., a CD28 polypeptide comprising the amino acid sequence set forth in one of SEQ ID NO:5, 6, or 7).

In some cases, a variant CD86 polypeptide has a binding affinity to CD28 that is from 100 nM to 100 µM. As another example, in some cases, a variant CD86 polypeptide of the present disclosure has a binding affinity for CD28 (e.g., a CD28 polypeptide comprising the amino acid sequence set forth in one of SEQ ID NOs:5, 6, or 7) that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

In some cases, a variant CD86 polypeptide has a single amino acid substitution compared to the CD86 amino acid sequence set forth in SEQ ID NO:8. In some cases, a variant CD86 polypeptide has from 2 to 10 amino acid substitutions compared to the CD86 amino acid sequence set forth in SEQ ID NO:8. In some cases, a variant CD86 polypeptide has 2 amino acid substitutions compared to the CD86 amino acid sequence set forth in SEQ ID NO:8. In some cases, a variant CD86 polypeptide has 3 amino acid substitutions compared to the CD86 amino acid sequence set forth in SEQ ID NO:8. In some cases, a variant CD86 polypeptide has 4 amino acid substitutions compared to the CD86 amino acid sequence set forth in SEQ ID NO:8. In some cases, a variant CD86 polypeptide has 5 amino acid substitutions compared to the CD86 amino acid sequence set forth in SEQ ID NO:8. In some cases, a variant CD86 polypeptide has 6 amino acid substitutions compared to the CD86 amino acid sequence set forth in SEQ ID NO:8. In some cases, a variant CD86 polypeptide has 7 amino acid substitutions compared to the CD86 amino acid sequence set forth in SEQ ID NO:8. In some cases, a variant CD86 polypeptide has 8 amino acid substitutions compared to the CD86 amino acid sequence set forth in SEQ ID NO:8. In some cases, a variant CD86 polypeptide has 9 amino acid substitutions compared to the CD86 amino acid sequence set forth in SEQ ID NO:8. In some cases, a variant CD86 polypeptide has 10 amino acid substitutions compared to the CD86 amino acid sequence set forth in SEQ ID NO:8.

In some cases, a variant CD86 polypeptide has a single amino acid substitution compared to the CD86 amino acid sequence set forth in SEQ ID NO:9. In some cases, a variant CD86 polypeptide has from 2 to 10 amino acid substitutions compared to the CD86 amino acid sequence set forth in SEQ ID NO:9. In some cases, a variant CD86 polypeptide has 2 amino acid substitutions compared to the CD86 amino acid sequence set forth in SEQ ID NO:9. In some cases, a variant CD86 polypeptide has 3 amino acid substitutions compared to the CD86 amino acid sequence set forth in SEQ ID NO:9. In some cases, a variant CD86 polypeptide has 4 amino acid substitutions compared to the CD86 amino acid sequence set forth in SEQ ID NO:9. In some cases, a variant CD86 polypeptide has 5 amino acid substitutions compared to the CD86 amino acid sequence set forth in SEQ ID NO:9. In some cases, a variant CD86 polypeptide has 6 amino acid substitutions compared to the CD86 amino acid sequence set forth in SEQ ID NO:9. In some cases, a variant CD86 polypeptide has 7 amino acid substitutions compared to the CD86 amino acid sequence set forth in SEQ ID NO:9. In some cases, a variant CD86 polypeptide has 8 amino acid substitutions compared to the CD86 amino acid sequence set forth in SEQ ID NO:9. In some cases, a variant CD86 polypeptide has 9 amino acid substitutions compared to the CD86 amino acid sequence set forth in SEQ ID NO:9. In some cases, a variant CD86 polypeptide has 10 amino acid substitutions compared to the CD86 amino acid sequence set forth in SEQ ID NO:9.

Suitable CD86 variants include a polypeptide that comprises an amino acid sequence having at least 90%, at least 95%, at least 98%, at least 99%, or 100%, amino acid sequence identity to any one of the following amino acid sequences:
APLKIQAYFNETADLPCQFANSQNQSLSELVVFWQDQENLVLNEVYLGKEKFDSVHSKYM **X**RTSFDSDSWTLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFS IELEDPQPPPDHIP (SEQ ID NO:73), where X is any amino acid other than Asn. In some cases, X is Ala;
APLKIQAYFNETADLPCQFANSQNQSLSELVVFWQDQENLVLNEVYLGKEKFDSVHSKYM NRTSF**X**SDSWTLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFS IELEDPQPPPDHIP (SEQ ID NO:74), where X is any amino acid other than Asp. In some cases, X is Ala;
APLKIQAYFNETADLPCQFANSQNQSLSELVVFWQDQENLVLNEVYLGKEKFDSVHSKYM NRTSFDSDS**X**TLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:75), where X is any amino acid other than Trp. In some cases, X is Ala;
APLKIQAYFNETADLPCQFANSQNQSLSELVVFWQDQENLVLNEVYLGKEKFDSVHSKYM NRTSFDSDSWTLRLHNLQIKDKCLYQCIIH**X**KKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFS IELEDPQPPPDHIP (SEQ ID NO:76), where X is any amino acid other than His. In some cases, X is Ala;
APLKIQAYFNETADLPCQFANSQNQSLSELVVFWQDQENLVLNEVYLGKEKFDSVHSKYM **X**RTSFDSDSWTLRLHNLQIKDKGLYQCIIHHKKPTGMIRIHQMNSELSVL (SEQ ID NO:77), where X is any amino acid other than Asn. In some cases, X is Ala;
APLKIQAYFNETADLPCQFANSQNQSLSELVVFWQDQENLVLNEVYLGKEKFDSVHSKYM NRTSF**X**SDSWTLRLHNLQIKDKGLYQCIIHHKKPTGMIRIHQMNSELSVL (SEQ ID NO:78), where X is any amino acid other than Asp. In some cases, X is Ala;
APLKIQAYFNETADLPCQFANSQNQSLSELVVFWQDQENLVLNEVYLGKEKFDSVHSKYM NRTSFDSDS**X**TLRLHNLQIKDKGLYQCIIHHKKPTGMIRIHQMNSELSVL (SEQ ID NO:79), where X is any amino acid other than Trp. In some cases, X is Ala;
APLKIQAYFNETADLPCQFANSQNQSLSELVVFWQDQENLVLNEVYLGKEKFDSVHSKYM NRTSFDSDSWTLRLHNLQIKDKGLYQCIIH**X**KKPTGMIRIHQMNSELSVL (SEQ ID NO:80), where X is any amino acid other than His. In some cases, X is Ala;
APLKIQAYFNETADLPCQFANSQNQSLSELVVFWQDQENL**X**LNEVYLGKEKFDSVHSKYM NRTSFDSDSWTLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:81), where X is any amino acid other than Val. In some cases, X is Ala;
APLKIQAYFNETADLPCQFANSQNQSLSELVVFWQDQENL**X**LNEVYLGKEKFDSVHSKYM NRTSFDSDSWTLRLHNLQIKDKGLYQCIIHHKKPTGMIRIHQMNSELSVL (SEQ ID NO:82), where X is any amino acid other than Val. In some cases, X is Ala;
APLKIQAYFNETADLPCQFANSQNQSLSELVVFW**X**DQENLVLNEVYLGKEKFDSVHSKYM NRTSFDSDSWTLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:83), where X is any amino acid other than Gln. In some cases, X is Ala;
APLKIQAYFNETADLPCQFANSQNQSLSELVVFW**X**DQENLVLNEVYLGKEKFDSVHSKYM NRTSFDSDSWTLRLHNLQIKDKGLYQCIIHHKKPTGMIRIHQMNSELSVL (SEQ ID NO:84), where X is any amino acid other than Gln. In some cases, X is Ala;
APLKIQAYFNETADLPCQFANSQNQSLSELVV**X**WQDQENLVLNEVYLGKEKFDSVHSKYM NRTSFDSDSWTLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:85), where X is any amino acid other than Phe. In some cases, X is Ala;
APLKIQAYFNETADLPCQFANSQNQSLSELVVXWQDQENLVLNEVYLGKEKFDSVHSKYM NRTSFDSDSWTLRLHNLQIKDKGLYQCIIHHKKPTGMIRIHQMNSELSVL (SEQ ID NO:86), where X is any amino acid other than Phe. In some cases, X is Ala;
APLKIQAYFNETADLPCQFANSQNQSLSELVVFWQDQENLVLNEVYLGKEKFDSVHSKYM NRTSFDSDSWT**X**RLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:87), where X is any amino acid other than Leu. In some cases, X is Ala;
APLKIQAYFNETADLPCQFANSQNQSLSELVVFWQDQENLVLNEVYLGKEKFDSVHSKYM NRTSFDSDSWT**X**RLHNLQIKDKGLYQCIIHHKKPTGMIRIHQMNSELSVL (SEQ ID NO:88), where X is any amino acid other than Leu. In some cases, X is Ala;
APLKIQAYFNETADLPCQFANSQNQSLSELVVFWQDQENLVLNEVYLGKEKFDSVHSK**X**M NRTSFDSDSWTLRLHNLQIKDKGLYQCIIHHKKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:89), where X is any amino acid other than Tyr. In some cases, X is Ala;
APLKIQAYFNETADLPCQFANSQNQSLSELVVFWQDQENLVLNEVYLGKEKFDSVHSK**X**M NRTSFDSDSWTLRLHNLQIKDKGLYQCIIHHKKPTGMIRIHQMNSELSVL (SEQ ID NO:90), where X is any amino acid other than Tyr. In some cases, X is Ala;
APLKIQAYFNETADLPCQFANSQNQSLSELVVFWQDQENLVLNEVYLGKEKFDSVHSKYM **X**RTSFDSDSWTLRLHNLQIKDKGLYQCIIH**X**KKPTGMIRIHQMNSELSVLANFSQPEIVPISNITENVYI NLTCSSIHGYPEPKKMSVLLRTKNSTIEYDGIMQKSQDNVTELYDVSISLSVSFPDVTSNMTIFCILETD KTRLLSSPFSIELEDPQPPPDHIP (SEQ ID NO:91), where the first X is any amino acid other than Asn and the second X is any amino acid other than His. In some cases, the first and the second X are both Ala;
APLKIQAYFNETADLPCQFANSQNQSLSELVVFWQDQENLVLNEVYLGKEKFDSVHSKYM **X**RTSFDSDSWTLRLHNLQIKDKGLYQCIIH**X**KKPTGMIRIHQMNSELSVL (SEQ ID NO:92), where the first X is any amino acid other than Asn and the second X is any amino acid other than His. In some cases, the first and the second X are both Ala;
APLKIQAYFNETADLPCQFANSQNQSLSELVVFWQDQENLVLNEVYLGKEKFDSVHSKYM NRTSF**X₁**SDSWTLRLHNLQIKDKGLYQCIIH**X₂**KKPTGMIRIHQMNSELSVLANFSQPEIVPISNITENVY INLTCSSIHGYPEPKKMSVLLRTKNSTIEYDGIMQKSQDNVTELYDVSISLSVSFPDVTSNMTIFCILET DKTRLLSSPFSIELEDPQPPPDHIP (SEQ ID NO:93), where X₁ is any amino acid other than Asp, and X₂ is any amino acid other than His . In some cases, X₁ is Ala and X₂ is Ala;
APLKIQAYFNETADLPCQFANSQNQSLSELVVFWQDQENLVLNEVYLGKEKFDSVHSKYM NRTSF**X₁**SDSWTLRLHNLQIKDKGLYQCIIH**X₂**KKPTGMIRIHQMNSELSVL (SEQ ID NO:94), where the first X is any amino acid other than Asn and the second X is any amino acid other than His. In some cases, the first and the second X are both Ala;
APLKIQAYFNETADLPCQFANSQNQSLSELVVFWQDQENLVLNEVYLGKEKFDSVHSKYM **X₁**RTSF**X₂**SDSWTLRLHNLQIKDKGLYQCIIH**X₃**KKPTGMIRIHQMNSELSVLANFSQPEIVPISNITENV YINLTCSSIHGYPEPKKMSVLLRTKNSTIEYDGIMQKSQDNVTELYDVSISLSVSFPDVTSNMTIFCILE TDKTRLLSSPFSIELEDPQPPPDHIP (SEQ ID NO:95), where X₁ is any amino acid other than Asn, X₂ is any amino acid other than Asp, and X₃ is any amino acid other than His . In some cases, X₁ is Ala, X₂ is Ala, and X₃ is Ala; and
APLKIQAYFNETADLPCQFANSQNQSLSELVVFWQDQENLVLNEVYLGKEKFDSVHSKYM **X₁**RTSF**X₂**SDSWTLRLHNLQIKDKGLYQCIIH**X₃**KKPTGMIRIHQMNSELSVL (SEQ ID NO:96), where X₁ is any amino acid other than Asn, X₂ is any amino acid other than Asp, and X₃ is any amino acid other than His . In some cases, X₁ is Ala, X₂ is Ala, and X₃ is Ala.

### 4-1BBL variants

In some cases, a variant immunomodulatory polypeptide present in a TMMP of the present disclosure is a variant 4-1BBL polypeptide. Wild-type 4-1BBL binds to 4-1BB (CD137).

A wild-type 4-1BBL amino acid sequence can be as follows: MEYASDASLD PEAPWPPAPR ARACRVLPWA LVAGLLLLLL LAAACAVFLA CPWAVSGARA SPGSAASPRL REGPELSPDD PAGLLDLRQG MFAQLVAQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:10).

In some cases, a variant 4-1BBL polypeptide is a variant of the tumor necrosis factor (TNF) homology domain (THD) of human 4-1BBL.

A wild-type amino acid sequence of the THD of human 4-1BBL can be, e.g., one of SEQ ID NOs:11-13, as follows:

PAGLLDLRQG MFAQLVAQNV LLIDGPLSWY SDPGLAGVSL TGGLSYKEDT
KELVVAKAGV YYVFFQLELR RVVAGEGSGS VSLALHLQPL RSAAGAAALA LTVDLPPASS
EARNSAFGFQ GRLLHLSAGQ RLGVHLHTEA RARHAWQLTQ GATVLGLFRV TPEIPAGLPS
PRSE (SEQ ID NO:11).

D PAGLLDLRQG MFAQLVAQNV LLIDGPLSWY SDPGLAGVSL TGGLSYKEDT
KELVVAKAGV YYVFFQLELR RVVAGEGSGS VSLALHLQPL RSAAGAAALA LTVDLPPASS

EARNSAFGFQ GRLLHLSAGQ RLGVHLHTEA RARHAWQLTQ GATVLGLFRV TPEIPAGLPS
PRSE (SEQ ID NO:12).

D PAGLLDLRQG MFAQLVAQNV LLIDGPLSWY SDPGLAGVSL TGGLSYKEDT
KELVVAKAGV YYVFFQLELR RVVAGEGSGS VSLALHLQPL RSAAGAAALA LTVDLPPASS
EARNSAFGFQ GRLLHLSAGQ RLGVHLHTEA RARHAWQLTQ GATVLGLFRV TPEIPA (SEQ ID
NO:13).

A wild-type 4-1BB amino acid sequence can be as follows: MGNSCYNIVA TLLLVLNFER TRSLQDPCSN CPAGTFCDNN RNQICSPCPP NSFSSAGGQR TCDICRQCKG VFRTRKECSS TSNAECDCTP GFHCLGAGCS MCEQDCKQGQ ELTKKGCKDC CFGTFNDQKR GICRPWTNCS LDGKSVLVNG TKERDVVCGP SPADLSPGAS SVTPPAPARE PGHSPQIISF FLALTSTALL FLLFFLTLRF SVVKRGRKKL LYIFKQPFMR PVQTTQEEDG CSCRFPEEEE GGCEL (SEQ ID NO:14). In some cases, where a TMMP of the present disclosure comprises a variant 4-1BBL polypeptide, a "cognate co-immunomodulatory polypeptide" is a 4-1BB polypeptide comprising the amino acid sequence of SEQ ID NO:14.

In some cases, a variant 4-1BBL polypeptide exhibits reduced binding affinity to 4-1BB, compared to the binding affinity of a 4-1BBL polypeptide comprising the amino acid sequence set forth in one of SEQ ID NOs:10-13. For example, in some cases, a variant 4-1BBL polypeptide of the present disclosure binds 4-1BB with a binding affinity that is at least 10% less, at least 15% less, at least 20% less, at least 25%, at least 30% less, at least 35% less, at least 40% less, at least 45% less, at least 50% less, at least 55% less, at least 60% less, at least 65% less, at least 70% less, at least 75% less, at least 80% less, at least 85% less, at least 90% less, at least 95% less, or more than 95% less, than the binding affinity of a 4-1BBL polypeptide comprising the amino acid sequence set forth in one of SEQ ID NOs:10-13 for a 4-1BB polypeptide (e.g., a 4-1BB polypeptide comprising the amino acid sequence set forth in SEQ ID NO:14), when assayed under the same conditions.

In some cases, a variant 4-1BBL polypeptide has a binding affinity to 4-1BB that is from 100 nM to 100 µM. As another example, in some cases, a variant 4-1BBL polypeptide has a binding affinity for 4-1BB (e.g., a 4-1BB polypeptide comprising the amino acid sequence set forth in SEQ ID NO:14) that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

In some cases, a variant 4-1BBL polypeptide has a single amino acid substitution compared to the 4-1BBL amino acid sequence set forth in one of SEQ ID NOs:10-13. In some cases, a variant 4-1BBL polypeptide has from 2 to 10 amino acid substitutions compared to the 4-1BBL amino acid sequence set forth in one of SEQ ID NOs:10-13. In some cases, a variant 4-1BBL polypeptide has 2 amino acid substitutions compared to the 4-1BBL amino acid sequence set forth in one of SEQ ID NOs:10-13. In some cases, a variant 4-1BBL polypeptide has 3 amino acid substitutions compared to the 4-1BBL amino acid sequence set forth in one of SEQ ID NOs:10-13. In some cases, a variant 4-1BBL polypeptide has 4 amino acid substitutions compared to the 4-1BBL amino acid sequence set forth in one of SEQ ID NOs:10-13. In some cases, a variant 4-1BBL polypeptide has 5 amino acid substitutions compared to the 4-1BBL amino acid sequence set forth in one of SEQ ID NOs:10-13. In some cases, a variant 4-1BBL polypeptide has 6 amino acid substitutions compared to the 4-1BBL amino acid sequence set forth in one of SEQ ID NOs:10-13. In some cases, a variant 4-1BBL polypeptide has 7 amino acid substitutions compared to the 4-1BBL amino acid sequence set forth in one of SEQ ID NOs:10-13. In some cases, a variant 4-1BBL polypeptide has 8 amino acid substitutions compared to the 4-1BBL amino acid sequence set forth in one of SEQ ID NOs:10-13. In some cases, a variant 4-1BBL polypeptide has 9 amino acid substitutions compared to the 4-1BBL amino acid sequence set forth in one of SEQ ID NOs:10-13. In some cases, a variant 4-1BBL polypeptide has 10 amino acid substitutions compared to the 4-1BBL amino acid sequence set forth in one of SEQ ID NOs:10-13.

Suitable 4-1BBL variants include a polypeptide that comprises an amino acid sequence having at least 90%, at least 95%, at least 98%, at least 99%, or 100%, amino acid sequence identity to any one of the following amino acid sequences:
PAGLLDLRQG MFAQLVAQNV LLIDGPLSWY SDPGLAGVSL TGGLSY**X**EDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO: 97), where X is any amino acid other than Lys. In some cases, X is Ala;
PAGLLDLRQG MFAQLVAQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAW**X**LTQ GATVLGLFRV TPEIPAGLPS PRSE (SEQ ID NO: 98), where X is any amino acid other than Gln. In some cases, X is Ala;
PAGLLDLRQG **X**FAQLVAQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO: 99), where X is any amino acid other than Met. In some cases, X is Ala;
PAGLLDLRQG M**X**AQLVAQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO: 100), where X is any amino acid other than Phe. In some cases, X is Ala;
PAGLLDLRQG MFA**X**LVAQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:101), where X is any amino acid other than Gln. In some cases, X is Ala;
PAGLLDLRQG MFAQ**X**VAQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:102), where X is any amino acid other than Leu. In some cases, X is Ala;
PAGLLDLRQG MFAQL**X**AQNV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:103), where X is any amino acid other than Val. In some cases, X is Ala;
PAGLLDLRQG MFAQLVA**X**NV LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:104), where X is any amino acid other than Gln. In some cases, X is Ala;
PAGLLDLRQG MFAQLVAQ**X**V LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:105), where X is any amino acid other than Asn. In some cases, X is Ala;
PAGLLDLRQG MFAQLVAQN**X** LLIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:106), where X is any amino acid other than Val. In some cases, X is Ala;
PAGLLDLRQG MFAQLVAQNV **X**LIDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:107), where X is any amino acid other than Leu. In some cases, X is Ala;
PAGLLDLRQG MFAQLVAQNV L**X**IDGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:108), where X is any amino acid other than Leu. In some cases, X is Ala;
PAGLLDLRQG MFAQLVAQNV LL**X**DGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:109), where X is any amino acid other than Ile. In some cases, X is Ala;
PAGLLDLRQG MFAQLVAQNV LLI**X**GPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:110), where X is any amino acid other than Asp. In some cases, X is Ala;
PAGLLDLRQG MFAQLVAQNV LLID**X**PLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:111), where X is any amino acid other than Gly. In some cases, X is Ala;
PAGLLDLRQG MFAQLVAQNV LLIGG**X**LSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:112), where X is any amino acid other than Pro. In some cases, X is Ala;
PAGLLDLRQG MFAQLVAQNV LLIGGP**X**SWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:113), where X is any amino acid other than Leu. In some cases, X is Ala;
PAGLLDLRQG MFAQLVAQNV LLIGGPL**X**WY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:114), where X is any amino acid other than Ser. In some cases, X is Ala;
PAGLLDLRQG MFAQLVAQNV LLIGGPLS**X**Y SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:115), where X is any amino acid other than Trp. In some cases, X is Ala;
PAGLLDLRQG MFAQLVAQNV LLIGGPLSW**X** SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:116), where X is any amino acid other than Tyr. In some cases, X is Ala;
PAGLLDLRQG MFAQLVAQNV LLIGGPLSWY **X**DPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:117), where X is any amino acid other than Ser. In some cases, X is Ala;
PAGLLDLRQG MFAQLVAQNV LLIGGPLSWY S**X**PGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:118), where X is any amino acid other than Asp. In some cases, X is Ala;
PAGLLDLRQG MFAQLVAQNV LLIGGPLSWY SD**X**GLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:119), where X is any amino acid other than Pro. In some cases, X is Ala;
PAGLLDLRQG MFAQLVAQNV LLIGGPLSWY SDP**X**LAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:120), where X is any amino acid other than Gly. In some cases, X is Ala;
PAGLLDLRQG MFAQLVAQNV LLIGGPLSWY SDPG**X**AGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:121), where X is any amino acid other than Leu. In some cases, X is Ala;
PAGLLDLRQG MFAQLVAQNV LLIGGPLSWY SDPGLA**X**VSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:122), where X is any amino acid other than Gly. In some cases, X is Ala;
PAGLLDLRQG MFAQLVAQNV LLIGGPLSWY SDPGLAG**X**SL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:123), where X is any amino acid other than Val. In some cases, X is Ala;
PAGLLDLRQG MFAQLVAQNV LLIGGPLSWY SDPGLAGV**X**L TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:124), where X is any amino acid other than Ser. In some cases, X is Ala;
PAGLLDLRQG MFAQLVAQNV LLIGGPLSWY SDPGLAGVS**X** TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:125), where X is any amino acid other than Leu. In some cases, X is Ala;
PAGLLDLRQG MFAQLVAQNV LLIGGPLSWY SDPGLAGVSL **X**GGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:126), where X is any amino acid other than Thr. In some cases, X is Ala;
PAGLLDLRQG MFAQLVAQNV LLIGGPLSWY SDPGLAGVSL T**X**GLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:127), where X is any amino acid other than Gly. In some cases, X is Ala;
PAGLLDLRQG MFAQLVAQNV LLIGGPLSWY SDPGLAGVSL TG**X**LSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:128), where X is any amino acid other than Gly. In some cases, X is Ala;
PAGLLDLRQG MFAQLVAQNV LLIGGPLSWY SDPGLAGVSL TGG**X**SYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:129), where X is any amino acid other than Leu. In some cases, X is Ala;
PAGLLDLRQG MFAQLVAQNV LLIGGPLSWY SDPGLAGVSL TGGL**X**YKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:130), where X is any amino acid other than Ser. In some cases, X is Ala;
PAGLLDLRQG MFAQLVAQNV LLIGGPLSWY SDPGLAGVSL TGGLS**X**KEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:131), where X is any amino acid other than Tyr. In some cases, X is Ala;
PAGLLDLRQG MFAQLVAQNV LLIGGPLSWY SDPGLAGVSL TGGLSYK**X**DT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:132), where X is any amino acid other than Glu. In some cases, X is Ala;
PAGLLDLRQG MFAQLVAQNV LLIGGPLSWY SDPGLAGVSL TGGLSYKE**X**T KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:133), where X is any amino acid other than Asp. In some cases, X is Ala;
PAGLLDLRQG MFAQLVAQNV LLIGGPLSWY SDPGLAGVSL TGGLSYKED**X** KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:134), where X is any amino acid other than Thr. In some cases, X is Ala;
PAGLLDLRQG MFAQLVAQNV LLIGGPLSWY SDPGLAGVSL TGGLSYKEDT XELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:135), where X is any amino acid other than Lys. In some cases, X is Ala;
PAGLLDLRQG MFAQLVAQNV LLIGGPLSWY SDPGLAGVSL TGGLSYKEDT K**X**LVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:136), where X is any amino acid other than Glu. In some cases, X is Ala;
PAGLLDLRQG MFAQLVAQNV LLIGGPLSWY SDPGLAGVSL TGGLSYKEDT KELVVAKAGV YYV**X**FQLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:137), where X is any amino acid other than Phe. In some cases, X is Ala;
PAGLLDLRQG MFAQLVAQNV LLIGGPLSWY SDPGLAGVSL TGGLSYKEDT KELVVAKAGV YYVF**X**QLELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:138), where X is any amino acid other than Phe. In some cases, X is Ala;
PAGLLDLRQG MFAQLVAQNV LLIGGPLSWY SDPGLAGVSL TGGLSYKEDT KELVVAKAGV YYVFF**X**LELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:139), where X is any amino acid other than Gln. In some cases, X is Ala;
PAGLLDLRQG MFAQLVAQNV LLIGGPLSWY SDPGLAGVSL TGGLSYKEDT KELVVAKAGV YYVFFQ**X**ELR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:140), where X is any amino acid other than Leu. In some cases, X is Ala;
PAGLLDLRQG MFAQLVAQNV LLIGGPLSWY SDPGLAGVSL TGGLSYKEDT KELVVAKAGV YYVFFQL**X**LR RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:141), where X is any amino acid other than Glu. In some cases, X is Ala;
PAGLLDLRQG MFAQLVAQNV LLIGGPLSWY SDPGLAGVSL TGGLSYKEDT KELVVAKAGV YYVFFQLE**X**R RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:142), where X is any amino acid other than Leu. In some cases, X is Ala;
PAGLLDLRQG MFAQLVAQNV LLIGGPLSWY SDPGLAGVSL TGGLSYKEDT KELVVAKAGV YYVFFQLEL**X** RVVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:143), where X is any amino acid other than Arg. In some cases, X is Ala;
PAGLLDLRQG MFAQLVAQNV LLIGGPLSWY SDPGLAGVSL TGGLSYKEDT KELVVAKAGV YYVFFQLELR **X**VVAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:144), where X is any amino acid other than Arg. In some cases, X is Ala;
PAGLLDLRQG MFAQLVAQNV LLIGGPLSWY SDPGLAGVSL TGGLSYKEDT KELVVAKAGV YYVFFQLELR R**X**VAGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:145), where X is any amino acid other than Val. In some cases, X is Ala;
PAGLLDLRQG MFAQLVAQNV LLIGGPLSWY SDPGLAGVSL TGGLSYKEDT KELVVAKAGV YYVFFQLELR RV**X**AGEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:146), where X is any amino acid other than Val. In some cases, X is Ala;
PAGLLDLRQG MFAQLVAQNV LLIGGPLSWY SDPGLAGVSL TGGLSYKEDT KELVVAKAGV YYVFFQLELR RVV**A**XEGSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:147), where X is any amino acid other than Gly. In some cases, X is Ala;
PAGLLDLRQG MFAQLVAQNV LLIGGPLSWY SDPGLAGVSL TGGLSYKEDT KELVVAKAGV YYVFFQLELR RVVAG**X**GSGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:148), where X is any amino acid other than Glu. In some cases, X is Ala;
PAGLLDLRQG MFAQLVAQNV LLIGGPLSWY SDPGLAGVSL TGGLSYKEDT KELVVAKAGV YYVFFQLELR RVVAGE**X**SGS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:149), where X is any amino acid other than Gly. In some cases, X is Ala;
PAGLLDLRQG MFAQLVAQNV LLIGGPLSWY SDPGLAGVSL TGGLSYKEDT KELVVAKAGV YYVFFQLELR RVVAGEG**X**GS VSLALHLQPL RSAAGAAALA LTVDLPPASS EARNSAFGFQ GRLLHLSAGQ RLGVHLHTEA RARHAWQLTQ GATVLGLFRV TPEIPAGLPS PRSE (SEQ ID NO:150), where X is any amino acid other than Ser. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTV**X**LPPASS EARNSAFGFQ GRLLHLSAGQ RLGVHLHTEA RARHAWQLTQ GATVLGLFRV TPEIPAGLPS PRSE (SEQ ID NO:151), where X is any amino acid other than Asp. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVD**X**PPASS EARNSAFGFQ GRLLHLSAGQ RLGVHLHTEA RARHAWQLTQ GATVLGLFRV TPEIPAGLPS PRSE (SEQ ID NO:152), where X is any amino acid other than Leu. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDL**X**PASS EARNSAFGFQ GRLLHLSAGQ RLGVHLHTEA RARHAWQLTQ GATVLGLFRV TPEIPAGLPS PRSE (SEQ ID NO:153), where X is any amino acid other than Pro. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPA**X**S EARNSAFGFQ GRLLHLSAGQ RLGVHLHTEA RARHAWQLTQ GATVLGLFRV TPEIPAGLPS PRSE (SEQ ID NO:154), where X is any amino acid other than Ser. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPAS**X** EARNSAFGFQ GRLLHLSAGQ RLGVHLHTEA RARHAWQLTQ GATVLGLFRV TPEIPAGLPS PRSE (SEQ ID NO:155), where X is any amino acid other than Ser. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS **X**ARNSAFGFQ GRLLHLSAGQ RLGVHLHTEA RARHAWQLTQ GATVLGLFRV TPEIPAGLPS PRSE (SEQ ID NO:156), where X is any amino acid other than Glu. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EA**X**NSAFGFQ GRLLHLSAGQ RLGVHLHTEA RARHAWQLTQ GATVLGLFRV TPEIPAGLPS PRSE (SEQ ID NO:157), where X is any amino acid other than Arg. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EAR**X**SAFGFQ GRLLHLSAGQ RLGVHLHTEA RARHAWQLTQ GATVLGLFRV TPEIPAGLPS PRSE (SEQ ID NO:158), where X is any amino acid other than Asn. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNXAFGFQ GRLLHLSAGQ RLGVHLHTEA RARHAWQLTQ GATVLGLFRV TPEIPAGLPS PRSE (SEQ ID NO:159), where X is any amino acid other than Ser. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSA**X**GFQ GRLLHLSAGQ RLGVHLHTEA RARHAWQLTQ GATVLGLFRV TPEIPAGLPS PRSE (SEQ ID NO:160), where X is any amino acid other than Phe. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAG**X** RLGVHLHTEA RARHAWQLTQ GATVLGLFRV TPEIPAGLPS PRSE (SEQ ID NO:161), where X is any amino acid other than Gln. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ **X**LGVHLHTEA RARHAWQLTQ GATVLGLFRV TPEIPAGLPS PRSE (SEQ ID NO:162), where X is any amino acid other than Arg. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ R**X**GVHLHTEA RARHAWQLTQ GATVLGLFRV TPEIPAGLPS PRSE (SEQ ID NO:163), where X is any amino acid other than Leu. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RL**X**VHLHTEA RARHAWQLTQ GATVLGLFRV TPEIPAGLPS PRSE (SEQ ID NO:164), where X is any amino acid other than Gly. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLG**X**HLHTEA RARHAWQLTQ GATVLGLFRV TPEIPAGLPS PRSE (SEQ ID NO:165), where X is any amino acid other than Val. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGV**X**LHTEA RARHAWQLTQ GATVLGLFRV TPEIPAGLPS PRSE (SEQ ID NO:166), where X is any amino acid other than His. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVH**X**HTEA RARHAWQLTQ GATVLGLFRV TPEIPAGLPS PRSE (SEQ ID NO:167), where X is any amino acid other than Leu. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHL**X**TEA RARHAWQLTQ GATVLGLFRV TPEIPAGLPS PRSE (SEQ ID NO:168), where X is any amino acid other than His. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLH**X**EA RARHAWQLTQ GATVLGLFRV TPEIPAGLPS PRSE (SEQ ID NO:169), where X is any amino acid other than Thr. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHT**X**A RARHAWQLTQ GATVLGLFRV TPEIPAGLPS PRSE (SEQ ID NO:170), where X is any amino acid other than Glu. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA **X**ARHAWQLTQ GATVLGLFRV TPEIPAGLPS PRSE (SEQ ID NO:171), where X is any amino acid other than Arg. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RA**X**HAWQLTQ GATVLGLFRV TPEIPAGLPS PRSE (SEQ ID NO:172), where X is any amino acid other than Arg. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RAR**X**AWQLTQ GATVLGLFRV TPEIPAGLPS PRSE (SEQ ID NO:173), where X is any amino acid other than His. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHA**X**QLTQ GATVLGLFRV TPEIPAGLPS PRSE (SEQ ID NO:174), where X is any amino acid other than Trp. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQ**X**TQ GATVLGLFRV TPEIPAGLPS PRSE (SEQ ID NO:175), where X is any amino acid other than Leu. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQL**X**Q GATVLGLFRV TPEIPAGLPS PRSE (SEQ ID NO:176), where X is any amino acid other than Thr. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLT**X** GATVLGLFRV TPEIPAGLPS PRSE (SEQ ID NO:177), where X is any amino acid other than Gln. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ **X**ATVLGLFRV TPEIPAGLPS PRSE (SEQ ID NO:178), where X is any amino acid other than Gly. In some cases, X is Ala;
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GA**X**VLGLFRV TPEIPAGLPS PRSE (SEQ ID NO:179), where X is any amino acid other than Thr. In some cases, X is Ala; and
PAGLLDLRQG MFAQLVAQNV LLIGGPLSWY SDPGLAGVSL TGGLSYKEDT KELVVAKAGV YYVFFQLELR RVVAGEGSGS VSLALHLQPL RSAAGAAALA LTVDLPPASS EARNSAFGFQ GRLLHLSAGQ RLGVHLHTEA RARHAWQLTQ GAT**X**LGLFRV TPEIPAGLPS PRSE (SEQ ID NO:180), where X is any amino acid other than Val. In some cases, X is Ala.

### IL-2 variants

In some cases, a variant immunomodulatory polypeptide present in a TMMP of the present disclosure is a variant IL-2 polypeptide. Wild-type IL-2 binds to IL-2 receptor (IL-2R), i.e., a heterotrimeric polypeptide comprising IL-2Rα, IL-2Rβ, and IL-2Rγ.

A wild-type IL-2 amino acid sequence can be as follows: APTSSSTKKT QLQL**EH**LLL**D** LQMILNGINN YKNPKLTRML T**F**KF**Y**MPKKA TELKHLQCLEEELKPLEEVL NLAQSKNFHL RPRDLISNIN VIVLELKGSE TTFMCEYADE TATIVEFLNRWITFCQSIIS TLT (SEQ ID NO: 15) .

Wild-type IL2 binds to an IL2 receptor (IL2R) on the surface of a cell. An IL2 receptor is in some cases a heterotrimeric polypeptide comprising an alpha chain (IL-2Rα; also referred to as CD25), a beta chain (IL-2Rβ; also referred to as CD122: and a gamma chain (IL-2Rγ; also referred to as CD132). Amino acid sequences of human IL-2Rα, IL2Rβ, and IL-2Rγ can be as follows.

Human IL-2Rα: ELCDDDPPE IPHATFKAMA YKEGTMLNCE CKRGFRRIKS
GSLYMLCTGN SSHSSWDNQC QCTSSATRNT TKQVTPQPEE QKERKTTEMQ SPMQPVDQAS
LPGHCREPPP WENEATERIY HFVVGQMVYY QCVQGYRALH RGPAESVCKM THGKTRWTQP

QLICTGEMET SQFPGEEKPQ ASPEGRPESE TSCLVTTTDF QIQTEMAATM ETSIFTTEYQ
VAVAGCVFLL ISVLLLSGLT WQRRQRKSRR TI (SEQ ID NO:16).

Human IL-2Rβ: VNG TSQFTCFYNS RANISCVWSQ DGALQDTSCQ
VHAWPDRRRW NQTCELLPVS QASWACNLIL GAPDSQKLTT VDIVTLRVLC REGVRWRVMA
IQDFKPFENL RLMAPISLQV VHVETHRCNI SWEISQASHY FERHLEFEAR TLSPGHTWEE
APLLTLKQKQ EWICLETLTP DTQYEFQVRV KPLQGEFTTW SPWSQPLAFR TKPAALGKDT
IPWLGHLLVG LSGAFGFIIL VYLLINCRNT GPWLKKVLKC NTPDPSKFFS QLSSEHGGDV
QKWLSSPFPS SSFSPGGLAP EISPLEVLER DKVTQLLLQQ DKVPEPASLS SNHSLTSCFT
NQGYFFFHLP DALEIEACQV YFTYDPYSEE DPDEGVAGAP TGSSPQPLQP LSGEDDAYCT
FPSRDDLLLF SPSLLGGPSP PSTAPGGSGA GEERMPPSLQ ERVPRDWDPQ PLGPPTPGVP
DLVDFQPPPE LVLREAGEEV PDAGPREGVS FPWSRPPGQG EFRALNARLP LNTDAYLSLQ
ELQGQDPTHL V (SEQ ID NO:17).

Human IL-2Rγ: LNTTILTP NGNEDTTADF FLTTMPTDSL SVSTLPLPEV
QCFVFNVEYM NCTWNSSSEP QPTNLTLHYW YKNSDNDKVQ KCSHYLFSEE ITSGCQLQKK
EIHLYQTFVV QLQDPREPRR QATQMLKLQN LVIPWAPENL TLHKLSESQL ELNWNNRFLN
HCLEHLVQYR TDWDHSWTEQ SVDYRHKFSL PSVDGQKRYT FRVRSRFNPL CGSAQHWSEW
SHPIHWGSNT SKENPFLFAL EAVVISVGSM GLIISLLCVY FWLERTMPRI PTLKNLEDLV
TEYHGNFSAW SGVSKGLAES LQPDYSERLC LVSEIPPKGG ALGEGPGASP CNQHSPYWAP
PCYTLKPET (SEQ ID NO:18).

In some cases, where a TMMP of the present disclosure comprises a variant IL-2 polypeptide, a "cognate co-immunomodulatory polypeptide" is an IL-2R comprising polypeptides comprising the amino acid sequences of SEQ ID NO:16, 17, and 18.

In some cases, a variant IL-2 polypeptide exhibits reduced binding affinity to IL-2R, compared to the binding affinity of a IL-2 polypeptide comprising the amino acid sequence set forth in SEQ ID NO:15. For example, in some cases, a variant IL-2 polypeptide binds IL-2R with a binding affinity that is at least 10% less, at least 15% less, at least 20% less, at least 25%, at least 30% less, at least 35% less, at least 40% less, at least 45% less, at least 50% less, at least 55% less, at least 60% less, at least 65% less, at least 70% less, at least 75% less, at least 80% less, at least 85% less, at least 90% less, at least 95% less, or more than 95% less, than the binding affinity of an IL-2 polypeptide comprising the amino acid sequence set forth in SEQ ID NO:15 for an IL-2R (e.g., an IL-2R comprising polypeptides comprising the amino acid sequence set forth in SEQ ID NOs:16-18), when assayed under the same conditions.

In some cases, a variant IL-2 polypeptide has a binding affinity to IL-2R that is from 100 nM to 100 µM. As another example, in some cases, a variant IL-2 polypeptide has a binding affinity for IL-2R (e.g., an IL-2R comprising polypeptides comprising the amino acid sequence set forth in SEQ ID NOs:16-18) that is from about 100 nM to 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 500 nM, from about 500 nM to about 600 nM, from about 600 nM to about 700 nM, from about 700 nM to about 800 nM, from about 800 nM to about 900 nM, from about 900 nM to about 1 µM, to about 1 µM to about 5 µM, from about 5 µM to about 10 µM, from about 10 µM to about 15 µM, from about 15 µM to about 20 µM, from about 20 µM to about 25 µM, from about 25 µM to about 50 µM, from about 50 µM to about 75 µM, or from about 75 µM to about 100 µM.

In some cases, a variant IL-2 polypeptide has a single amino acid substitution compared to the IL-2 amino acid sequence set forth in SEQ ID NO:15. In some cases, a variant IL-2 polypeptide has from 2 to 10 amino acid substitutions compared to the IL-2 amino acid sequence set forth in SEQ ID NO:15. In some cases, a variant IL-2 polypeptide has 2 amino acid substitutions compared to the IL-2 amino acid sequence set forth in SEQ ID NO:15. In some cases, a variant IL-2 polypeptide has 3 amino acid substitutions compared to the IL-2 amino acid sequence set forth in SEQ ID NO:15. In some cases, a variant IL-2 polypeptide has 4 amino acid substitutions compared to the IL-2 amino acid sequence set forth in SEQ ID NO:15. In some cases, a variant IL-2 polypeptide has 5 amino acid substitutions compared to the IL-2 amino acid sequence set forth in SEQ ID NO:15. In some cases, a variant IL-2 polypeptide has 6 amino acid substitutions compared to the IL-2 amino acid sequence set forth in SEQ ID NO:15. In some cases, a variant IL-2 polypeptide has 7 amino acid substitutions compared to the IL-2 amino acid sequence set forth in SEQ ID NO:15. In some cases, a variant IL-2 polypeptide has 8 amino acid substitutions compared to the IL-2 amino acid sequence set forth in SEQ ID NO:15. In some cases, a variant IL-2 polypeptide has 9 amino acid substitutions compared to the IL-2 amino acid sequence set forth in SEQ ID NO:15. In some cases, a variant IL-2 polypeptide has 10 amino acid substitutions compared to the IL-2 amino acid sequence set forth in SEQ ID NO:15.

Suitable IL-2 variants include a polypeptide that comprises an amino acid sequence having at least 90%, at least 95%, at least 98%, at least 99%, or 100%, amino acid sequence identity to any one of the following amino acid sequences:
APTSSSTKKT QLQLEHLLLD LQMILNGINN YKNPKLTRML T**X**KFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:181), where X is any amino acid other than Phe. In some cases, X is Ala. In some cases, X is Met. In some cases, X is Pro. In some cases, X is Ser. In some cases, X is Thr. In some cases, X is Trp. In some cases, X is Tyr. In some cases, X is Val. In some cases, X is His;
APTSSSTKKT QLQLEHLLL**X** LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:182), where X is any amino acid other than Asp. In some cases, X is Ala;
APTSSSTKKT QLQL**X**HLLLD LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:183), where X is any amino acid other than Glu. In some cases, X is Ala.
APTSSSTKKT QLQLE**X**LLLD LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:184), where X is any amino acid other than His. In some cases, X is Ala. In some cases, X is Thr. In some cases, X is Asn. In some cases, X is Cys. In some cases, X is Gln. In some cases, X is Met. In some cases, X is Val. In some cases, X is Trp;
APTSSSTKKT QLQLE**X**LLLD LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:185), where X is any amino acid other than His. In some cases, X is Ala. In some cases, X is Arg. In some cases, X is Asn. In some cases, X is Asp. In some cases, X is Cys. In some cases, X is Glu. In some cases, X is Gln. In some cases, X is Gly. In some cases, X is Ile. I n some cases, X is Lys. In some cases, X is Leu. In some cases, X is Met. In some cases, X is Phe. In some cases, X is Pro. In some cases, X is Ser. In some cases, X is Thr. In some cases, X is Tyr. In some cases, X is Trp. In some cases, X is Val;
APTSSSTKKT QLQLEHLLLD LQMILNGINN YKNPKLTRML TFKFXMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:186), where X is any amino acid other than Tyr. In some cases, X is Ala;
APTSSSTKKT QLQLEHLLLD LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITFC**X**SIIS TLT (SEQ ID NO:187), where X is any amino acid other than Gln. In some cases, X is Ala;
APTSSSTKKT QLQLE**X₁**LLLD LQMILNGINN YKNPKLTRML T**X₂**KFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:188), where X₁ is any amino acid other than His, and where X₂ is any amino acid other than Phe. In some cases, X₁ is Ala. In some cases, X₂ is Ala. In some cases, X₁ is Ala; and X₂ is Ala. In some cases, X₁ is Thr; and X₂ is Ala;
APTSSSTKKT QLQLEHLLL**X₁** LQMILNGINN YKNPKLTRML T**X₂**KFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:189), where X₁ is any amino acid other than Asp; and where X₂ is any amino acid other than Phe. In some cases, X₁ is Ala. In some cases, X₂ is Ala. In some cases, X₁ is Ala; and X₂ is Ala;
APTSSSTKKT QLQL**X₁**HLLL**X₂** LQMILNGINN YKNPKLTRML T**X₃**KFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:190), where X₁ is any amino acid other than Glu; where X₂ is any amino acid other than Asp; and where X₃ is any amino acid other than Phe. In some cases, X₁ is Ala. In some cases, X₂ is Ala. In some cases, X₃ is Ala. In some cases, X₁ is Ala; X₂ is Ala; and X₃ is Ala;
APTSSSTKKT QLQLE**X₁**LLL**X₂** LQMILNGINN YKNPKLTRML T**X₃**KFYMPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:191), where X₁ is any amino acid other than His; where X₂ is any amino acid other than Asp; and where X₃ is any amino acid other than Phe. In some cases, X₁ is Ala. In some cases, X₂ is Ala. In some cases, X₃ is Ala. In some cases, X₁ is Ala; X₂ is Ala; and X₃ is Ala;
APTSSSTKKT QLQLEHLLL**X₁** LQMILNGINN YKNPKLTRML T**X₂**KFYMPKKA TELKHLQCLE EELKPLEEVL NLAQSKNFHL RPRDLISNIN VIVLELKGSE TTFMCEYADE TATIVEFLNR WITFC**X₃**SIIS TLT (SEQ ID NO:192), where X₁ is any amino acid other than Asp; where X₂ is any amino acid other than Phe; and where X₃ is any amino acid other than Gln. In some cases, X₁ is Ala. In some cases, X₂ is Ala. In some cases, X₃ is Ala. In some cases, X₁ is Ala; X₂ is Ala; and X₃ is Ala;
APTSSSTKKT QLQLEHLLL**X₁** LQMILNGINN YKNPKLTRML T**X₂**KF**X₃**MPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:193), where X₁ is any amino acid other than Asp; where X₂ is any amino acid other than Phe; and where X₃ is any amino acid other than Tyr. In some cases, X₁ is Ala. In some cases, X₂ is Ala. In some cases, X₃ is Ala. In some cases, X₁ is Ala; X₂ is Ala; and X₃ is Ala;
APTSSSTKKT QLQLE**X₁**LLL**X₂** LQMILNGINN YKNPKLTRML T**X₃**KF**X₄**MPKKA TELKHLQCLE EELKPLEEVL NLAQSKNFHL RPRDLISNIN VIVLELKGSE TTFMCEYADE TATIVEFLNR WITFCQSIIS TLT (SEQ ID NO:194), where X₁ is any amino acid other than His; where X₂ is any amino acid other than Asp; where X₃ is any amino acid other than Phe; and where X₄ is any amino acid other than Tyr. In some cases, X₁ is Ala. In some cases, X₂ is Ala. In some cases, X₃ is Ala. In some cases, X₄ is Ala. In some cases, X₁ is Ala; X₂ is Ala; X₃ is Ala; and X₄ is Ala;
APTSSSTKKT QLQLEHLLL**X₁** LQMILNGINN YKNPKLTRML T**X₂**KF**X₃**MPKKA TELKHLQCLE EELKPLEEVL NLAQSKNFHL RPRDLISNIN VIVLELKGSE TTFMCEYADE TATIVEFLNR WITFC**X₄**SIIS TLT (SEQ ID NO:195), where X₁ is any amino acid other than Asp; where X₂ is any amino acid other than Phe; where X₃ is any amino acid other than Tyr; and where X₄ is any amino acid other than Gln. In some cases, X₁ is Ala. In some cases, X₂ is Ala. In some cases, X₃ is Ala. In some cases, X₄ is Ala. In some cases, X₁ is Ala; X₂ is Ala; X₃ is Ala; and X₄ is Ala;
APTSSSTKKT QLQLE**X₁**LLL**X₂** LQMILNGINN YKNPKLTRML T**X₃**KF**X₄**MPKKA TELKHLQCLE EELKPLEEVL NLAQSKNFHL RPRDLISNIN VIVLELKGSE TTFMCEYADE TATIVEFLNR WITFC**X₅**SIIS TLT (SEQ ID NO:196), where X₁ is any amino acid other than His; where X₂ is any amino acid other than Asp; where X₃ is any amino acid other than Phe; where X₄ is any amino acid other than Tyr; and where X₅ is any amino acid other than Gln. In some cases, X₁ is Ala. In some cases, X₂ is Ala. In some cases, X₃ is Ala. In some cases, X₄ is Ala. In some cases, X₅ is Ala. In some cases, X₁ is Ala; X₂ is Ala; X₃ is Ala; X₄ is Ala; X₅ is Ala; and
APTSSSTKKT QLQLE**X₁**LLLD LQMILNGINN YKNPKLTRML T**X₂**KFYMPKKA TELKHLQCLE EELKPLEEVL NLAQSKNFHL RPRDLISNIN VIVLELKGSE TTFMCEYADE TATIVEFLNR WITFC**X₃**SIIS TLT (SEQ ID NO:197), where X₁ is any amino acid other than His; where X₂ is any amino acid other than Phe; and where X₃ is any amino acid other than Gln. In some cases, X₁ is Ala. In some cases, X₂ is Ala. In some cases, X₃ is Ala. In some cases, X₁ is Ala; X₂ is Ala; and X₃ is Ala.

### Additional polypeptides

A polypeptide chain of a TMMP of the present disclosure can include one or more polypeptides in addition to those described above. Suitable additional polypeptides include epitope tags and affinity domains. The one or more additional polypeptide can be included at the N-terminus of a polypeptide chain of a TMMP, at the C-terminus of a polypeptide chain of a TMMP, or internally within a polypeptide chain of a TMMP.

### Epitope tag

Suitable epitope tags include, but are not limited to, hemagglutinin (HA; e.g., YPYDVPDYA (SEQ ID NO:271); FLAG (e.g., DYKDDDDK (SEQ ID NO:272); c-myc (e.g., EQKLISEEDL; SEQ ID NO:273), and the like.

### Affinity domain

Affinity domains include peptide sequences that can interact with a binding partner, e.g., such as one immobilized on a solid support, useful for identification or purification. DNA sequences encoding multiple consecutive single amino acids, such as histidine, when fused to the expressed protein, may be used for one-step purification of the recombinant protein by high affinity binding to a resin column, such as nickel sepharose. Exemplary affinity domains include His5 (HHHHH) (SEQ ID NO:271), HisX6 (HHHHHH) (SEQ ID NO:275), C-myc (EQKLISEEDL) (SEQ ID NO:276), Flag (DYKDDDDK) (SEQ ID NO:277), StrepTag (WSHPQFEK) (SEQ ID NO:278), hemagglutinin, e.g., HA Tag (YPYDVPDYA) (SEQ ID NO:279), glutathione-S-transferase (GST), thioredoxin, cellulose binding domain, RYIRS (SEQ ID NO:280), Phe-His-His-Thr (SEQ ID NO:281), chitin binding domain, S-peptide, T7 peptide, SH2 domain, C-end RNA tag, WEAAAREACCRECCARA (SEQ ID NO:282), metal binding domains, e.g., zinc binding domains or calcium binding domains such as those from calcium-binding proteins, e.g., calmodulin, troponin C, calcineurin B, myosin light chain, recoverin, S-modulin, visinin, VILIP, neurocalcin, hippocalcin, frequenin, caltractin, calpain large-subunit, S100 proteins, parvalbumin, calbindin D9K, calbindin D28K, and calretinin, inteins, biotin, streptavidin, MyoD, Id, leucine zipper sequences, and maltose binding protein.

### Drug conjugates

A polypeptide chain of a TMMP of the present disclosure can comprise a small molecule drug linked (e.g., covalently attached) to the polypeptide chain. For example, where a TMMP of the present disclosure comprises an Fc polypeptide, the Fc polypeptide can comprise a covalently linked small molecule drug. In some cases, the small molecule drug is a cancer chemotherapeutic agent, e.g., a cytotoxic agent. A polypeptide chain of a TMMP of the present disclosure can comprise a cytotoxic agent linked (e.g., covalently attached) to the polypeptide chain. For example, where a TMMP of the present disclosure comprises an Fc polypeptide, the Fc polypeptide can comprise a covalently linked cytotoxic agent. Cytotoxic agents include prodrugs.

A drug (e.g., a cancer chemotherapeutic agent) can be linked directly or indirectly to a polypeptide chain of a TMMP of the present disclosure. For example, where a TMMP of the present disclosure comprises an Fc polypeptide, a drug (e.g., a cancer chemotherapeutic agent) can be linked directly or indirectly to the Fc polypeptide. Direct linkage can involve linkage directly to an amino acid side chain. Indirect linkage can be linkage via a linker. A drug (e.g., a cancer chemotherapeutic agent) can be linked to a polypeptide chain (e.g., an Fc polypeptide) of a TMMP of the present disclosure via a thioether bond, an amide bond, a carbamate bond, a disulfide bond, or an ether bond.

Linkers include cleavable linkers and non-cleavable linkers. In some cases, the linker is a protease-cleavable linker. Suitable linkers include, e.g., peptides (e.g., from 2 to 10 amino acids in length; e.g., 2, 3, 4, 5, 6, 7, 8, 9, or 10 amino acids in length), alkyl chains, poly(ethylene glycol), disulfide groups, thioether groups, acid labile groups, photolabile groups, peptidase labile groups, and esterase labile groups. Non-limiting example of suitable linkers are: i) N-succinimidyl-[(N-maleimidopropionamido)-tetraethyleneglycol]ester (NHS-PEG4-maleimide); ii) N-succinimidyl 4-(2-pyridyldithio)butanoate (SPDB); N-succinimidyl 4-(2-pyridyldithio)2-sulfobutanoate (sulfo-SPDB); N-succinimidyl 4-(2-pyridyldithio) pentanoate (SPP); N-succinimidyl-4-(N-maleimidomethyl)-cyclohexane-1-carboxy-(6-amidocaproate) (LC-SMCC); κ-maleimidoundecanoic acid N-succinimidyl ester (KMUA); γ-maleimide butyric acid N-succinimidyl ester (GMBS); ε-maleimidocaproic acid N-hydroxysuccinimide ester (EMCS); m-maleimide benzoyl-N-hydroxysuccinimide ester (MBS); N-(α-maleimidoacetoxy)-succinimide ester (AMAS); succinimidyl-6-(β-maleimidopropionamide)hexanoate (SMPH); N-succinimidyl 4-(p-maleimidophenyl)butyrate (SMPB); N-(p-maleimidophenyl)isocyanate (PMPI); N-succinimidyl 4(2-pyridylthio)pentanoate (SPP); N-succinimidyl(4-iodo-acetyl)aminobenzoate (SIAB); 6-maleimidocaproyl (MC); maleimidopropanoyl (MP); p-aminobenzyloxycarbonyl (PAB); N-succinimidyl 4-(maleimidomethyl)cyclohexanecarboxylate (SMCC); N-succinimidyl-4-(N-maleimidomethyl)-cyclohexane-1-carboxy-(6-amidocaproate), a "long chain" analog of SMCC (LC-SMCC); 3-maleimidopropanoic acid N-succinimidyl ester (BMPS); N-succinimidyl iodoacetate (SIA); N-succinimidyl bromoacetate (SBA); and N-succinimidyl 3-(bromoacetamido)propionate (SBAP).

A polypeptide (e.g., an Fc polypeptide) can be modified with crosslinking reagents such as succinimidyl 4-(N-maleimidomethyl)-cyclohexane-1-carboxylate (SMCC), sulfo-SMCC, maleimidobenzoyl-N-hydroxysuccinimide ester (MBS), sulfo-MBS or succinimidyl-iodoacetate, as described in the literature, to introduce 1-10 reactive groups. The modified Fc polypeptide is then reacted with a thiol-containing cytotoxic agent to produce a conjugate.

For example, where a TMMP of the present disclosure comprises an Fc polypeptide, the polypeptide chain comprising the Fc polypeptide can be of the formula (A)-(L)-(C), where (A) is the polypeptide chain comprising the Fc polypeptide; where (L), if present, is a linker; and where (C) is a cytotoxic agent. (L), if present, links (A) to (C). In some cases, the polypeptide chain comprising the Fc polypeptide can comprise more than one cytotoxic agent (e.g., 2, 3, 4, or 5, or more than 5, cytotoxic agents).

Suitable drugs include, e.g., rapamycin. Suitable drugs include, e.g., retinoids, such as all-trans retinoic acid (ATRA); vitamin D3; a vitamin D3 analog; and the like. As noted above, in some cases, a drug is a cytotoxic agent. Cytotoxic agents are known in the art. A suitable cytotoxic agent can be any compound that results in the death of a cell, or induces cell death, or in some manner decreases cell viability, and includes, for example, maytansinoids and maytansinoid analogs, benzodiazepines, taxoids, CC-1065 and CC-1065 analogs, duocarmycins and duocarmycin analogs, enediynes, such as calicheamicins, dolastatin and dolastatin analogs including auristatins, tomaymycin derivatives, leptomycin derivatives, methotrexate, cisplatin, carboplatin, daunorubicin, doxorubicin, vincristine, vinblastine, melphalan, mitomycin C, chlorambucil and morpholino doxorubicin.

For example, in some cases, the cytotoxic agent is a compound that inhibits microtubule formation in eukaryotic cells. Such agents include, e.g., maytansinoid, benzodiazepine, taxoid, CC-1065, duocarmycin, a duocarmycin analog, calicheamicin, dolastatin, a dolastatin analog, auristatin, tomaymycin, and leptomycin, or a pro-drug of any one of the foregoing. Maytansinoid compounds include, e.g., N(2')-deacetyl-N(2')-(3-mercapto-1-oxopropyl)-maytansine (DM1); N(2')-deacetyl-N(2')-(4-mercapto-1-oxopentyl)-maytansine (DM3); and N(2')-deacetyl-N2-(4-mercapto-4-methyl-1-oxopentyl)-maytansine (DM4). Benzodiazepines include, e.g., indolinobenzodiazepines and oxazolidinobenzodiazepines.

Cytotoxic agents include taxol; cytochalasin B; gramicidin D; ethidium bromide; emetine; mitomycin; etoposide; tenoposide; vincristine; vinblastine; colchicin; doxorubicin; daunorubicin; dihydroxy anthracin dione; maytansine or an analog or derivative thereof; an auristatin or a functional peptide analog or derivative thereof; dolastatin 10 or 15 or an analogue thereof; irinotecan or an analogue thereof; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite; 6 mercaptopurine; 6 thioguanine; cytarabine; fludarabin; 5 fluorouracil; decarbazine; hydroxyurea; asparaginase; gemcitabine; cladribine; an alkylating agent; a platinum derivative; duocarmycin A; duocarmycin SA; rachelmycin (CC-1065) or an analog or derivative thereof; an antibiotic; pyrrolo[2,1-c][1,4]-benzodiazepines (PDB); diphtheria toxin; ricin toxin; cholera toxin; a Shiga-like toxin; LT toxin; C3 toxin; Shiga toxin; pertussis toxin; tetanus toxin; soybean Bowman-Birk protease inhibitor; Pseudomonas exotoxin; alorin; saporin; modeccin; gelanin; abrin A chain; modeccin A chain; alpha-sarcin; *Aleurites fordii* proteins; dianthin proteins; *Phytolacca americana* proteins; momordica charantia inhibitor; curcin; crotin; sapaonaria officinalis inhibitor; gelonin; mitogellin; restrictocin; phenomycin; enomycin toxins; ribonuclease (RNase); DNase I; Staphylococcal enterotoxin A; pokeweed antiviral protein; diphtherin toxin; and Pseudomonas endotoxin.

### Exemplary TMMPs

A TMMP of the present disclosure comprises at least one heterodimer comprising: a) a first polypeptide comprising: i) a WT-1 peptide epitope; and ii) first MHC polypeptide; b) a second polypeptide comprising a second MHC polypeptide, and c) at least one immunomodulatory polypeptide, where the first and/or the second polypeptide comprises the immunomodulatory polypeptide. Thus, in some cases, a TMMP of the present disclosure comprises at least one heterodimer comprising: a) a first polypeptide comprising: i) a WT-1 peptide epitope; ii) first MHC polypeptide; and iii) at least one immunomodulatory polypeptide; and b) a second polypeptide comprising a second MHC polypeptide. In other instances, a TMMP of the present disclosure comprises at least one heterodimer comprising: a) a first polypeptide comprising: i) a WT-1 peptide epitope; and ii) first MHC polypeptide; and b) a second polypeptide comprising: i) a second MHC polypeptide; and ii) at least one immunomodulatory polypeptide. In some cases, a TMMP of the present disclosure comprises at least one heterodimer comprising: a) a first polypeptide comprising: i) a WT-1 peptide epitope; ii) first MHC polypeptide; and iii) at least one immunomodulatory polypeptide; and b) a second polypeptide comprising: i) a second MHC polypeptide; and ii) at least one immunomodulatory polypeptide. In some cases, the at least one immunomodulatory polypeptide is a wild-type immunomodulatory polypeptide. In other cases, the at least one immunomodulatory polypeptide is a variant immunomodulatory polypeptide that exhibits reduced affinity for a co-immunomodulatory polypeptide, compared to the affinity of a corresponding wild-type immunomodulatory polypeptide for the co-immunomodulatory polypeptide. In some cases, a TMMP of the present disclosure comprises two immunomodulatory polypeptides, where the two immunomodulatory polypeptides have the same amino acid sequence.

In some cases, a TMMP of the present disclosure comprises: a) a first polypeptide comprising, in order from N-terminus to C-terminus: i) a WT-1 peptide epitope; ii) a first MHC polypeptide; and iii) at least one immunomodulatory polypeptide; and b) a second polypeptide comprising, in order from N-terminus to C-terminus: i) a second MHC polypeptide; and ii) an Ig Fc polypeptide. In some cases, the first MHC polypeptide is a β2M polypeptide; and the second MHC polypeptide is an HLA heavy chain polypeptide. In some cases, the HLA heavy chain polypeptide is an HLA-A24 polypeptide. In some cases, the HLA heavy chain polypeptide is an HLA-A24 polypeptide with an A236C substitution. In some cases, the first polypeptide comprises, in order from N-terminus to C-terminus: i) a WT-1 peptide epitope; ii) a first MHC polypeptide; and iii) two immunomodulatory polypeptides, where the two immunomodulatory polypeptides have the same amino acid sequence. In some cases, the Ig Fc polypeptide is a human IgG1 Fc polypeptide. In some cases, the Ig Fc polypeptide is an IgG1 Fc polypeptide comprising L234A and L235A substitutions. In some cases, the first and the second polypeptides are disulfide linked to one another. In some cases, the immunomodulatory polypeptide is a variant IL-2 polypeptide comprising H16A and F42A substitutions. In some cases, the immunomodulatory polypeptide is a variant IL-2 polypeptide comprising H16T and F42A substitutions. In some cases, a peptide linker is between one or more of: i) the second MHC polypeptide and the Ig Fc polypeptide; ii) the epitope and the first MHC polypeptide; iii) the first MHC polypeptide and the immunomodulatory polypeptide; and (where the TMMP comprises two immunomodulatory polypeptides on the first polypeptide chain) iv) between the two immunomodulatory polypeptides. In some cases, the peptide linker comprises the amino acid sequence AAAGG (SEQ ID NO: 283). In some cases, the peptide linker comprises the amino acid sequence (GGGGS)n (SEQ ID NO: 284), where n is an integer from 1 to 10 (e.g., where n is 2, 3, or 4). In some cases, the WT-1 peptide epitope is CMTWNQMN (SEQ ID NO: 261). In some cases, the WT-1 peptide epitope is CYTWNQMNL (SEQ ID NO: 286).

In some cases, a TMMP of the present disclosure comprises: a) a first polypeptide comprising, in order from N-terminus to C-terminus: i) a WT-1 peptide epitope; and ii) a first MHC polypeptide; and b) a second polypeptide comprising, in order from N-terminus to C-terminus: i) at least one immunomodulatory polypeptide; ii) a second MHC polypeptide; and iii) an Ig Fc polypeptide. In some cases, the first MHC polypeptide is a β2M polypeptide; and the second MHC polypeptide is an HLA heavy chain polypeptide. In some cases, the HLA heavy chain polypeptide is an HLA-A24 polypeptide. In some cases, the HLA heavy chain polypeptide is an HLA-A24 polypeptide with an A236C substitution. In some cases, the second polypeptide comprises, in order from N-terminus to C-terminus: i) two immunomodulatory polypeptides, where the two immunomodulatory polypeptides have the same amino acid sequence; ii) a second MHC polypeptide; and iii) an Ig Fc polypeptide. In some cases, the Ig Fc polypeptide is a human IgG1 Fc polypeptide. In some cases, the Ig Fc polypeptide is an IgG1 Fc polypeptide comprising L234A and L235A substitutions. In some cases, the first and the second polypeptides are disulfide linked to one another. In some cases, the immunomodulatory polypeptide is a variant IL-2 polypeptide comprising H16A and F42A substitutions. In some cases, the immunomodulatory polypeptide is a variant IL-2 polypeptide comprising H16T and F42A substitutions. In some cases, a peptide linker is between one or more of: i) the second MHC polypeptide and the Ig Fc polypeptide; ii) the epitope and the first MHC polypeptide; iii) the first MHC polypeptide and the immunomodulatory polypeptide; and (where the TMMP comprises two immunomodulatory polypeptides on the second polypeptide chain) iv) between the two immunomodulatory polypeptides. In some cases, the peptide linker comprises the amino acid sequence AAAGG (SEQ ID NO: 283). In some cases, the peptide linker comprises the amino acid sequence (GGGGS)n (SEQ ID NO: 284), where n is an integer from 1 to 10 (e.g., where n is 2, 3, or 4). In some cases, the WT-1 peptide epitope is CMTWNQMN (SEQ ID NO: 261). In some cases, the WT-1 peptide epitope is CYTWNQMNL (SEQ ID NO: 286).

In some cases, a TMMP of the present disclosure comprises: a) a first polypeptide comprising, in order from N-terminus to C-terminus: i) a WT-1 peptide epitope; and ii) a first MHC polypeptide; and b) a second polypeptide comprising, in order from N-terminus to C-terminus: i) a second MHC polypeptide; ii) an Ig Fc polypeptide; and iii) at least one immunomodulatory polypeptide. In some cases, the first MHC polypeptide is a β2M polypeptide; and the second MHC polypeptide is an HLA heavy chain polypeptide. In some cases, the HLA heavy chain polypeptide is an HLA-A24 polypeptide. In some cases, the HLA heavy chain polypeptide is an HLA-A24 polypeptide with an A236C substitution. In some cases, the second polypeptide comprises, in order from N-terminus to C-terminus: i) a second MHC polypeptide; ii) an Ig Fc polypeptide; and iii) two immunomodulatory polypeptides, where the two immunomodulatory polypeptides have the same amino acid sequence. In some cases, the Ig Fc polypeptide is a human IgG1 Fc polypeptide. In some cases, the Ig Fc polypeptide is an IgG1 Fc polypeptide comprising L234A and L235A substitutions. In some cases, the first and the second polypeptides are disulfide linked to one another. In some cases, the immunomodulatory polypeptide is a variant IL-2 polypeptide comprising H16A and F42A substitutions. In some cases, the immunomodulatory polypeptide is a variant IL-2 polypeptide comprising H16T and F42A substitutions. In some cases, a peptide linker is between one or more of: i) the second MHC polypeptide and the Ig Fc polypeptide; ii) the epitope and the first MHC polypeptide; iii) the Ig Fc polypeptide and the immunomodulatory polypeptide; and (where the TMMP comprises two immunomodulatory polypeptides on the second polypeptide chain) iv) between the two immunomodulatory polypeptides. In some cases, the peptide linker comprises the amino acid sequence AAAGG (SEQ ID NO: 283). In some cases, the peptide linker comprises the amino acid sequence (GGGGS)n (SEQ ID NO: 284), where n is an integer from 1 to 10 (e.g., where n is 2, 3, or 4). In some cases, the WT-1 peptide epitope is CMTWNQMN (SEQ ID NO: 261). In some cases, the WT-1 peptide epitope is CYTWNQMNL (SEQ ID NO: 286).

In some cases, a TMMP of the present disclosure comprises: a) a first polypeptide comprising, in order from N-terminus to C-terminus: i) a WT-1 peptide epitope; and ii) a first MHC polypeptide; and b) a second polypeptide comprising, in order from N-terminus to C-terminus: i) at least one immunomodulatory polypeptide; ii) a second MHC polypeptide; and iii) an Ig Fc polypeptide. In some cases, the first MHC polypeptide is a β2M polypeptide; and the second MHC polypeptide is an HLA heavy chain polypeptide. In some cases, the HLA heavy chain polypeptide is an HLA-A24 polypeptide. In some cases, the HLA heavy chain polypeptide is an HLA-A24 polypeptide with an A236C substitution. In some cases, the Ig Fc polypeptide is a human IgG1 Fc polypeptide. In some cases, the Ig Fc polypeptide is an IgG1 Fc polypeptide comprising L234A and L235A substitutions. In some cases, the first and the second polypeptides are disulfide linked to one another. In some cases, the immunomodulatory polypeptide is a variant IL-2 polypeptide comprising H16A and F42A substitutions. In some cases, the immunomodulatory polypeptide is a variant IL-2 polypeptide comprising H16T and F42A substitutions. In some cases, the WT-1 peptide epitope is CMTWNQMN (SEQ ID NO: 261). In some cases, the WT-1 peptide epitope is CYTWNQMNL (SEQ ID NO: 286).

In some cases, a TMMP of the present disclosure comprises: a) a first polypeptide comprising, in order from N-terminus to C-terminus: i) at least one immunomodulatory polypeptide; ii) a WT-1 peptide epitope; and iii) a first MHC polypeptide; and b) a second polypeptide comprising, in order from N-terminus to C-terminus: i) a second MHC polypeptide; and ii) an Ig Fc polypeptide. In some cases, the first MHC polypeptide is a β2M polypeptide; and the second MHC polypeptide is an HLA heavy chain polypeptide. In some cases, the HLA heavy chain polypeptide is an HLA-A24 polypeptide. In some cases, the HLA heavy chain polypeptide is an HLA-A24 polypeptide with an A236C substitution. In some cases, the first polypeptide comprises, in order from N-terminus to C-terminus: i) two immunomodulatory polypeptides, where the two immunomodulatory polypeptides have the same amino acid sequence; ii) a WT-1 peptide epitope; and iii) a first MHC polypeptide. In some cases, the Ig Fc polypeptide is a human IgG1 Fc polypeptide. In some cases, the Ig Fc polypeptide is an IgG1 Fc polypeptide comprising L234A and L235A substitutions. In some cases, the first and the second polypeptides are disulfide linked to one another. In some cases, the immunomodulatory polypeptide is a variant IL-2 polypeptide comprising H16A and F42A substitutions. In some cases, the immunomodulatory polypeptide is a variant IL-2 polypeptide comprising H16T and F42A substitutions. In some cases, a peptide linker is between one or more of: i) the second MHC polypeptide and the Ig Fc polypeptide; ii) the epitope and the first MHC polypeptide; iii) the immunomodulatory polypeptide and the epitope; and (where the TMMP comprises two immunomodulatory polypeptides on the first polypeptide chain) iv) between the two immunomodulatory polypeptides. In some cases, the peptide linker comprises the amino acid sequence AAAGG (SEQ ID NO: 283). In some cases, the peptide linker comprises the amino acid sequence (GGGGS)n (SEQ ID NO: 284), where n is an integer from 1 to 10 (e.g., where n is 2, 3, or 4). In some cases, the WT-1 peptide epitope is CMTWNQMN (SEQ ID NO: 261). In some cases, the WT-1 peptide epitope is CYTWNQMNL (SEQ ID NO: 286).

In some cases, a TMMP of the present disclosure comprises: a) a first polypeptide comprising, in order from N-terminus to C-terminus: i) a WT-1 peptide epitope; and ii) a first MHC polypeptide; and b) a second polypeptide comprising, in order from N-terminus to C-terminus: i) a second MHC polypeptide; ii) at least one immunomodulatory polypeptide; and iii) an Ig Fc polypeptide. In some cases, the first MHC polypeptide is a β2M polypeptide; and the second MHC polypeptide is an HLA heavy chain polypeptide. In some cases, the HLA heavy chain polypeptide is an HLA-A24 polypeptide. In some cases, the HLA heavy chain polypeptide is an HLA-A24 polypeptide with an A236C substitution. In some cases, the second polypeptide comprises, in order from N-terminus to C-terminus: i) a second MHC polypeptide; ii) two immunomodulatory polypeptides, where the two immunomodulatory polypeptides have the same amino acid sequence; and iii) an Ig Fc polypeptide. In some cases, the Ig Fc polypeptide is a human IgG1 Fc polypeptide. In some cases, the Ig Fc polypeptide is an IgG1 Fc polypeptide comprising L234A and L235A substitutions. In some cases, the first and the second polypeptides are disulfide linked to one another. In some cases, the immunomodulatory polypeptide is a variant IL-2 polypeptide comprising H16A and F42A substitutions. In some cases, the immunomodulatory polypeptide is a variant IL-2 polypeptide comprising H16T and F42A substitutions. In some cases, a peptide linker is between one or more of: i) the second MHC polypeptide and the immunomodulatory polypeptide; ii) the immunomodulatory polypeptide and the Ig Fc polypeptide; iii) the epitope and the first MHC polypeptide; iii) the first MHC polypeptide and the immunomodulatory polypeptide; and (where the TMMP comprises two immunomodulatory polypeptides on the second polypeptide chain) iv) between the two immunomodulatory polypeptides. In some cases, the peptide linker comprises the amino acid sequence AAAGG (SEQ ID NO: 283). In some cases, the peptide linker comprises the amino acid sequence (GGGGS)n (SEQ ID NO: 284), where n is an integer from 1 to 10 (e.g., where n is 2, 3, or 4). In some cases, the WT-1 peptide epitope is CMTWNQMN (SEQ ID NO: 261). In some cases, the WT-1 peptide epitope is CYTWNQMNL (SEQ ID NO: 286).

As noted above, and as depicted schematically in FIG. 19, an immunomodulatory polypeptide (i.e., one or more immunomodulatory polypeptides) can be present in a TMMP of the present disclosure at any of a variety of positions. FIG. 19 depicts the position of two copies of a variant IL-2 polypeptide; however, the immunomodulatory polypeptide can be any of a variety of immunomodulatory polypeptide, as described herein. As depicted in FIG. 19, an immunomodulatory polypeptide can be: 1) N-terminal to the MHC class I heavy chain (position 1); 2) C-terminal to the MHC class I heavy chain and N-terminal to the Ig Fc polypeptide; in other words, between the MHC class I heavy chain and the Ig Fc polypeptide (position 2); 3) C-terminal to the Ig Fc polypeptide (position 3); 4) N-terminal to the peptide epitope (position 4); or 5) C-terminal to the β2M polypeptide (position 5). "Position 1" refers to a position of the immunomodulatory polypeptide on the same polypeptide chain as the class I MHC heavy chain and N-terminal to the class I MHC heavy chain; e.g., where the TMMP comprises: a) a first polypeptide comprising, in order from N-terminus to C-terminus: i) a peptide epitope (e.g., a WT-1 peptide); and ii) a β2M polypeptide; and b) a second polypeptide comprising, in order from N-terminus to C-terminus: i) one or more immunomodulatory polypeptides; and ii) a class I MHC heavy chain polypeptide. "Position 2" refers to a position of the immunomodulatory polypeptide on the same polypeptide chain as the class I MHC heavy chain and C-terminal to the class I MHC heavy chain, but not at the C-terminus of the polypeptide chain; e.g., where the TMMP comprises: a) a first polypeptide comprising, in order from N-terminus to C-terminus: i) a peptide epitope (e.g., a WT-1 peptide); and ii) a β2M polypeptide; and b) a second polypeptide comprising, in order from N-terminus to C-terminus: i) a class I MHC heavy chain polypeptide; ii) one or more immunomodulatory polypeptides; and iii) an Ig Fc polypeptide. "Position 3" refers to a position of the immunomodulatory polypeptide on the same polypeptide chain as the class I MHC heavy chain and at the C-terminus of the polypeptide chain; e.g., where the TMMP comprises: a) a first polypeptide comprising, in order from N-terminus to C-terminus: i) a peptide epitope (e.g., a WT-1 peptide); and ii) a β2M polypeptide; and b) a second polypeptide comprising, in order from N-terminus to C-terminus: i) a class I MHC heavy chain polypeptide; ii) an Ig Fc polypeptide; and iii) one or more immunomodulatory polypeptides. "Position 4" refers to a position of the immunomodulatory polypeptide on the same polypeptide chain as the β2M polypeptide and N-terminal to the peptide epitope and the β2M polypeptide; e.g., where the TMMP comprises: a) a first polypeptide comprising, in order from N-terminus to C-terminus: i) one or more immunomodulatory polypeptides; ii) a peptide epitope (e.g., a WT-1 peptide); and iii) a β2M polypeptide; and b) a second polypeptide comprising a class I MHC heavy chain polypeptide (e.g., a second polypeptide comprising, in order from N-terminus to C-terminus: i) a class I MHC heavy chain polypeptide; and ii) an Ig Fc polypeptide. "Position 5" refers to a position of the immunomodulatory polypeptide on the same polypeptide chain as the β2M polypeptide and C-terminal to the β2M polypeptide (e.g., at the C-terminus of the polypeptide chain); e.g., where the TMMP comprises: a) a first polypeptide comprising, in order from N-terminus to C-terminus: i) a peptide epitope (e.g., a WT-1 peptide); ii) a β2M polypeptide; and iii) one or more immunomodulatory polypeptides; and b) a second polypeptide comprising a class I MHC heavy chain polypeptide (e.g., a second polypeptide comprising, in order from N-terminus to C-terminus: i) a class I MHC heavy chain polypeptide; and ii) an Ig Fc polypeptide.

Furthermore, as discussed above and as depicted schematically in FIG. 18A-18C, the first polypeptide chain and the second polypeptide chain of a TMMP of the present disclosure can be linked by one or more disulfide bonds. For example, a TMMMP of the present disclosure can comprise: a) a first polypeptide chain comprising an β2M polypeptide having an R12C substitution; and b) a second polypeptide chain comprising a class I MHC heavy chain polypeptide having an A236C substitution; such that a disulfide bond forms between the Cys at position 12 of the β2M polypeptide in the first polypeptide chain and the Cys at position 236 of the class I MHC heavy chain polypeptide in the second polypeptide chain. As another example, a TMMMP of the present disclosure can comprise: a) a first polypeptide comprising, in order from N-terminus to C-terminus: i) a peptide epitope; ii) a peptide linker comprising a GCGG(G4S)ₙ (SEQ ID NO:393) sequence, where n is 1, 2, or 3; and iii) a β2M polypeptide; and b) a second polypeptide comprising a class I MHC heavy chain polypeptide having a Y84C substitution, such that a disulfide bond forms between the Cys in the peptide linker in the first polypeptide chain and the Cys at position 84 of the class I MHC heavy chain polypeptide in the second polypeptide chain. In other examples, a TMMP of the present disclosure can comprise: a) a first polypeptide comprising, in order from N-terminus to C-terminus: i) a peptide epitope; ii) a peptide linker comprising a GCGG(G4S)ₙ (SEQ ID NO:393) sequence, where n is 1, 2, or 3; and iii) a β2M polypeptide having an R12C substitution; and b) a second polypeptide comprising a class I MHC heavy chain polypeptide having a Y84C substitution and an A236C substitution; such that: i) a first disulfide bond forms between the Cys in the peptide linker in the first polypeptide chain and the Cys at position 84 of the class I MHC heavy chain polypeptide in the second polypeptide chain; and ii) a second disulfide bond forms between the Cys at position 12 of the β2M polypeptide in the first polypeptide chain and the Cys at position 236 of the class I MHC heavy chain polypeptide in the second polypeptide chain. For simplicity, the first disulfide bond is referred to as "G2C/Y84C"; and the second disulfide bond is referred to as "R12C/A236C." A TMMP of the present disclosure can include: a) a G2C/Y84C disulfide bond and not an R12C/A236C disulfide bond; b) an R12C/A236C disulfide bond and not a G2C/Y84C disulfide bond; or c) a G2C/Y84C disulfide bond and an R12C/A236C disulfide bond.

A TMMP of the present disclosure can include: a) a G2C/Y84C disulfide bond and not an R12C/A236C disulfide bond; and b) at least one immunomodulatory polypeptide at position 1. A TMMP of the present disclosure can include: a) a G2C/Y84C disulfide bond and not an R12C/A236C disulfide bond; and b) at least one immunomodulatory polypeptide at position 2. A TMMP of the present disclosure can include: a) a G2C/Y84C disulfide bond and not an R12C/A236C disulfide bond; and b) at least one immunomodulatory polypeptide at position 3. A TMMP of the present disclosure can include: a) a G2C/Y84C disulfide bond and not an R12C/A236C disulfide bond; and b) at least one immunomodulatory polypeptide at position 4. A TMMP of the present disclosure can include: a) a G2C/Y84C disulfide bond and not an R12C/A236C disulfide bond; and b) at least one immunomodulatory polypeptide at position 5.

A TMMP of the present disclosure can include: a) an R12C/A236C disulfide bond and not a G2C/Y84C disulfide bond; and at least one immunomodulatory polypeptide at position 1. A TMMP of the present disclosure can include: a) an R12C/A236C disulfide bond and not a G2C/Y84C disulfide bond; and at least one immunomodulatory polypeptide at position 2. A TMMP of the present disclosure can include: a) an R12C/A236C disulfide bond and not a G2C/Y84C disulfide bond; and at least one immunomodulatory polypeptide at position 3. A TMMP of the present disclosure can include: a) an R12C/A236C disulfide bond and not a G2C/Y84C disulfide bond; and at least one immunomodulatory polypeptide at position 4. A TMMP of the present disclosure can include: a) an R12C/A236C disulfide bond and not a G2C/Y84C disulfide bond; and at least one immunomodulatory polypeptide at position 5.

A TMMP of the present disclosure can include: a) a G2C/Y84C disulfide bond and an R12C/A236C disulfide bond; and b) and at least one immunomodulatory polypeptide at position 1. A TMMP of the present disclosure can include: a) a G2C/Y84C disulfide bond and an R12C/A236C disulfide bond; and b) and at least one immunomodulatory polypeptide at position 2. A TMMP of the present disclosure can include: a) a G2C/Y84C disulfide bond and an R12C/A236C disulfide bond; and b) and at least one immunomodulatory polypeptide at position 3. A TMMP of the present disclosure can include: a) a G2C/Y84C disulfide bond and an R12C/A236C disulfide bond; and b) and at least one immunomodulatory polypeptide at position 4. A TMMP of the present disclosure can include: a) a G2C/Y84C disulfide bond and an R12C/A236C disulfide bond; and b) and at least one immunomodulatory polypeptide at position 5.

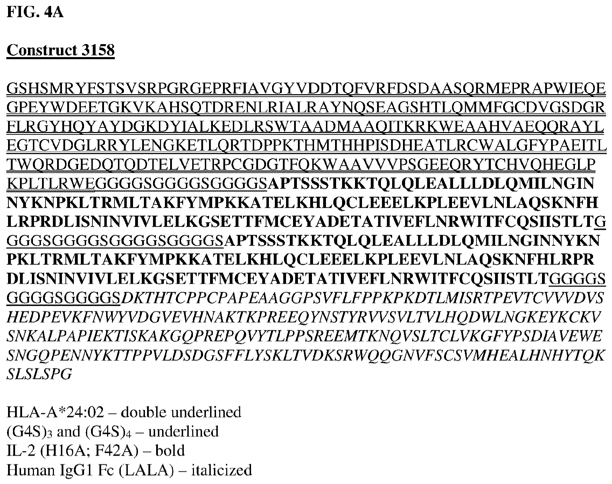

Non-limiting examples of amino acid sequences of first and second polypeptide chains of a TMMP of the present disclosure are provided in FIGs. 4A-4K and FIGs. 20A-20R.

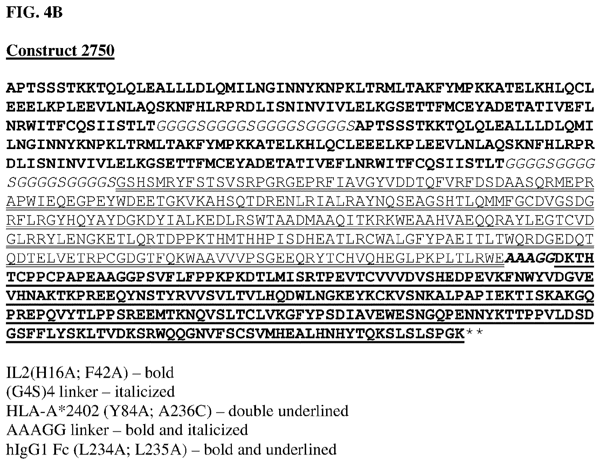

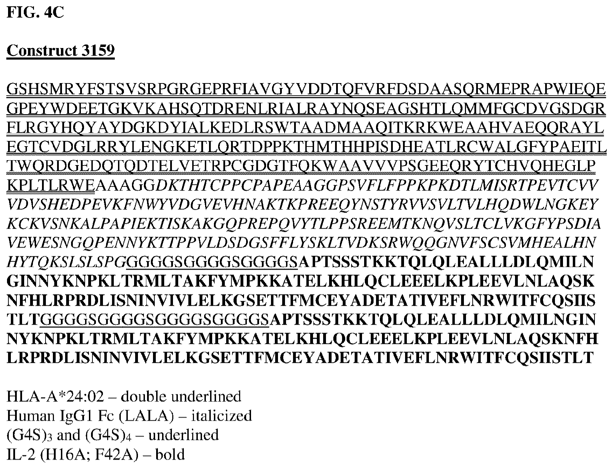

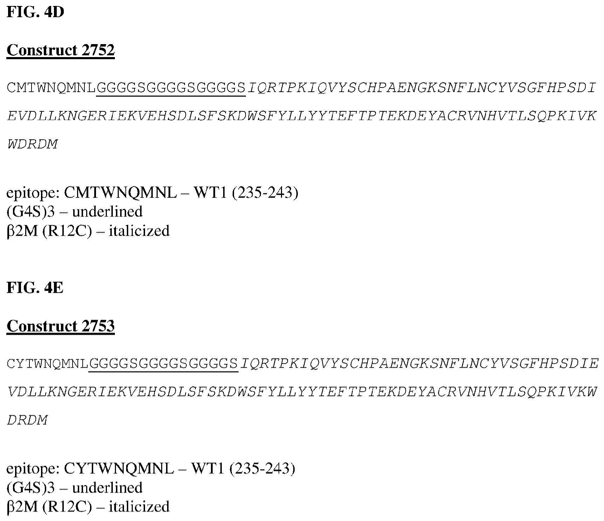

In some cases, a TMMP of the present disclosure comprises: a) a first polypeptide chain comprising the amino acid sequence designated "2752" as depicted in FIG. 4D; and b) a second polypeptide chain comprising the amino acid sequence designated "3159" as depicted in FIG. 4C.

In some cases, a TMMP of the present disclosure comprises: a) a first polypeptide chain comprising the amino acid sequence designated "2753" as depicted in FIG. 4E; and b) a second polypeptide chain comprising the amino acid sequence designated "3159" as depicted in FIG. 4C. Such a TMMP comprises: a) an immunomodulatory polypeptide at position 3 as depicted in FIG. 19; and b) an R12C/A236C disulfide bond (but not a G2C/Y84C disulfide bond).

In some cases, a TMMP of the present disclosure comprises: a) a first polypeptide chain comprising the amino acid sequence designated "2752" as depicted in FIG. 4D; and b) a second polypeptide chain comprising the amino acid sequence designated "2750" as depicted in FIG. 4B.

In some cases, a TMMP of the present disclosure comprises: a) a first polypeptide chain comprising the amino acid sequence designated "2753" as depicted in FIG. 4E; and b) a second polypeptide chain comprising the amino acid sequence designated "2750" as depicted in FIG. 4B. Such a TMMP comprises: a) an immunomodulatory polypeptide at position 1 as depicted in FIG. 19; and b) an R12C/A236C disulfide bond (but not a G2C/Y84C disulfide bond).

In some cases, a TMMP of the present disclosure comprises: a) a first polypeptide chain comprising the amino acid sequence designated "2752" as depicted in FIG. 4D; and b) a second polypeptide chain comprising the amino acid sequence designated "3158" as depicted in FIG. 4A.

In some cases, a TMMP of the present disclosure comprises: a) a first polypeptide chain comprising the amino acid sequence designated "2753" as depicted in FIG. 4E; and b) a second polypeptide chain comprising the amino acid sequence designated "3158" as depicted in FIG. 4A.

In some cases, a TMMP of the present disclosure comprises: a) a first polypeptide chain comprising the amino acid sequence designated "2380" as depicted in FIG. 14B; and b) a second polypeptide chain comprising the amino acid sequence designated "1715" as depicted in FIG. 14A.

In some cases, a TMMP of the present disclosure comprises: a) a first polypeptide chain comprising the amino acid sequence designated "2381" as depicted in FIG. 14B; and b) a second polypeptide chain comprising the amino acid sequence designated "1715" as depicted in FIG. 14A.

In some cases, a TMMP of the present disclosure comprises: a) a first polypeptide chain comprising the amino acid sequence designated "2380" as depicted in FIG. 14B; and b) a second polypeptide chain comprising the amino acid sequence designated "2405" as depicted in FIG. 14D.

In some cases, a TMMP of the present disclosure comprises: a) a first polypeptide chain comprising the amino acid sequence designated "2381" as depicted in FIG. 14B; and b) a second polypeptide chain comprising the amino acid sequence designated "2405" as depicted in FIG. 14D. In some cases, a TMMP of the present disclosure comprises: a) a first polypeptide chain comprising the amino acid sequence designated "2762" as depicted in FIG. 14F; and b) a second polypeptide chain comprising the amino acid sequence designated "2405" as depicted in FIG. 14D.

In some cases, a TMMP of the present disclosure comprises: a) a first polypeptide chain comprising the amino acid sequence designated "2380" as depicted in FIG. 14B; and b) a second polypeptide chain comprising the amino acid sequence designated "1380" as depicted in FIG. 14E.

In some cases, a TMMP of the present disclosure comprises: a) a first polypeptide chain comprising the amino acid sequence designated "2381" as depicted in FIG. 14B; and b) a second polypeptide chain comprising the amino acid sequence designated "1380" as depicted in FIG. 14E.

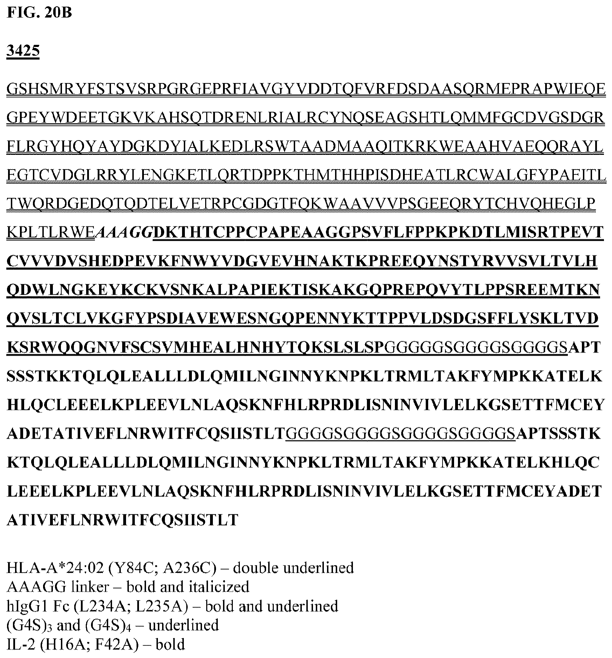

In some cases, a TMMP of the present disclosure comprises: a) a first polypeptide chain comprising the amino acid sequence designated "3592" as depicted in FIG. 20A; and b) a second polypeptide chain comprising the amino acid sequence designated "3188" as depicted in FIG. 20H. Such a TMMP comprises: a) an immunomodulatory polypeptide at position 1 as depicted in FIG. 19; and b) both a G2C/Y84C disulfide bond and an R12C/A236C disulfide bond.

In some cases, a TMMP of the present disclosure comprises: a) a first polypeptide chain comprising the amino acid sequence designated "3425" as depicted in FIG. 20B; and b) a second polypeptide chain comprising the amino acid sequence designated "3188" as depicted in FIG. 20H. Such a TMMP comprises: a) an immunomodulatory polypeptide at position 3 as depicted in FIG. 19; and b) both a G2C/Y84C disulfide bond and an R12C/A236C disulfide bond.

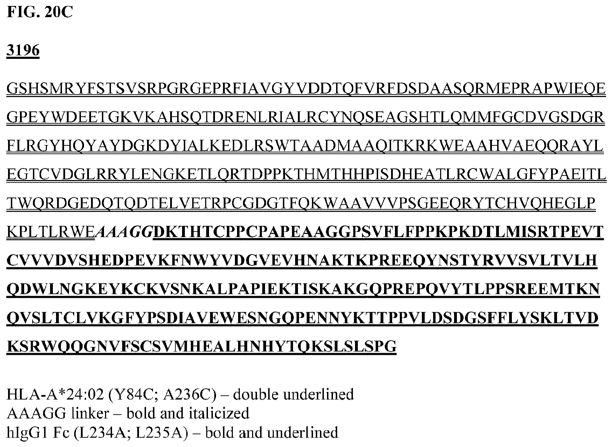

In some cases, a TMMP of the present disclosure comprises: a) a first polypeptide chain comprising the amino acid sequence designated "3196" as depicted in FIG. 20C; and b) a second polypeptide chain comprising the amino acid sequence designated "3604" as depicted in FIG. 20I. Such a TMMP comprises: a) an immunomodulatory polypeptide at position 5 as depicted in FIG. 19; and b) both a G2C/Y84C disulfide bond and an R12C/A236C disulfide bond.

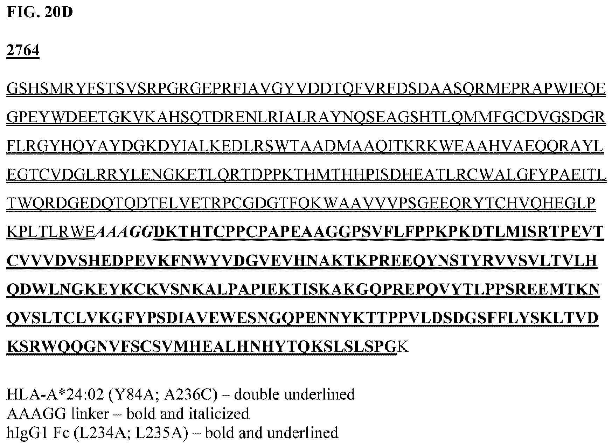

In some cases, a TMMP of the present disclosure comprises: a) a first polypeptide chain comprising the amino acid sequence designated "2764" as depicted in FIG. 20D; and b) a second polypeptide chain comprising the amino acid sequence designated "3603" as depicted in FIG. 20J. Such a TMMP comprises: a) an immunomodulatory polypeptide at position 5 as depicted in FIG. 19; and b) an R12C/A236C disulfide bond (but not a G2C/Y84C disulfide bond).

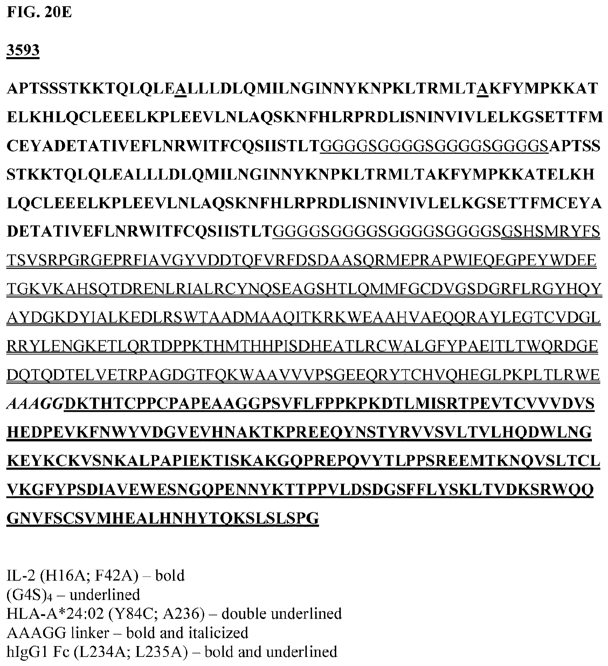

In some cases, a TMMP of the present disclosure comprises: a) a first polypeptide chain comprising the amino acid sequence designated "3593" as depicted in FIG. 20E; and b) a second polypeptide chain comprising the amino acid sequence designated "3192" as depicted in FIG. 20K. Such a TMMP comprises: a) an immunomodulatory polypeptide at position 1 as depicted in FIG. 19; and b) a G2C/Y84C disulfide bond (but not an R12C/A236C disulfide bond).

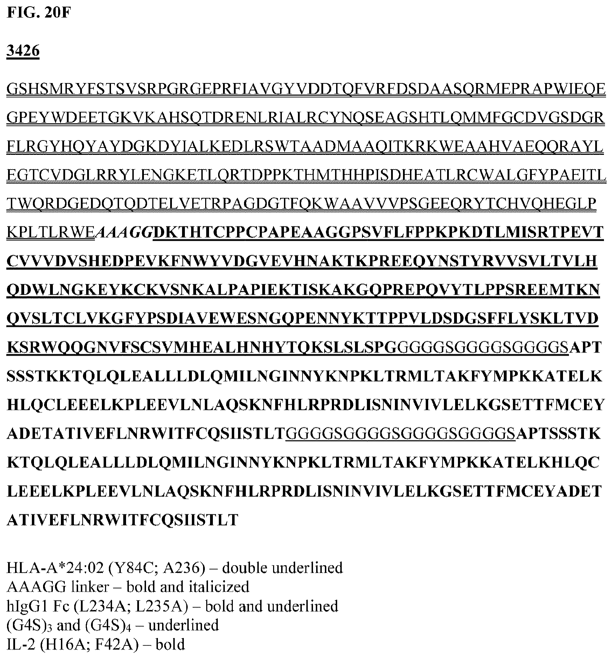

In some cases, a TMMP of the present disclosure comprises: a) a first polypeptide chain comprising the amino acid sequence designated "3426" as depicted in FIG. 20F; and b) a second polypeptide chain comprising the amino acid sequence designated "3192" as depicted in FIG. 20K. Such a TMMP comprises: a) an immunomodulatory polypeptide at position 3 as depicted in FIG. 19; and b) a G2C/Y84C disulfide bond (but not an R12C/A236C disulfide bond).

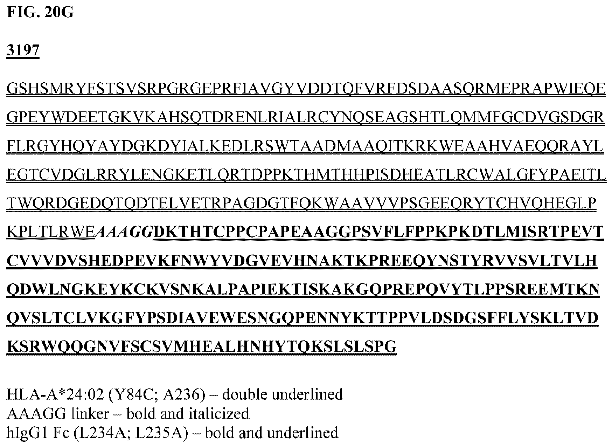

In some cases, a TMMP of the present disclosure comprises: a) a first polypeptide chain comprising the amino acid sequence designated "3197" as depicted in FIG. 20G; and b) a second polypeptide chain comprising the amino acid sequence designated "3605" as depicted in FIG. 20L. Such a TMMP comprises: a) an immunomodulatory polypeptide at position 5 as depicted in FIG. 19; and b) a G2C/Y84C disulfide bond (but not an R12C/A236C disulfide bond).

In some cases, a TMMP of the present disclosure comprises: a) a first polypeptide chain comprising the amino acid sequence designated "3592" as depicted in FIG. 20A; and b) a second polypeptide chain comprising the amino acid sequence designated "3529" as depicted in FIG. 20M. Such a TMMP comprises: a) an immunomodulatory polypeptide at position 1 as depicted in FIG. 19; and b) both a G2C/Y84C disulfide bond and an R12C/A236C disulfide bond.

In some cases, a TMMP of the present disclosure comprises: a) a first polypeptide chain comprising the amino acid sequence designated "3425" as depicted in FIG. 20B; and b) a second polypeptide chain comprising the amino acid sequence designated "3529" as depicted in FIG. 20M. Such a TMMP comprises: a) an immunomodulatory polypeptide at position 3 as depicted in FIG. 19; and b) both a G2C/Y84C disulfide bond and an R12C/A236C disulfide bond; and also comprises a WT1 239-247 (Q240Y) epitope.

In some cases, a TMMP of the present disclosure comprises: a) a first polypeptide chain comprising the amino acid sequence designated "3196" as depicted in FIG. 20C; and b) a second polypeptide chain comprising the amino acid sequence designated "3709" as depicted in FIG. 20N. Such a TMMP comprises: a) an immunomodulatory polypeptide at position 3 as depicted in FIG. 19; and b) both a G2C/Y84C disulfide bond and an R12C/A236C disulfide bond.

In some cases, a TMMP of the present disclosure comprises: a) a first polypeptide chain comprising the amino acid sequence designated "2750" as depicted in FIG. 4B; and b) a second polypeptide chain comprising the amino acid sequence designated "3528" as depicted in FIG. 20O. Such a TMMP comprises: a) an immunomodulatory polypeptide at position 1 as depicted in FIG. 19; and b) an R12C/A236C disulfide bond (but not a G2C/Y84C disulfide bond).

In some cases, a TMMP of the present disclosure comprises: a) a first polypeptide chain comprising the amino acid sequence designated "3159" as depicted in FIG. 4C; and b) a second polypeptide chain comprising the amino acid sequence designated "3528" as depicted in FIG. 20O. Such a TMMP comprises: a) an immunomodulatory polypeptide at position 3 as depicted in FIG. 19; and b) an R12C/A236C disulfide bond (but not a G2C/Y84C disulfide bond).

In some cases, a TMMP of the present disclosure comprises: a) a first polypeptide chain comprising the amino acid sequence designated "2764" as depicted in FIG. 20D; and b) a second polypeptide chain comprising the amino acid sequence designated "3708" as depicted in FIG. 20P. Such a TMMP comprises: a) an immunomodulatory polypeptide at position 5 as depicted in FIG. 19; and b) an R12C/A236C disulfide bond (but not a G2C/Y84C disulfide bond).

In some cases, a TMMP of the present disclosure comprises: a) a first polypeptide chain comprising the amino acid sequence designated "3593" as depicted in FIG. 20E; and b) a second polypeptide chain comprising the amino acid sequence designated "3530" as depicted in FIG. 20Q. Such a TMMP comprises: a) an immunomodulatory polypeptide at position 1 as depicted in FIG. 19; and b) a G2C/Y84C disulfide bond (but not an R12C/A236C disulfide bond).

In some cases, a TMMP of the present disclosure comprises: a) a first polypeptide chain comprising the amino acid sequence designated "3426" as depicted in FIG. 20F; and b) a second polypeptide chain comprising the amino acid sequence designated "3530" as depicted in FIG. 20Q. Such a TMMP comprises: a) an immunomodulatory polypeptide at position 3 as depicted in FIG. 19; and b) a G2C/Y84C disulfide bond (but not an R12C/A236C disulfide bond).

In some cases, a TMMP of the present disclosure comprises: a) a first polypeptide chain comprising the amino acid sequence designated "3197" as depicted in FIG. 20G; and b) a second polypeptide chain comprising the amino acid sequence designated "3710" as depicted in FIG. 20R. Such a TMMP comprises: a) an immunomodulatory polypeptide at position 5 as depicted in FIG. 19; and b) a G2C/Y84C disulfide bond (but not an R12C/A236C disulfide bond).

In some cases, a TMMP of the present disclosure comprises: a) a first polypeptide chain comprising the amino acid sequence designated "3426" as depicted in FIG. 20F; and b) a second polypeptide chain comprising the amino acid sequence designated "3529" as depicted in FIG. 20M. Such a TMMP comprises: a) an immunomodulatory polypeptide at position 3 as depicted in FIG. 19; and b) a G2C/Y84C disulfide bond (but not an R12C/A236C disulfide bond), and also includes a WT1 239-247 (Q240Y) epitope.

In some cases, a TMMP of the present disclosure comprises: a) a first polypeptide chain comprising the amino acid sequence designated "3425" as depicted in FIG. 20B; and b) a second polypeptide chain comprising the amino acid sequence designated "3528" as depicted in FIG. 20O. Such a TMMP comprises: a) an immunomodulatory polypeptide at position 3 as depicted in FIG. 19; and b) an R12C/A236C disulfide bond (but not a G2C/Y84C disulfide bond) and also includes a WT1 239-247 (Q240Y) epitope.

In some cases, a TMMP of the present disclosure comprises: a) a first polypeptide chain comprising the amino acid sequence designated "3425" as depicted in FIG. 20B; and b) a second polypeptide chain comprising the amino acid sequence designated "3530" as depicted in FIG. 20Q. Such a TMMP comprises: a) an immunomodulatory polypeptide at position 3 as depicted in FIG. 19; and b) a G2C/Y84C disulfide bond (but not an R12C/A236C disulfide bond), and also includes a WT1 239-247 (Q240Y) epitope.

In some cases, a TMMP of the present disclosure comprises: a) a first polypeptide chain comprising the amino acid sequence designated "3159" as depicted in FIG. 4C; and b) a second polypeptide chain comprising the amino acid sequence designated "3188" as depicted in FIG. 20H. Such a TMMP comprises: a) an immunomodulatory polypeptide at position 3 as depicted in FIG. 19; and b) an R12C/A236C disulfide bond (but not a G2C/Y84C disulfide bond), and also includes a WT1 235-243 (M236Y) epitope.

### METHODS OF GENERATING A MULTIMERIC T-CELL MODULATORY POLYPEPTIDE

The present disclosure provides a method of obtaining a TMMP comprising one or more variant immunomodulatory polypeptides that exhibit lower affinity for a cognate co-immunomodulatory polypeptide compared to the affinity of the corresponding parental wild-type immunomodulatory polypeptide for the co-immunomodulatory polypeptide, the method comprising: A) generating a library of TMMPs comprising a plurality of members, wherein each member comprises: a) a first polypeptide comprising: i) an epitope; and ii) a first major MHC polypeptide; and b) a second polypeptide comprising: i) a second MHC polypeptide; and ii) optionally an Ig Fc polypeptide or a non-Ig scaffold, wherein each member comprises a different variant immunomodulatory polypeptide on the first polypeptide, the second polypeptide, or both the first and the second polypeptide; B) determining the affinity of each member of the library for a cognate co-immunomodulatory polypeptide; and C) selecting a member that exhibits reduced affinity for the cognate co-immunomodulatory polypeptide. In some cases, the affinity is determined by bio-layer interferometry (BLI) using purified TMMP library members and the cognate co-immunomodulatory polypeptide. BLI methods are well known to those skilled in the art. A BLI assay is described above. See, e.g., Lad et al. (2015) J. Biomol. Screen. 20(4): 498-507; and Shah and Duncan (2014) J. Vis. Exp. 18:e51383.

The present disclosure provides a method of obtaining a TMMP that exhibits selective binding to a T-cell, the method comprising: A) generating a library of TMMPs comprising a plurality of members, wherein each member comprises: a) a first polypeptide comprising: i) an epitope; and ii) a first MHC polypeptide; and b) a second polypeptide comprising: i) a second MHC polypeptide; and ii) optionally an immunoglobulin (Ig) Fc polypeptide or a non-Ig scaffold, wherein each member comprises a different variant immunomodulatory polypeptide on the first polypeptide, the second polypeptide, or both the first and the second polypeptide, wherein the variant immunomodulatory polypeptide differs in amino acid sequence by from 1 amino acid to 10 amino acids from a parental wild-type immunomodulatory polypeptide; B) contacting a TMMP library member with a target T-cell expressing on its surface: i) a cognate co-immunomodulatory polypeptide that binds the parental wild-type immunomodulatory polypeptide; and ii) a T-cell receptor that binds to the epitope, wherein the TMMP library member comprises an epitope tag, such that the TMMP library member binds to the target T-cell; C) contacting the TMMP library member bound to the target T-cell with a fluorescently labeled binding agent that binds to the epitope tag, generating a TMMP library member/target T-cell/binding agent complex; D) measuring the mean fluorescence intensity (MFI) of the TMMP library member/target T-cell/binding agent complex using flow cytometry, wherein the MFI measured over a range of concentrations of the TMMP library member provides a measure of the affinity and apparent avidity; and E) selecting a TMMP library member that selectively binds the target T cell, compared to binding of the TMMP library member to a control T cell that comprises: i) the cognate co-immunomodulatory polypeptide that binds the parental wild-type immunomodulatory polypeptide; and ii) a T-cell receptor that binds to an epitope other than the epitope present in the TMMP library member. In some cases, a TMMP library member that is identified as selectively binds to a target T cell is isolated from the library.

In some cases, a parental wild-type immunomodulatory polypeptide and cognate immunomodulatory polypeptide pairs are selected from:
IL-2 and IL-2 receptor;
4-1BBL and 4-1BB;
PD-L1 and PD-1;
CD70 and CD27;
TGFβ and TGFβ receptor;
CD80 and CD28;
CD86 and CD28;
OX40L and OX40;
FasL and Fas;
ICOS-L and ICOS;
ICAM and LFA-1;
JAG1 and Notch;
JAG1 and CD46;
CD80 and CTLA4; and
CD86 and CTLA4.

The present disclosure provides a method of obtaining a TMMP comprising one or more variant immunomodulatory polypeptides that exhibit reduced affinity for a cognate co-immunomodulatory polypeptide compared to the affinity of the corresponding parental wild-type immunomodulatory polypeptide for the co-immunomodulatory polypeptide, the method comprising selecting, from a library of TMMPs comprising a plurality of members, a member that exhibits reduced affinity for the cognate co-immunomodulatory polypeptide, wherein the plurality of member comprises: a) a first polypeptide comprising: i) an epitope; and ii) a first MHC polypeptide; and b) a second polypeptide comprising: i) a second MHC polypeptide; and ii) optionally an Ig Fc polypeptide or a non-Ig scaffold, wherein the members of the library comprise a plurality of variant immunomodulatory polypeptide present in the first polypeptide, the second polypeptide, or both the first and the second polypeptide. In some cases, the selecting step comprises determining the affinity, using bio-layer interferometry, of binding between TMMP library members and the cognate co-immunomodulatory polypeptide. In some cases, the TMMP is as described above.

In some cases, the method further comprises: a) contacting the selected TMMP library member with a target T-cell expressing on its surface: i) a cognate co-immunomodulatory polypeptide that binds the parental wild-type immunomodulatory polypeptide; and ii) a T-cell receptor that binds to the epitope, wherein the TMMP library member comprises an epitope tag, such that the TMMP library member binds to the target T-cell; b) contacting the selected TMMP library member bound to the target T-cell with a fluorescently labeled binding agent that binds to the epitope tag, generating a selected TMMP library member/target T-cell/binding agent complex; and c) measuring the mean fluorescence intensity (MFI) of the selected TMMP library member/target T-cell/binding agent complex using flow cytometry, wherein the MFI measured over a range of concentrations of the selected TMMP library member provides a measure of the affinity and apparent avidity. A selected TMMP library member that selectively binds the target T cell, compared to binding of the TMMP library member to a control T cell that comprises: i) the cognate co-immunomodulatory polypeptide that binds the parental wild-type immunomodulatory polypeptide; and ii) a T-cell receptor that binds to an epitope other than the epitope present in the TMMP library member, is identified as selectively binding to the target T cell. In some cases, the binding agent is an antibody specific for the epitope tag. In some cases, the variant immunomodulatory polypeptide comprises from 1 to 20 amino acid substitutions (e.g., 1, 2, 3, 4, 5, 6, 7, 8, 9, 10, 11, 12, 13, 14, 15, 16, 17, 18, 19, or 20 amino acid substitutions) compared to the corresponding parental wild-type immunomodulatory polypeptide. In some cases, the TMMP comprises two variant immunomodulatory polypeptides. In some cases, the two variant immunomodulatory polypeptides comprise the same amino acid sequence. In some cases, the first polypeptide comprises one of the two variant immunomodulatory polypeptides and wherein the second polypeptide comprises the second of the two variant immunomodulatory polypeptides. In some cases, the two variant immunomodulatory polypeptides are on the same polypeptide chain of the TMMP. In some cases, the two variant immunomodulatory polypeptides are on the first polypeptide of the TMMP. In some cases, the two variant immunomodulatory polypeptides are on the second polypeptide of the TMMP.

In some cases, the method further comprises isolating the selected TMMP library member from the library. In some cases, the method further comprises providing a nucleic acid comprising a nucleotide sequence encoding the selected TMMP library member. In some cases, the nucleic acid is present in a recombinant expression vector. In some cases, the nucleotide sequence is operably linked to a transcriptional control element that is functional in a eukaryotic cell. In some cases, the method further comprises introducing the nucleic acid into a eukaryotic host cell, and culturing the cell in a liquid medium to synthesize the encoded selected TMMP library member in the cell. In some cases, the method further comprises isolating the synthesized selected TMMP library member from the cell or from liquid culture medium comprising the cell. In some cases, the selected TMMP library member comprises an Ig Fc polypeptide. In some cases, the method further comprises conjugating a drug to the Ig Fc polypeptide. In some cases, the drug is a cytotoxic agent is selected from maytansinoid, benzodiazepine, taxoid, CC-1065, duocarmycin, a duocarmycin analog, calicheamicin, dolastatin, a dolastatin analog, auristatin, tomaymycin, and leptomycin, or a pro-drug of any one of the foregoing. In some cases, the drug is a retinoid. In some cases, the parental wild-type immunomodulatory polypeptide and the cognate immunomodulatory polypeptides are selected from: IL-2 and IL-2 receptor; 4-1BBL and 4-1BB; PD-L1 and PD-1; CD70 and CD27; TGFβ and TGFβ receptor; CD80 and CD28; CD86 and CD28; OX40L and OX40; FasL and Fas; ICOS-L and ICOS; ICAM and LFA-1; JAG1 and Notch; JAG1 and CD46; CD80 and CTLA4; and CD86 and CTLA4.

The present disclosure provides a method of obtaining a TMMP comprising one or more variant immunomodulatory polypeptides that exhibit reduced affinity for a cognate co-immunomodulatory polypeptide compared to the affinity of the corresponding parental wild-type immunomodulatory polypeptide for the co-immunomodulatory polypeptide, the method comprising: A) providing a library of TMMPs comprising a plurality of members, wherein the plurality of member comprises: a) a first polypeptide comprising: i) an epitope; and ii) a first MHC polypeptide; and b) a second polypeptide comprising: i) a second MHC polypeptide; and ii) optionally an Ig Fc polypeptide or a non-Ig scaffold, wherein the members of the library comprise a plurality of variant immunomodulatory polypeptide present in the first polypeptide, the second polypeptide, or both the first and the second polypeptide; and B) selecting from the library a member that exhibits reduced affinity for the cognate co-immunomodulatory polypeptide. In some cases, the selecting step comprises determining the affinity, using bio-layer interferometry, of binding between TMMP library members and the cognate co-immunomodulatory polypeptide. In some cases, the TMMP is as described above.

In some cases, the method further comprises: a) contacting the selected TMMP library member with a target T-cell expressing on its surface: i) a cognate co-immunomodulatory polypeptide that binds the parental wild-type immunomodulatory polypeptide; and ii) a T-cell receptor that binds to the epitope, wherein the TMMP library member comprises an epitope tag, such that the TMMP library member binds to the target T-cell; b) contacting the selected TMMP library member bound to the target T-cell with a fluorescently labeled binding agent that binds to the epitope tag, generating a selected TMMP library member/target T-cell/binding agent complex; and c) measuring the mean fluorescence intensity (MFI) of the selected TMMP library member/target T-cell/binding agent complex using flow cytometry, wherein the MFI measured over a range of concentrations of the selected TMMP library member provides a measure of the affinity and apparent avidity. A selected TMMP library member that selectively binds the target T cell, compared to binding of the TMMP library member to a control T cell that comprises: i) the cognate co-immunomodulatory polypeptide that binds the parental wild-type immunomodulatory polypeptide; and ii) a T-cell receptor that binds to an epitope other than the epitope present in the TMMP library member, is identified as selectively binding to the target T cell. In some cases, the binding agent is an antibody specific for the epitope tag. In some cases, the variant immunomodulatory polypeptide comprises from 1 to 20 amino acid substitutions (e.g., 1, 2, 3, 4, 5, 6, 7, 8, 9, 10, 11, 12, 13, 14, 15, 16, 17, 18, 19, or 20 amino acid substitutions) compared to the corresponding parental wild-type immunomodulatory polypeptide. In some cases, the TMMP comprises two variant immunomodulatory polypeptides. In some cases, the two variant immunomodulatory polypeptides comprise the same amino acid sequence. In some cases, the first polypeptide comprises one of the two variant immunomodulatory polypeptides and wherein the second polypeptide comprises the second of the two variant immunomodulatory polypeptides. In some cases, the two variant immunomodulatory polypeptides are on the same polypeptide chain of the TMMP. In some cases, the two variant immunomodulatory polypeptides are on the first polypeptide of the TMMP. In some cases, the two variant immunomodulatory polypeptides are on the second polypeptide of the TMMP.

In some cases, the method further comprises isolating the selected TMMP library member from the library. In some cases, the method further comprises providing a nucleic acid comprising a nucleotide sequence encoding the selected TMMP library member. In some cases, the nucleic acid is present in a recombinant expression vector. In some cases, the nucleotide sequence is operably linked to a transcriptional control element that is functional in a eukaryotic cell. In some cases, the method further comprises introducing the nucleic acid into a eukaryotic host cell, and culturing the cell in a liquid medium to synthesize the encoded selected TMMP library member in the cell. In some cases, the method further comprises isolating the synthesized selected TMMP library member from the cell or from liquid culture medium comprising the cell. In some cases, the selected TMMP library member comprises an Ig Fc polypeptide. In some cases, the method further comprises conjugating a drug to the Ig Fc polypeptide. In some cases, the drug is a cytotoxic agent is selected from maytansinoid, benzodiazepine, taxoid, CC-1065, duocarmycin, a duocarmycin analog, calicheamicin, dolastatin, a dolastatin analog, auristatin, tomaymycin, and leptomycin, or a pro-drug of any one of the foregoing. In some cases, the drug is a retinoid. In some cases, the parental wild-type immunomodulatory polypeptide and the cognate immunomodulatory polypeptides are selected from IL-2 and IL-2 receptor; 4-1BBL and 4-1BB; PD-L1 and PD-1; TGFβ and TGFβ receptor; CD80 and CD28; CD86 and CD28; OX40L and OX40; FasL and Fas; ICOS-L and ICOS; CD70 and CD27; ICAM and LFA-1; JAG1 and Notch; JAG1 and CD46; CD80 and CTLA4; and CD86 and CTLA4.

### NUCLEIC ACIDS

The present disclosure provides a nucleic acid comprising a nucleotide sequence encoding a TMMP of the present disclosure. The present disclosure provides a nucleic acid comprising a nucleotide sequence encoding a TMMP of the present disclosure.

The present disclosure provides nucleic acids comprising nucleotide sequences encoding a TMMP of the present disclosure. In some cases, the individual polypeptide chains of a TMMP of the present disclosure are encoded in separate nucleic acids. In some cases, all polypeptide chains of a TMMP of the present disclosure are encoded in a single nucleic acid. In some cases, a first nucleic acid comprises a nucleotide sequence encoding a first polypeptide of a TMMP of the present disclosure; and a second nucleic acid comprises a nucleotide sequence encoding a second polypeptide of a TMMP of the present disclosure. In some cases, single nucleic acid comprises a nucleotide sequence encoding a first polypeptide of a TMMP of the present disclosure and a second polypeptide of a TMMP of the present disclosure.

### Separate nucleic acids encoding individual polypeptide chains of a multimeric polypeptide

The present disclosure provides nucleic acids comprising nucleotide sequences encoding a TMMP of the present disclosure. As noted above, in some cases, the individual polypeptide chains of a TMMP of the present disclosure are encoded in separate nucleic acids. In some cases, nucleotide sequences encoding the separate polypeptide chains of a TMMP of the present disclosure are operably linked to transcriptional control elements, e.g., promoters, such as promoters that are functional in a eukaryotic cell, where the promoter can be a constitutive promoter or an inducible promoter.

The present disclosure provides a first nucleic acid and a second nucleic acid, where the first nucleic acid comprises a nucleotide sequence encoding a first polypeptide of a TMMP of the present disclosure, where the first polypeptide comprises, in order from N-terminus to C-terminus: a) an epitope (e.g., a T-cell epitope); b) a first MHC polypeptide; and c) an immunomodulatory polypeptide (e.g., a reduced-affinity variant, as described above); and where the second nucleic acid comprises a nucleotide sequence encoding a second polypeptide of a TMMP of the present disclosure, where the second polypeptide comprises, in order from N-terminus to C-terminus: a) a second MHC polypeptide; and b) an Ig Fc polypeptide. Suitable T-cell epitopes, MHC polypeptides, immunomodulatory polypeptides, and Ig Fc polypeptides, are described above. In some cases, the nucleotide sequences encoding the first and the second polypeptides are operably linked to transcriptional control elements. In some cases, the transcriptional control element is a promoter that is functional in a eukaryotic cell. In some cases, the nucleic acids are present in separate expression vectors.

The present disclosure provides a first nucleic acid and a second nucleic acid, where the first nucleic acid comprises a nucleotide sequence encoding a first polypeptide of a TMMP of the present disclosure, where the first polypeptide comprises, in order from N-terminus to C-terminus: a) an epitope (e.g., a T-cell epitope); and b) a first MHC polypeptide; and where the second nucleic acid comprises a nucleotide sequence encoding a second polypeptide of a TMMP of the present disclosure, where the second polypeptide comprises, in order from N-terminus to C-terminus: a) an immunomodulatory polypeptide (e.g., a reduced-affinity variant as described above); b) a second MHC polypeptide; and c) an Ig Fc polypeptide. Suitable T-cell epitopes, MHC polypeptides, immunomodulatory polypeptides, and Ig Fc polypeptides, are described above. In some cases, the nucleotide sequences encoding the first and the second polypeptides are operably linked to transcriptional control elements. In some cases, the transcriptional control element is a promoter that is functional in a eukaryotic cell. In some cases, the nucleic acids are present in separate expression vectors.

### Nucleic acid encoding two or more polypeptides present in a multimeric polypeptide

The present disclosure provides a nucleic acid comprising nucleotide sequences encoding at least the first polypeptide and the second polypeptide of a TMMP of the present disclosure. In some cases, where a TMMP of the present disclosure includes a first, second, and third polypeptide, the nucleic acid includes a nucleotide sequence encoding the first, second, and third polypeptides. In some cases, the nucleotide sequences encoding the first polypeptide and the second polypeptide of a TMMP of the present disclosure includes a proteolytically cleavable linker interposed between the nucleotide sequence encoding the first polypeptide and the nucleotide sequence encoding the second polypeptide. In some cases, the nucleotide sequences encoding the first polypeptide and the second polypeptide of a TMMP of the present disclosure includes an internal ribosome entry site (IRES) interposed between the nucleotide sequence encoding the first polypeptide and the nucleotide sequence encoding the second polypeptide. In some cases, the nucleotide sequences encoding the first polypeptide and the second polypeptide of a TMMP of the present disclosure includes a ribosome skipping signal (or cis-acting hydrolase element, CHYSEL (SEQ ID NO: 394)) interposed between the nucleotide sequence encoding the first polypeptide and the nucleotide sequence encoding the second polypeptide. Examples of nucleic acids are described below, where a proteolytically cleavable linker is provided between nucleotide sequences encoding the first polypeptide and the second polypeptide of a TMMP of the present disclosure; in any of these embodiments, an IRES or a ribosome skipping signal can be used in place of the nucleotide sequence encoding the proteolytically cleavable linker.

In some cases, a first nucleic acid (e.g., a recombinant expression vector, an mRNA, a viral RNA, etc.) comprises a nucleotide sequence encoding a first polypeptide chain of a TMMP of the present disclosure; and a second nucleic acid (e.g., a recombinant expression vector, an mRNA, a viral RNA, etc.) comprises a nucleotide sequence encoding a second polypeptide chain of a TMMP of the present disclosure. In some cases, the nucleotide sequence encoding the first polypeptide, and the second nucleotide sequence encoding the second polypeptide, are each operably linked to transcriptional control elements, e.g., promoters, such as promoters that are functional in a eukaryotic cell, where the promoter can be a constitutive promoter or an inducible promoter.

The present disclosure provides a nucleic acid comprising a nucleotide sequence encoding a recombinant polypeptide, where the recombinant polypeptide comprises, in order from N-terminus to C-terminus: a) an epitope (e.g., a T-cell epitope); b) a first MHC polypeptide; c) an immunomodulatory polypeptide (e.g., a reduced-affinity variant as described above); d) a proteolytically cleavable linker; e) a second MHC polypeptide; and f) an immunoglobulin (Ig) Fc polypeptide. The present disclosure provides a nucleic acid comprising a nucleotide sequence encoding a recombinant polypeptide, where the recombinant polypeptide comprises, in order from N-terminus to C-terminus: a) a first leader peptide; b) the epitope; c) the first MHC polypeptide; d) the immunomodulatory polypeptide (e.g., a reduced-affinity variant as described above); e) the proteolytically cleavable linker; f) a second leader peptide; g) the second MHC polypeptide; and h) the Ig Fc polypeptide. The present disclosure provides a nucleic acid comprising a nucleotide sequence encoding a recombinant polypeptide, where the recombinant polypeptide comprises, in order from N-terminus to C-terminus: a) an epitope; b) a first MHC polypeptide; c) a proteolytically cleavable linker; d) an immunomodulatory polypeptide (e.g., a reduced-affinity variant as described above); e) a second MHC polypeptide; and f) an Ig Fc polypeptide. In some cases, the first leader peptide and the second leader peptide are a β2-M leader peptide. In some cases, the nucleotide sequence is operably linked to a transcriptional control element. In some cases, the transcriptional control element is a promoter that is functional in a eukaryotic cell.

Suitable MHC polypeptides are described above. In some cases, the first MHC polypeptide is a β2-microglobulin polypeptide; and wherein the second MHC polypeptide is an MHC class I heavy chain polypeptide. In some cases, the β2-microglobulin polypeptide comprises an amino acid sequence having at least 85% amino acid sequence identity to a β2M amino acid sequence depicted in FIG. 6. In some cases, the MHC class I heavy chain polypeptide is an HLA-A, HLA-B, HLA-C, HLA-E, HLA-F, HLA-G, HLA-K, or HLA-L heavy chain. In some cases, the MHC class I heavy chain polypeptide comprises an amino acid sequence having at least 85% amino acid sequence identity to the amino acid sequence depicted in any one of FIG. 3A-3C.

Suitable Fc polypeptides are described above. In some cases, the Ig Fc polypeptide is an IgG1 Fc polypeptide, an IgG2 Fc polypeptide, an IgG3 Fc polypeptide, an IgG4 Fc polypeptide, an IgA Fc polypeptide, or an IgM Fc polypeptide. In some cases, the Ig Fc polypeptide comprises an amino acid sequence having at least 85% amino acid sequence identity to an amino acid sequence depicted in Figures 5A-5G.

Suitable immunomodulatory polypeptides are described above.

Suitable proteolytically cleavable linkers are described above. In some cases, the proteolytically cleavable linker comprises an amino acid sequence selected from: a) LEVLFQGP (SEQ ID NO:388); b) ENLYTQS (SEQ ID NO:389); c) DDDDK (SEQ ID NO:390); d) LVPR (SEQ ID NO:391); and e) GSGATNFSLLKQAGDVEENPGP (SEQ ID NO:392).

In some cases, a linker between the epitope and the first MHC polypeptide comprises a first Cys residue, and the second MHC polypeptide comprises an amino acid substitution to provide a second Cys residue, such that the first and the second Cys residues provide for a disulfide linkage between the linker and the second MHC polypeptide. In some cases, first MHC polypeptide comprises an amino acid substitution to provide a first Cys residue, and the second MHC polypeptide comprises an amino acid substitution to provide a second Cys residue, such that the first Cys residue and the second Cys residue provide for a disulfide linkage between the first MHC polypeptide and the second MHC polypeptide.

### Recombinant expression vectors

The present disclosure provides recombinant expression vectors comprising nucleic acids of the present disclosure. In some cases, the recombinant expression vector is a non-viral vector. In some cases, the recombinant expression vector is a viral construct, e.g., a recombinant adeno-associated virus construct (see, e.g., U.S. Patent No. 7,078,387), a recombinant adenoviral construct, a recombinant lentiviral construct, a recombinant retroviral construct, a non-integrating viral vector, etc.

Suitable expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90:10613-10617); SV40; herpes simplex virus; human immunodeficiency virus (see, e.g., Miyoshi et al., PNAS 94:10319 23, 1997; Takahashi et al., J Virol 73:7812 7816, 1999); a retroviral vector (e.g., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell.

Depending on the host/vector system utilized, any of a number of suitable transcription and translation control elements, including constitutive and inducible promoters, transcription enhancer elements, transcription terminators, etc. may be used in the expression vector (see e.g., Bitter et al. (1987) Methods in Enzymology, 153:516-544).

In some cases, a nucleotide sequence encoding a DNA-targeting RNA and/or a site-directed modifying polypeptide is operably linked to a control element, e.g., a transcriptional control element, such as a promoter. The transcriptional control element may be functional in either a eukaryotic cell, e.g., a mammalian cell; or a prokaryotic cell (e.g., bacterial or archaeal cell). In some cases, a nucleotide sequence encoding a DNA-targeting RNA and/or a site-directed modifying polypeptide is operably linked to multiple control elements that allow expression of the nucleotide sequence encoding a DNA-targeting RNA and/or a site-directed modifying polypeptide in both prokaryotic and eukaryotic cells.

Non-limiting examples of suitable eukaryotic promoters (promoters functional in a eukaryotic cell) include those from cytomegalovirus (CMV) immediate early, herpes simplex virus (HSV) thymidine kinase, early and late SV40, long terminal repeats (LTRs) from retrovirus, and mouse metallothionein-I. Selection of the appropriate vector and promoter is well within the level of ordinary skill in the art. The expression vector may also contain a ribosome binding site for translation initiation and a transcription terminator. The expression vector may also include appropriate sequences for amplifying expression.

### GENETICALLY MODIFIED HOST CELLS

The present disclosure provides a genetically modified host cell, where the host cell is genetically modified with a nucleic acid of the present disclosure.

Suitable host cells include eukaryotic cells, such as yeast cells, insect cells, and mammalian cells. In some cases, the host cell is a cell of a mammalian cell line. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, and the like.

In some cases, the host cell is a mammalian cell that has been genetically modified such that it does not synthesize endogenous MHC β2-M.

In some cases, the host cell is a mammalian cell that has been genetically modified such that it does not synthesize endogenous MHC Class I heavy chain. In some cases, the host cell is a mammalian cell that has been genetically modified such that it does not synthesize endogenous MHC β2-M and such that it does not synthesize endogenous MHC Class I heavy chain.

### COMPOSITIONS

The present disclosure provides compositions, including pharmaceutical compositions, comprising a TMMP (synTac) of the present disclosure. The present disclosure provides compositions, including pharmaceutical compositions, comprising a TMMP of the present disclosure. The present disclosure provides compositions, including pharmaceutical compositions, comprising a nucleic acid or a recombinant expression vector of the present disclosure.

### Compositions comprising a multimeric polypeptide

A composition of the present disclosure can comprise, in addition to a TMMP of the present disclosure, one or more of: a salt, e.g., NaCl, MgCl₂, KCl, MgSO₄, etc.; a buffering agent, e.g., a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), N-tris[Hydroxymethyl]methyl-3-aminopropanesulfonic acid (TAPS), etc.; a solubilizing agent; a detergent, e.g., a non-ionic detergent such as Tween-20, etc.; a protease inhibitor; glycerol; and the like.

The composition may comprise a pharmaceutically acceptable excipient, a variety of which are known in the art and need not be discussed in detail herein. Pharmaceutically acceptable excipients have been amply described in a variety of publications, including, for example, "Remington: The Science and Practice of Pharmacy", 19th Ed. (1995), or latest edition, Mack Publishing Co; A. Gennaro (2000) "Remington: The Science and Practice of Pharmacy", 20th edition, Lippincott, Williams, & Wilkins; Pharmaceutical Dosage Forms and Drug Delivery Systems (1999) H.C. Ansel et al., eds 7th ed., Lippincott, Williams, & Wilkins; and Handbook of Pharmaceutical Excipients (2000) A.H. Kibbe et al., eds., 3rd ed. Amer. Pharmaceutical Assoc.

A pharmaceutical composition can comprise a TMMP of the present disclosure, and a pharmaceutically acceptable excipient. In some cases, a subject pharmaceutical composition will be suitable for administration to a subject, e.g., will be sterile. For example, in some cases, a subject pharmaceutical composition will be suitable for administration to a human subject, e.g., where the composition is sterile and is free of detectable pyrogens and/or other toxins.

The protein compositions may comprise other components, such as pharmaceutical grades of mannitol, lactose, starch, magnesium stearate, sodium saccharin, talcum, cellulose, glucose, sucrose, magnesium, carbonate, and the like. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate, hydrochloride, sulfate salts, solvates (e.g., mixed ionic salts, water, organics), hydrates (e.g., water), and the like.

For example, compositions may include aqueous solution, powder form, granules, tablets, pills, suppositories, capsules, suspensions, sprays, and the like. The composition may be formulated according to the various routes of administration described below.

Where a TMMP of the present disclosure is administered as an injectable (e.g. subcutaneously, intraperitoneally, intramuscularly, and/or intravenously) directly into a tissue, a formulation can be provided as a ready-to-use dosage form, or as non-aqueous form (e.g. a reconstitutable storage-stable powder) or aqueous form, such as liquid composed of pharmaceutically acceptable carriers and excipients. The protein-containing formulations may also be provided so as to enhance serum half-life of the TMMP following administration. For example, the TMMP may be provided in a liposome formulation, prepared as a colloid, or other conventional techniques for extending serum half-life. A variety of methods are available for preparing liposomes, as described in, e.g., Szoka et al. 1980 Ann. Rev. Biophys. Bioeng. 9:467, U.S. Pat. Nos. 4,235,871, 4,501,728 and 4,837,028. The preparations may also be provided in controlled release or slow-release forms.

Other examples of formulations suitable for parenteral administration include isotonic sterile injection solutions, anti-oxidants, bacteriostats, and solutes that render the formulation isotonic with the blood of the intended recipient, suspending agents, solubilizers, thickening agents, stabilizers, and preservatives. For example, a subject pharmaceutical composition can be present in a container, e.g., a sterile container, such as a syringe. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets.

The concentration of a TMMP of the present disclosure in a formulation can vary widely (e.g., from less than about 0.1%, usually at or at least about 2% to as much as 20% to 50% or more by weight) and will usually be selected primarily based on fluid volumes, viscosities, and patient-based factors in accordance with the particular mode of administration selected and the patient's needs.

The present disclosure provides a container comprising a composition of the present disclosure, e.g., a liquid composition. The container can be, e.g., a syringe, an ampoule, and the like. In some cases, the container is sterile. In some cases, both the container and the composition are sterile.

The present disclosure provides compositions, including pharmaceutical compositions, comprising a TMMP of the present disclosure. A composition can comprise: a) a TMMP of the present disclosure; and b) an excipient, as described above. In some cases, the excipient is a pharmaceutically acceptable excipient.

In some cases, a TMMP of the present disclosure is present in a liquid composition. Thus, the present disclosure provides compositions (e.g., liquid compositions, including pharmaceutical compositions) comprising a TMMP of the present disclosure. In some cases, a composition of the present disclosure comprises: a) a TMMP of the present disclosure; and b) saline (e.g., 0.9% NaCl). In some cases, the composition is sterile. In some cases, the composition is suitable for administration to a human subject, e.g., where the composition is sterile and is free of detectable pyrogens and/or other toxins. Thus, the present disclosure provides a composition comprising: a) a TMMP of the present disclosure; and b) saline (e.g., 0.9% NaCl), where the composition is sterile and is free of detectable pyrogens and/or other toxins.

### Compositions comprising a nucleic acid or a recombinant expression vector

The present disclosure provides compositions, e.g., pharmaceutical compositions, comprising a nucleic acid or a recombinant expression vector of the present disclosure. A wide variety of pharmaceutically acceptable excipients is known in the art and need not be discussed in detail herein. Pharmaceutically acceptable excipients have been amply described in a variety of publications, including, for example, A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., eds 7th ed., Lippincott, Williams, & Wilkins; and Handbook of Pharmaceutical Excipients (2000) A. H. Kibbe et al., eds., 3rd ed. Amer. Pharmaceutical Assoc.

A composition of the present disclosure can include: a) one or more nucleic acids or one or more recombinant expression vectors comprising nucleotide sequences encoding a TMMP; and b) one or more of: a buffer, a surfactant, an antioxidant, a hydrophilic polymer, a dextrin, a chelating agent, a suspending agent, a solubilizer, a thickening agent, a stabilizer, a bacteriostatic agent, a wetting agent, and a preservative. Suitable buffers include, but are not limited to, (such as N,N-bis(2-hydroxyethyl)-2-aminoethanesulfonic acid (BES), bis(2-hydroxyethyl)amino-tris(hydroxymethyl)methane (BIS-Tris), N-(2-hydroxyethyl)piperazine-N'3-propanesulfonic acid (EPPS or HEPPS), glycylglycine, N-2-hydroxyehtylpiperazine-N'-2-ethanesulfonic acid (HEPES), 3-(N-morpholino)propane sulfonic acid (MOPS), piperazine-N,N'-bis(2-ethane-sulfonic acid) (PIPES), sodium bicarbonate, 3-(N-tris(hydroxymethyl)-methyl-amino)-2-hydroxy-propanesulfonic acid) TAPSO, (N-tris(hydroxymethyl)methyl-2-aminoethanesulfonic acid (TES), N-tris(hydroxymethyl)methyl-glycine (Tricine), tris(hydroxymethyl)-aminomethane (Tris), etc.). Suitable salts include, e.g., NaCl, MgCl₂, KCl, MgSO₄, etc.

A pharmaceutical formulation of the present disclosure can include a nucleic acid or recombinant expression vector of the present disclosure in an amount of from about 0.001% to about 90% (w/w). In the description of formulations, below, "subject nucleic acid or recombinant expression vector" will be understood to include a nucleic acid or recombinant expression vector of the present disclosure. For example, in some cases, a subject formulation comprises a nucleic acid or recombinant expression vector of the present disclosure.

A subject nucleic acid or recombinant expression vector can be admixed, encapsulated, conjugated or otherwise associated with other compounds or mixtures of compounds; such compounds can include, e.g., liposomes or receptor-targeted molecules. A subject nucleic acid or recombinant expression vector can be combined in a formulation with one or more components that assist in uptake, distribution and/or absorption.

A subject nucleic acid or recombinant expression vector composition can be formulated into any of many possible dosage forms such as, but not limited to, tablets, capsules, gel capsules, liquid syrups, soft gels, suppositories, and enemas. A subject nucleic acid or recombinant expression vector composition can also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

A formulation comprising a subject nucleic acid or recombinant expression vector can be a liposomal formulation. As used herein, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes that can interact with negatively charged DNA molecules to form a stable complex. Liposomes that are pH sensitive or negatively charged are believed to entrap DNA rather than complex with it. Both cationic and noncationic liposomes can be used to deliver a subject nucleic acid or recombinant expression vector.

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Pat. No. 6,287,860, which is incorporated herein by reference in its entirety.

The formulations and compositions of the present disclosure may also include surfactants. The use of surfactants in drug products, formulations and in emulsions is well known in the art. Surfactants and their uses are further described in U.S. Pat. No. 6,287,860.

In one embodiment, various penetration enhancers are included, to effect the efficient delivery of nucleic acids. In addition to aiding the diffusion of non-lipophilic drugs across cell membranes, penetration enhancers also enhance the permeability of lipophilic drugs. Penetration enhancers may be classified as belonging to one of five broad categories, i.e., surfactants, fatty acids, bile salts, chelating agents, and non-chelating non-surfactants. Penetration enhancers and their uses are further described in U.S. Pat. No. 6,287,860, which is incorporated herein by reference in its entirety.

Compositions and formulations for oral administration include powders or granules, microparticulates, nanoparticulates, suspensions or solutions in water or non-aqueous media, capsules, gel capsules, sachets, tablets, or minitablets. Thickeners, flavoring agents, diluents, emulsifiers, dispersing aids or binders may be desirable. Suitable oral formulations include those in which a subject antisense nucleic acid is administered in conjunction with one or more penetration enhancers surfactants and chelators. Suitable surfactants include, but are not limited to, fatty acids and/or esters or salts thereof, bile acids and/or salts thereof. Suitable bile acids/salts and fatty acids and their uses are further described in U.S. Pat. No. 6,287,860. Also suitable are combinations of penetration enhancers, for example, fatty acids/salts in combination with bile acids/salts. An exemplary suitable combination is the sodium salt of lauric acid, capric acid, and UDCA. Further penetration enhancers include, but are not limited to, polyoxyethylene-9-lauryl ether, and polyoxyethylene-20-cetyl ether. Suitable penetration enhancers also include propylene glycol, dimethylsulfoxide, triethanoiamine, N,N-dimethylacetamide, N,N-dimethylformamide, 2-pyrrolidone and derivatives thereof, tetrahydrofurfuryl alcohol, and AZONE^{™}.

### METHODS OF MODULATING T CELL ACTIVITY

The present disclosure provides a method of selectively modulating the activity of an epitope-specific T cell, the method comprising contacting the T cell with a TMMP of the present disclosure, where contacting the T cell with a TMMP of the present disclosure selectively modulates the activity of the epitope-specific T cell. In some cases, the contacting occurs *in vitro.* In some cases, the contacting occurs *in vivo.* In some cases, the contacting occurs *ex vivo.*

In some cases, e.g., where the target T cell is a CD8⁺ T cell, the TMMP comprises Class I MHC polypeptides (e.g., β2-microglobulin and Class I MHC heavy chain).

Where a TMMP of the present disclosure includes an immunomodulatory polypeptide that is an activating polypeptide, contacting the T cell with the TMMP activates the epitope-specific T cell. In some instances, the epitope-specific T cell is a T cell that is specific for an epitope present on a cancer cell, and contacting the epitope-specific T cell with the TMMP increases cytotoxic activity of the T cell toward the cancer cell. In some instances, the epitope-specific T cell is a T cell that is specific for an epitope present on a cancer cell, and contacting the epitope-specific T cell with the TMMP increases the number of the epitope-specific T cells.

In some instances, the epitope-specific T cell is a T cell that is specific for an epitope present on a virus-infected cell, and contacting the epitope-specific T cell with the TMMP increases cytotoxic activity of the T cell toward the virus-infected cell. In some instances, the epitope-specific T cell is a T cell that is specific for an epitope present on a virus-infected cell, and contacting the epitope-specific T cell with the TMMP increases the number of the epitope-specific T cells.

Where a TMMP of the present disclosure includes an immunomodulatory polypeptide that is an inhibiting polypeptide, contacting the T cell with the TMMP inhibits the epitope-specific T cell. In some instances, the epitope-specific T cell is a self-reactive T cell that is specific for an epitope present in a self antigen, and the contacting reduces the number of the self-reactive T cells.

The present disclosure provides a method of modulating an immune response in an individual, the method comprising administering to the individual an effective amount of a TMMP of the present disclosure. Administering the TMMP induces an epitope-specific T cell response (e.g., a WT-1 epitope-specific T-cell response) and an epitope-non-specific T cell response, where the ratio of the epitope-specific T cell response to the epitope-non-specific T cell response is at least 2:1. In some cases, the ratio of the epitope-specific T cell response to the epitope-non-specific T cell response is at least 5:1. In some cases, the ratio of the epitope-specific T cell response to the epitope-non-specific T cell response is at least 10:1. In some cases, the ratio of the epitope-specific T cell response to the epitope-non-specific T cell response is at least 25:1. In some cases, the ratio of the epitope-specific T cell response to the epitope-non-specific T cell response is at least 50:1. In some cases, the ratio of the epitope-specific T cell response to the epitope-non-specific T cell response is at least 100: 1. In some cases, the individual is a human. In some cases, the modulating increases a cytotoxic T-cell response to a cancer cell, e.g., a WT-1-expressing cancer cell. In some cases, the administering is intravenous, subcutaneous, intramuscular, systemic, intralymphatic, distal to a treatment site, local, or at or near a treatment site.

The present disclosure provides a method of delivering a costimulatory (i.e., immunomodulatory) polypeptide selectively to target T cell, the method comprising contacting a mixed population of T cells with a TMMP of the present disclosure, where the mixed population of T cells comprises the target T cell and non-target T cells, where the target T cell is specific for the epitope present within the TMMP (e.g., where the target T cell is specific for the WT-1 epitope present within the TMMP), and where the contacting step delivers the one or more costimulatory polypeptides (immunomodulatory polypeptides) present within the TMMP to the target T cell. In some cases, the population of T cells is *in vitro.* In some cases, the population of T cells is *in vivo* in an individual. In some cases, the method comprises administering the TMMP to the individual. In some case, the T cell is a cytotoxic T cell. In some cases, the mixed population of T cells is an *in vitro* population of mixed T cells obtained from an individual, and the contacting step results in activation and/or proliferation of the target T cell, generating a population of activated and/or proliferated target T cells; in some of these instances, the method further comprises administering the population of activated and/or proliferated target T cells to the individual.

The present disclosure provides a method of detecting, in a mixed population of T cells obtained from an individual, the presence of a target T cell that binds an epitope of interest (e.g., a WT-1 epitope), the method comprising: a) contacting *in vitro* the mixed population of T cells with a TMMP of the present disclosure, wherein the TMMP comprises the epitope of interest (e.g., the WT-1 epitope); and b) detecting activation and/or proliferation of T cells in response to said contacting, wherein activated and/or proliferated T cells indicates the presence of the target T cell.

### TREATMENT METHODS

The present disclosure provides a method of treatment of an individual, the method comprising administering to the individual an amount of a TMMP of the present disclosure, or one or more nucleic acids encoding the TMMP, effective to treat the individual. Also provided is a TMMP of the present disclosure for use in a method of treatment of the human or animal body. In some cases, a treatment method of the present disclosure comprises administering to an individual in need thereof one or more recombinant expression vectors comprising nucleotide sequences encoding a TMMP of the present disclosure. In some cases, a treatment method of the present disclosure comprises administering to an individual in need thereof one or more mRNA molecules comprising nucleotide sequences encoding a TMMP of the present disclosure. In some cases, a treatment method of the present disclosure comprises administering to an individual in need thereof a TMMP of the present disclosure. Conditions that can be treated include, e.g., cancer and autoimmune disorders, as described below.

In some cases, a TMMP of the present disclosure, when administered to an individual in need thereof, induces both an epitope-specific T cell response and an epitope non-specific T cell response. In other words, in some cases, a TMMP of the present disclosure, when administered to an individual in need thereof, induces an epitope-specific T cell response by modulating the activity of a first T cell that displays both: i) a TCR specific for the epitope present in the TMMP; ii) a co-immunomodulatory polypeptide that binds to the immunomodulatory polypeptide present in the TMMP; and induces an epitope non-specific T cell response by modulating the activity of a second T cell that displays: i) a TCR specific for an epitope other than the epitope present in the TMMP; and ii) a co-immunomodulatory polypeptide that binds to the immunomodulatory polypeptide present in the TMMP. The ratio of the epitope-specific T cell response to the epitope-non-specific T cell response is at least 2:1, at least 5:1, at least 10:1, at least 15:1, at least 20:1, at least 25:1, at least 50:1, or at least 100:1. The ratio of the epitope-specific T cell response to the epitope-non-specific T cell response is from about 2:1 to about 5:1, from about 5:1 to about 10:1, from about 10:1 to about 15:1, from about 15:1 to about 20:1, from about 20:1 to about 25:1, from about 25:1 to about 50:1, or from about 50:1 to about 100:1, or more than 100:1. "Modulating the activity" of a T cell can include one or more of: i) activating a cytotoxic (e.g., CD8⁺) T cell; ii) inducing cytotoxic activity of a cytotoxic (e.g., CD8⁺) T cell; iii) inducing production and release of a cytotoxin (e.g., a perforin; a granzyme; a granulysin) by a cytotoxic (e.g., CD8⁺) T cell; iv) inhibiting activity of an autoreactive T cell; and the like.

The combination of the reduced affinity of the immunomodulatory polypeptide for its cognate co-immunomodulatory polypeptide, and the affinity of the epitope for a TCR, provides for enhanced selectivity of a TMMP of the present disclosure. Thus, for example, a TMMP of the present disclosure binds with higher avidity to a first T cell that displays both: i) a TCR specific for the epitope present in the TMMP; and ii) a co-immunomodulatory polypeptide that binds to the immunomodulatory polypeptide present in the TMMP, compared to the avidity to which it binds to a second T cell that displays: i) a TCR specific for an epitope other than the epitope present in the TMMP; and ii) a co-immunomodulatory polypeptide that binds to the immunomodulatory polypeptide present in the TMMP.

The present disclosure provides a method of selectively modulating the activity of an epitope-specific T cell in an individual, the method comprising administering to the individual an effective amount of a TMMP of the present disclosure, or one or more nucleic acids (e.g., expression vectors; mRNA; etc.) comprising nucleotide sequences encoding the TMMP, where the TMMP selectively modulates the activity of the epitope-specific T cell in the individual. Selectively modulating the activity of an epitope-specific T cell can treat a disease or disorder in the individual. Thus, the present disclosure provides a treatment method comprising administering to an individual in need thereof an effective amount of a TMMP of the present disclosure.

In some cases, the immunomodulatory polypeptide ("MOD") is an activating polypeptide, and the TMMP activates the epitope-specific T cell. In some cases, the epitope is a cancer-associated epitope, and the TMMP increases the activity of a T cell specific for the cancer-associate epitope. In some cases, the MOD is an activating polypeptide, and the TMMP activates a WT-1 epitope-specific T-cell. In some cases, the T cells are T-helper cells (CD4⁺ cells), cytotoxic T-cells (CD8⁺ cells), or NK-T-cells. In some cases, the epitope is a WT-1 epitope, and the TMMP increases the activity of a T-cell specific for a cancer cell expressing the WT-1 epitope (e.g., T-helper cells (CD4⁺ cells), cytotoxic T-cells (CD8⁺ cells), and/or NK-T-cells). Activation of CD4⁺ T cells can include increasing proliferation of CD4⁺ T cells and/or inducing or enhancing release cytokines by CD4⁺ T cells. Activation of NK-T-cells and/or CD8⁺ cells can include: increasing proliferation of NK-T-cells and/or CD8⁺ cells; and/or inducing release of cytokines such as interferon γ by NK-T-cells and/or CD8⁺ cells. In some cases, a TMMP of the present disclosure reduces proliferation and/or activity of a regulatory T (Treg) cell. Tregs are FoxP3⁺, CD4⁺ T cells. In some cases, e.g., where a TMMP of the present disclosure comprises an inhibitory immunomodulatory polypeptide (e.g., PD-L1, FasL, and the like), the TMMP reduces the proliferation and/or activity of a Treg.

In some cases, the immunomodulatory polypeptide is an activating polypeptide, and the TMMP activates the epitope-specific T cell. In some cases, the epitope is a cancer-associated epitope, and the TMMP increases the activity of a T cell specific for the cancer-associate epitope.

Where a TMMP of the present disclosure comprises a WT-1 peptide epitope, the TMMP can be administered to an individual having a WT-1-expressing cancer. WT1-expressing cancers include a leukemia, a desmoplastic small round cell tumor, a gastric cancer, a colon cancer, a lung cancer, a breast cancer, a germ cell tumor, an ovarian cancer, a uterine cancer, a thyroid cancer, a liver cancer, a renal cancer, a Kaposi's sarcoma, a sarcoma, a hepatocellular carcinoma, a Wilms' tumor, an acute myelogenous leukemia (AML), a myelodysplastic syndrome (MDS), an a non-small cell lung cancer (NSCLC), a myeloma, pancreatic cancer, colorectal cancer, a mesothelioma, a soft tissue sarcoma, a neuroblastoma, and a nephroblastoma.

Where a TMMP of the present disclosure comprises a WT-1 peptide epitope, the TMMP can be administered to an individual in need thereof to treat acute myeloid leukemia (AML) in the individual. Where a TMMP of the present disclosure comprises a WT-1 peptide epitope, the TMMP can be administered to an individual in need thereof to treat a myeloma in the individual. Where a TMMP of the present disclosure comprises a WT-1 peptide epitope, the TMMP can be administered to an individual in need thereof to treat ovarian cancer in the individual. Where a TMMP of the present disclosure comprises a WT-1 peptide epitope, the TMMP can be administered to an individual in need thereof to treat pancreatic cancer in the individual. Where a TMMP of the present disclosure comprises a WT-1 peptide epitope, the TMMP can be administered to an individual in need thereof to treat non-small cell lung cancer (NSCLC) in the individual. Where a TMMP of the present disclosure comprises a WT-1 peptide epitope, the TMMP can be administered to an individual in need thereof to treat colorectal cancer (CRC) in the individual. Where a TMMP of the present disclosure comprises a WT-1 peptide epitope, the TMMP can be administered to an individual in need thereof to treat breast cancer in the individual. Where a TMMP of the present disclosure comprises a WT-1 peptide epitope, the TMMP can be administered to an individual in need thereof to treat a Wilms tumor in the individual. Where a TMMP of the present disclosure comprises a WT-1 peptide epitope, the TMMP can be administered to an individual in need thereof to treat mesothelioma in the individual. Where a TMMP of the present disclosure comprises a WT-1 peptide epitope, the TMMP can be administered to an individual in need thereof to treat soft tissue sarcoma in the individual. Where a TMMP of the present disclosure comprises a WT-1 peptide epitope, the TMMP can be administered to an individual in need thereof to treat a neuroblastoma in the individual. Where a TMMP of the present disclosure comprises a WT-1 peptide epitope, the TMMP can be administered to an individual in need thereof to treat a nephroblastoma in the individual.

The present disclosure provides a method of treating cancer in an individual, the method comprising administering to the individual an effective amount of a TMMP of the present disclosure, or one or more nucleic acids (e.g., expression vectors; mRNA; etc.) comprising nucleotide sequences encoding the TMMP, where the TMMP comprises a T-cell epitope that is a cancer epitope, and where the TMMP comprises a stimulatory immunomodulatory polypeptide. In some cases, an "effective amount" of a TMMP of the present disclosure is an amount that, when administered in one or more doses to an individual in need thereof, reduces the number of cancer cells in the individual. For example, in some cases, an "effective amount" of a TMMP of the present disclosure is an amount that, when administered in one or more doses to an individual in need thereof, reduces the number of cancer cells in the individual by at least 10%, at least 15%, at least 20%, at least 25%, at least 30%, at least 40%, at least 50%, at least 60%, at least 70%, at least 80%, at least 90%, or at least 95%, compared to the number of cancer cells in the individual before administration of the TMMP, or in the absence of administration with the TMMP. In some cases, an "effective amount" of a TMMP of the present disclosure is an amount that, when administered in one or more doses to an individual in need thereof, reduces the number of cancer cells in the individual to undetectable levels.

In some cases, an "effective amount" of a TMMP of the present disclosure is an amount that, when administered in one or more doses to an individual in need thereof, reduces the tumor mass in the individual. For example, in some cases, an "effective amount" of a TMMP of the present disclosure is an amount that, when administered in one or more doses to an individual in need thereof (an individual having a tumor), reduces the tumor mass in the individual by at least 10%, at least 15%, at least 20%, at least 25%, at least 30%, at least 40%, at least 50%, at least 60%, at least 70%, at least 80%, at least 90%, or at least 95%, compared to the tumor mass in the individual before administration of the TMMP, or in the absence of administration with the TMMP. In some cases, an "effective amount" of a TMMP of the present disclosure is an amount that, when administered in one or more doses to an individual in need thereof (an individual having a tumor), reduces the tumor volume in the individual. For example, in some cases, an "effective amount" of a TMMP of the present disclosure is an amount that, when administered in one or more doses to an individual in need thereof (an individual having a tumor), reduces the tumor volume in the individual by at least 10%, at least 15%, at least 20%, at least 25%, at least 30%, at least 40%, at least 50%, at least 60%, at least 70%, at least 80%, at least 90%, or at least 95%, compared to the tumor volume in the individual before administration of the TMMP, or in the absence of administration with the TMMP. In some cases, an "effective amount" of a TMMP of the present disclosure is an amount that, when administered in one or more doses to an individual in need thereof, increases survival time of the individual. For example, in some cases, an "effective amount" of a TMMP of the present disclosure is an amount that, when administered in one or more doses to an individual in need thereof, increases survival time of the individual by at least 1 month, at least 2 months, at least 3 months, from 3 months to 6 months, from 6 months to 1 year, from 1 year to 2 years, from 2 years to 5 years, from 5 years to 10 years, or more than 10 years, compared to the expected survival time of the individual in the absence of administration with the TMMP.

In some instances, the epitope-specific T cell is a T cell that is specific for an epitope present on a virus-infected cell, and contacting the epitope-specific T cell with the TMMP increases cytotoxic activity of the T cell toward the virus-infected cell. In some instances, the epitope-specific T cell is a T cell that is specific for an epitope present on a virus-infected cell, and contacting the epitope-specific T cell with the TMMP increases the number of the epitope-specific T cells.

Thus, the present disclosure provides a method of treating a virus infection in an individual, the method comprising administering to the individual an effective amount of a TMMP of the present disclosure, or one or more nucleic acids comprising nucleotide sequences encoding the TMMP, where the TMMP comprises a T-cell epitope that is a viral epitope, and where the TMMP comprises a stimulatory immunomodulatory polypeptide. In some cases, an "effective amount" of a TMMP is an amount that, when administered in one or more doses to an individual in need thereof, reduces the number of virus-infected cells in the individual. For example, in some cases, an "effective amount" of a TMMP of the present disclosure is an amount that, when administered in one or more doses to an individual in need thereof, reduces the number of virus-infected cells in the individual by at least 10%, at least 15%, at least 20%, at least 25%, at least 30%, at least 40%, at least 50%, at least 60%, at least 70%, at least 80%, at least 90%, or at least 95%, compared to the number of virus-infected cells in the individual before administration of the TMMP, or in the absence of administration with the TMMP. In some cases, an "effective amount" of a TMMP of the present disclosure is an amount that, when administered in one or more doses to an individual in need thereof, reduces the number of virus-infected cells in the individual to undetectable levels.

Thus, the present disclosure provides a method of treating an infection in an individual, the method comprising administering to the individual an effective amount of a TMMP of the present disclosure, or one or more nucleic acids comprising nucleotide sequences encoding the TMMP, where the TMMP comprises a T-cell epitope that is a pathogen-associated epitope, and where the TMMP comprises a stimulatory immunomodulatory polypeptide. In some cases, an "effective amount" of a TMMP of the present disclosure is an amount that, when administered in one or more doses to an individual in need thereof, reduces the number of pathogens in the individual. For example, in some cases, an "effective amount" of a TMMP of the present disclosure is an amount that, when administered in one or more doses to an individual in need thereof, reduces the number of pathogens in the individual by at least 10%, at least 15%, at least 20%, at least 25%, at least 30%, at least 40%, at least 50%, at least 60%, at least 70%, at least 80%, at least 90%, or at least 95%, compared to the number of pathogens in the individual before administration of the TMMP, or in the absence of administration with the TMMP. In some cases, an "effective amount" of a TMMP of the present disclosure is an amount that, when administered in one or more doses to an individual in need thereof, reduces the number of pathogens in the individual to undetectable levels. Pathogens include viruses, bacteria, protozoans, and the like.

In some cases, the immunomodulatory polypeptide is an inhibitory polypeptide, and the TMMP inhibits activity of the epitope-specific T cell. In some cases, the epitope is a self-epitope, and the TMMP selectively inhibits the activity of a T cell specific for the self-epitope.

The present disclosure provides a method of treating an autoimmune disorder in an individual, the method comprising administering to the individual an effective amount of a TMMP of the present disclosure, or one or more nucleic acids comprising nucleotide sequences encoding the TMMP, where the TMMP comprises a T-cell epitope that is a self epitope, and where the TMMP comprises an inhibitory immunomodulatory polypeptide. In some cases, an "effective amount" of a TMMP of the present disclosure is an amount that, when administered in one or more doses to an individual in need thereof, reduces the number self-reactive T cells by at least 10%, at least 15%, at least 20%, at least 25%, at least 30%, at least 40%, at least 50%, at least 60%, at least 70%, at least 80%, at least 90%, or at least 95%, compared to number of self-reactive T cells in the individual before administration of the TMMP, or in the absence of administration with the TMMP. In some cases, an "effective amount" of a TMMP is an amount that, when administered in one or more doses to an individual in need thereof, reduces production of Th2 cytokines in the individual. In some cases, an "effective amount" of a TMMP of the present disclosure is an amount that, when administered in one or more doses to an individual in need thereof, ameliorates one or more symptoms associated with an autoimmune disease in the individual.

As noted above, in some cases, in carrying out a subject treatment method, a TMMP of the present disclosure is administered to an individual in need thereof, as the TMMP *per se.* In other instances, in carrying out a subject treatment method, one or more nucleic acids comprising nucleotide sequences encoding a TMMP of the present disclosure is/are administering to an individual in need thereof. Thus, in other instances, one or more nucleic acids of the present disclosure, e.g., one or more recombinant expression vectors of the present disclosure, is/are administered to an individual in need thereof.

### Formulations

Suitable formulations are described above, where suitable formulations include a pharmaceutically acceptable excipient. In some cases, a suitable formulation comprises: a) a TMMP of the present disclosure; and b) a pharmaceutically acceptable excipient. In some cases, a suitable formulation comprises: a) a nucleic acid comprising a nucleotide sequence encoding a TMMP of the present disclosure; and b) a pharmaceutically acceptable excipient; in some instances, the nucleic acid is an mRNA. In some cases, a suitable formulation comprises: a) a first nucleic acid comprising a nucleotide sequence encoding the first polypeptide of a TMMP of the present disclosure; b) a second nucleic acid comprising a nucleotide sequence encoding the second polypeptide of a TMMP of the present disclosure; and c) a pharmaceutically acceptable excipient. In some cases, a suitable formulation comprises: a) a recombinant expression vector comprising a nucleotide sequence encoding a TMMP of the present disclosure; and b) a pharmaceutically acceptable excipient. In some cases, a suitable formulation comprises: a) a first recombinant expression vector comprising a nucleotide sequence encoding the first polypeptide of a TMMP of the present disclosure; b) a second recombinant expression vector comprising a nucleotide sequence encoding the second polypeptide of a TMMP of the present disclosure; and c) a pharmaceutically acceptable excipient.

Suitable pharmaceutically acceptable excipients are described above.

### Dosages

A suitable dosage can be determined by an attending physician or other qualified medical personnel, based on various clinical factors. As is well known in the medical arts, dosages for any one patient depend upon many factors, including the patient's size, body surface area, age, the particular polypeptide or nucleic acid to be administered, sex of the patient, time, and route of administration, general health, and other drugs being administered concurrently. A TMMP of the present disclosure may be administered in amounts between 1 ng/kg body weight and 20 mg/kg body weight per dose, e.g. between 0.1 mg/kg body weight to 10 mg/kg body weight, e.g. between 0.5 mg/kg body weight to 5 mg/kg body weight; however, doses below or above this exemplary range are envisioned, especially considering the aforementioned factors. If the regimen is a continuous infusion, it can also be in the range of 1 µg to 10 mg per kilogram of body weight per minute. A TMMP of the present disclosure can be administered in an amount of from about 1 mg/kg body weight to 50 mg/kg body weight, e.g., from about 1 mg/kg body weight to about 5 mg/kg body weight, from about 5 mg/kg body weight to about 10 mg/kg body weight, from about 10 mg/kg body weight to about 15 mg/kg body weight, from about 15 mg/kg body weight to about 20 mg/kg body weight, from about 20 mg/kg body weight to about 25 mg/kg body weight, from about 25 mg/kg body weight to about 30 mg/kg body weight, from about 30 mg/kg body weight to about 35 mg/kg body weight, from about 35 mg/kg body weight to about 40 mg/kg body weight, or from about 40 mg/kg body weight to about 50 mg/kg body weight.

In some cases, a suitable dose of a TMMP of the present disclosure is from 0.01 µg to 100 g per kg of body weight, from 0.1 µg to 10 g per kg of body weight, from 1 µg to 1 g per kg of body weight, from 10 µg to 100 mg per kg of body weight, from 100 µg to 10 mg per kg of body weight, or from 100 µg to 1 mg per kg of body weight. Persons of ordinary skill in the art can easily estimate repetition rates for dosing based on measured residence times and concentrations of the administered agent in bodily fluids or tissues. Following successful treatment, it may be desirable to have the patient undergo maintenance therapy to prevent the recurrence of the disease state, wherein a TMMP of the present disclosure is administered in maintenance doses, ranging from 0.01 µg to 100 g per kg of body weight, from 0.1 µg to 10 g per kg of body weight, from 1 µg to 1 g per kg of body weight, from 10 µg to 100 mg per kg of body weight, from 100 µg to 10 mg per kg of body weight, or from 100 µg to 1 mg per kg of body weight.

Those of skill will readily appreciate that dose levels can vary as a function of the specific TMMP, the severity of the symptoms and the susceptibility of the subject to side effects. Preferred dosages for a given compound are readily determinable by those of skill in the art by a variety of means.

In some cases, multiple doses of a TMMP of the present disclosure, a nucleic acid of the present disclosure, or a recombinant expression vector of the present disclosure are administered. The frequency of administration of a TMMP of the present disclosure, a nucleic acid of the present disclosure, or a recombinant expression vector of the present disclosure can vary depending on any of a variety of factors, e.g., severity of the symptoms, etc. For example, in some cases, a TMMP of the present disclosure, a nucleic acid of the present disclosure, or a recombinant expression vector of the present disclosure is administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid).

The duration of administration of a TMMP of the present disclosure, a nucleic acid of the present disclosure, or a recombinant expression vector of the present disclosure, e.g., the period of time over which a TMMP of the present disclosure, a nucleic acid of the present disclosure, or a recombinant expression vector of the present disclosure is administered, can vary, depending on any of a variety of factors, e.g., patient response, etc. For example, a TMMP of the present disclosure, a nucleic acid of the present disclosure, or a recombinant expression vector of the present disclosure can be administered over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

### Routes of administration

An active agent (a TMMP of the present disclosure, a nucleic acid of the present disclosure, or a recombinant expression vector of the present disclosure) is administered to an individual using any available method and route suitable for drug delivery, including *in vivo* and *ex vivo* methods, as well as systemic and localized routes of administration.

Conventional and pharmaceutically acceptable routes of administration include intratumoral, peritumoral, intramuscular, intralymphatic, intratracheal, intracranial, subcutaneous, intradermal, topical application, intravenous, intraarterial, rectal, nasal, oral, and other enteral and parenteral routes of administration. Routes of administration may be combined, if desired, or adjusted depending upon the TMMP and/or the desired effect. A TMMP of the present disclosure, or a nucleic acid or recombinant expression vector of the present disclosure, can be administered in a single dose or in multiple doses.

In some cases, a TMMP of the present disclosure, a nucleic acid of the present disclosure, or a recombinant expression vector of the present disclosure is administered intravenously. In some cases, a TMMP of the present disclosure, a nucleic acid of the present disclosure, or a recombinant expression vector of the present disclosure is administered intramuscularly. In some cases, a TMMP of the present disclosure, a nucleic acid of the present disclosure, or a recombinant expression vector of the present disclosure is administered intralymphatically. In some cases, a TMMP of the present disclosure, a nucleic acid of the present disclosure, or a recombinant expression vector of the present disclosure is administered locally. In some cases, a TMMP of the present disclosure, a nucleic acid of the present disclosure, or a recombinant expression vector of the present disclosure is administered intratumorally. In some cases, a TMMP of the present disclosure, a nucleic acid of the present disclosure, or a recombinant expression vector of the present disclosure is administered peritumorally. In some cases, a TMMP of the present disclosure, a nucleic acid of the present disclosure, or a recombinant expression vector of the present disclosure is administered intracranially. In some cases, a TMMP of the present disclosure, a nucleic acid of the present disclosure, or a recombinant expression vector of the present disclosure is administered subcutaneously.

In some cases, a TMMP of the present disclosure is administered intravenously. In some cases, a TMMP of the present disclosure is administered intramuscularly. In some cases, a TMMP of the present disclosure is administered locally. In some cases, a TMMP the present disclosure is administered intratumorally. In some cases, a TMMP of the present disclosure is administered peritumorally. In some cases, a TMMP of the present disclosure is administered intracranially. In some cases, a TMMP is administered subcutaneously. In some cases, a TMMP of the present disclosure is administered intralymphatically.

A TMMP of the present disclosure, a nucleic acid of the present disclosure, or a recombinant expression vector of the present disclosure can be administered to a host using any available conventional methods and routes suitable for delivery of conventional drugs, including systemic or localized routes. In general, routes of administration contemplated for use in a method of the present disclosure include, but are not necessarily limited to, enteral, parenteral, and inhalational routes.

Parenteral routes of administration other than inhalation administration include, but are not necessarily limited to, topical, transdermal, subcutaneous, intramuscular, intraorbital, intracapsular, intraspinal, intrasternal, intratumoral, intralymphatic, peritumoral, and intravenous routes, *i.e.,* any route of administration other than through the alimentary canal. Parenteral administration can be carried to effect systemic or local delivery of a TMMP of the present disclosure, a nucleic acid of the present disclosure, or a recombinant expression vector of the present disclosure. Where systemic delivery is desired, administration typically involves invasive or systemically absorbed topical or mucosal administration of pharmaceutical preparations.

### Combination therapies

In some cases, a method of the present disclosure for treating cancer in an individual comprises: a) administering a TMMP of the present disclosure; and b) administering at least one additional therapeutic agent or therapeutic treatment. Suitable additional therapeutic agents include, but are not limited to, a small molecule cancer chemotherapeutic agent, and an immune checkpoint inhibitor. Suitable additional therapeutic treatments include, e.g., radiation, surgery (e.g., surgical resection of a tumor), and the like.

A treatment method of the present disclosure can comprise co-administration of a TMMP of the present disclosure and at least one additional therapeutic agent. By "co-administration" is meant that both a TMMP of the present disclosure and at least one additional therapeutic agent are administered to an individual, although not necessarily at the same time, in order to achieve a therapeutic effect that is the result of having administered both the TMMP and the at least one additional therapeutic agent. The administration of the TMMP and the at least one additional therapeutic agent can be substantially simultaneous, e.g., the TMMP can be administered to an individual within about 1 minute to about 24 hours (e.g., within about 1 minute, within about 5 minutes, within about 15 minutes, within about 30 minutes, within about 1 hour, within about 4 hours, within about 8 hours, within about 12 hours, or within about 24 hours) of administration of the at least one additional therapeutic agent. In some cases, a TMMP of the present disclosure is administered to an individual who is undergoing treatment with, or who has undergone treatment with, the at least one additional therapeutic agent. The administration of the TMMP can occur at different times and/or at different frequencies.

As an example, a treatment method of the present disclosure can comprise co-administration of a TMMP of the present disclosure and an immune checkpoint inhibitor such as an antibody specific for an immune checkpoint. By "co-administration" is meant that both a TMMP of the present disclosure and an immune checkpoint inhibitor (e.g., an antibody specific for an immune checkpoint polypeptide) are administered to an individual, although not necessarily at the same time, in order to achieve a therapeutic effect that is the result of having administered both the TMMP and the immune checkpoint inhibitor (e.g., an antibody specific for an immune checkpoint polypeptide). The administration of the TMMP and the immune checkpoint inhibitor (e.g., an antibody specific for an immune checkpoint polypeptide) can be substantially simultaneous, e.g., the TMMP can be administered to an individual within about 1 minute to about 24 hours (e.g., within about 1 minute, within about 5 minutes, within about 15 minutes, within about 30 minutes, within about 1 hour, within about 4 hours, within about 8 hours, within about 12 hours, or within about 24 hours) of administration of the immune checkpoint inhibitor (e.g., an antibody specific for an immune checkpoint polypeptide). In some cases, a TMMP of the present disclosure is administered to an individual who is undergoing treatment with, or who has undergone treatment with, an immune checkpoint inhibitor (e.g., an antibody specific for an immune checkpoint polypeptide). The administration of the TMMP and the immune checkpoint inhibitor (e.g., an antibody specific for an immune checkpoint polypeptide) can occur at different times and/or at different frequencies.

Exemplary immune checkpoint inhibitors include inhibitors that target an immune checkpoint polypeptide such as CD27, CD28, CD40, CD122, CD96, CD73, CD47, OX40, GITR, CSF1R, JAK, PI3K delta, PI3K gamma, TAM, arginase, CD137 (also known as 4-1BB), ICOS, A2AR, B7-H3, B7-H4, BTLA, CTLA-4, LAG3, TIM3, VISTA, CD96, TIGIT, CD122, PD-1, PD-L1 and PD-L2. In some cases, the immune checkpoint polypeptide is a stimulatory checkpoint molecule selected from CD27, CD28, CD40, ICOS, OX40, GITR, CD122 and CD137. In some cases, the immune checkpoint polypeptide is an inhibitory checkpoint molecule selected from A2AR, B7-H3, B7-H4, BTLA, CTLA-4, IDO, KIR, LAG3, PD-1, TIM3, CD96, TIGIT and VISTA.

In some cases, the immune checkpoint inhibitor is an antibody specific for an immune checkpoint polypeptide. In some cases, the anti-immune checkpoint antibody is a monoclonal antibody. In some cases, the anti-immune checkpoint antibody is humanized, or de-immunized such that the antibody does not substantially elicit an immune response in a human. In some cases, the anti-immune checkpoint antibody is a humanized monoclonal antibody. In some cases, the anti-immune checkpoint antibody is a de-immunized monoclonal antibody. In some cases, the anti-immune checkpoint antibody is a fully human monoclonal antibody. In some cases, the anti-immune checkpoint antibody inhibits binding of the immune checkpoint polypeptide to a ligand for the immune checkpoint polypeptide. In some cases, the anti-immune checkpoint antibody inhibits binding of the immune checkpoint polypeptide to a receptor for the immune checkpoint polypeptide.

Suitable anti-immune checkpoint antibodies include, but are not limited to, nivolumab (Bristol-Myers Squibb), pembrolizumab (Merck), pidilizumab (Curetech), AMP-224 (GlaxoSmithKline/Amplimmune), MPDL3280A (Roche), MDX-1105 (Medarex, Inc./Bristol Myer Squibb), MEDI-4736 (Medimmune/AstraZeneca), arelumab (Merck Serono), ipilimumab (YERVOY, (Bristol-Myers Squibb), tremelimumab (Pfizer), pidilizumab (CureTech, Ltd.), IMP321 (Immutep S.A.), MGA271 (Macrogenics), BMS-986016 (Bristol-Meyers Squibb), lirilumab (Bristol-Myers Squibb), urelumab (Bristol-Meyers Squibb), PF-05082566 (Pfizer), IPH2101 (Innate Pharma/Bristol-Myers Squibb), MEDI-6469 (MedImmune/AZ), CP-870,893 (Genentech), Mogamulizumab (Kyowa Hakko Kirin), Varlilumab (CelIDex Therapeutics), Avelumab (EMD Serono), Galiximab (Biogen Idec), AMP-514 (Amplimmune/AZ), AUNP 12 (Aurigene and Pierre Fabre), Indoximod (NewLink Genetics), NLG-919 (NewLink Genetics), INCB024360 (Incyte); KN035; and combinations thereof. For example, in some cases, the immune checkpoint inhibitor is an anti-PD-1 antibody. Suitable anti-PD-1 antibodies include, e.g., nivolumab, pembrolizumab (also known as MK-3475), pidilizumab, SHR-1210, PDR001, and AMP-224. In some cases, the anti-PD-1 monoclonal antibody is nivolumab, pembrolizumab or PDR001. Suitable anti-PD1 antibodies are described in U.S. Patent Publication No. 2017/0044259. For pidilizumab, see, e.g., Rosenblatt et al. (2011) J. Immunother. 34:409-18. In some cases, the immune checkpoint inhibitor is an anti-CTLA-4 antibody. In some cases, the anti-CTLA-4 antibody is ipilimumab or tremelimumab. For tremelimumab, see, e.g., Ribas et al. (2013) J. Clin. Oncol. 31:616-22. In some cases, the immune checkpoint inhibitor is an anti-PD-L1 antibody. In some cases, the anti-PD-L1 monoclonal antibody is BMS-935559, MEDI4736, MPDL3280A (also known as RG7446), KN035, or MSB0010718C. In some embodiments, the anti-PD-L1 monoclonal antibody is MPDL3280A (atezolizumab) or MEDI4736 (durvalumab). For durvalumab, see, e.g., WO 2011/066389. For atezolizumab, see, e.g., U.S. Patent No. 8,217,149.

### Subjects suitable for treatment

Subjects suitable for treatment with a method of the present disclosure include individuals who have cancer, including individuals who have been diagnosed as having cancer, individuals who have been treated for cancer but who failed to respond to the treatment, and individuals who have been treated for cancer and who initially responded but subsequently became refractory to the treatment. Subjects suitable for treatment with a method of the present disclosure include individuals who have an infection (e.g., an infection with a pathogen such as a bacterium, a virus, a protozoan, etc.), including individuals who have been diagnosed as having an infection, and individuals who have been treated for an infection but who failed to respond to the treatment. Subjects suitable for treatment with a method of the present disclosure include individuals who have bacterial infection, including individuals who have been diagnosed as having a bacterial infection, and individuals who have been treated for a bacterial infection but who failed to respond to the treatment. Subjects suitable for treatment with a method of the present disclosure include individuals who have a viral infection, including individuals who have been diagnosed as having a viral infection, and individuals who have been treated for a viral infection but who failed to respond to the treatment. Subjects suitable for treatment with a method of the present disclosure include individuals who have an autoimmune disease, including individuals who have been diagnosed as having an autoimmune disease, and individuals who have been treated for an autoimmune disease but who failed to respond to the treatment.

### Examples of Non-Limiting Aspects of the Disclosure

### ASPECTS SET A

Aspects, including embodiments, of the present subject matter described above may be beneficial alone or in combination, with one or more other aspects or embodiments. Without limiting the foregoing description, certain non-limiting aspects of the disclosure numbered 1-95 are provided below. As will be apparent to those of skill in the art upon reading this disclosure, each of the individually numbered aspects may be used or combined with any of the preceding or following individually numbered aspects. This is intended to provide support for all such combinations of aspects and is not limited to combinations of aspects explicitly provided below:
Aspect 1. T-cell modulatory multimeric polypeptide comprising: at least one heterodimer comprising: a) a first polypeptide comprising: i) a Wilms tumor-1 (WT-1) peptide epitope; and ii) first major histocompatibility complex (MHC) polypeptide; b) a second polypeptide comprising a second MHC polypeptide, and c) at least one immunomodulatory polypeptide, wherein the first and/or the second polypeptide comprises the immunomodulatory polypeptide.
Aspect 2. A T-cell modulatory multimeric polypeptide of aspect 1, wherein at least one of the one or more immunomodulatory domains is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide, and wherein the epitope binds to a T-cell receptor (TCR) on a T cell with an affinity of at least 10⁻⁷ M, such that: i) the T-cell modulatory multimeric polypeptide binds to a first T cell with an affinity that is at least 25% higher than the affinity with which the T-cell modulatory multimeric polypeptide binds a second T cell, wherein the first T cell expresses on its surface the cognate co-immunomodulatory polypeptide and a TCR that binds the epitope with an affinity of at least 10⁻⁷ M, and wherein the second T cell expresses on its surface the cognate co-immunomodulatory polypeptide but does not express on its surface a TCR that binds the epitope with an affinity of at least 10⁻⁷ M; and/or ii) the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is in a range of from 1.5:1 to 10⁶:1.
Aspect 3. A T-cell modulatory multimeric polypeptide of aspect 2, wherein: a) the T-cell modulatory multimeric polypeptide binds to the first T cell with an affinity that is at least 50%, at least 2-fold, at least 5-fold, or at least 10-fold higher than the affinity with which it binds the second T cell; and/or b) the variant immunomodulatory polypeptide binds the co-immunomodulatory polypeptide with an affinity of from about 10⁻⁴ M to about 10⁻⁷ M, from about 10⁻⁴ M to about 10⁻⁶ M, from about 10⁻⁴ M to about 10⁻⁵ M; and/or c) wherein the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is at least 10:1, at least 50:1, at least 10²:1, or at least 10³:1.
Aspect 4. A T-cell modulatory multimeric polypeptide of any one of aspects 1-3, wherein the first or the second polypeptide comprises an immunoglobulin (Ig) Fc polypeptide.
Aspect 5. A T-cell modulatory multimeric polypeptide of aspect 4, wherein the Ig Fc polypeptide is an IgG1 Fc polypeptide.
Aspect 6. A T-cell modulatory multimeric polypeptide of aspect 5, wherein IgG1 Fc polypeptide comprises one or more amino acid substitutions selected from N297A, L234A, L235A, L234F, L235E, and P331S.
Aspect 7. A T-cell modulatory multimeric polypeptide of any one of aspects 1-6, wherein: a1) the first polypeptide comprises, in order from N-terminus to C-terminus: i) the WT-1 peptide epitope; ii) the first MHC polypeptide; and iii) at least one immunomodulatory polypeptide; and b2) the second polypeptide comprises, in order from N-terminus to C-terminus: i) the second MHC polypeptide; and ii) an immunoglobulin (Ig) Fc polypeptide; or a2) the first polypeptide comprises, in order from N-terminus to C-terminus: i) the WT-1 peptide epitope; and ii) the first MHC polypeptide; and b2) the second polypeptide comprises, in order from N-terminus to C-terminus: i) at least one immunomodulatory polypeptide; ii) the second MHC polypeptide; and iii) an Ig Fc polypeptide; or a3) the first polypeptide comprises, in order from N-terminus to C-terminus: i) the WT-1 peptide epitope; and ii) the first MHC polypeptide; and b3) the second polypeptide comprises, in order from N-terminus to C-terminus: i) the second MHC polypeptide; and ii) an Ig Fc polypeptide; and iii) at least one immunomodulatory polypeptide; or a4) the first polypeptide comprises, in order from N-terminus to C-terminus: i) the WT-1 peptide epitope; and ii) the first MHC polypeptide; and b4) the second polypeptide comprises, in order from N-terminus to C-terminus: i) the second MHC polypeptide; and ii) at least one immunomodulatory polypeptide; or a5) the first polypeptide comprises, in order from N-terminus to C-terminus: i) the WT-1 peptide epitope; and ii) the first MHC polypeptide; and b5) a second polypeptide comprises, in order from N-terminus to C-terminus: i) at least one immunomodulatory polypeptide; and ii) the second MHC polypeptide; or a6) the first polypeptide comprises, in order from N-terminus to C-terminus: i) the WT-1 peptide epitope; ii) the first MHC polypeptide; and iii) at least one immunomodulatory polypeptide; and b6) the second polypeptide comprises: i) the second MHC polypeptide.
Aspect 8. A T-cell modulatory multimeric polypeptide of any one of aspects 1-7, wherein the first polypeptide comprises a peptide linker between the WT-1 epitope and the first MHC polypeptide and/or wherein the second polypeptide comprises a peptide linker between the immunomodulatory polypeptide and the second MHC polypeptide.
Aspect 9. A T-cell modulatory multimeric polypeptide of aspect 8, wherein the peptide linker comprises the amino acid sequence (GGGGS)n (SEQ ID NO:284), where n is an integer from 1 to 10.
Aspect 10. A T-cell modulatory multimeric polypeptide of any one of aspects 1-9, wherein the first MHC polypeptide is a β2-microglobulin polypeptide; and wherein the second MHC polypeptide is an MHC class I heavy chain polypeptide.
Aspect 11. A T-cell modulatory multimeric polypeptide of any one of aspects 1-10, wherein the at least one immunomodulatory polypeptide is selected from the group consisting of a cytokine (e.g., an IL2 polypeptide, an IL7 polypeptide, an IL12 polypeptide, an IL15 polypeptide, an IL17 polypeptide, an IL21 polypeptide, an IL27 polypeptide, an IL-23 polypeptide, a TGFβ polypeptide, and the like; and including all family members, e.g., IL17A, IL-17B, IL-17C, IL-17D, IL-17E, IL-17F, IL-17E), a 4-1BBL polypeptide, an ICOS-L polypeptide, an OX-40L polypeptide, a CD80 polypeptide, a CD86 polypeptide, (CD80 and CD86 are also known as B7-1 and B7-2, respectively), a CD40 polypeptide, a CD70 polypeptide, a JAG1 (CD339) polypeptide, an ICAM (CD540 polypeptide, a PD-L1 polypeptide, a FasL polypeptide, a PD-L2 polypeptide, a PD-1H (VISTA) polypeptide, an ICOS-L (CD275) polypeptide, a GITRL polypeptide, an HVEM polypeptide, a CXCL10 polypeptide, a CXCL9 polypeptide, a CXCL11 polypeptide, a CXCL13 polypeptide, and a CX3CL1 polypeptide, a Galectin-9 polypeptide, a CD83 polypeptide, a CD30L polypeptide, a HLA-G polypeptide, a MICA polypeptide, a MICB polypeptide, a HVEM (CD270) polypeptide, a lymphotoxin beta receptor polypeptide, a 3/TR6 polypeptide, an ILT3 polypeptide, an ILT4 polypeptide, a CXCL10 polypeptide, a CXCL9 polypeptide, a CXCL11 polypeptide, a CXCL13 polypeptide, and a CX3CL1 polypeptide, and combinations thereof.
Aspect 12. A T-cell modulatory multimeric polypeptide of any one of aspects 1-11, wherein the at least one immunomodulatory polypeptide is an IL-2 polypeptide.
Aspect 13. A T-cell modulatory multimeric polypeptide of any one of aspects 1-12, wherein the multimeric polypeptide comprises at least two immunomodulatory polypeptides, and wherein at least two of the immunomodulatory polypeptides are the same.
Aspect 14. A T-cell modulatory multimeric polypeptide of aspect 13, wherein the 2 or more immunomodulatory polypeptides are in tandem.
Aspect 15. A T-cell modulatory multimeric polypeptide of any one of aspects 1-14, wherein the first polypeptide and the second polypeptide are covalently linked to one another.
Aspect 16. A T-cell modulatory multimeric polypeptide of aspect 15, wherein the covalent linkage is via a disulfide bond.
Aspect 17. A T-cell modulatory multimeric polypeptide of any one of aspects 1-16, wherein the first MHC polypeptide or a linker between the epitope and the first MHC polypeptide comprises an amino acid substitution to provide a first Cys residue, wherein the second MHC polypeptide comprises an amino acid substitution to provide a second Cys residue, and wherein the disulfide linkage is between the first and the second Cys residues.
Aspect 18. A T-cell modulatory multimeric polypeptide of any one of aspects 1-17, wherein the WT-1 peptide epitope has a length of from about 4 amino acids to about 25 amino acids.
Aspect 19. A T-cell modulatory multimeric polypeptide of any one of aspects 1-18, wherein the WT-1 peptide epitope comprises an amino acid sequence selected from the group consisting of: NLMNLGATL (SEQ ID NO:258), NYMNLGATL (SEQ ID NO:263), CMTWNQMNLGATLKG (SEQ ID NO:223), WNQMNLGATLKGVAA (SEQ ID NO:224), CMTWNYMNLGATLKG (SEQ ID NO:225), WNYMNLGATLKGVAA (SEQ ID NO:226), MTWNQMNLGATLKGV (SEQ ID NO:227), TWNQMNLGATLKGVA (SEQ ID NO:228), CMTWNLMNLGATLKG (SEQ ID NO:229), MTWNLMNLGATLKGV (SEQ ID NO:230), TWNLMNLGATLKGVA (SEQ ID NO:231), WNLMNLGATLKGVAA (SEQ ID NO:232), MNLGATLK (SEQ ID NO:233), MTWNYMNLGATLKGV SEQ ID NO:234), TWNYMNLGATLKGVA (SEQ ID NO:235), CMTWNQMNLGATLKGVA (SEQ ID NO:236), CMTWNLMNLGATLKGVA (SEQ ID NO:237), CMTWNYMNLGATLKGVA (SEQ ID NO:238), GYLRNPTAC (SEQ ID NO:239), GALRNPTAL (SEQ ID NO:240), YALRNPTAC (SEQ ID NO:241), GLLRNPTAC (SEQ ID NO:242), RYRPHPGAL (SEQ ID NO:243), YQRPHPGAL (SEQ ID NO:244), RLRPHPGAL (SEQ ID NO:245), RIRPHPGAL (SEQ ID NO:246), QFPNHSFKHEDPMGQ (SEQ ID NO:247), HSFKHEDPY (SEQ ID NO:248), QFPNHSFKHEDPM (SEQ ID NO:249), QFPNHSFKHEDPY (SEQ ID NO:250), KRPFMCAYPGCNK (SEQ ID NO:251), KRPFMCAYPGCYK (SEQ ID NO:252), FMCAYPGCY (SEQ ID NO:253), FMCAYPGCK (SEQ ID NO:254), KRPFMCAYPGCNKRY (SEQ ID NO:255), SEKRPFMCAYPGCNK (SEQ ID NO:256), KRPFMCAYPGCYKRY (SEQ ID NO:257), NLMNLGATL (SEQ ID NO:258), VLDFAPPGA (SEQ ID NO:259), RMFPNAPYL (SEQ ID NO:260), CMTWNQMN (SEQ ID NO:261), CYTWNQMNL (SEQ ID NO:269), NYMNLGATL (SEQ ID NO:263), YMFPNAPYL (SEQ ID NO:264), SLGEQQYSV (SEQ ID NO:265), CMTWNQMNL (SEQ ID NO:266), and NQMNLGATL (SEQ ID NO:267).
Aspect 20. A T-cell modulatory multimeric polypeptide of any one of aspects 1-18, wherein the WT-1 peptide comprises the amino acid sequence CMTWNQMNL (SEQ ID NO:266) or CYTWNQMNL (SEQ ID NO:269).
Aspect 21. A T-cell modulatory multimeric polypeptide of any one of aspects 1-20, wherein the first or the second MHC polypeptide comprises: a) an amino acid sequence having at least 95% amino acid sequence identity to the HLA-A*0101, HLA-A*0201, HLA-A*0201, HLA-A*1101, HLA-A*2301, HLA-A*2402, HLA-A*2407, HLA-A*3303, or HLA-A*3401 amino acid sequence depicted in FIG. 9A; or b) an amino acid sequence having at least 95% amino acid sequence identity to the HLA-B*0702, HLA-B*0801, HLA-B*1502, HLA-B*3802, HLA-B*4001, HLA-B*4601, or HLA-B*5301 amino acid sequence depicted in FIG. 10A; or c) an amino acid sequence having at least 95% amino acid sequence identity to the HLA-C*0102, HLA-C*0303, HLA-C*0304, HLA-C*0401, HLA-C*0602, HLA-C*0701, HLA-C*0702, HLA-C*0801, or HLA-C*1502 depicted in FIG. 11A.
Aspect 22. A T-cell modulatory multimeric polypeptide of any one of aspects 1-20, wherein the first MHC polypeptide is a β2M polypeptide, and wherein the second MHC polypeptide comprises an amino acid sequence having at least 95% amino acid sequence identity to an HLA-A*2402 polypeptide.
Aspect 23. A T-cell modulatory multimeric polypeptide of any one of aspects 1-20, wherein the first MHC polypeptide is a β2M polypeptide, and wherein the second MHC polypeptide is an HLA-A*1101 polypeptide.
Aspect 24. A T-cell modulatory multimeric polypeptide of any one of aspects 1-20, wherein the first MHC polypeptide is a β2M polypeptide, and wherein the second MHC polypeptide comprises an amino acid sequence having at least 95% amino acid sequence identity to an HLA-A*3303 polypeptide.
Aspect 25. A T-cell modulatory multimeric polypeptide of any one of aspects 1-20, wherein the first MHC polypeptide is a β2M polypeptide, and wherein the second MHC polypeptide comprises an amino acid sequence having at least 95% amino acid sequence identity to an HLA-A*0201 polypeptide.
Aspect 26. A T-cell modulatory multimeric polypeptide of any one of aspects 21-25, wherein the MHC heavy chain polypeptide comprises a Cys at position 236.
Aspect 27. A T-cell modulatory multimeric polypeptide of any one of aspects 21-26, wherein the β2M polypeptide comprises a Cys at position 12.
Aspect 28. A T-cell modulatory multimeric polypeptide of any one of aspects 1-27, wherein the immunomodulatory polypeptide is a variant IL-2 polypeptide comprising: i) an H16A substitution and an F42A substation; or ii) an H16T substitution and an F42A substitution.
Aspect 29. A T-cell modulatory multimeric polypeptide of any one of aspects 4-28, wherein the multimeric polypeptide comprises a first and a second heterodimer, and wherein the first and second heterodimers are covalently bound by one or more disulfide bonds between the Ig Fc polypeptides of the first and second heterodimers.
Aspect 30. A nucleic acid comprising a nucleotide sequence encoding a first or second polypeptide according to any one of aspects 1-28, wherein the first or second polypeptide comprises at least one immunomodulatory domain.
Aspect 31. An expression vector comprising the nucleic acid of aspect 30.
Aspect 32. A method of selectively modulating the activity of T cell specific for a Wilms tumor-1 (WT-1) epitope, the method comprising contacting the T cell with a T-cell modulatory multimeric polypeptide according to any one of aspects 1-29, wherein said contacting selectively modulates the activity of the WT-1 epitope-specific T cell.
Aspect 22. A method of treating a patient having a cancer, the method comprising administering to the patient an effective amount of a pharmaceutical composition comprising T-cell modulatory multimeric polypeptide according to any one of aspects 1-29.
Aspect 34. The method of aspect 33, wherein the cancer is hepatocellular carcinoma, pancreatic cancer, stomach cancer, colorectal cancer, hepatoblastoma, or an ovarian yolk sac tumor.
Aspect 35. The method of aspect 33 or aspect 34, wherein said administering is intramuscular.
Aspect 36. The method of aspect 33 or aspect 34, wherein said administering is intravenous.
Aspect 37. A method of modulating an immune response in an individual, the method comprising administering to the individual an effective amount of the T-cell modulatory multimeric polypeptide (TMMP) of any one of aspects 1-29, wherein said administering induces an epitope-specific T cell response (e.g., a T cell response specific for the WT-1 epitope present in the TMMP) and an epitope-non-specific T cell response, wherein the ratio of the epitope-specific T cell response to the epitope-non-specific T cell response is at least 2:1.
Aspect 38 The method of aspect 37, wherein the ratio of the epitope-specific T cell response to the epitope-non-specific T cell response is at least 5:1.
Aspect 39. The method of aspect 37, wherein the ratio of the epitope-specific T cell response to the epitope-non-specific T cell response is at least 10:1.
Aspect 40. The method of aspect 37, wherein the ratio of the epitope-specific T cell response to the epitope-non-specific T cell response is at least 25:1.
Aspect 41. The method of aspect 37, wherein the ratio of the epitope-specific T cell response to the epitope-non-specific T cell response is at least 50:1.
Aspect 42. The method of aspect 37, wherein the ratio of the epitope-specific T cell response to the epitope-non-specific T cell response is at least 100:1.
Aspect 43. The method of any one of aspects 37-42, wherein the individual is a human.
Aspect 44. The method of any one of aspects 37-43, wherein said modulating comprises increasing a cytotoxic T-cell response to a cancer cell (e.g., a WT-1-expressing cancer cell).
Aspect 45. The method of any one of aspects 37-44, wherein said administering is intravenous, subcutaneous, intramuscular, systemic, intralymphatic, distal to a treatment site, local, or at or near a treatment site.
Aspect 46. The method of any one of aspects 37-45, wherein the epitope non-specific T-cell response is less than the epitope non-specific T-cell response that would be induced by a control T-cell modulatory multimeric polypeptide comprising a corresponding wild-type immunomodulatory polypeptide.
Aspect 47. A method of delivering a costimulatory (i.e., immunomodulatory) polypeptide selectively to target T cell, the method comprising contacting a mixed population of T cells with a T-cell modulatory multimeric polypeptide (TMMP) of any one of aspects 1-29, wherein the mixed population of T cells comprises the target T cell and non-target T cells, wherein the target T cell is specific for the epitope present within the TMMP (e.g., wherein the target T cell is specific for the WT-1 epitope present within the TMMP), and wherein said contacting delivers the one or more costimulatory polypeptides present within the TMMP to the target T cell.
Aspect 48. The method of aspect 47, wherein the population of T cells is *in vitro.*
Aspect 49. The method of aspect 47, wherein the population of T cells is *in vivo* in an individual.
Aspect 50. The method of aspect 49, comprising administering the multimeric polypeptide to the individual.
Aspect 51. The method of any one of aspects 47-50, wherein the target T cell is a cytotoxic T cell.
Aspect 52. The method of aspect 47, wherein the mixed population of T cells is an *in vitro* population of mixed T cells obtained from an individual, and wherein said contacting results in activation and/or proliferation of the target T cell, generating a population of activated and/or proliferated target T cells.
Aspect 53. The method of aspect 52, further comprising administering the population of activated and/or proliferated target T cells to the individual.
Aspect 54. A method of detecting, in a mixed population of T cells obtained from an individual, the presence of a target T cell that binds a WT-1 epitope of interest, the method comprising: a) contacting *in vitro* the mixed population of T cells with T-cell modulatory multimeric polypeptide (TMMP) of any one of aspects 1-29, wherein the TMMP comprises the WT-1 epitope of interest; and b) detecting activation and/or proliferation of T cells in response to said contacting, wherein activated and/or proliferated T cells indicates the presence of the target T cell.
Aspect 55. A T-cell modulatory multimeric polypeptide comprising: at least one heterodimer comprising: a) a first polypeptide comprising: i) a Wilms tumor-1 (WT-1) peptide epitope, wherein the WT-1 peptide has a length of from about 4 amino acids to about 25 amino acids; and ii) first major histocompatibility complex (MHC) class I polypeptide; b) a second polypeptide comprising a second MHC class I polypeptide, and c) at least one immunomodulatory polypeptide, wherein the first and/or the second polypeptide comprises the immunomodulatory polypeptide, and wherein the first and the second polypeptides are covalently linked to one another via at least 2 disulfide bonds.
Aspect 56. A T-cell modulatory multimeric polypeptide of aspect 55, wherein at least one of the at least one immunomodulatory polypeptides is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide, and wherein the epitope binds to a T-cell receptor (TCR) on a T cell with an affinity of at least 10⁻⁷ M, such that: i) the T-cell modulatory multimeric polypeptide binds to a first T cell with an affinity that is at least 25% higher than the affinity with which the T-cell modulatory multimeric polypeptide binds a second T cell, wherein the first T cell expresses on its surface the cognate co-immunomodulatory polypeptide and a TCR that binds the epitope with an affinity of at least 10⁻⁷ M, and wherein the second T cell expresses on its surface the cognate co-immunomodulatory polypeptide but does not express on its surface a TCR that binds the epitope with an affinity of at least 10⁻⁷ M; and/or ii) the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is in a range of from 1.5:1 to 10⁶:1.
Aspect 57. A T-cell modulatory multimeric polypeptide of aspect 56, wherein: a) the T-cell modulatory multimeric polypeptide binds to the first T cell with an affinity that is at least 50%, at least 2-fold, at least 5-fold, or at least 10-fold higher than the affinity with which it binds the second T cell; and/or b) the variant immunomodulatory polypeptide binds the co-immunomodulatory polypeptide with an affinity of from about 10⁻⁴ M to about 10⁻⁷ M, from about 10⁻⁴ M to about 10⁻⁶ M, from about 10⁻⁴ M to about 10⁻⁵ M; and/or c) wherein the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is at least 10:1, at least 50:1, at least 10²:1, or at least 10³:1.
Aspect 58. A T-cell modulatory multimeric polypeptide of any one of aspects 55-57, wherein the first or the second polypeptide comprises an immunoglobulin (Ig) Fc polypeptide.
Aspect 59. A T-cell modulatory multimeric polypeptide of aspect 58, wherein the Ig Fc polypeptide is an IgG1 Fc polypeptide.
Aspect 60. A T-cell modulatory multimeric polypeptide of aspect 58, wherein the Ig Fc polypeptide is an IgG4 Fc polypeptide.
Aspect 61. T-cell modulatory multimeric polypeptide of aspect 59, wherein IgG1 Fc polypeptide comprises one or more amino acid substitutions selected from N297A, L234A, L235A, L234F, L235E, and P331S.
Aspect 62. A T-cell modulatory multimeric polypeptide of any one of aspects 55-61, wherein
   a1) the first polypeptide comprises, in order from N-terminus to C-terminus:
      i) the WT-1 peptide epitope;
      ii) the first class I MHC polypeptide; and
      iii) at least one immunomodulatory polypeptide; and
   b1) the second polypeptide comprises, in order from N-terminus to C-terminus:
      i) the second class I MHC polypeptide; and
      ii) an immunoglobulin (Ig) Fc polypeptide; or
   a2) the first polypeptide comprises, in order from N-terminus to C-terminus:
      i) the WT-1 peptide epitope; and
      ii) the first class I MHC polypeptide; and
   b2) the second polypeptide comprises, in order from N-terminus to C-terminus:
      i) at least one immunomodulatory polypeptide;
      ii) the second class I MHC polypeptide; and
      iii) an Ig Fc polypeptide; or
   a3) the first polypeptide comprises, in order from N-terminus to C-terminus:
      i) the WT-1 peptide epitope; and
      ii) the first class I MHC polypeptide; and
   b3) the second polypeptide comprises, in order from N-terminus to C-terminus:
      i) the second class I MHC polypeptide; and
      ii) an Ig Fc polypeptide; and
      iii) at least one immunomodulatory polypeptide; or
   a4) the first polypeptide comprises, in order from N-terminus to C-terminus:
      i) the WT-1 peptide epitope; and
      ii) the first class I MHC polypeptide; and
   b4) the second polypeptide comprises, in order from N-terminus to C-terminus:
      i) the second class I MHC polypeptide; and
      ii) at least one immunomodulatory polypeptide; or
   a5) the first polypeptide comprises, in order from N-terminus to C-terminus:
      i) the WT-1 peptide epitope; and
      ii) the first class I MHC polypeptide; and
   b5) a second polypeptide comprises, in order from N-terminus to C-terminus:
      i) at least one immunomodulatory polypeptide; and
      ii) the second class I MHC polypeptide; or
   a6) the first polypeptide comprises, in order from N-terminus to C-terminus:
      i) the WT-1 peptide epitope;
      ii) the first class I MHC polypeptide; and
      iii) at least one immunomodulatory polypeptide; and
   b6) the second polypeptide comprises:
      i) the second class I MHC polypeptide.
Aspect 63. A T-cell modulatory multimeric polypeptide of any one of aspects 55-62, wherein the first MHC polypeptide is a β2-microglobulin polypeptide; and wherein the second MHC polypeptide is an MHC class I heavy chain polypeptide.
Aspect 64. A T-cell modulatory multimeric polypeptide of any one of aspects 55-63, wherein the at least one immunomodulatory polypeptide is selected from the group consisting of a cytokine (e.g., an IL2 polypeptide, an IL7 polypeptide, an IL12 polypeptide, an IL15 polypeptide, an IL17 polypeptide, an IL21 polypeptide, an IL27 polypeptide, an IL-23 polypeptide, a TGFβ polypeptide, and the like; and including all family members, e.g., IL17A, IL-17B, IL-17C, IL-17D, IL-17E, IL-17F, IL-17E), a 4-1BBL polypeptide, an ICOS-L polypeptide, an OX-40L polypeptide, a CD80 polypeptide, a CD86 polypeptide, (CD80 and CD86 are also known as B7-1 and B7-2, respectively), a CD40 polypeptide, a CD70 polypeptide, a JAG1 (CD339) polypeptide, an ICAM (CD540 polypeptide, a PD-L1 polypeptide, a FasL polypeptide, a PD-L2 polypeptide, a PD-1H (VISTA) polypeptide, an ICOS-L (CD275) polypeptide, a GITRL polypeptide, an HVEM polypeptide, a CXCL10 polypeptide, a CXCL9 polypeptide, a CXCL11 polypeptide, a CXCL13 polypeptide, and a CX3CL1 polypeptide, a Galectin-9 polypeptide, a CD83 polypeptide, a CD30L polypeptide, a HLA-G polypeptide, a MICA polypeptide, a MICB polypeptide, a HVEM (CD270) polypeptide, a lymphotoxin beta receptor polypeptide, a 3/TR6 polypeptide, an ILT3 polypeptide, an ILT4 polypeptide, a CXCL10 polypeptide, a CXCL9 polypeptide, a CXCL11 polypeptide, a CXCL13 polypeptide, and a CX3CL1 polypeptide, and combinations thereof.
Aspect 65. A T-cell modulatory multimeric polypeptide of any one of aspects 55-63, wherein the at least one immunomodulatory polypeptide is an IL-2 polypeptide.
Aspect 66. A T-cell modulatory multimeric polypeptide of any one of aspects 55-65, wherein the multimeric polypeptide comprises at least two immunomodulatory polypeptides, and wherein at least two of the immunomodulatory polypeptides are the same.
Aspect 67. A T-cell modulatory multimeric polypeptide of aspect 66, wherein the 2 or more immunomodulatory polypeptides are in tandem.
Aspect 68. The T-cell modulatory multimeric polypeptide of any one of aspects 55-67, wherein: a) a first disulfide bond is between: i) a Cys present in a linker between the WT-1 peptide epitope and the first MHC class I polypeptide, wherein the first MHC class I polypeptide is a β2M polypeptide; and ii) a Cys residue introduced via a Y84C substitution in the second MHC class I polypeptide, wherein the second MHC class I polypeptide is a MHC Class I heavy chain polypeptide; and b) a second disulfide bond is between: i) a Cys residue introduced into the β2M polypeptide via an R12C substitution; and ii) a Cys residue introduced into the MHC Class I heavy chain polypeptide via an A236C substitution.
Aspect 69. A T-cell modulatory multimeric polypeptide of aspect 68, wherein the linker comprises the amino acid sequence GCGGS (SEQ ID NO:318).
Aspect 70. A T-cell modulatory multimeric polypeptide of aspect 69, wherein the linker comprises the amino acid sequence GCGGS(GGGGS)n (SEQ ID NO:342), where n is an integer from 1 to 10.
Aspect 71. A T-cell modulatory multimeric polypeptide of any one of aspects 55-70, wherein the WT-1 peptide epitope has a length of from about 4 amino acids to about 15 amino acids.
Aspect 72. A T-cell modulatory multimeric polypeptide of any one of aspects 55-71, wherein the WT-1 peptide epitope comprises an amino acid sequence selected from the group consisting of: NLMNLGATL (SEQ ID NO:258), NYMNLGATL (SEQ ID NO:263), CMTWNQMNLGATLKG (SEQ ID NO:223), WNQMNLGATLKGVAA (SEQ ID NO:224), CMTWNYMNLGATLKG (SEQ ID NO:225), WNYMNLGATLKGVAA (SEQ ID NO:226), MTWNQMNLGATLKGV (SEQ ID NO:227), TWNQMNLGATLKGVA (SEQ ID NO:228), CMTWNLMNLGATLKG (SEQ ID NO:229), MTWNLMNLGATLKGV (SEQ ID NO:230), TWNLMNLGATLKGVA (SEQ ID NO:231), WNLMNLGATLKGVAA (SEQ ID NO:232), MNLGATLK (SEQ ID NO:233), MTWNYMNLGATLKGV SEQ ID NO:234), TWNYMNLGATLKGVA (SEQ ID NO:235), CMTWNQMNLGATLKGVA (SEQ ID NO:236), CMTWNLMNLGATLKGVA (SEQ ID NO:237), CMTWNYMNLGATLKGVA (SEQ ID NO:238), GYLRNPTAC (SEQ ID NO:239), GALRNPTAL (SEQ ID NO:240), YALRNPTAC (SEQ ID NO:241), GLLRNPTAC (SEQ ID NO:242), RYRPHPGAL (SEQ ID NO:243), YQRPHPGAL (SEQ ID NO:244), RLRPHPGAL (SEQ ID NO:245), RIRPHPGAL (SEQ ID NO:246), QFPNHSFKHEDPMGQ (SEQ ID NO:247), HSFKHEDPY (SEQ ID NO:248), QFPNHSFKHEDPM (SEQ ID NO:249), QFPNHSFKHEDPY (SEQ ID NO:250), KRPFMCAYPGCNK (SEQ ID NO:251), KRPFMCAYPGCYK (SEQ ID NO:252), FMCAYPGCY (SEQ ID NO:253), FMCAYPGCK (SEQ ID NO:254), KRPFMCAYPGCNKRY (SEQ ID NO:255), SEKRPFMCAYPGCNK (SEQ ID NO:256), KRPFMCAYPGCYKRY (SEQ ID NO:257), NLMNLGATL (SEQ ID NO:258), VLDFAPPGA (SEQ ID NO:259), RMFPNAPYL (SEQ ID NO:260), CMTWNQMN (SEQ ID NO:261), CYTWNQMNL (SEQ ID NO:269), NYMNLGATL (SEQ ID NO:263), YMFPNAPYL (SEQ ID NO:264), SLGEQQYSV (SEQ ID NO:265), CMTWNQMNL (SEQ ID NO:266), and NQMNLGATL (SEQ ID NO:267).
Aspect 73. A T-cell modulatory multimeric polypeptide of any one of aspects 55-71, wherein the WT-1 peptide comprises the amino acid sequence VLDFAPPGA (SEQ ID NO:259) or RMFPNAPYL (SEQ ID NO:260).
Aspect 74. A T-cell modulatory multimeric polypeptide of any one of aspects 55-73, wherein the first or the second MHC class I polypeptide comprises: a) an amino acid sequence having at least 95% amino acid sequence identity to the HLA-A*0101, HLA-A*0201, HLA-A*0201, HLA-A*1101, HLA-A*2301, HLA-A*2402, HLA-A*2407, HLA-A*3303, or HLA-A*3401 amino acid sequence depicted in FIG. 9A; or b) an amino acid sequence having at least 95% amino acid sequence identity to the HLA-B*0702, HLA-B*0801, HLA-B*1502, HLA-B*3802, HLA-B*4001, HLA-B*4601, or HLA-B*5301 amino acid sequence depicted in FIG. 10A; or c) an amino acid sequence having at least 95% amino acid sequence identity to the HLA-C*0102, HLA-C*0303, HLA-C*0304, HLA-C*0401, HLA-C*0602, HLA-C*0701, HLA-C*0702, HLA-C*0801, or HLA-C*1502 depicted in FIG. 11A.
Aspect 75. A T-cell modulatory multimeric polypeptide of any one of aspects 55-74, wherein the first MHC polypeptide is a β2M polypeptide, and wherein the second MHC polypeptide comprises an amino acid sequence having at least 95% amino acid sequence identity to an HLA-A*2402 polypeptide.
Aspect 76. A T-cell modulatory multimeric polypeptide of any one of aspects 55-74, wherein the first MHC polypeptide is a β2M polypeptide, and wherein the second MHC polypeptide is an HLA-A*1101 polypeptide.
Aspect 77. A T-cell modulatory multimeric polypeptide of any one of aspects 55-74, wherein the first MHC polypeptide is a β2M polypeptide, and wherein the second MHC polypeptide comprises an amino acid sequence having at least 95% amino acid sequence identity to an HLA-A*3303 polypeptide.
Aspect 78. A T-cell modulatory multimeric polypeptide of any one of aspects 55-74, wherein the first MHC polypeptide is a β2M polypeptide, and wherein the second MHC polypeptide comprises an amino acid sequence having at least 95% amino acid sequence identity to an HLA-A*0201 polypeptide.
Aspect 79. A T-cell modulatory multimeric polypeptide of any one of aspects 55-78, wherein the at least one immunomodulatory polypeptide is a variant IL-2 polypeptide comprising: i) an H16A substitution and an F42A substation; or ii) an H16T substitution and an F42A substitution.
Aspect 80. A T-cell modulatory multimeric polypeptide of any one of aspects 55-79, wherein the multimeric polypeptide comprises a first and a second heterodimer.
Aspect 81. A nucleic acid comprising a nucleotide sequence encoding a first or second polypeptide according to any one of aspects 55-80, wherein the first or second polypeptide comprises at least one immunomodulatory domain.
Aspect 82. An expression vector comprising the nucleic acid of aspect 81.
Aspect 83. A method of selectively modulating the activity of T cell specific for a Wilms tumor-1 (WT-1) epitope, the method comprising contacting the T cell with a T-cell modulatory multimeric polypeptide according to any one of aspects 55-80, wherein said contacting selectively modulates the activity of the WT-1 epitope-specific T cell.
Aspect 84. A method of treating a patient having a cancer, the method comprising administering to the patient an effective amount of a pharmaceutical composition comprising T-cell modulatory multimeric polypeptide according to any one of aspects 55-80.
Aspect 85. The method of aspect 84, wherein the cancer expresses a WT-1 protein.
Aspect 86. The method of aspect 84 or aspect 85, wherein the cancer is acute myeloid leukemia, myeloma, ovarian cancer, pancreatic cancer, non-small cell lung cancer, colorectal cancer, breast cancer, Wilms tumor, mesothelioma, soft tissue sarcoma, neuroblastoma, or nephroblastoma.
Aspect 87. The method of any one of aspects 84-86, wherein said administering is intramuscular.
Aspect 88. The method of any one of aspects 84-86, wherein said administering is intravenous.
Aspect 89. A method of any one of aspects 84-88, further comprising administering one or more checkpoint inhibitors to the individual.
Aspect 90. A method according to aspect 89, wherein the checkpoint inhibitor is an antibody that binds to a polypeptide selected from the group consisting of CD27, CD28, CD40, CD122, CD96, CD73, CD47, OX40, GITR, CSF1R, JAK, PI3K delta, PI3K gamma, TAM, arginase, CD137, ICOS, A2AR, B7-H3, B7-H4, BTLA, CTLA-4, LAG3, TIM3, VISTA, CD96, TIGIT, CD122, PD-1, PD-L1, and PD-L2.
Aspect 91. A method according to aspect 90, wherein the checkpoint inhibitor is an antibody specific for PD-1, PD-L1, or CTLA4.
Aspect 92. A method according to aspect 89, wherein the one or more checkpoint inhibitors is selected from the group consisting of nivolumab, pembrolizumab, pidilizumab, AMP-224, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, Mogamulizumab, Varlilumab, Avelumab, Galiximab, AMP-514, AUNP 12, Indoximod, NLG-919, INCB024360, KN035, and combinations thereof.
Aspect 93. A method of modulating an immune response in an individual, the method comprising administering to the individual an effective amount of the T-cell modulatory multimeric polypeptide of any one of aspects 55-80, wherein said administering induces an epitope-specific T cell response and an epitope-non-specific T cell response, wherein the ratio of the epitope-specific T cell response to the epitope-non-specific T cell response is at least 2:1.
Aspect 94. A method of delivering an immunomodulatory polypeptide selectively to a target T cell, the method comprising contacting a mixed population of T cells with a T-cell modulatory multimeric polypeptide of any one of aspects 55-80, wherein the mixed population of T cells comprises the target T cell and non-target T cells, wherein the target T cell is specific for the WT-1 epitope present within the T-cell modulatory multimeric polypeptide, and wherein said contacting delivers the one or more immunomodulatory polypeptides present within the T-cell modulatory multimeric polypeptide to the target T cell.
Aspect 95. A method of detecting, in a mixed population of T cells obtained from an individual, the presence of a target T cell that binds a Wilms tumor-1 (WT-1) epitope, the method comprising: a) contacting *in vitro* the mixed population of T cells with the T-cell modulatory multimeric polypeptide of any one of aspects 55-80, wherein the T-cell modulatory multimeric polypeptide comprises the WT-1 epitope; and b) detecting activation and/or proliferation of T cells in response to said contacting, wherein activated and/or proliferated T cells indicates the presence of the target T cell.

### ASPECTS SET B

Aspects, including embodiments, of the present subject matter described above may be beneficial alone or in combination, with one or more other aspects or embodiments. Without limiting the foregoing description, certain non-limiting aspects of the disclosure numbered 1-36 are provided below. As will be apparent to those of skill in the art upon reading this disclosure, each of the individually numbered aspects may be used or combined with any of the preceding or following individually numbered aspects. This is intended to provide support for all such combinations of aspects and is not limited to combinations of aspects explicitly provided below:
Aspect 1. A T-cell modulatory multimeric polypeptide comprising: at least one heterodimer comprising: a) a first polypeptide comprising: i) a Wilms tumor-1 (WT-1) peptide epitope, wherein the WT-1 peptide has a length of at least 4 amino acids; and ii) first class I major histocompatibility complex (MHC) polypeptide; b) a second polypeptide comprising a second class I MHC polypeptide, and c) at least one activating immunomodulatory, wherein the first and/or the second polypeptide comprises the immunomodulatory polypeptide, and wherein the WT-1 peptide epitope comprises an amino acid sequence selected from the group consisting of: NLMNLGATL (SEQ ID NO:258), NYMNLGATL (SEQ ID NO:263), CMTWNQMNLGATLKG (SEQ ID NO:223), WNQMNLGATLKGVAA (SEQ ID NO:224), CMTWNYMNLGATLKG (SEQ ID NO:225), WNYMNLGATLKGVAA (SEQ ID NO:226), MTWNQMNLGATLKGV (SEQ ID NO:227), TWNQMNLGATLKGVA (SEQ ID NO:228), CMTWNLMNLGATLKG (SEQ ID NO:229), MTWNLMNLGATLKGV (SEQ ID NO:230), TWNLMNLGATLKGVA (SEQ ID NO:231), WNLMNLGATLKGVAA (SEQ ID NO:232), MNLGATLK (SEQ ID NO:233), MTWNYMNLGATLKGV SEQ ID NO:234), TWNYMNLGATLKGVA (SEQ ID NO:235), CMTWNQMNLGATLKGVA (SEQ ID NO:236), CMTWNLMNLGATLKGVA (SEQ ID NO:237), CMTWNYMNLGATLKGVA (SEQ ID NO:238), GYLRNPTAC (SEQ ID NO:239), GALRNPTAL (SEQ ID NO:240), YALRNPTAC (SEQ ID NO:241), GLLRNPTAC (SEQ ID NO:242), RYRPHPGAL (SEQ ID NO:243), YQRPHPGAL (SEQ ID NO:244), RLRPHPGAL (SEQ ID NO:245), RIRPHPGAL (SEQ ID NO:246), QFPNHSFKHEDPMGQ (SEQ ID NO:247), HSFKHEDPY (SEQ ID NO:248), QFPNHSFKHEDPM (SEQ ID NO:249), QFPNHSFKHEDPY (SEQ ID NO:250), KRPFMCAYPGCNK (SEQ ID NO:251), KRPFMCAYPGCYK (SEQ ID NO:252), FMCAYPGCY (SEQ ID NO:253), FMCAYPGCK (SEQ ID NO:254), KRPFMCAYPGCNKRY (SEQ ID NO:255), SEKRPFMCAYPGCNK (SEQ ID NO:256), KRPFMCAYPGCYKRY (SEQ ID NO:257), NLMNLGATL (SEQ ID NO:258), VLDFAPPGA (SEQ ID NO:259), RMFPNAPYL (SEQ ID NO:260), CMTWNQMN (SEQ ID NO:261), CYTWNQMNL (SEQ ID NO:269), NYMNLGATL (SEQ ID NO:263), YMFPNAPYL (SEQ ID NO:264), SLGEQQYSV (SEQ ID NO:265), CMTWNQMNL (SEQ ID NO:266), and NQMNLGATL (SEQ ID NO:267), optionally wherein the first or the second polypeptide comprises an immunoglobulin (Ig) Fc polypeptide.
Aspect 2. A T-cell modulatory multimeric polypeptide of aspect 1, wherein at least one of the one or more immunomodulatory domains is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide, and wherein the epitope binds to a T-cell receptor (TCR) on a T cell with an affinity of at least 10⁻⁷ M, such that:
   i) the T-cell modulatory multimeric polypeptide binds to a first T cell with an affinity that is at least 25% higher than the affinity with which the T-cell modulatory multimeric polypeptide binds a second T cell, wherein the first T cell expresses on its surface the cognate co-immunomodulatory polypeptide and a TCR that binds the epitope with an affinity of at least 10⁻⁷ M, and wherein the second T cell expresses on its surface the cognate co-immunomodulatory polypeptide but does not express on its surface a TCR that binds the epitope with an affinity of at least 10⁻⁷ M; and/or
   ii) the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is in a range of from 1.5:1 to 10⁶:1.
Aspect 3. A T-cell modulatory multimeric polypeptide of aspect 2, wherein:
   a) the T-cell modulatory multimeric polypeptide binds to the first T cell with an affinity that is at least 50%, at least 2-fold, at least 5-fold, or at least 10-fold higher than the affinity with which it binds the second T cell; and/or
   b) the variant immunomodulatory polypeptide binds the co-immunomodulatory polypeptide with an affinity of from about 10⁻⁴ M to about 10⁻⁷ M, from about 10⁻⁴ M to about 10⁻⁶ M, from about 10⁻⁴ M to about 10⁻⁵ M; and/or
   c) wherein the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is at least 10:1, at least 50:1, at least 10²:1, or at least 10³:1.
Aspect 4. A T-cell modulatory multimeric polypeptide of any one of aspects 1-3, wherein
   a1) the first polypeptide comprises, in order from N-terminus to C-terminus:
      i) the WT-1 peptide epitope;
      ii) the first MHC polypeptide; and
      iii) at least one immunomodulatory polypeptide; and
   b1) the second polypeptide comprises, in order from N-terminus to C-terminus:
      i) the second MHC polypeptide; and
      ii) an immunoglobulin (Ig) Fc polypeptide; or
   a2) the first polypeptide comprises, in order from N-terminus to C-terminus:
      i) the WT-1 peptide epitope; and
      ii) the first MHC polypeptide; and
   b2) the second polypeptide comprises, in order from N-terminus to C-terminus:
      i) at least one immunomodulatory polypeptide;
      ii) the second MHC polypeptide; and
      iii) an Ig Fc polypeptide; or
   a3) the first polypeptide comprises, in order from N-terminus to C-terminus:
      i) the WT-1 peptide epitope; and
      ii) the first MHC polypeptide; and
   b3) the second polypeptide comprises, in order from N-terminus to C-terminus:
      i) the second MHC polypeptide; and
      ii) an Ig Fc polypeptide; and
      iii) at least one immunomodulatory polypeptide; or
   a4) the first polypeptide comprises, in order from N-terminus to C-terminus:
      i) the WT-1 peptide epitope; and
      ii) the first MHC polypeptide; and
   b4) the second polypeptide comprises, in order from N-terminus to C-terminus:
      i) the second MHC polypeptide; and
      ii) at least one immunomodulatory polypeptide; or
   a5) the first polypeptide comprises, in order from N-terminus to C-terminus:
      i) the WT-1 peptide epitope; and
      ii) the first MHC polypeptide; and
   b5) a second polypeptide comprises, in order from N-terminus to C-terminus:
      i) at least one immunomodulatory polypeptide; and
      ii) the second MHC polypeptide; or
   a6) the first polypeptide comprises, in order from N-terminus to C-terminus:
      i) the WT-1 peptide epitope;
      ii) the first MHC polypeptide; and
      iii) at least one immunomodulatory polypeptide; and
   b6) the second polypeptide comprises:
      i) the second MHC polypeptide.
Aspect 5. A T-cell modulatory multimeric polypeptide of any one of aspects 1-4, wherein:
   a) the first MHC polypeptide is a β2-microglobulin polypeptide; and the second MHC polypeptide is an MHC class I heavy chain polypeptide; or
   b) the first MHC polypeptide is an MHC class I heavy chain polypeptide; and the second MHC polypeptide is a β2-microglobulin polypeptide.
Aspect 6. A T-cell modulatory multimeric polypeptide of aspect 5, wherein:
   a) the first polypeptide comprises, in order from N-terminus to C-terminus:
      i) the WT-1 peptide epitope; and
      ii) the β2-microglobulin polypeptide; and
   b) the second polypeptide comprises, in order from N-terminus to C-terminus:
      i) at least one immunomodulatory polypeptide;
      ii) the MHC class I heavy chain polypeptide; and
      iii) an Ig Fc polypeptide.
Aspect 7. A T-cell modulatory multimeric polypeptide of aspect 5, wherein:
   a) the first polypeptide comprises, in order from N-terminus to C-terminus:
      i) the WT-1 peptide epitope; and
      ii) the β2-microglobulin polypeptide; and
   b) the second polypeptide comprises, in order from N-terminus to C-terminus:
      i) the MHC class I heavy chain polypeptide; and
      ii) an Ig Fc polypeptide; and
      iii) at least one immunomodulatory polypeptide
Aspect 8. A T-cell modulatory multimeric polypeptide of any one of aspects 1-7, wherein the at least one immunomodulatory polypeptide is selected from the group consisting of a cytokine, a 4-1BBL polypeptide, an ICOS-L polypeptide, an OX-40L polypeptide, a CD80 polypeptide, a CD86 polypeptide, a CD40 polypeptide, a CD70 polypeptide, and combinations thereof.
Aspect 9. A T-cell modulatory multimeric polypeptide of any one of aspects 1-8, wherein the at least one immunomodulatory polypeptide is an IL-2 polypeptide.
Aspect 10. A T-cell modulatory multimeric polypeptide of any one of aspects 1-9, wherein the multimeric polypeptide comprises at least two immunomodulatory polypeptides, and wherein at least two of the immunomodulatory polypeptides are the same, optionally wherein the 2 or more immunomodulatory polypeptides are in tandem.
Aspect 11. A T-cell modulatory multimeric polypeptide of any one of aspects 1-10, wherein the immunomodulatory polypeptide is a variant IL-2 polypeptide that exhibits reduced affinity to an IL-2 receptor compared to the affinity of a wild-type IL-2 polypeptide for the IL-2 receptor.
Aspect 12. A T-cell modulatory multimeric polypeptide of aspect 11, wherein the variant IL-2 polypeptide comprises: i) an H16A substitution and an F42A substitution; or ii) an H16T substitution and an F42A substitution.
Aspect 13. A T-cell modulatory multimeric polypeptide of any one of aspects 1-12, wherein the first polypeptide and the second polypeptide are covalently linked to one another, optionally wherein the covalent linkage is via a disulfide bond.
Aspect 14. A T-cell modulatory multimeric polypeptide of any one of aspects 1-13, wherein the first MHC polypeptide or a linker between the epitope and the first MHC polypeptide comprises an amino acid substitution to provide a first Cys residue, wherein the second MHC polypeptide comprises an amino acid substitution to provide a second Cys residue, and wherein the disulfide linkage is between the first and the second Cys residues.
Aspect 15. The T-cell modulatory multimeric polypeptide of any one of aspects 1-14, wherein the polypeptide comprises a disulfide bond between: i) a Cys present in a linker between the WT-1 peptide epitope and the first MHC class I polypeptide, wherein the first MHC class I polypeptide is a β2M polypeptide; and ii) a Cys residue introduced via a Y84C substitution in the second MHC class I polypeptide, wherein the second MHC class I polypeptide is a MHC Class I heavy chain polypeptide.
Aspect 16. The T-cell modulatory multimeric polypeptide of any one of aspects 1-14, wherein the polypeptide comprises a disulfide bond between i) a Cys residue introduced into the first MHC class I polypeptide via an R12C substitution, wherein the first MHC class I polypeptide is a β2M polypeptide; and ii) a Cys residue introduced into the second MHC class I polypeptide, via an A236C substitution, wherein second MHC class I polypeptide is an MHC Class I heavy chain polypeptide.
Aspect 17. The T-cell modulatory multimeric polypeptide of any one of aspects 1-14, wherein the polypeptide comprises a first disulfide bond between: i) a Cys present in a linker between the WT-1 peptide epitope and the first MHC class I polypeptide, wherein the first MHC class I polypeptide is a β2M polypeptide; and ii) a Cys residue introduced via a Y84C substitution in the second MHC class I polypeptide, wherein the second MHC class I polypeptide is a MHC Class I heavy chain polypeptide, and a second disulfide bond between i) a Cys residue introduced into the β2M polypeptide via an R12C substitution; and ii) a Cys residue introduced into the MHC Class I heavy chain polypeptide via an A236C substitution.
Aspect 18. A T-cell modulatory multimeric polypeptide of aspect 15 or aspect 17, wherein the linker between the WT-1 peptide epitope and the first MHC is GCGGS(G4S)n (SEQ ID NO:315), where n is 1, 2, 3, 4, 5, 6, 7, 8, or 9.
Aspect 19. A T-cell modulatory multimeric polypeptide of any one of aspects 1-18, wherein the WT-1 peptide epitope has a length of from about 4 amino acids to about 25 amino acids.
Aspect 20. A T-cell modulatory multimeric polypeptide of any one of aspects 1-19, wherein the WT-1 peptide comprises the amino acid sequence CMTWNQMNL (SEQ ID NO: 266), CYTWNQMNL (SEQ ID NO:269), NYMNLGATL (SEQ ID NO:263), VLDFAPPGA (SEQ ID NO:259), YMFPNAPYL (SEQ ID NO:264), SLGEQQYSV (SEQ ID NO:265), RMFPNAPYL (SEQ ID NO:260), and NLMNLGATL (SEQ ID NO:258).
Aspect 21. A T-cell modulatory multimeric polypeptide of any one of aspects 1-20, wherein the first or the second MHC polypeptide comprises:
   a) an amino acid sequence having at least 95% amino acid sequence identity to the HLA-A*0101, HLA-A*0201, HLA-A*0201, HLA-A*1101, HLA-A*2301, HLA-A*2402, HLA-A*2407, HLA-A*3303, or HLA-A*3401 amino acid sequence depicted in FIG. 9A; or
   b) an amino acid sequence having at least 95% amino acid sequence identity to the HLA-B*0702, HLA-B*0801, HLA-B*1502, HLA-B*3802, HLA-B*4001, HLA-B*4601, or HLA-B*5301 amino acid sequence depicted in FIG. 10A; or
   c) an amino acid sequence having at least 95% amino acid sequence identity to the HLA-C*0102, HLA-C*0303, HLA-C*0304, HLA-C*0401, HLA-C*0602, HLA-C*0701, HLA-C*0702, HLA-C*0801, or HLA-C*1502 depicted in FIG. 11A.
Aspect 22. A T-cell modulatory multimeric polypeptide of any one of aspects 1-21, wherein the first MHC polypeptide is a β2M polypeptide, and wherein the second MHC polypeptide comprises an amino acid sequence having at least 95% amino acid sequence identity to an HLA-A*2402 polypeptide, and wherein the epitope is selected from the group consisting of: RMFPNAPYL (SEQ ID NO:260), CYTWNQMNL (SEQ ID NO:269), and NYMNLGATL (SEQ ID NO:263).
Aspect 23. A T-cell modulatory multimeric polypeptide of any one of aspects 1-21, wherein the first MHC polypeptide is a β2M polypeptide, and wherein the second MHC polypeptide comprises an amino acid sequence having at least 95% amino acid sequence identity to an HLA-A*0201 polypeptide, and wherein the epitope is selected from the group consisting of: VLDFAPPGA (SEQ ID NO:259), RMFPNAPYL (SEQ ID NO:260), and YMFPNAPYL (SEQ ID NO:264).
Aspect 24. A T-cell modulatory multimeric polypeptide of any one of aspects 1-23, wherein the multimeric polypeptide comprises a first and a second heterodimer, and wherein the first and second heterodimers are covalently bound by one or more disulfide bonds between the Ig Fc polypeptides of the first and second heterodimers.
Aspect 25. A nucleic acid comprising a nucleotide sequence encoding a first or second polypeptide according to any one of aspects 1-24.
Aspect 26. An expression vector comprising the nucleic acid of aspect 25.
Aspect 27. A method of selectively modulating the activity of T cell specific for a Wilms tumor-1 (WT-1) epitope, the method comprising contacting the T cell with a T-cell modulatory multimeric polypeptide according to any one of aspects 1-24, wherein said contacting selectively modulates the activity of the WT-1 epitope-specific T cell.
Aspect 28. A method of treating a patient having a cancer, the method comprising administering to the patient an effective amount of a pharmaceutical composition comprising T-cell modulatory multimeric polypeptide according to any one of aspects 1-24.
Aspect 29. The method of aspect 28, wherein the cancer is acute myeloid leukemia, myeloma, ovarian cancer, pancreatic cancer, non-small cell lung cancer, colorectal cancer, breast cancer, Wilms tumor, mesothelioma, soft tissue sarcoma, neuroblastoma, or nephroblastoma.
Aspect 30. A method of aspect 28 or aspect 29, further comprising administering one or more checkpoint inhibitors to the individual.
Aspect 31. A method according to aspect 30, wherein the checkpoint inhibitor is an antibody that binds to a polypeptide selected from the group consisting of CD27, CD28, CD40, CD122, CD96, CD73, CD47, OX40, GITR, CSF1R, JAK, PI3K delta, PI3K gamma, TAM, arginase, CD137, ICOS, A2AR, B7-H3, B7-H4, BTLA, CTLA-4, LAG3, TIM3, VISTA, CD96, TIGIT, CD122, PD-1, PD-L1, and PD-L2.
Aspect 32. A method according to aspect 31, wherein the checkpoint inhibitor is an antibody specific for PD-1, PD-L1, or CTLA4.
Aspect 33. A method according to aspect 30, wherein the one or more checkpoint inhibitors is selected from the group consisting of nivolumab, pembrolizumab, pidilizumab, AMP-224, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, Mogamulizumab, Varlilumab, Avelumab, Galiximab, AMP-514, AUNP 12, Indoximod, NLG-919, INCB024360, KN035, and combinations thereof.
Aspect 34. A method of modulating an immune response in an individual, the method comprising administering to the individual an effective amount of the T-cell modulatory multimeric polypeptide of any one of aspects 1-24, wherein said administering induces an epitope-specific T cell response and an epitope-non-specific T cell response, and wherein the ratio of the epitope-specific T cell response to the epitope-non-specific T cell response is at least 2:1.
Aspect 35. A method of delivering an immunomodulatory polypeptide selectively to a target T cell, the method comprising contacting a mixed population of T cells with a T-cell modulatory multimeric polypeptide of any one of aspects 1-24, wherein the mixed population of T cells comprises the target T cell and non-target T cells, wherein the target T cell is specific for the WT-1 epitope present within the T-cell modulatory multimeric polypeptide, and wherein said contacting delivers the one or more immunomodulatory polypeptides present within the T-cell modulatory multimeric polypeptide to the target T cell.
Aspect 36. A method of detecting, in a mixed population of T cells obtained from an individual, the presence of a target T cell that binds a WT-1 epitope, the method comprising: a) contacting *in vitro* the mixed population of T cells with the T-cell modulatory multimeric polypeptide of any one of aspects 1-24, wherein the T-cell modulatory multimeric polypeptide comprises the WT-1 epitope; and b) detecting activation and/or proliferation of T cells in response to said contacting, wherein activated and/or proliferated T cells indicates the presence of the target T cell.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Example 1

The effect of linking the two polypeptide chains of a TMMP heterodimer via two disulfide bonds on stability and production was tested.

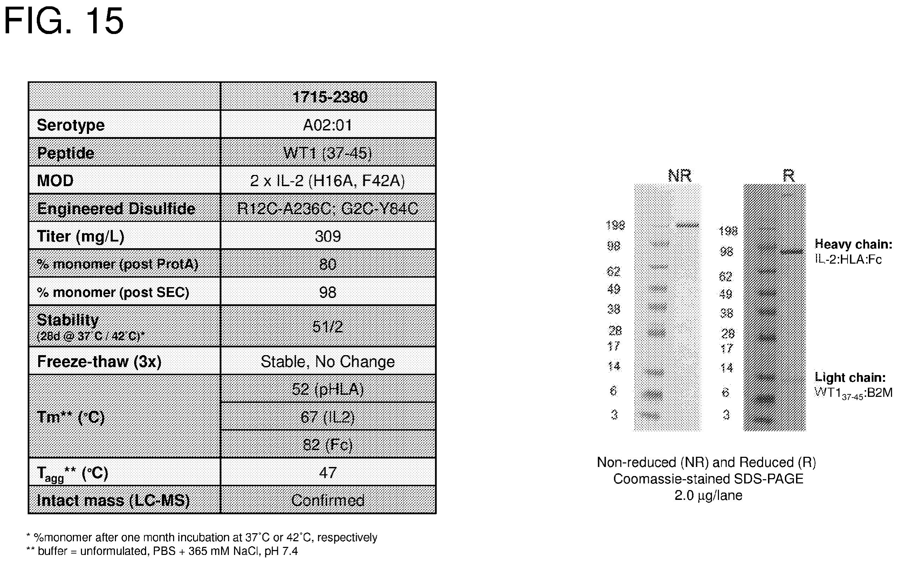

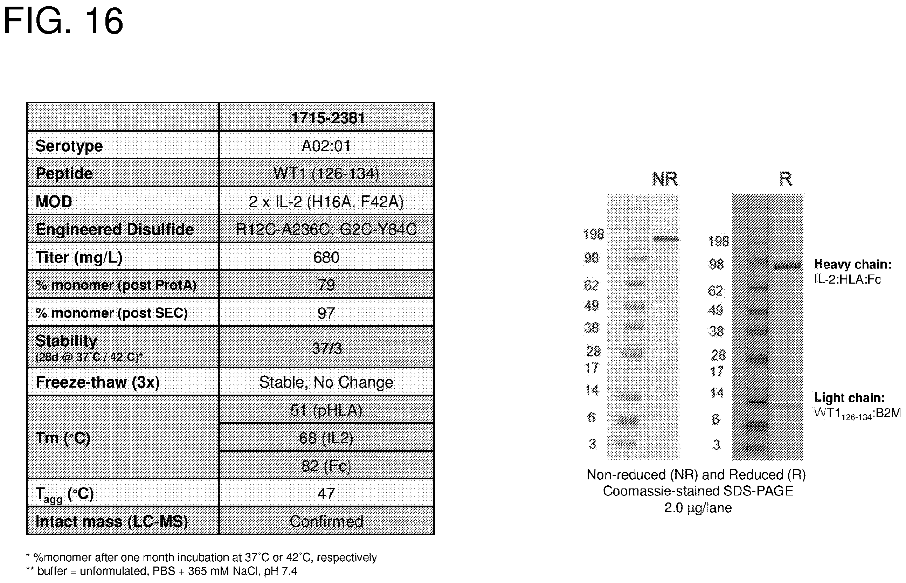

The following TMMPs were generated: a) a TMMP comprising 1715 + 2380 polypeptides; and b) a TMMP comprising 1715 and 2381 polypeptides. The amino acid sequences of the polypeptide chains are provided in FIG. 14A-14C. As shown in FIG. 15 and FIG. 16, the TMMPs included: i) Class I HLA-A heavy chain polypeptides of the A02:01 allele; and ii) two copies of IL2 (H16A; F42A) immunomodulatory ("MOD") polypeptides. The 2380 polypeptide comprises the WT1 peptide WT1(37-45), while the 2381 polypeptide comprises the WT1 peptide WT1(126-134). The 1715-2380 TMMP is a homodimer of a heterodimer comprising the 1715 polypeptide and the 2380 polypeptide. Likewise, the 1715-2381 TMMP is a homodimer of a heterodimer comprising the 1715 polypeptide and the 2381 polypeptide. Thus, the TMMPs included: i) 2 copies of the 1715 + 2380 heterodimer, linked by 2 disulfide bonds between the IgFc polypeptide present in the 1715 polypeptides; or ii) 2 copies of the 1715 + 2381 heterodimer, linked by 2 disulfide bonds between the IgFc polypeptide present in the 1715 polypeptides. This arrangement is depicted schematically in FIG. 17C.

TMMP 1715 + 2380 is a double disulfide-linked heterodimer: a) a first disulfide linkage is between: i) the Cys present in the linker between the WT1 peptide and the β2M chain in the 2380 polypeptide; and ii) the Cys introduced by the Y84C substitution in the Class I heavy chain present in the 1715 polypeptide; and b) a second disulfide linkages is between: i) the Cys introduced by the R12C substitution in the β2M polypeptide present in the 2380 polypeptide; and ii) the Cys introduced by the A236C substitution in the Class I heavy chain present in the 1715 polypeptide.

TMMP 1715 + 2381 is a double disulfide-linked heterodimer: a) a first disulfide linkage is between: i) the Cys present in the linker between the WT1 peptide and the β2M chain in the 2381 polypeptide; and ii) the Cys introduced by the Y84C substitution in the Class I heavy chain present in the 1715 polypeptide; and b) a second disulfide linkages is between: i) the Cys introduced by the R12C substitution in the β2M polypeptide present in the 2381 polypeptide; and ii) the Cys introduced by the A236C substitution in the Class I heavy chain present in the 1715 polypeptide.

The TMMPs were produced in ExpiCHO cells (adapted from Chinese hamster ovary (CHO) cells; ThermoFisher; see, e.g., Jain et al. (2017) Protein Expr. Purif. 134:38) and were purified from the cell culture medium in which the cells were grown. Two purification steps were carried out. In a first step, the cell culture medium was clarified, and the clarified cell culture medium was subjected to Protein A column purification. In the second purification step, the eluate from the Protein A column was subjected to size exclusion chromatography.

The stability of the purified TMMPs was tested. The amount of heterodimeric TMMP present was determined after storage of the purified TMMPs in a liquid solution (phosphate-buffered saline (PBS) containing 365 mM NaCl, pH 7.4) for 28 days at 37°C or for 28 days at 42°C.

In addition, the purified TMMPs were subjected to 3 freeze/thaw cycles.

The results are depicted in FIG. 15 and FIG. 16.

As shown in FIG. 15, a homodimer of the 1715-2380 heterodimer (referred to in FIG. 15 as "monomer") represented 80% of the eluate from the Protein A column. As shown in FIG. 16, a homodimer of the 1715-2381 heterodimer (referred to in FIG. 16 as "monomer") represented 79% of the eluate from the Protein A column.

Homodimers of heterodimers 1715-2380 and 1715-2381 were found to be stable to 3 freeze/thaw cycles.

Unfolding temperatures of the peptide/HLA, IL-2, and Fc domains of various TMMPs, expressed as Tₘ (°C), are provided in FIG. 15 and FIG. 16. In addition, the temperature at which aggregation occurs (Tₘ °C) is provided in FIG. 15 and FIG. 16.

### Example 2: Biochemical characterization of TMMPs comprising WT1 epitopes, HLA-A*02 heavy chains, either one or two disulfide bonds between the 2 polypeptide chains of the heterodimer, and variant IL-2 immunomodulatory polypeptides at position 1

The constructs used in this study are summarized in Table 2.

**Table 2**

| **Constructs** | **Epitope** | **S-S bond(s)** | **IL-2 position** |
|---|---|---|---|
| 2405 + 2762 | WT-1 (37-45) | G2C | 1 |
| 1715 + 2380 | WT-1 (37-45) | G2C + R12C | 1 |
| 2405 + 2763 | WT-1 (126-134) | G2C | 1 |
| 1715 + 2381 | WT-1 (126-134) | G2C + R12C | 1 |
| 2405 + 3626 | WT-1 (126-134 (R126Y)) | G2C | 1 |
| 1715 + 3625 | WT-1 (126-134 (R126Y)) | G2C + R12C | 1 |

Amino acid sequences of the polypeptide chains of the constructs are provided in FIG. 14A-14I.

"G2C" indicates that the TMMP includes a disulfide bond between: i) a Cys in the peptide linker between the peptide epitope and the β2M polypeptide; and ii) a Cys at position 84 of the MHC class I heavy chain, where the MHC class I heavy chain has a Y84C substitution.

"R12C" indicates that the TMMP includes a disulfide bond between: i) a Cys at position 12 in the β2M polypeptide, where the β2M polypeptide has an R12C substitution; and ii) a Cys at position 236 of the MHC class I heavy chain, where the MHC class I heavy chain has an A236C substitution.

"G2C + R12C" indicates that the TMMP includes both the "G2C" disulfide bond and the "R12C" disulfide bond.

WT-1 (37-45) is VLDFAPPGA (SEQ ID NO:259).

WT-1 (126-134) is RMFPNAPYL (SEQ ID NO:260).

WT-1 (126-134 (R126Y)) is YMFPNAPYL (SEQ ID NO:264) and is also referred to as "126-134 mimotope."

IL-2 "position 1" is depicted schematically in FIG. 19.

### RESULTS

The ability of TMMPs to stimulate antigen-specific proliferation of CD8⁺ T cells was tested. The TMMPs included, as the epitope: i) WT1 37-45; ii) WT1 126-134; or iii) WT1 126-134 (R126Y). All TMMPs included A02 allele MHC class I heavy chains.

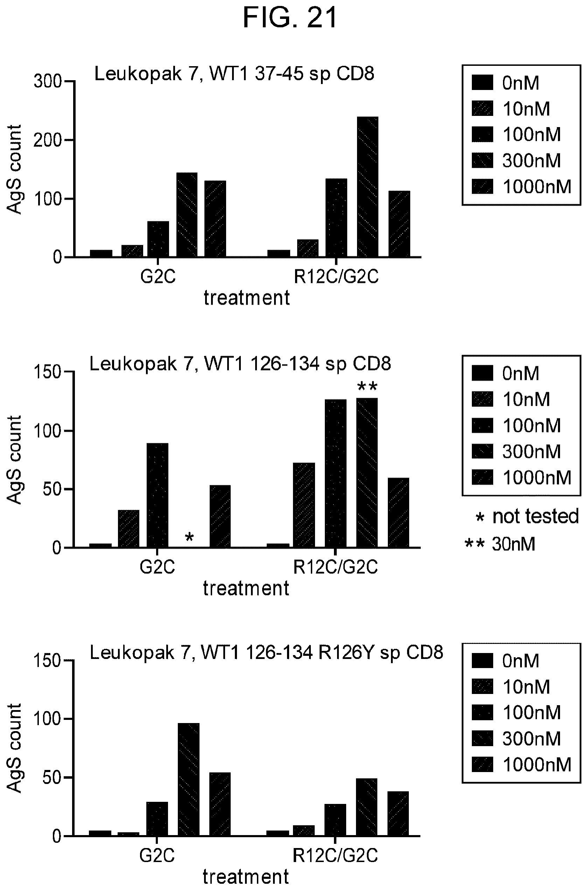

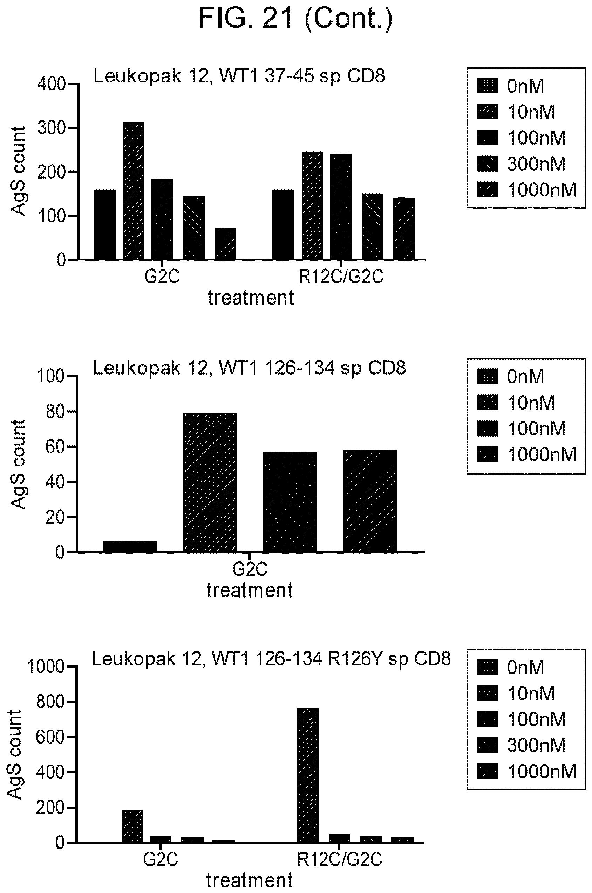

Peripheral blood mononuclear cells (PBMCs) obtained from human donors were incubated *in vitro* with the TMMPs at various concentrations (0 nM, 10 nM, 100 nM, 300 nM, or 1000 nM) for 10 days. After the 10-day incubation period, the number of cells specific for the epitope was determined. Data from PBMCs from two donors ("Leukopak 7 and Leukopak 12") are shown in **FIG. 21****.**

The data presented in FIG. 21 demonstrate, in two donors, that WT1-specific TMMPs can induce expansion of WT1-specific CD8+ T cells from total PBMCs over a course of a 10-day stimulation culture. This expansion was achieved in PBMCs that have low or no detectable WT1-specific T cell precursors, indicating that the TMMPs were able to induce antigen-specific responses in donors from an unprimed or naive repertoire. The data demonstrate that TMMPs specific for any of the 3 selected WT1 peptides (37-45, 126-134, and 126-134 R126Y) and on either of the two tested disulfide frameworks (G2C and R12C/G2C) induce expansion of WT1-specific CD8+ T cells from total PBMCs.

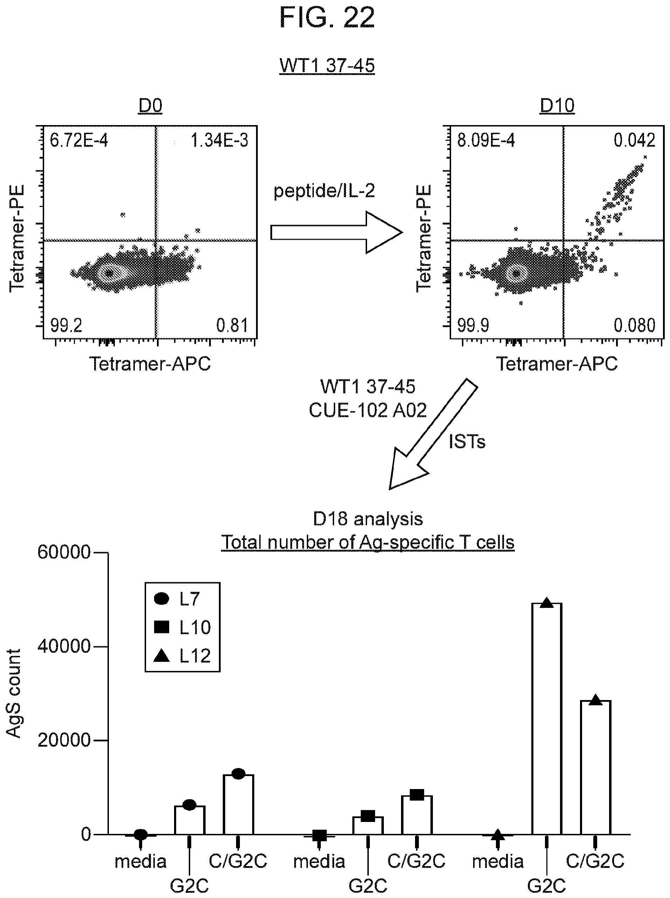

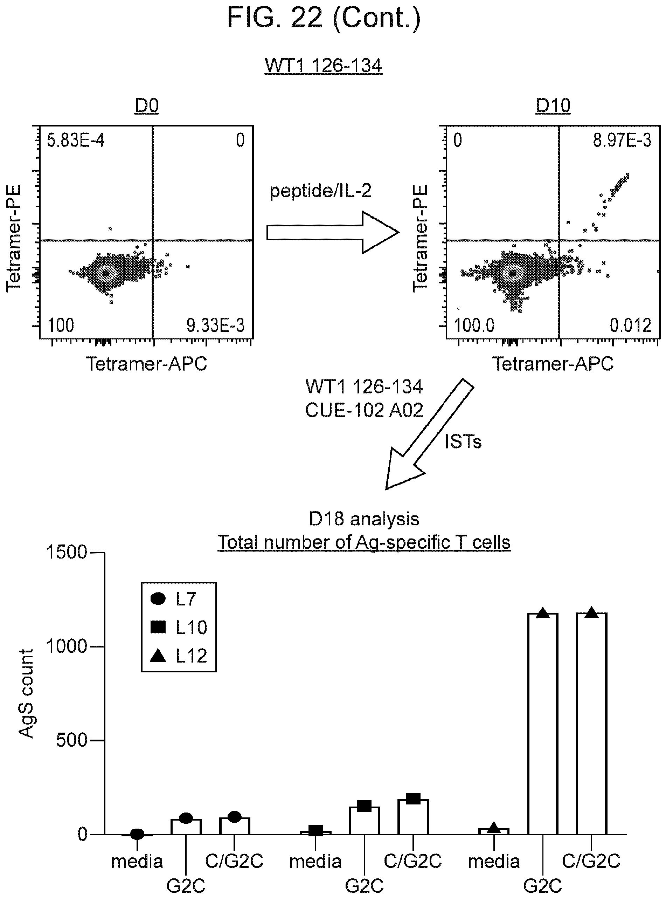

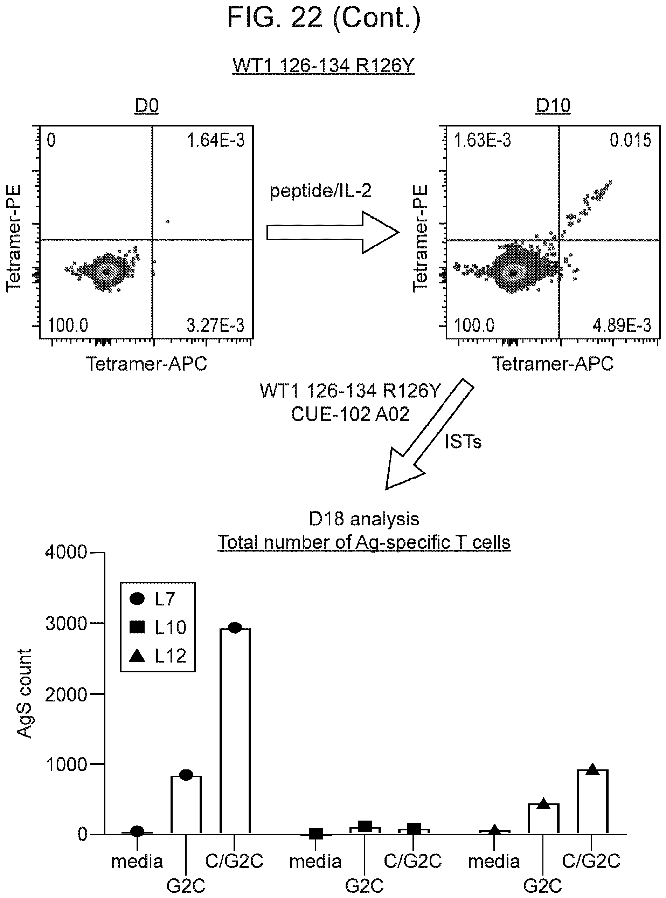

PBMCs from different human donors (L7, L10, and L12) were stimulated for 10 days *in vitro* with the indicated WT1 peptides in the presence of recombinant human IL-2 and then re-stimulated for 8 days with TMMPs, containing the same peptides, and containing either the G2C disulfide bond or both the R12C and G2C disulfide bonds. The data are depicted in **FIG. 22****.**

The data presented in FIG. 22 demonstrate, in PBMCs from three donors, that WT1-containing TMMPs can expand WT1-specific CD8+ T cells from total PBMCs over a course of an 8-day re-stimulation culture following a 10-day priming culture. This expansion occurred from cells that have a detectable number of WT1-specific T cell precursors, indicating that the TMMPs were able to expand antigen-specific T cells in donors with a primed/preexisting WT-1 specific T cell repertoire. The data demonstrate that TMMPs specific for any of the 3 WT1 peptides (37-45, 126-134, and 126-134 R126Y) and on either of the two tested disulfide frameworks (G2C and R12C/G2C) induce such expansions.

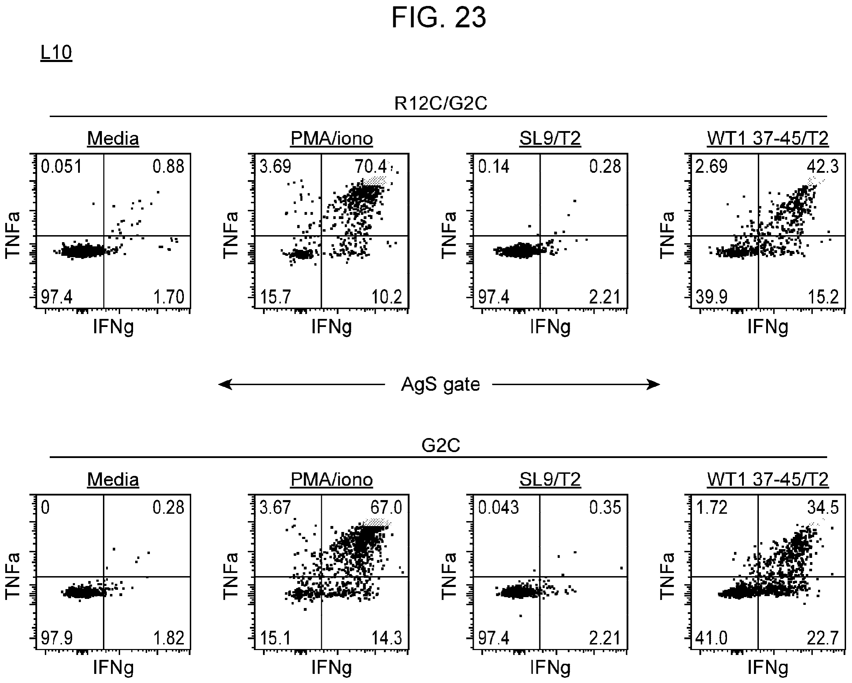

The ability of the CD8⁺ T cells expanded by contacting with TMMPs containing the WT1 37-45 peptide and containing either the G2C disulfide bond or both the R12C and G2C disulfide bonds, to produce TNF-α and IFN-γ in response to target cells (T2 cells) presenting the WT1-37-45 peptide or an irrelevant peptide (SL9) was tested. Phorbol 12-myristate 13-acetate (PMA) and ionomycin ("iono") were used as a positive control. CD8⁺ T cells treated with media were used as a negative control. CD8⁺ T cells expanded by TMMPs were incubated with 'target cells' (T2 cells) that were loaded with either a WT1 peptide or with an irrelevant peptide (the SL9 peptide from HIV). The response, as indicated by production of IFN-γ and TNF-α, of the CD8+ T cells to the T2 cells pulsed with WT 37-45 peptide was compared to the response to T2 cells pulsed with SL9 peptide. The data are shown in **FIG. 23****.**

The data presented in FIG. 23 demonstrate the selective polyfunctionality of the WT1 37-45-specific CD8+ T cells expanded with WT1 37-45 containing TMMPs having either the G2C or R12C/G2C framework. The response measured (TNF-α and IFN-γ production) was observed only upon recognition of target cells presenting the WT1 37-45 peptide but not the SL9 peptide. The positive and negative control wells show that there is no baseline activity in the CD8+ T cells in the absence of stimulation (as seen in the media-only wells) and that both antigen-specific and non-antigen-specific cells are capable of showing functional responses upon strong, antigen-non-specific stimulation (PMA + ionomycin).

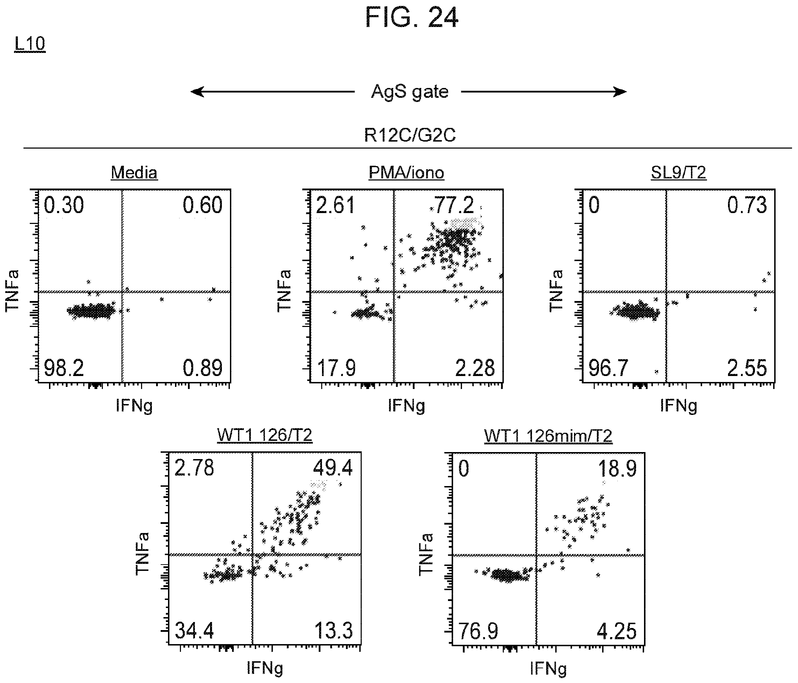

Using the same assay, the ability of the CD8 T cells expanded by TMMPs containing the WT1 126-134 peptide ("WT1 126") and containing the R12C and G2C disulfide bonds, to produce TNF-α and IFN-γ in response to target cells (T2 cells) presenting the WT1-126-134 peptide, WT1-126-134 R126Y peptide or an irrelevant peptide (SL9) was tested. The data are shown in **FIG. 24****.**

The data presented in FIG. 24 demonstrate the selective polyfunctionality of the WT1 126-134-specific CD8+ T cells expanded with WT1 126-134 specific Immuno-STATs on the R12C/G2C framework. The response measured (TNF-α and IFN-γ production) was observed only upon recognition of target cells presenting the WT1 126-134 peptide or the WT1 126-134 R126Y peptide but not the SL9 peptide. The positive and negative control wells show that there is no baseline activity in the CD8+ T cells in the absence of stimulation (as seen in the media-only wells) and that both antigen-specific and non-antigen-specific cells are capable of showing functional responses upon strong, antigen-non-specific stimulation (PMA + ionomycin).

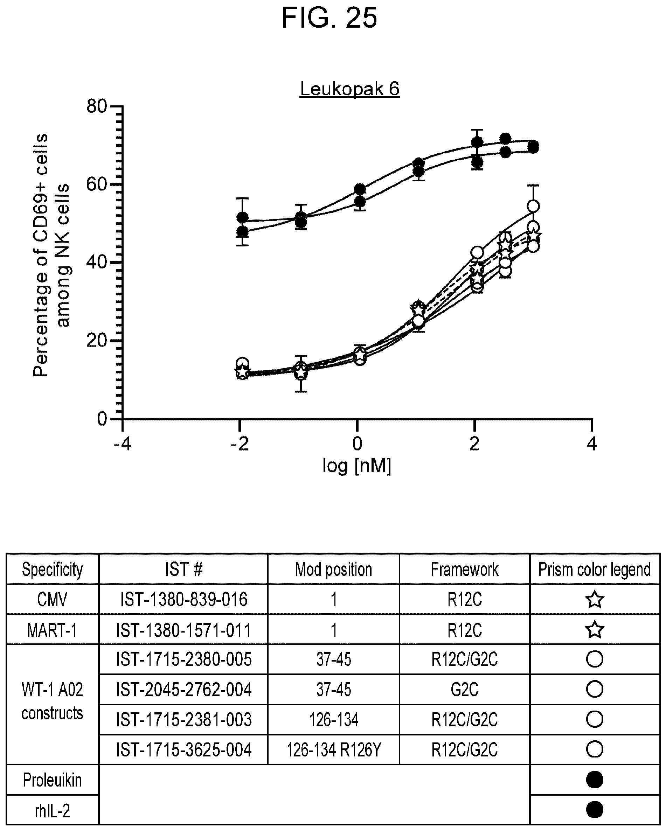

The effect of disulfide bonds on IL-2-driven immune cell activation was tested. CD69 is an early activation marker on most lymphocytes and some other immune cells. Cells upregulate CD69 upon different types of stimulatory conditions, including IL-2 stimulation. CD69 upregulation on both NK cells, CD4⁺ T cells, and CD8⁺ T cells was assessed. CD69 upregulation demonstrates that the IL-2 polypeptides present in the TMMPs are active and that their function is attenuated compared to control (recombinant human IL-2). PBMCs from different human donors were incubated with various TMMPs ("ISTs") set out in the table. TMMPs comprising a CMV epitope or a MART-1 epitope were included as controls. Data from one human donor (Leukopak 6) are shown. The data are shown in **FIG. 25.** FIG. 25 shows the upregulation of CD69 on NK cells as a relevant and representative example of a cell that readily upregulates CD69 in response to IL-2. Similar data was observed on CD8+ T cell gates and CD4+ T cell gates.

The data presented in FIG. 25 demonstrates the IL-2 immunomodulatory polypeptide engineered on position 1 into HLA-A02-specific Immuno-STATs built on various disulfide frameworks (R12C, G2C and R12C/G2C) is functional (as observed by the induction of CD69 on the surface of a relevant immune cell) and attenuated compared to wild-type recombinant human IL-2.

To evaluate the potency of the variant IL-2 immunomodulatory polypeptides present in the TMMPs, a CTLL-2 proliferation assay was carried out. CTLL-2 cells are dependent on IL-2 for growth; thus, CTLL-2 proliferation is a measure of the amount and/or potency of IL-2 present in the culture medium (e.g., where the IL-2 is produced by T cells contacted with a TMMP). Gillis et al. (1978) J. Immunol. 120:2027.

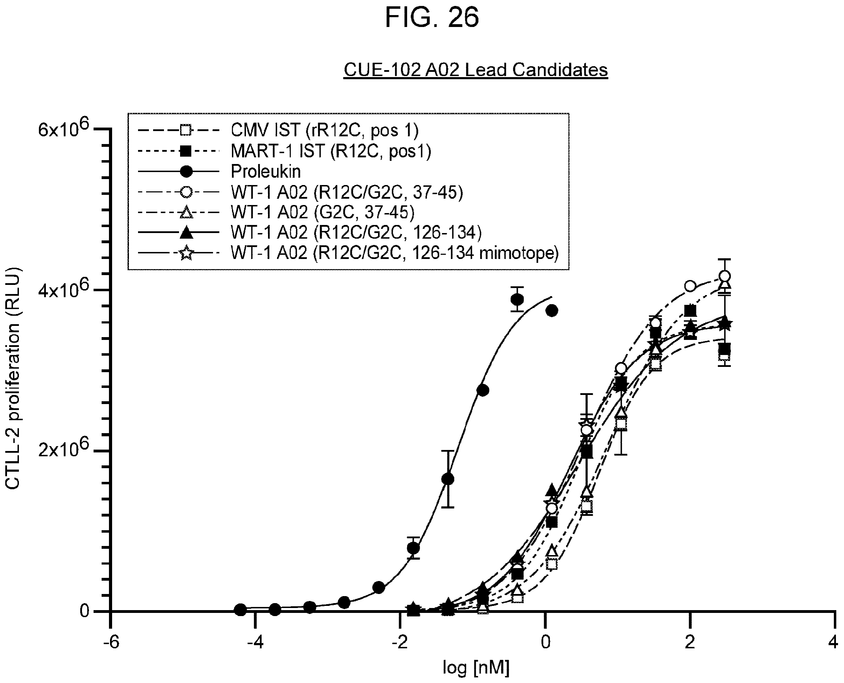

The data are shown in **FIG. 26****.** The data presented in FIG. 26 demonstrate that the IL-2 immunomodulatory polypeptide engineered on position 1 into HLA-A02-specific Immuno-STATs (TMMPs) built on various disulfide frameworks (R12C, G2C and R12C/G2C) is functional (as observed by the induction of CTLL-2 proliferation) and attenuated compared to proleukin.

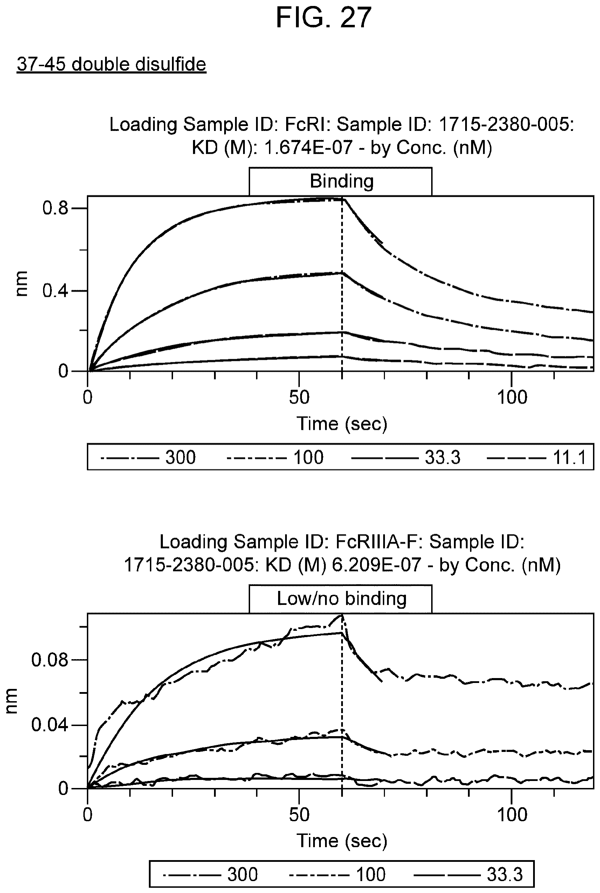

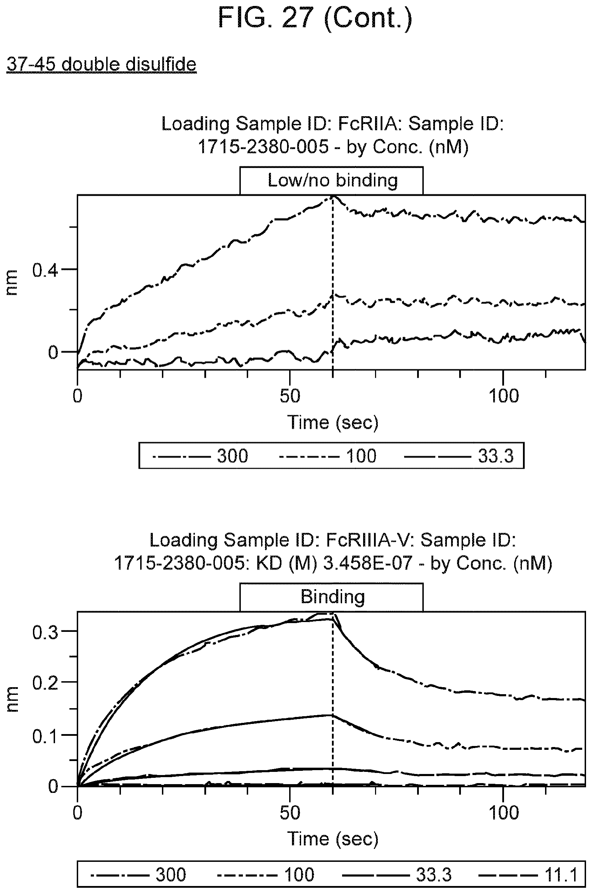

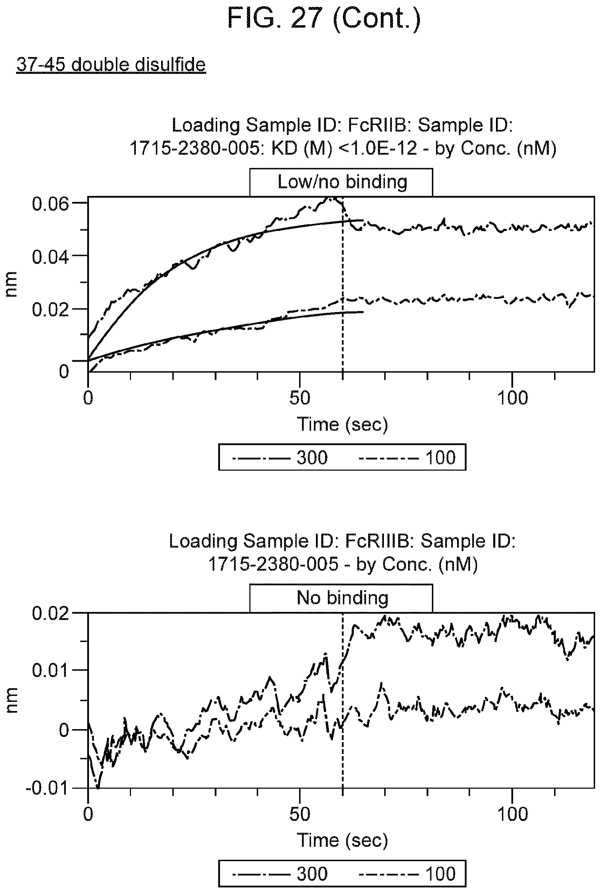

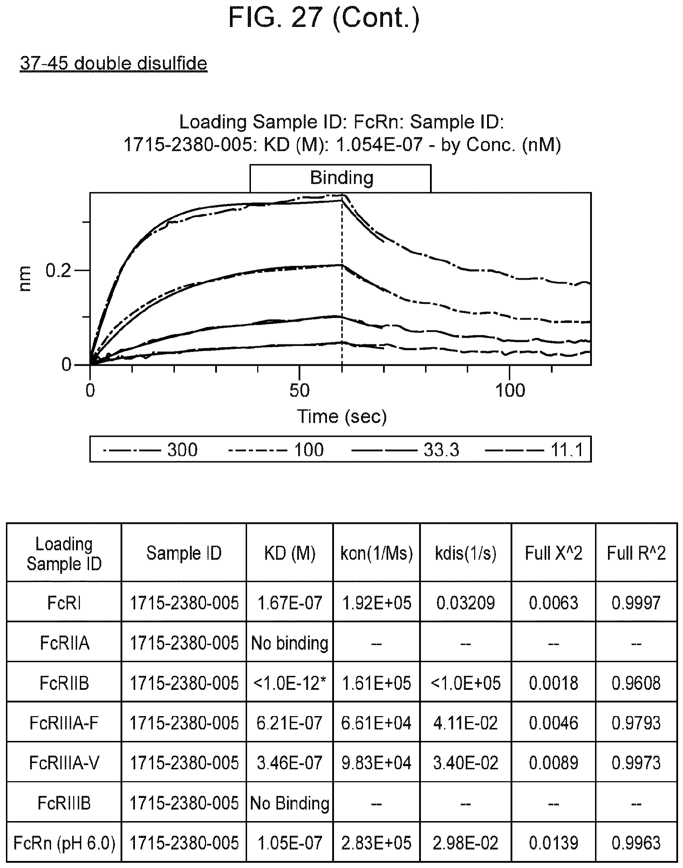

The ability of the TMMPs used in these experiments to bind to FcRn was tested. The TMMPs include an Ig Fc region that can bind to FcRn. Binding to FcRn is an indication of prolonged *in vivo* half-life. Souders et al. (2015) MAbs 7:912. The data for the "1715 + 2380" TMPP are shown in **FIG. 27****.**

The ability of the "1715 + 2380" TMPP to bind to other Fc receptors was tested. The "1715 + 2380" TMPP includes an Ig Fc region with LALA substitutions, which reduce binding of the Ig Fc to FcRI, RIIA, IIB, IIIA-F, and IIIB, thereby reducing Ig Fc-mediated effector functions. The data are shown in **FIG. 27****.**

### MATERIALS AND METHODS

### FIG. 21

Leukopaks from two healthy donors were obtained using apheresis machines. Leukopaks were diluted with an equal volume of room temperature phosphate-buffered saline (PBS). PBMCs were isolated from diluted leukopaks by density gradient centrifugation as follows: 30 mL of diluted leukopak was underlayed with 13 mL of Ficoll-Paque in a 50 mL conical tube and centrifugated at 400 g for 30 minutes at room temperature in a swinging bucket rotor without brake. Mononuclear cell layer (lymphocytes, monocytes and thrombocytes) was collected from the plasma-Ficoll interface, transferred to new 50 mL conical tube and washed with 3-fold excess PBS by centrifugation at 300 g for 10 minutes at room temperature. After careful removal of supernatant, cells were resuspended and washed with 50 mL of PBS by centrifugation at 200 g for 10 minutes at room temperature to remove platelets. Upon washing and platelet removal, obtained PBMCs were pooled from the 50 mL tubes, resuspended in PBS, counted, pelleted by centrifugation at 300 g for 10 minutes and resuspended at a final concentration of 50x10⁶ cells per ml in cell freezing media.

Human healthy donor PBMCs were prepared from two leukopaks as described above. On the day of the experiment, the cells were thawed in A 37°C water bath and washed in warm ImmunoCult^{™}-XF Cell Expansion Media (Stemcell Technologies) by centrifugation at 350 x g for 6 minutes. The supernatant was removed, and the cells were resuspended in ImmunoCult^{™} media. Live cell count was assessed using the Countess automated cell counter (Invitrogen, CA). The media volume was adjusted to bring the cell concentration to 5x10⁶ cells/ml and 2 mL of cells (equivalent to 10x10⁶ cells) were seeded per well in a 6-well plate. PBMCs were stimulated with the indicated amounts of Immuno-STATs or with media alone in a total volume of 4 ml of media. Cells were stimulated for 10 days at 37°C, 5% CO₂ with media replacement on days 5 and 7 by aspirating 2 mL of culture supernatant from the wells and adding back 2 mL of fresh media.

Upon culture, the cells were harvested and pelleted by centrifugation at 350 x g for 5 minutes, live cell counts were determined by the Countess automated cell counter (Invitrogen, CA), and cells were processed for flow cytometry by staining with: a viability stain, appropriate WT1-peptide-specific HLA-A*0201 tetramers (MBL International) and antibodies against CD69, CD3, CD14, CD19, CD127, CD56, CD4 (Biolegend), CD8, CD25 (BD Biosciences) Stained cells were washed and analyzed by flow cytometry.

Data acquisition was performed using the Attune NxT flow cytometer instrument (Invitrogen). The acquired data was exported as fcs files and analyzed using the Flowjo software (Tree Star, OR).

Based on the frequency of antigen-specific T cells, volumes and events analyzed by flow cytometry and total volume and number of cells harvested at the end of the culture, the number of antigen specific T cells per well was calculated and plotted in the graphs shown.

### FIG. 22

PBMCs from two donors were expanded for 10 days with 10 micrograms/ml of the indicated peptides and 50U/ml of recombinant human IL-2. Expansion was done in 6-well plates with a total of 10 million cells in 4 ml of Immunocult media per well. Cells were stimulated for 10 days at 37°C, 5% CO₂ with media replacement on days 5 and 7 by aspirating 2 mL of culture supernatant from the wells and adding back 2 mL of fresh media with 50 U/ml of recombinant human IL-2. Upon culture, the cells were harvested and pelleted by centrifugation at 350 g for 5 minutes, live cell counts were determined by the Countess automated cell counter (Invitrogen, CA). Each stimulation condition was performed in at least 3 wells of a six well plate. PBMCs from one well were used to estimate the frequency/amount of WT1-specific CD8+ T cells in the culture by flow cytometry upon staining with: a viability stain, appropriate WT1-peptide-specific HLA-A*0201 tetramers (MBL International) and antibodies against CD69, CD3, CD14, CD19, CD127, CD56, CD4 (Biolegend), CD8, CD25 (BD Biosciences). Stained cells were washed and analyzed by flow cytometry. At least two wells were used to enrich for CD8+ T cells using a CD8+ T cell negative selection kit from Stem Cell Technologies. The purified CD8+ T cells were restimulated for 8 days with the indicated TMMPs in the presence of autologous PBMCs, previously treated with mitomycin C, in a 1:2 ratio, with a final 5-10 million cells in a volume of 4 ml of Immunocult media per well. TMMPs were used at concentrations previously established to be optimal for the combination of a donor and a particular TMMP.

Upon culture, the cells were harvested and pelleted by centrifugation at 350 g for 5 minutes, live cell counts were determined by the Countess automated cell counter (Invitrogen, CA) and processed for flow cytometry by staining with: a viability stain, appropriate WT1-peptide-specific HLA-A*0201 tetramers (MBL International) and antibodies against CD69, CD3, CD14, CD19, CD127, CD56, CD4 (Biolegend), CD8, CD25 (BD Biosciences) Stained cells were washed and analyzed by flow cytometry.

Data acquisition was performed using the Attune NxT flow cytometer instrument (Invitrogen). The acquired data was exported as fcs files and analyzed using the Flowjo software (Tree Star, OR).

Based on the frequency of antigen-specific T cells, volumes and events analyzed by flow cytometry and total volume and number of cells harvested at the end of the culture, the number of antigen specific T cells per well was calculated and plotted in the graphs shown.

### FIG. 23

PBMCs from two donors were expanded for 10 days with 10 micrograms/ml of the WT1 37-45 peptide and 50U/ml of recombinant human IL-2. Expansion was done in 6-well plates with a total of 10 million cells in 4 ml of Immunocult media per well. Cells were stimulated for 10 days at 37°C, 5% CO₂ with media replacement on days 5 and 7 by aspirating 2 mL of culture supernatant from the wells and adding back 2 mL of fresh media with 50 U/ml of recombinant human IL-2. Upon culture, the cells were harvested and pelleted by centrifugation at 350 g for 5 minutes, live cell counts were determined by the Countess automated cell counter (Invitrogen, CA). The stimulation was performed in at least 3 wells of a six well plate. PBMCs from one well were used to estimate the frequency/amount of WT1 37-45 peptide-specific CD8+ T cells in the culture by flow cytometry upon staining with: a viability stain, appropriate WT1 37-45-peptide-specific HLA-A*0201 tetramers (MBL International) and antibodies against CD69, CD3, CD14, CD19, CD127, CD56, CD4 (Biolegend), CD8, CD25 (BD Biosciences). Stained cells were washed and analyzed by flow cytometry. Data acquisition was performed using the Attune NxT flow cytometer instrument (Invitrogen). The acquired data was exported as fcs files and analyzed using the Flowjo software (Tree Star, OR).

At least two wells were used to enrich for CD8+ T cells using a CD8+ T cell negative selection kit from Stem Cell Technologies. The purified CD8+ T cells were restimulated for 8 days with the indicated WT1 37-45 specific Immuno-STATs on either the G2C or the R12C/G2C framework in the presence of autologous PBMCs, previously treated with mitomycin C, in a 1:2 ratio, with a final 5-10 million cells in a volume of 4 ml of Immunocult media per well. WT1 37-45 specific Immuno-STAT on either the G2C or the R12C/G2C framework was used at the concentration previously established to be optimal for that donor.

Upon culture, the cells were harvested and pelleted by centrifugation at 350 g for 5 minutes, live cell counts were determined by the Countess automated cell counter (Invitrogen, CA) and CD8+ T cells were enriched using a CD8+ T cell negative selection kit from Stem Cell Technologies.

Target cells, T2 cells (ATCC), were pulsed with 5 µg/mL of the WT1 37-45 peptide or the human immunodeficiency virus-1 (HIV-1) Gag₇₇₋₈₅ SL9 peptide for 2 hours at 37°C, 5% CO₂. Post-peptide loading, the T2 cells were washed twice and resuspend in ImmunoCult^{™}-XF Cell Expansion Media (Stemcell Technologies).

The enriched CD8⁺ T cells and the peptide-loaded T2 cells were mixed at a 1:1 ratio (1x10⁶ cells each) in a final volume of 200 µL per well in 96-well plates. Media and Phorbol 12-myristate; 13-acetate (PMA)/ionomycin was added to control wells as negative and positive controls, respectively. At 0.5 to 1 hour post-stimulation, the staining antibody against CD107a was added directly to the cells. Cell were stimulated for 5 hours, washed with PBS and stained for viability using the FVS780 for 10 minutes on ice. The cells were washed stained with the WT1 37-45 peptide-specific tetramers (labeled with APC and PE) for 15 minutes at room temperature. Subsequently, the cells were washed and stained antibodies against CD3 and CD8 for 30 minutes on ice. Stained cells were washed twice and resuspended in intracellular (IC) fixation buffer overnight at 4°C. The following day, the cells were washed and resuspended in permeabilization buffer and incubated for 5 minutes at room temperature. Permeabilized cells were washed and stained with antibodies against interferon-γ (IFN-γ), tumor-necrosis factor-α (TNF-α), resuspended in permeabilization buffer, for 30 minutes at room temperature. Stained cells were washed, resuspended in 2 mL of FACS buffer, and transferred to a 96-well deep plate. Data acquisition was performed using the Attune NxT flow cytometer instrument (Thermofisher Scientific, MA). The acquired data was exported as fcs files and analyzed using the Flowjo software (Tree Star, OR).

### FIG. 24

PBMCs from two donors were expanded for 10 days with 10 micrograms/ml of the WT1 126-134 peptide and 50U/ml of recombinant human IL-2. Expansion was done in 6-well plates with a total of 10 million cells in 4 ml of Immunocult media per well. Cells were stimulated for 10 days at 37°C, 5% CO₂ with media replacement on days 5 and 7 by aspirating 2 mL of culture supernatant from the wells and adding back 2 mL of fresh media with 50 U/ml of recombinant human IL-2. Upon culture, the cells were harvested and pelleted by centrifugation at 350 g for 5 minutes, live cell counts were determined by the Countess automated cell counter (Invitrogen, CA). The stimulation was performed in at least 3 wells of a six well plate. PBMCs from one well were used to estimate the frequency/amount of WT1 126-134 peptide-specific CD8+ T cells in the culture by flow cytometry upon staining with: a viability stain, appropriate WT1 126-134-peptide-specific HLA-A*0201 tetramers (MBL International) and antibodies against CD69, CD3, CD14, CD19, CD127, CD56, CD4 (Biolegend), CD8, CD25 (BD Biosciences). Stained cells were washed and analyzed by flow cytometry. Data acquisition was performed using the Attune NxT flow cytometer instrument (Invitrogen). The acquired data was exported as fcs files and analyzed using the Flowjo software (Tree Star, OR).

At least two wells were used to enrich for CD8+ T cells using a CD8+ T cell negative selection kit from Stem Cell Technologies. The purified CD8+ T cells were restimulated for 8 days with the WT1 126-134 specific Immuno-STATs on the R12C/G2C framework in the presence of autologous PBMCs, previously treated with mitomycin C, in a 1:2 ratio, with a final 5-10 million cells in a volume of 4 ml of Immunocult media per well. WT1 126-134 specific Immuno-STAT on the R12C/G2C framework was used at the concentration previously established to be optimal for that donor.

Upon culture, the cells were harvested and pelleted by centrifugation at 350 g for 5 minutes, live cell counts were determined by the Countess automated cell counter (Invitrogen, CA) and CD8+ T cells were enriched using a CD8+ T cell negative selection kit from Stem Cell Technologies.

Target cells, T2 cells (ATCC), were pulsed with 5 µg/mL of the WT1 126-134 peptide, the WT1 126-134 R126Y peptide or the human immunodeficiency virus-1 (HIV-1) Gag₇₇₋₉₅ SL9 peptide for 2 hours at 37°C, 5% CO₂. Post-peptide loading, the T2 cells were washed twice and resuspend in ImmunoCult^{™}-XF Cell Expansion Media (Stemcell Technologies).

The enriched CD8⁺ T cells and the peptide-loaded T2 cells were mixed at a 1:1 ratio (1x10⁶ cells each) in a final volume of 200 µL per well in 96-well plates. Media and Phorbol 12-myristate; 13-acetate (PMA)/ionomycin was added to control wells as negative and positive controls, respectively. At 0.5 to 1 hour post-stimulation, the staining antibody against CD107a was added directly to the cells. Cell were stimulated for 5 hours, washed with PBS and stained for viability using the FVS780 for 10 minutes on ice. The cells were washed stained with the WT1 126-134 peptide-specific tetramers (labeled with APC and PE) for 15 minutes at room temperature. Subsequently, the cells were washed and stained antibodies against CD3 and CD8 for 30 minutes on ice. Stained cells were washed twice and resuspended in intracellular (IC) fixation buffer overnight at 4°C. The following day, the cells were washed and resuspended in permeabilization buffer and incubated for 5 minutes at room temperature. Permeabilized cells were washed and stained with antibodies against interferon-γ (IFN-γ), tumor-necrosis factor-α (TNF-α), resuspended in permeabilization buffer, for 30 minutes at room temperature. Stained cells were washed, resuspended in 2 mL of FACS buffer, and transferred to a 96-well deep plate. Data acquisition was performed using the Attune NxT flow cytometer instrument (Thermofisher Scientific, MA). The acquired data was exported as fcs files and analyzed using the Flowjo software (Tree Star, OR).

### FIG. 25

Human healthy donor PBMCs were prepared from leukopaks obtained from Hemacare (Northridge, CA) and kept cryopreserved at -150°C until the day of experiment.

The cells were thawed on the day of the experiment in a water bath for 1 minute, washed with 10 mL of warm ImmunoCult^{™}-XF Cell Expansion Media (Stemcell Technologies, Vancouver, Canada), pelleted by centrifugation (350 g, 5 minutes), and resuspended in 10 mL media. Cells were counted using the Countess automated cell counter (Invitrogen, CA), the media volume was adjusted to bring the cell concentration to 3.8x10⁶ cell/mL and 237.5 uL of the cell suspensions were added into round bottom 96-well plates.

20X dilution series of the indicated Immuno-STATs and of rh-IL-2, were prepared in Immunocult media. To stimulate the PBMCs, 12.5 µL of the 20X dilution series was added to the wells containing the cells and mixed to obtain the final assay drug concentrations. The PBMCs were incubated at 37°C, 5% CO₂ for 20 to 24 hours.

Upon stimulation, the cells were pelleted by centrifugation at 350 g for 5 minutes. Supernatants were collected, frozen and stored at -20°C until further analysis. Pelleted PBMCs were washed twice with PBS and stained for 10 minutes at 4°C in 50 µL of Fixable live/dead FVS780 stain. The staining was quenched with 200 µL of stain buffer and the cells were pelleted by centrifugation (350 x g, 5 minutes). The cells were stained for 30 minutes at 4°C with antibodies against CD3, CD4, CD8, CD14, CD19, CD56, and CD69 in 50 µL volume. Upon staining cells were washed with stain buffer, pelleted by centrifugation (350 x g, 5 minutes), resuspended in 130 µL of stain buffer and analyzed by Flow Cytometry using the Attune^{®} Flow Cytometer. The acquired data was exported as fcs files and analyzed using the Flowjo software (Tree Star, OR).

CD69 upregulation was assessed on different cell subsets that are sensitive to upregulate CD69 upon IL-2 stimulation. Based on expression levels of the surface markers used in the staining, gates were made to identify NK cells, CD8+ T cells, CD4+ T cells.

### FIG. 26

One day before the assay, CTLL-2 cells were washed with media and cultured at 1x10⁵ cells /ml in a 75-T flask for 24 hours at 37C, 5% CO₂ for IL-2 starvation. After the 24 hour starvation culture, cells were seeded at 5000 cells per well in 100 microliters/well of a 96 well cell culture cluster flat bottom plate with lid (Costar corning, Cat # 3599). Cell viability and count were checked before stimulation using Vi cell viability analyzer (Beckman-Coulter).

Dilution series (10 points; 3-fold dilution steps) of the indicated Immuno-STATs (TMMPs) or proleukin (Prometheus Therapeutics) were prepared in complete RPMI supplemented with 10% HI FBS as 2X stocks of the final assay concentrations. 100 µL of this 2X dilution series were added to cells previously seeded in 96 well plates and mixed to obtain the final assay drug concentrations. Each concentration was tested in triplicates. Cells were incubated for three days at 37C, 5% CO₂.

After three days in culture 100 µL of cells from each well was transferred into a flat bottom white tissue culture treated 96 well plate. 100 µL of CellTiter-Glo^{®} Reagent was prepared using CellTiter-Glo Luminescent Cell viability assay kit (Promega cat # G7571) following instructions provided by the manufacturer and were added to the cells. Cells and CellTiter-Glo^{®} Reagent were mixed by placing the plates on an orbital shaker for 2 minutes to induce cell lysis. Then plates were incubated at room temperature for 10 minutes to stabilize luminescent signal. The luminescence was measured and recorded on Biotek synergy neo2 multimode reader, software Gen5 3.04.

### FIG. 27

All experiments were performed on the Octet HTX system (ForteBio). Anti-penta-his (HIS1K) kinetic grade biosensors (ForteBio, #18-5122) were hydrated in assay buffer and preconditioned in pH 1.7 glycine. The assay buffer was used for all assays except for FcRn. The buffer used for FcRn was PBS, 0.1% bovine serum albumin (BSA), 0.02% Tween-20, pH 7.2. The assay buffer used for the FcRn reagents was PBS, 0.1% BSA, 0.02% Tween-20, pH 6.

Each His-tagged receptor was immobilized onto HIS1K biosensors at a concentration of 5 µg/mL (except for FcRI: 10 ug/mL) for 120 seconds. The antigen-loaded HIS1K biosensors were then dipped into a 7-point, 1:3 dilution series of each individual antibody starting from 300 nM. A well containing only assay buffer was used to test for non-specific binding between the buffer and loaded biosensors. Association was observed for 60 seconds, followed by 60 seconds of dissociation. A short baseline (60 seconds) was established using dissociation buffer after HIS1K loading.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.
The following numbered clauses, describing aspects of the invention, are part of the description
1. A T-cell modulatory multimeric polypeptide comprising:
   at least one heterodimer comprising:
      a) a first polypeptide comprising:
         i) a Wilms tumor-1 (WT-1) peptide epitope, wherein the WT-1 peptide has a length of at least 4 amino acids; and
         ii) first class I major histocompatibility complex (MHC) polypeptide;
      b) a second polypeptide comprising a second class I MHC polypeptide, and
      c) at least one activating immunomodulatory,
   wherein the first and/or the second polypeptide comprises the immunomodulatory polypeptide, and
   wherein the WT-1 peptide epitope comprises an amino acid sequence selected from the group consisting of: NLMNLGATL (SEQ ID NO:258), NYMNLGATL (SEQ ID NO:263), CMTWNQMNLGATLKG (SEQ ID NO:223), WNQMNLGATLKGVAA (SEQ ID NO:224), CMTWNYMNLGATLKG (SEQ ID NO:225), WNYMNLGATLKGVAA (SEQ ID NO:226), MTWNQMNLGATLKGV (SEQ ID NO:227), TWNQMNLGATLKGVA (SEQ ID NO:228), CMTWNLMNLGATLKG (SEQ ID NO:229), MTWNLMNLGATLKGV (SEQ ID NO:230), TWNLMNLGATLKGVA (SEQ ID NO:231), WNLMNLGATLKGVAA (SEQ ID NO:232), MNLGATLK (SEQ ID NO:233), MTWNYMNLGATLKGV SEQ ID NO:234), TWNYMNLGATLKGVA (SEQ ID NO:235), CMTWNQMNLGATLKGVA (SEQ ID NO:236), CMTWNLMNLGATLKGVA (SEQ ID NO:237), CMTWNYMNLGATLKGVA (SEQ ID NO:238), GYLRNPTAC (SEQ ID NO:239), GALRNPTAL (SEQ ID NO:240), YALRNPTAC (SEQ ID NO:241), GLLRNPTAC (SEQ ID NO:242), RYRPHPGAL (SEQ ID NO:243), YQRPHPGAL (SEQ ID NO:244), RLRPHPGAL (SEQ ID NO:245), RIRPHPGAL (SEQ ID NO:246), QFPNHSFKHEDPMGQ (SEQ ID NO:247), HSFKHEDPY (SEQ ID NO:248), QFPNHSFKHEDPM (SEQ ID NO:249), QFPNHSFKHEDPY (SEQ ID NO:250), KRPFMCAYPGCNK (SEQ ID NO:251), KRPFMCAYPGCYK (SEQ ID NO:252), FMCAYPGCY (SEQ ID NO:253), FMCAYPGCK (SEQ ID NO:254), KRPFMCAYPGCNKRY (SEQ ID NO:255), SEKRPFMCAYPGCNK (SEQ ID NO:256), KRPFMCAYPGCYKRY (SEQ ID NO:257), NLMNLGATL (SEQ ID NO:258), VLDFAPPGA (SEQ ID NO:259), RMFPNAPYL (SEQ ID NO:260), CMTWNQMN (SEQ ID NO:261), CYTWNQMNL (SEQ ID NO:269), NYMNLGATL (SEQ ID NO:263), YMFPNAPYL (SEQ ID NO:264), SLGEQQYSV (SEQ ID NO:265), CMTWNQMNL (SEQ ID NO:266), and NQMNLGATL (SEQ ID NO:267),
   optionally wherein the first or the second polypeptide comprises an immunoglobulin (Ig) Fc polypeptide.
2. A T-cell modulatory multimeric polypeptide of clause 1, wherein at least one of the one or more immunomodulatory domains is a variant immunomodulatory polypeptide that exhibits reduced affinity to a cognate co-immunomodulatory polypeptide compared to the affinity of a corresponding wild-type immunomodulatory polypeptide for the cognate co-immunomodulatory polypeptide,
   and wherein the epitope binds to a T-cell receptor (TCR) on a T cell with an affinity of at least 10⁻⁷ M, such that:
   i) the T-cell modulatory multimeric polypeptide binds to a first T cell with an affinity that is at least 25% higher than the affinity with which the T-cell modulatory multimeric polypeptide binds a second T cell, wherein the first T cell expresses on its surface the cognate co-immunomodulatory polypeptide and a TCR that binds the epitope with an affinity of at least 10⁻⁷ M, and
      wherein the second T cell expresses on its surface the cognate co-immunomodulatory polypeptide but does not express on its surface a TCR that binds the epitope with an affinity of at least 10⁻⁷ M; and/or
   ii) the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is in a range of from 1.5:1 to 10⁶:1.
3. A T-cell modulatory multimeric polypeptide of clause 2, wherein:
   a) the T-cell modulatory multimeric polypeptide binds to the first T cell with an affinity that is at least 50%, at least 2-fold, at least 5-fold, or at least 10-fold higher than the affinity with which it binds the second T cell; and/or
   b) the variant immunomodulatory polypeptide binds the co-immunomodulatory polypeptide with an affinity of from about 10⁻⁴ M to about 10⁻⁷ M, from about 10⁻⁴ M to about 10⁻⁶ M, from about 10⁻⁴ M to about 10⁻⁵ M; and/or
   c) wherein the ratio of the binding affinity of a control T-cell modulatory multimeric polypeptide, wherein the control comprises a wild-type immunomodulatory polypeptide, to a cognate co-immunomodulatory polypeptide to the binding affinity of the T-cell modulatory multimeric polypeptide comprising a variant of the wild-type immunomodulatory polypeptide to the cognate co-immunomodulatory polypeptide, when measured by bio-layer interferometry, is at least 10:1, at least 50:1, at least 10²:1, or at least 10³:1.
4. A T-cell modulatory multimeric polypeptide of any one of clauses 1-3, wherein
   a1) the first polypeptide comprises, in order from N-terminus to C-terminus:
      i) the WT-1 peptide epitope;
      ii) the first MHC polypeptide; and
      iii) at least one immunomodulatory polypeptide; and
   b1) the second polypeptide comprises, in order from N-terminus to C-terminus:
      i) the second MHC polypeptide; and
      ii) an immunoglobulin (Ig) Fc polypeptide; or
   a2) the first polypeptide comprises, in order from N-terminus to C-terminus:
      i) the WT-1 peptide epitope; and
      ii) the first MHC polypeptide; and
   b2) the second polypeptide comprises, in order from N-terminus to C-terminus:
      i) at least one immunomodulatory polypeptide;
      ii) the second MHC polypeptide; and
      iii) an Ig Fc polypeptide; or
   a3) the first polypeptide comprises, in order from N-terminus to C-terminus:
      i) the WT-1 peptide epitope; and
      ii) the first MHC polypeptide; and
   b3) the second polypeptide comprises, in order from N-terminus to C-terminus:
      i) the second MHC polypeptide; and
      ii) an Ig Fc polypeptide; and
      iii) at least one immunomodulatory polypeptide; or
   a4) the first polypeptide comprises, in order from N-terminus to C-terminus:
      i) the WT-1 peptide epitope; and
      ii) the first MHC polypeptide; and
   b4) the second polypeptide comprises, in order from N-terminus to C-terminus:
      i) the second MHC polypeptide; and
      ii) at least one immunomodulatory polypeptide; or
   a5) the first polypeptide comprises, in order from N-terminus to C-terminus:
      i) the WT-1 peptide epitope; and
      ii) the first MHC polypeptide; and
   b5) a second polypeptide comprises, in order from N-terminus to C-terminus:
      i) at least one immunomodulatory polypeptide; and
      ii) the second MHC polypeptide; or
   a6) the first polypeptide comprises, in order from N-terminus to C-terminus:
      i) the WT-1 peptide epitope;
      ii) the first MHC polypeptide; and
      iii) at least one immunomodulatory polypeptide; and
   b6) the second polypeptide comprises:
      i) the second MHC polypeptide.
5. A T-cell modulatory multimeric polypeptide of any one of clauses 1-4, wherein:
   a) the first MHC polypeptide is a β2-microglobulin polypeptide; and the second MHC polypeptide is an MHC class I heavy chain polypeptide; or
   b) the first MHC polypeptide is an MHC class I heavy chain polypeptide; and the second MHC polypeptide is a β2-microglobulin polypeptide.
6. A T-cell modulatory multimeric polypeptide of clause 5, wherein:
   a) the first polypeptide comprises, in order from N-terminus to C-terminus:
      i) the WT-1 peptide epitope; and
      ii) the β2-microglobulin polypeptide; and
   b) the second polypeptide comprises, in order from N-terminus to C-terminus:
      i) at least one immunomodulatory polypeptide;
      ii) the MHC class I heavy chain polypeptide; and
      iii) an Ig Fc polypeptide.
7. A T-cell modulatory multimeric polypeptide of clause 5, wherein:
   a) the first polypeptide comprises, in order from N-terminus to C-terminus:
      i) the WT-1 peptide epitope; and
      ii) the β2-microglobulin polypeptide; and
   b) the second polypeptide comprises, in order from N-terminus to C-terminus:
      i) the MHC class I heavy chain polypeptide; and
      ii) an Ig Fc polypeptide; and
      iii) at least one immunomodulatory polypeptide
8. A T-cell modulatory multimeric polypeptide of any one of clauses 1-7, wherein the at least one immunomodulatory polypeptide is selected from the group consisting of a cytokine, a 4-1BBL polypeptide, an ICOS-L polypeptide, an OX-40L polypeptide, a CD80 polypeptide, a CD86 polypeptide, a CD40 polypeptide, a CD70 polypeptide, and combinations thereof.
9. A T-cell modulatory multimeric polypeptide of any one of clauses 1-8, wherein the at least one immunomodulatory polypeptide is an IL-2 polypeptide.
10. A T-cell modulatory multimeric polypeptide of any one of clauses 1-9, wherein the multimeric polypeptide comprises at least two immunomodulatory polypeptides, and wherein at least two of the immunomodulatory polypeptides are the same, optionally wherein the 2 or more immunomodulatory polypeptides are in tandem.
11. A T-cell modulatory multimeric polypeptide of any one of clauses 1-10, wherein the immunomodulatory polypeptide is a variant IL-2 polypeptide that exhibits reduced affinity to an IL-2 receptor compared to the affinity of a wild-type IL-2 polypeptide for the IL-2 receptor.
12. A T-cell modulatory multimeric polypeptide of clause 11, wherein the variant IL-2 polypeptide comprises: i) an H16A substitution and an F42A substitution; or ii) an H16T substitution and an F42A substitution.
13. A T-cell modulatory multimeric polypeptide of any one of clauses 1-12, wherein the first polypeptide and the second polypeptide are covalently linked to one another, optionally wherein the covalent linkage is via a disulfide bond.
14. A T-cell modulatory multimeric polypeptide of any one of clauses 1-13, wherein the first MHC polypeptide or a linker between the epitope and the first MHC polypeptide comprises an amino acid substitution to provide a first Cys residue, wherein the second MHC polypeptide comprises an amino acid substitution to provide a second Cys residue, and wherein the disulfide linkage is between the first and the second Cys residues.
15. The T-cell modulatory multimeric polypeptide of any one of clauses 1-14, wherein the polypeptide comprises a disulfide bond between: i) a Cys present in a linker between the WT-1 peptide epitope and the first MHC class I polypeptide, wherein the first MHC class I polypeptide is a β2M polypeptide; and ii) a Cys residue introduced via a Y84C substitution in the second MHC class I polypeptide, wherein the second MHC class I polypeptide is a MHC Class I heavy chain polypeptide.
16. The T-cell modulatory multimeric polypeptide of any one of clauses 1-14, wherein the polypeptide comprises a disulfide bond between i) a Cys residue introduced into the first MHC class I polypeptide via an R12C substitution, wherein the first MHC class I polypeptide is a β2M polypeptide; and ii) a Cys residue introduced into the second MHC class I polypeptide, via an A236C substitution, wherein second MHC class I polypeptide is an MHC Class I heavy chain polypeptide.
17. The T-cell modulatory multimeric polypeptide of any one of clauses 1-14, wherein the polypeptide comprises a first disulfide bond between: i) a Cys present in a linker between the WT-1 peptide epitope and the first MHC class I polypeptide, wherein the first MHC class I polypeptide is a β2M polypeptide; and ii) a Cys residue introduced via a Y84C substitution in the second MHC class I polypeptide, wherein the second MHC class I polypeptide is a MHC Class I heavy chain polypeptide, and a second disulfide bond between i) a Cys residue introduced into the β2M polypeptide via an R12C substitution; and ii) a Cys residue introduced into the MHC Class I heavy chain polypeptide via an A236C substitution.
18. A T-cell modulatory multimeric polypeptide of clause 15 or clause 17, wherein the linker between the WT-1 peptide epitope and the first MHC is GCGGS(G4S)n (SEQ ID NO:315), where n is 1, 2, 3, 4, 5, 6, 7, 8, or 9.
19. A T-cell modulatory multimeric polypeptide of any one of clauses 1-18, wherein the WT-1 peptide epitope has a length of from about 4 amino acids to about 25 amino acids.
20. A T-cell modulatory multimeric polypeptide of any one of clauses 1-19, wherein the WT-1 peptide comprises the amino acid sequence CMTWNQMNL (SEQ ID NO: 266), CYTWNQMNL (SEQ ID NO:269), NYMNLGATL (SEQ ID NO:263), VLDFAPPGA (SEQ ID NO:259), YMFPNAPYL (SEQ ID NO:264), SLGEQQYSV (SEQ ID NO:265), RMFPNAPYL (SEQ ID NO:260), and NLMNLGATL (SEQ ID NO:258).
21. A T-cell modulatory multimeric polypeptide of any one of clauses 1-20, wherein the first or the second MHC polypeptide comprises:
   a) an amino acid sequence having at least 95% amino acid sequence identity to the HLA-A*0101, HLA-A*0201, HLA-A*0201, HLA-A*1101, HLA-A*2301, HLA-A*2402, HLA-A*2407, HLA-A*3303, or HLA-A*3401 amino acid sequence depicted in FIG. 9A; or
   b) an amino acid sequence having at least 95% amino acid sequence identity to the HLA-B*0702, HLA-B*0801, HLA-B*1502, HLA-B*3802, HLA-B*4001, HLA-B*4601, or HLA-B*5301 amino acid sequence depicted in FIG. 10A; or
   c) an amino acid sequence having at least 95% amino acid sequence identity to the HLA-C*0102, HLA-C*0303, HLA-C*0304, HLA-C*0401, HLA-C*0602, HLA-C*0701, HLA-C*0702, HLA-C*0801, or HLA-C*1502 depicted in FIG. 11A.
22. A T-cell modulatory multimeric polypeptide of any one of clauses 1-21, wherein the first MHC polypeptide is a β2M polypeptide, and wherein the second MHC polypeptide comprises an amino acid sequence having at least 95% amino acid sequence identity to an HLA-A*2402 polypeptide, and wherein the epitope is selected from the group consisting of: RMFPNAPYL (SEQ ID NO:260), CYTWNQMNL (SEQ ID NO:269), and NYMNLGATL (SEQ ID NO:263).
23. A T-cell modulatory multimeric polypeptide of any one of clauses 1-21, wherein the first MHC polypeptide is a β2M polypeptide, and wherein the second MHC polypeptide comprises an amino acid sequence having at least 95% amino acid sequence identity to an HLA-A*0201 polypeptide, and wherein the epitope is selected from the group consisting of: VLDFAPPGA (SEQ ID NO:259), RMFPNAPYL (SEQ ID NO:260), and YMFPNAPYL (SEQ ID NO:264).
24. A T-cell modulatory multimeric polypeptide of any one of clauses 1-23, wherein the multimeric polypeptide comprises a first and a second heterodimer, and wherein the first and second heterodimers are covalently bound by one or more disulfide bonds between the Ig Fc polypeptides of the first and second heterodimers.
25. A nucleic acid comprising a nucleotide sequence encoding a first or second polypeptide according to any one of clauses 1-24.
26. An expression vector comprising the nucleic acid of clause 25.
27. A method of selectively modulating the activity of T cell specific for a Wilms tumor-1 (WT-1) epitope, the method comprising contacting the T cell with a T-cell modulatory multimeric polypeptide according to any one of clauses 1-24, wherein said contacting selectively modulates the activity of the WT-1 epitope-specific T cell.
28. A method of treating a patient having a cancer, the method comprising administering to the patient an effective amount of a pharmaceutical composition comprising T-cell modulatory multimeric polypeptide according to any one of clauses 1-24.
29. The method of clause 28, wherein the cancer is acute myeloid leukemia, myeloma, ovarian cancer, pancreatic cancer, non-small cell lung cancer, colorectal cancer, breast cancer, Wilms tumor, mesothelioma, soft tissue sarcoma, neuroblastoma, or nephroblastoma.
30. A method of clause 28 or clause 29, further comprising administering one or more checkpoint inhibitors to the individual.
31. A method according to clause 30, wherein the checkpoint inhibitor is an antibody that binds to a polypeptide selected from the group consisting of CD27, CD28, CD40, CD122, CD96, CD73, CD47, OX40, GITR, CSF1R, JAK, PI3K delta, PI3K gamma, TAM, arginase, CD137, ICOS, A2AR, B7-H3, B7-H4, BTLA, CTLA-4, LAG3, TIM3, VISTA, CD96, TIGIT, CD122, PD-1, PD-L1, and PD-L2.
32. A method according to clause 31, wherein the checkpoint inhibitor is an antibody specific for PD-1, PD-L1, or CTLA4.
33. A method according to clause 30, wherein the one or more checkpoint inhibitors is selected from the group consisting of nivolumab, pembrolizumab, pidilizumab, AMP-224, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, Mogamulizumab, Varlilumab, Avelumab, Galiximab, AMP-514, AUNP 12, Indoximod, NLG-919, INCB024360, KN035, and combinations thereof.
34. A method of modulating an immune response in an individual, the method comprising
   administering to the individual an effective amount of the T-cell modulatory multimeric polypeptide of any one of clauses 1-24,
   wherein said administering induces an epitope-specific T cell response and an epitope-non-specific T cell response, and
   wherein the ratio of the epitope-specific T cell response to the epitope-non-specific T cell response is at least 2:1.
35. A method of delivering an immunomodulatory polypeptide selectively to a target T cell, the method comprising contacting a mixed population of T cells with a T-cell modulatory multimeric polypeptide of any one of clauses 1-24, wherein the mixed population of T cells comprises the target T cell and non-target T cells, wherein the target T cell is specific for the WT-1 epitope present within the T-cell modulatory multimeric polypeptide, and wherein said contacting delivers the one or more immunomodulatory polypeptides present within the T-cell modulatory multimeric polypeptide to the target T cell.
36. A method of detecting, in a mixed population of T cells obtained from an individual, the presence of a target T cell that binds a WT-1 epitope, the method comprising:
   a) contacting *in vitro* the mixed population of T cells with the T-cell modulatory multimeric polypeptide of any one of clauses 1-24, wherein the T-cell modulatory multimeric polypeptide comprises the WT-1 epitope; and
   b) detecting activation and/or proliferation of T cells in response to said contacting, wherein activated and/or proliferated T cells indicates the presence of the target T cell.

## Claims

1. A T-cell modulatory multimeric polypeptide (TMMP) comprising:
at least one heterodimer comprising:
a) a first polypeptide comprising:
i) a Wilms tumor-1 (WT-1) peptide epitope, wherein the WT-1 peptide has a length of from 4 to 25 amino acids; and
ii) a first class I major histocompatibility complex (MHC) polypeptide, wherein the first class I MHC polypeptide is a β2-microglobulin (β2M) polypeptide;
b) a second polypeptide comprising:
i) a second class I MHC polypeptide, wherein the second class I MHC polypeptide is an MHC class I heavy chain polypeptide;
ii) one or more immunomodulatory polypeptides, wherein the one or more immunomodulatory polypeptides is one or more variant IL-2 polypeptides that exhibit reduced affinity to an IL-2 receptor and that comprise a substitution of H16, or a substitution of H16 and a substitution of F42, based on the amino acid numbering of SEQ ID NO:15; and
iii) an immunoglobulin (Ig) Fc polypeptide, wherein the Ig Fc polypeptide is an IgG1 Fc polypeptide,
wherein the first polypeptide and the second polypeptide are covalently linked to one another via at least a first and second disulfide bond, wherein the first disulfide bond is formed between (i) a Cys residue in a Cys-containing linker between the WT-1 peptide epitope and the β2M polypeptide, and (ii) a Cys residue in the MHC class I heavy chain polypeptide; and the second disulfide bond is formed between a Cys residue in the β2M polypeptide and a Cys residue in the MHC class I heavy chain polypeptide.

2. A TMMP of claim 1, wherein:
a) the first polypeptide comprises, in order from N-terminus to C-terminus:
i) the WT-1 peptide epitope;
ii) the Cys-containing linker; and
iii) the β2M polypeptide; and
b) the second polypeptide comprises, in order from N-terminus to C-terminus:
i) the one or more variant IL-2 polypeptides;
ii) an optional linker;
iii) the MHC class I heavy chain polypeptide;
iv) an optional linker; and
v) the Ig Fc polypeptide,
wherein when the TMMP comprises more than one variant IL-2 polypeptide, the TMMP may comprise one or more linkers between the variant IL-2 polypeptides.

3. A TMMP of claim 1, wherein:
a) the first polypeptide comprises, in order from N-terminus to C-terminus:
i) the WT-1 peptide epitope;
ii) the Cys-containing linker; and
iii) the β2M polypeptide; and
b) the second polypeptide comprises, in order from N-terminus to C-terminus:
i) the MHC class I heavy chain polypeptide;
ii) an optional linker;
iii) the Ig Fc polypeptide;
iv) an optional linker; and
v) the one or more variant IL-2 polypeptides,
wherein when the TMMP comprises more than one variant IL-2 polypeptide, the TMMP may comprise one or more linkers between the variant IL-2 polypeptides.

4. A TMMP of any one of claims 1-3, wherein the variant IL-2 polypeptide comprises: i) an H16A substitution and an F42A substitution; or ii) an H16T substitution and an F42A substitution.

5. A TMMP of any one of claims 1-4, wherein the MHC class I heavy chain polypeptide comprises an amino acid sequence having at least 95% amino acid sequence identity to SEQ ID NO:199, SEQ ID NO:201 or SEQ ID NO:203.

6. A TMMP of claim 2, wherein the TMMP comprises two variant IL-2 polypeptides that are in tandem and optionally joined by a linker, and wherein each of the variant IL-2 polypeptides comprises an amino acid sequence having at least 95% amino acid sequence identity to the amino acid sequence set forth in SEQ ID NO: 188, wherein X₁ is Ala and X₂ is Ala, or wherein X₁ is Thr and X₂ is Ala,
wherein the first disulfide bond is formed between (i) a Cys residue in a linker between the WT-1 peptide epitope and the β2M polypeptide, and (ii) a Cys residue at position 84 in the MHC Class I heavy chain polypeptide, and wherein the second disulfide bond is formed between a Cys residue at position 12 of the β2M polypeptide and a Cys residue at position 236 of the MHC class I heavy chain polypeptide,
wherein the MHC class I heavy chain polypeptide comprises an amino acid sequence having at least 95% amino acid sequence identity to the amino acid sequence set forth in any one of SEQ ID Nos: 198-222, where amino acid 84 is a Cys and amino acid 236 is a Cys, and
wherein the Ig Fc polypeptide has at least 95% amino acid sequence identity to the amino acid sequence set forth in any one of SEQ ID NO:418-421.

7. A TMMP of claim 6, wherein the variant IL-2 polypeptides are joined by a linker, and each of the variant IL-2 polypeptides has the amino acid sequence set forth in SEQ ID NO:188, wherein X₁ is Ala and X₂ is Ala.

8. A TMMP of claim 6 or claim 7, wherein the MHC class I heavy chain polypeptide comprises an amino acid sequence having at least 95% amino acid sequence identity to SEQ ID NO:199, and wherein the epitope is VLDFAPPGA (SEQ ID NO:259).

9. A TMMP of claim 3, wherein the TMMP comprises two variant IL-2 polypeptides that are in tandem and optionally joined by a linker, and wherein each of the variant IL-2 polypeptides comprises an amino acid sequence having at least 95% amino acid sequence identity to the amino acid sequence set forth in SEQ ID NO: 188, wherein X₁ is Ala and X₂ is Ala, or wherein X₁ is Thr and X₂ is Ala,
wherein the first disulfide bond is formed between (i) a Cys residue in a linker between the WT-1 peptide epitope and the β2M polypeptide, and (ii) a Cys residue at position 84 in the MHC Class I heavy chain polypeptide, and wherein the second disulfide bond is formed between a Cys residue at position 12 of the β2M polypeptide and a Cys residue at position 236 of the MHC class I heavy chain polypeptide,
wherein the MHC class I heavy chain polypeptide comprises an amino acid sequence having at least 95% amino acid sequence identity to the amino acid sequence set forth in any one of SEQ ID Nos: 198-222, where amino acid 84 is a Cys and amino acid 236 is a Cys, and
wherein the Ig Fc polypeptide has at least 95% amino acid sequence identity to the amino acid sequence set forth in any one of SEQ ID NO:418-421.

10. A TMMP of claim 9, wherein the MHC class I heavy chain polypeptide comprises an amino acid sequence having at least 95% amino acid sequence identity to set forth in any one of SEQ ID Nos: 198-206.

11. A TMMP of claim 9 or 10, wherein the variant IL-2 polypeptides are joined by a linker, and each of the variant IL-2 polypeptides has the amino acid sequence set forth in SEQ ID NO:188, wherein X₁ is Ala and X₂ is Ala.

12. A TMMP of any one of claims 1-11, wherein the linker between the WT-1 peptide epitope and the β2M polypeptide comprises the amino acid sequence set forth in SEQ ID NO:316 or SEQ ID NO:317.

13. A TMMP of claim 7, wherein
the first polypeptide comprises the amino acid sequence set forth in SEQ ID NO:423,
the MHC class I heavy chain polypeptide comprises an amino acid sequence having at least 98% amino acid sequence identity to the amino acid sequence set forth in any one of SEQ ID Nos: 198-222, and
the Ig Fc polypeptide comprises an amino acid sequence having at least 98% amino acid sequence identity to the amino acid sequence set forth in SEQ ID NO:421.

14. A TMMP comprising a first and a second heterodimer according to any one of claims 1-13, wherein the first and second heterodimers are identical in amino acid sequence and are covalently bound to each other by one or more disulfide bonds between the Ig Fc polypeptides of the first and second heterodimers.

15. A TMMP of claim 14, wherein two disulfide bonds link the Ig Fc polypeptide of one heterodimer to the Ig Fc polypeptide of the other heterodimer.
